# EUROPEAN PATENT APPLICATION

(11) **EP 3 722 441 A1**
(43) Date of publication of application: **14.10.2020**
(21) Application number: 20166281.4
(22) Date of filing: 01.06.2016
(51) Int. Cl.: C12Q 1/68

(54) **METHOD OF DIAGNOSING A CANCER FOR A TREATMENT WITH A TRK INHIBITOR**

(30) Priority: 01.06.2015 US 201562169443 P
(62) Divisional of application: 16731698.3
(71) Applicant: Loxo Oncology, Inc., Stamford, Connecticut 06901 (US)
(72) Inventor: TUCH, Brian B., Stamford, CT 06901 (US); BILENKER, Josh, Stamford, CT 06901 (US)
(74) Representative: HGF

(57) **Abstract**

A variety of different point mutations in NTRK1, NTRK2, and NTRK3 were identified in biopsy samples from a subjects having a variety of different cancers. Provided herein are methods of treating a subject having a cancer, methods of selecting a treatment including a therapeutically effective amount of a Trk inhibitor for a subject, methods of determining the likelihood that a subject having a cancer will have a positive response to a treatment with a Trk inhibitor, methods of predicting the efficacy of a Trk inhibitor in a subject having a cancer, methods of determining a subject's risk for developing a cancer, and methods of assisting in the diagnosis of cancer that are based on the identification of a subject having a cell that has at least one of the point mutations in NTRK1, NTRK2, and/or NTRK3, or the determination that a subject has a cell that has at least one of the point mutations in NTRK1, NTRK2, and/or NTRK3. Also provided are kits that allow for the detection of at least one of the point mutations in NTRK1, NTRK2, and/or NTRK3.

## Description

### CROSS-REFERENCE TO RELATED APPLICATION

This application claims priority to U.S. Provisional Patent Application Serial No. 62/169,443, filed June 1, 2015; the entire content of which is herein incorporated by reference.

### TECHNICAL FIELD

This invention relates to methods of genetics, diagnostics, and pharmacogenetics.

### BACKGROUND

Tropomyosin-related kinase (TRK) is a receptor tyrosine kinase family of neurotrophin receptors that are found in multiple tissues types. Three members of the TRK proto-oncogene family have been described: TrkA, TrkB, and TrkC, coded by the *NTRK1, NTRK2,* and *NTRK3* genes, respectively. The TRK receptor family is involved in neuronal development, including the growth and function of neuronal synapses, memory development, and maintenance, and the protection of neurons after ischemia or other types of injury (Nakagawara, Cancer Lett. 169:107-114, 2001).

TRK was originally identified from a colorectal cancer as an oncogene fusion containing 5' sequences from tropomyosin-3 (TPM3) gene and the kinase domain encoded by the 3' region of the neurotrophic tyrosine kinase, receptor, type 1 gene (NTRK1) (Pulciani et al., Nature 300:539-542, 1982; Martin-Zanca et al., Nature 319:743-748, 1986). TRK gene fusions follow the well-established paradigm of other oncogenic fusions, such as those involving ALK and ROS1 that have been shown to drive the growth of tumors and can be successfully inhibited in the clinic by targeted drugs (Shaw et al., New Engl. J. Med. 371:1963-1971, 2014; Shaw et al., New Engl. J. Med. 370:1189-1197, 2014). Oncogenic TRK fusions induce cancer cell proliferation and engage critical cancer-related downstream signaling pathways such as MAPK and AKT (Vaishnavi et al., Cancer Discov. 5:25-34, 2015). Numerous oncogenic rearrangements involving *NTRK1* and its related TRK family members *NTRK2* and *NTRK3* have been discovered (Vaishnavi et al., Cancer Disc. 5:25-34, 2015; Vaishnavi et al., Nature Med. 19:1469-1472, 2013). Although there are numerous different 5' gene fusion partners identified, all share an in-frame, intact TRK kinase domain. A variety of different Trk inhibitors have been developed to treat cancer (see, e.g., U.S. Patent Application Publication No. 62/080,374, International Application Publication Nos. WO 11/006074, WO 11/146336, WO 10/033941, and WO 10/048314, and U.S. Patent Nos. 8,933,084, 8,791,123, 8,637,516, 8,513,263, 8,450,322, 7,615,383, 7,384,632, 6,153,189, 6,027,927, 6,025,166, 5,910,574, 5,877,016, and 5,844,092).

### SUMMARY

The present invention is based on the discovery that a biopsy samples from subjects having a variety of different cancers include a cancer cell that has at least one point mutation in a NTRK1, NTRK2, and/or NTRK3 gene that results in the expression of a TrkA, TrkB, and/or TrkC protein comprising a mutation at one or more amino acid position(s) (e.g., a substitution or a deletion). In view of this discovery, provided herein are methods of treating a subject having a cancer that include administering to a subject identified as having a cancer cell that has at least one point mutation in NTRK1, NTRK2, and/or NTRK3 (e.g., any of the point mutations in NTRK1, NTRK2, and/or NTRK3 described herein) a therapeutically effective amount of a Trk inhibitor (e.g., any of the Trk inhibitors described herein or known in the art), methods of selecting a treatment including a therapeutically effective amount of a Trk inhibitor (e.g., any of the Trk inhibitors described herein or known in the art) for a subject identified as having a cancer cell that has at least one point mutation in NTRK1, NTRK2, and/or NTRK (e.g., any of the point mutations in NTRK1, NTRK2, and/or NTRK3 described herein), methods of determining the likelihood that a subject having a cancer will have a positive response to a treatment based upon whether the subject has a cancer cell that has at least one point mutation in NTRK1, NTRK2, and/or NTRK3 (e.g., any of the point mutations in NTRK1, NTRK2, and/or NTRK3 described herein), methods of predicting the efficacy of a Trk inhibitor (e.g., any of the Trk inhibitors described herein or known in the art) in a subject having a cancer based upon whether the subject has a cancer cell that has at least one point mutation in NTRK1, NTRK2, and/or NTRK3 (e.g., any of the point mutations in NTRK1, NTRK2, and/or NTRK3 described herein), methods of determining a subject's risk for developing a cancer (e.g., any of the cancers described herein) based upon whether the subject has a cell that has at least one point mutation in NTRK1, NTRK2, and/or NTRK3 (e.g., any of the point mutations in NTRK1, NTRK2, and/or NTRK3 described herein), and methods of assisting in the diagnosis of cancer (e.g., any of the cancers described herein) in a subject based upon whether the subject has a cell that has at least one point mutation in NTRK1, NTRK2, and/or NTRK3 (e.g., any of the point mutations in NTRK1, NTRK2, and/or NTRK3 described herein).

Provided herein are methods of treating a subject having a cancer (e.g., any of the cancers described herein) that include identifying a subject having a cancer cell that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein); and administering to the identified subject a therapeutically effective amount of a Trk inhibitor (e.g., any of the Trk inhibitors described herein).

Also provided herein are methods of treating a subject having a cancer (e.g., any of the cancers described herein) that include identifying a subject having a cancer cell that has at least one (e.g., two, three, four, five, six, seven, eight, nine, ten, eleven, or twelve) point mutation in a NTRK2 gene that results in the expression of a TrkB protein comprising a mutation at one or more (e.g., one, two, three, four, five, six, seven, eight, nine, ten, eleven, or twelve) amino acid position(s) selected from the group consisting of: 240, 241, 242, 264, 314, 315, 401, 426, 427, 428, 440, and 689; and administering to the identified subject a therapeutically effective amount of a Trk inhibitor (e.g., any of the Trk inhibitors described herein).

Also provided herein are methods of treating a subject identified as having a cancer cell that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), that includes administering to the subject a therapeutically effective amount of a Trk inhibitor (e.g., any of the Trk inhibitors described herein).

Also provided are methods of treating a subject identified as having a cancer cell that has at least one (e.g., two, three, four, five, six, seven, eight, nine, ten, eleven, or twelve) point mutations in a NTRK2 gene that results in the expression of a TrkB protein comprising a mutation at one or more amino acid position(s) selected from the group consisting of: 240, 241, 242, 264, 314, 315, 401, 426, 427, 428, 440, and 689, the method including administering to the subject a therapeutically effective amount of a Trk inhibitor.

Also provided are methods of selecting a treatment for a subject having a cancer (e.g., any of the cancers described herein) that include identifying a subject having a cancer cell that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), and selecting a treatment including a therapeutically effective amount of a Trk inhibitor (e.g., any of the Trk inhibitors described herein) for the identified subject.

Also provided are methods of selecting a treatment for a subject having a cancer (e.g., any of the cancers described herein) that include identifying a subject having a cancer cell that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of: 240, 241, 242, 264, 314, 315, 401, 426, 427, 428, 440, and 689; and selecting a treatment comprising a therapeutically effective amount of a Trk inhibitor (e.g., any of the Trk inhibitors described herein) for the identified subject.

Also provided are methods of selecting a treatment for a subject having a cancer (e.g., any of the cancers described herein) that include selecting a treatment including a therapeutically effective amount of a Trk inhibitor (e.g., any of the Trk inhibitors described herein) for a subject identified as having a cancer cell that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein).

Also provided are methods of selecting a treatment for a subject having a cancer (e.g., any of the cancers described herein) that include selecting a treatment comprising a therapeutically effective amount of a Trk inhibitor (e.g., any of the Trk inhibitors described herein) for a subject identified as having a cancer cell that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of: 240, 241, 242, 264, 314, 315, 401, 426, 427, 428, 440, and 689.

Some embodiments of these methods further include administering a therapeutically effective amount of the selected treatment to the identified subject. Some embodiments of these methods further include recording the selected treatment in the identified subject's clinical record (e.g., a computer readable medium).

Also provided are methods of determining the likelihood that a subject having a cancer (e.g., any of the cancers described herein) will have a positive response to treatment with a Trk inhibitor (e.g., any of the Trk inhibitors described herein) that include determining whether a cancer cell in a sample obtained from the subject (e.g., a biopsy sample) has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), and determining that a subject having a cancer cell that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), has an increased likelihood of having a positive response to treatment with a Trk inhibitor.

Also provided are methods of determining the likelihood that a subject having a cancer will have a positive response to treatment with a Trk inhibitor (e.g., any of the Trk inhibitors described herein) that include determining whether a cancer cell in a sample obtained from the subject (e.g., a biopsy sample) has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of: 240, 241, 242, 264, 314, 315, 401, 426, 427, 428, 440, and 689; and determining that a subject having a cancer cell that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of: 240, 241, 242, 264, 314, 315, 401, 426, 427, 428, 440, and 689, has an increased likelihood of having a positive response to treatment with a Trk inhibitor.

Also provided are methods of determining the likelihood that a subject having cancer (e.g., any of the cancers described herein) will have a positive response to treatment with a Trk inhibitor (e.g., any of the Trk inhibitors described herein) that include determining that a subject having a cancer cell that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), has an increased likelihood of having a positive response to treatment with a Trk inhibitor.

Also provided are methods of determining the likelihood that a subject having cancer (e.g., any of the cancers described herein) will have a positive response to treatment with a Trk inhibitor (e.g., any of the Trk inhibitors described herein) that include determining that a subject having a cancer cell that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of: 240, 241, 242, 264, 314, 315, 401, 426, 427, 428, 440, and 689, has an increased likelihood of having a positive response to treatment with a Trk inhibitor.

Some embodiments of these methods further include administering a therapeutically effective amount of a Trk inhibitor (e.g., any of the Trk inhibitors described herein) to a subject determined to have an increased likelihood of having a positive response to treatment with a Trk inhibitor.

Also provided are methods of predicting the efficacy of a Trk inhibitor (e.g., any of the Trk inhibitors described herein) in a subject having cancer (e.g., any of the cancers described herein) that include determining whether a cancer cell in a sample obtained from the subject (e.g., a biopsy sample) has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), and determining that a Trk inhibitor is more likely to be effective in a subject having a cancer cell in a sample obtained from the subject that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein).

Also provided are methods of predicting the efficacy of a Trk inhibitor (e.g., any of the Trk inhibitors described herein) in a subject having cancer (e.g., any of the cancers described herein) that include determining whether a cancer cell in a sample obtained from the subject has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of: 240, 241, 242, 264, 314, 315, 401, 426, 427, 428, 440, and 689; and determining that a Trk inhibitor is more likely to be effective in a subject having a cancer cell in a sample obtained from the subject that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of: 240, 241, 242, 264, 314, 315, 401, 426, 427, 428, 440, and 689.

Also provided are methods of predicting the efficacy of a Trk inhibitor (e.g., any of the Trk inhibitors described herein) in a subject having a cancer (e.g., any of the cancers described herein) that include determining that a Trk inhibitor is more likely to be effective in a subject having a cancer cell in a sample obtained from the subject that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein).

Also provided are methods of predicting the efficacy of a Trk inhibitor (e.g., any of the Trk inhibitors described herein) in a subject having a cancer (e.g., any of the cancers described herein) that include determining that a Trk inhibitor is more likely to be effective in a subject having a cancer cell in a sample obtained from the subject that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of: 240, 241, 242, 264, 314, 315, 401, 426, 427, 428, 440, and 689.

In some embodiments of any of these methods, the subject is previously identified or diagnosed as having the cancer.

In some embodiments, the step of identifying a subject having a cancer cell that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), comprises performing an assay to determine the presence of the at least one point mutation in a NTRK1, NTRK2, and/or NTRK3 gene in a cancer cell in a sample from the subject.

In some embodiments, the step of identifying a subject having a cancer cell that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group of: 240, 241, 242, 264, 314, 315, 401, 426, 427, 428, 440, and 689, comprises performing an assay to determine the presence of the at least one point mutation in a NTRK2 gene in a cancer cell in a sample from the subject.

In some embodiments of these methods, the assay is selected from the group of: denaturing gradient gel electrophoresis (DGGE), temperature gradient gel electrophoresis (TGGE), temperature gradient capillary electrophoresis, a single strand conformational polymorphism assay, a molecular beacon assay, a dynamic hybridization assay, a PCR-based assay, and denaturing high performance liquid chromatography. In some embodiments of these methods, the assay includes sequencing a segment of the NTRK1, NTRK2, and/or NTRK3 gene comprising the at least one point mutation.

In any of the methods described herein, the Trk inhibitor a crystalline form of the compound of Formula I: 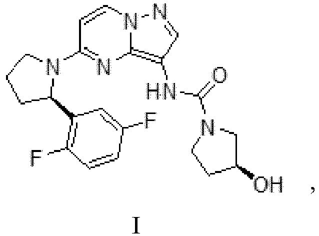 or a hydrogen sulfate salt thereof.

Also provided are methods of determining a subject's risk for developing a cancer (e.g., any of the cancers described herein) that include determining whether a cell in a sample obtained from the subject has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), and identifying a subject having a cell that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein) as having an increased likelihood of developing a cancer.

Also provided are methods of determining a subject's risk for developing a cancer (e.g., any of the cancers described herein) that include determining whether a cell in a sample obtained from the subject has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of: 240, 241, 242, 264, 314, 315, 401, 426, 427, 428, 440, and 689, and identifying a subject having a cell that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of: 240, 241, 242, 264, 314, 315, 401, 426, 427, 428, 440, and 689, as having an increased likelihood of developing a cancer.

Also provided are methods of determining a subject's risk of developing a cancer (e.g., any of the cancers described herein) that include identifying a subject having a cell that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein) as having an increased likelihood of developing a cancer.

Also provided are methods of determining a subject's risk of developing a cancer (e.g., any of the cancers described herein) that include identifying a subject having a cell that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of: 240, 241, 242, 264, 314, 315, 401, 426, 427, 428, 440, and 689, as having an increased likelihood of developing a cancer.

Also provided are methods of assisting in the diagnosis of a cancer (e.g., any of the cancers described herein) in a subject that include determining whether a cell in a sample obtained from the subject has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), and determining that a subject having a cell that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), has an increased likelihood of having a cancer.

Also provided are methods of assisting in the diagnosis of a cancer (e.g., any of the cancers described herein) in a subject that include determining whether a cell in a sample obtained from the subject has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of: 240, 241, 242, 264, 314, 315, 401, 426, 427, 428, 440, and 689, and determining that a subject having a cell that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of: 240, 241, 242, 264, 314, 315, 401, 426, 427, 428, 440, and 689, has an increased likelihood of having a cancer.

Also provided are methods of assisting in the diagnosis of a cancer (e.g., any of the cancers described herein) in a subject that include determining that a subject having a cell that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), has an increased likelihood of having a cancer.

Also provided are methods of assisting in the diagnosis of a cancer (e.g., any of the cancers described herein) in a subject that include determining that a subject having a cell that has has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of: 240, 241, 242, 264, 314, 315, 401, 426, 427, 428, 440, and 689, has an increased likelihood of having a cancer.

In some embodiments of these methods, the subject is identified as having been exposed to a significant level of carcinogen(s). In some embodiments of these methods, the subject is suspected of having a cancer. In some embodiments of these methods, the subject has one or more symptoms of cancer.

In any of the methods described herein, the cancer is selected from the group of: adenocarcinoma, adrenal gland cortical carcinoma, adrenal gland neuroblastoma, anus squamous cell carcinoma, appendix adenocarcinoma, bladder urothelial carcinoma, bile duct adenocarcinoma, bladder carcinoma, bladder urothelial carcinoma, bone chordoma, bone marrow leukemia lymphocytic chronic, bone marrow leukemia non-lymphocytic acute myelocytic, bone marrow lymph proliferative disease, bone marrow multiple myeloma, bone sarcoma, brain astrocytoma, brain glioblastoma, brain medulloblastoma, brain meningioma, brain oligodendroglioma, breast adenoid cystic carcinoma, breast carcinoma, breast ductal carcinoma in situ, breast invasive ductal carcinoma, breast invasive lobular carcinoma, breast metaplastic carcinoma, cervix neuroendocrine carcinoma, cervix squamous cell carcinoma, colon adenocarcinoma, colon carcinoid tumor, duodenum adenocarcinoma, endometrioid tumor, esophagus adenocarcinoma, esophagus and stomach carcinoma, eye intraocular melanoma, eye intraocular squamous cell carcinoma, eye lacrimal duct carcinoma, fallopian tube serous carcinoma, gallbladder adenocarcinoma, gallbladder glomus tumor, gastroesophageal junction adenocarcinoma, head and neck adenoid cystic carcinoma, head and neck carcinoma, head and neck neuroblastoma, head and neck squamous cell carcinoma, kidney chromophore carcinoma, kidney medullary carcinoma, kidney renal cell carcinoma, kidney renal papillary carcinoma, kidney sarcomatoid carcinoma, kidney urothelial carcinoma, kidney carcinoma, leukemia lymphocytic, leukemia lymphocytic chronic, liver cholangiocarcinoma, liver hepatocellular carcinoma, liver carcinoma, lung adenocarcinoma, lung adenosquamous carcinoma, lung atypical carcinoid, lung carcinosarcoma, lung large cell neuroendocrine carcinoma, lung non-small cell lung carcinoma, lung sarcoma, lung sarcomatoid carcinoma, lung small cell carcinoma, lung small cell undifferentiated carcinoma, lung squamous cell carcinoma, upper aerodigestive tract squamous cell carcinoma, upper aerodigestive tract carcinoma, lymph node lymphoma diffuse large B cell, lymph node lymphoma follicular lymphoma, lymph node lymphoma mediastinal B-cell, lymph node lymphoma plasmablastic lung adenocarcinoma, lymphoma follicular lymphoma, lymphoma, non-Hodgkins, nasopharynx and paranasal sinuses undifferentiated carcinoma, ovary carcinoma, ovary carcinosarcoma, ovary clear cell carcinoma, ovary epithelial carcinoma, ovary granulosa cell tumor, ovary serous carcinoma, pancreas carcinoma, pancreas ductal adenocarcinoma, pancreas neuroendocrine carcinoma, peritoneum mesothelioma, peritoneum serous carcinoma, placenta choriocarcinoma, pleura mesothelioma, prostate acinar adenocarcinoma, prostate carcinoma, rectum adenocarcinoma, rectum squamous cell carcinoma, skin adnexal carcinoma, skin basal cell carcinoma, skin melanoma, skin Merkel cell carcinoma, skin squamous cell carcinoma, small intestine adenocarcinoma, small intestine gastrointestinal stromal tumors (GISTs), large intestine/colon carcinoma, large intestine adenocarcinoma, soft tissue angiosarcoma, soft tissue Ewing sarcoma, soft tissue hemangioendothelioma, soft tissue inflammatory myofibroblastic tumor, soft tissue leiomyosarcoma, soft tissue liposarcoma, soft tissue neuroblastoma, soft tissue paraganglioma, soft tissue perivascular epitheliod cell tumor, soft tissue sarcoma, soft tissue synovial sarcoma, stomach adenocarcinoma, stomach adenocarcinoma diffuse-type, stomach adenocarcinoma intestinal type, stomach adenocarcinoma intestinal type, stomach leiomyosarcoma, thymus carcinoma, thymus thymoma lymphocytic, thyroid papillary carcinoma, unknown primary adenocarcinoma, unknown primary carcinoma, unknown primary malignant neoplasm, lymphoid neoplasm, unknown primary melanoma, unknown primary sarcomatoid carcinoma, unknown primary squamous cell carcinoma, unknown undifferentiated neuroendocrine carcinoma, unknown primary undifferentiated small cell carcinoma, uterus carcinosarcoma, uterus endometrial adenocarcinoma, uterus endometrial adenocarcinoma endometrioid, uterus endometrial adenocarcinoma papillary serous, and uterus leiomyosarcoma.

In some embodiments of these methods, the step of determining whether a cell in a sample obtained from the subject has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein) includes performing an assay to determine the presence of the at least one point mutation in a N TRK1, NTRK2, and/or NTRk3 gene in a cell in the sample.

In some embodiments of these methods, the step of determining whether a cell in a sample obtained from the subject has at least one point (e.g., two, three, four, five, six, seven, eight, nine, ten, eleven, or twelve) mutation in a NTRK2 gene that results in the expression of a TrkB protein including a mutation at one or more (e.g., two, three, four, five, six, seven, eight, nine, ten, eleven, or twelve) amino acid position(s) selected from the group of: 240, 241, 242, 264, 314, 315, 401, 426, 427, 428, 440, and 689, includes performing an assay to determine the presence of the at least one point mutation in a NTRK2 gene in a cell in the sample.

In some embodiments, the assay is selected from the group of: denaturing gradient gel electrophoresis (DGGE), temperature gradient gel electrophoresis (TGGE), temperature gradient capillary electrophoresis, a single strand conformational polymorphism assay, a molecular beacon assay, a dynamic hybridization assay, a PCR-based assay, denaturing high performance liquid chromatography. In some embodiments, the assay includes sequencing a segment of the NTRK1, NTRK2, and/or NTRK3 gene including the at least one point mutation.

Some methods further include confirming the diagnosis of a cancer in a subject determined to have an increased likelihood of having a cancer.

In some embodiments of any of the methods claims described herein, the TrkB protein comprises a mutation at one or more (e.g., two, three, four, five, six, seven, eight, nine, ten, eleven, or twelve) amino acid position(s) selected from the group of: M240I, N241D, E242K, I264M, A314E, A314G, A314V, L315F, G401A, G401E, G401R, T426I, G427S, R428Q, A440S, A440T, A440V, and V689M.

Also provided are kits including one or more (e.g., two, three, four, five, six, seven, eight, nine, ten, eleven, or twelve) probes that each specifically hybridize to a segment of a NTRK1, NTRK2, or NTRK3 gene that encodes a mutation at one of the amino acid positions in TrkA, TrkB, or TrkC (e.g., any of the specific mutations in TrkA, TrkB, or TrkC described herein).

Also provided are kits including one or more (e.g., two, three, four, five, six, seven, eight, nine, ten, eleven, or twelve) probes that each specifically hybridize to a segment of a NTRK3 gene that encodes a mutation at one of amino acid positions 240, 241, 242, 264, 314, 315, 401, 426, 427, 428, 440, and 689 in TrkB protein. In some examples, the kit includes one or more (e.g., two, three, four, five, six, seven, eight, nine, ten, eleven, or twelve) probes that each specifically hybridize to a segment of a NTRK3 gene that encodes a mutation selected from the group of M240I, N241D, E242K, I264M, A314E, A314G, A314V, L315F, G401A, G401E, G401R, T426I, G427S, R428Q, A440S, A440T, A440V, and V689M in TrkB protein.

In some embodiments of the kits, the one or more probes are labeled with a detectable probe. In some embodiments of the kits, the one or more probes are covalently attached to a substrate (e.g., a film, a plate, or a bead).

Also provided herein are methods of treating a subject having a cancer that include:
(a) identifying a subject having a cancer cell that has: at least one point mutation in a NTRK1 gene that results in the expression of a TrkA protein including a mutation at one or more amino acid position(s) selected from the group of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750; at least one point mutation in a NTRK2 gene that results in the expression of a TrkB protein including a mutation at one or more amino acid position(s) selected from the group of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or at least one point mutation in a NTRK3 gene that results in the expression of a TrkC protein including a mutation at one or more amino acid position(s) selected from the group of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810; and
(b) administering to the identified subject a therapeutically effective amount of a Trk inhibitor.

Also provided herein are methods of treating a subject identified as having a cancer cell that has: at least one point mutation in a NTRK1 gene that results in the expression of a TrkA protein including a mutation at one or more amino acid position(s) selected from the group of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750; at least one point mutation in a NTRK2 gene that results in the expression of a TrkB protein including a mutation at one or more amino acid position(s) selected from the group of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or at least one point mutation in a NTRK3 gene that results in the expression of a TrkC protein including a mutation at one or more amino acid position(s) selected from the group of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810, the method comprising administering to the subject a therapeutically effective amount of a Trk inhibitor.

Also provided herein are methods of selecting a treatment for a subject having a cancer that include: (a) identifying a subject having a cancer cell that has: at least one point mutation in a NTRK1 gene that results in the expression of a TrkA protein including a mutation at one or more amino acid position(s) selected from the group of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750; at least one point mutation in a NTRK2 gene that results in the expression of a TrkB protein including a mutation at one or more amino acid position(s) selected from the group of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or at least one point mutation in a NTRK3 gene that results in the expression of a TrkC protein including a mutation at one or more amino acid position(s) selected from the group of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810; and (b) selecting a treatment comprising a therapeutically effective amount of a Trk inhibitor for the identified subject.

Also provided herein are methods of selecting a treatment for a subject having a cancer that include selecting a treatment comprising a therapeutically effective amount of a Trk inhibitor for a subject identified as having a cancer cell that has: at least one point mutation in a NTRK1 gene that results in the expression of a TrkA protein including a mutation at one or more amino acid position(s) selected from the group of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750; at least one point mutation in a NTRK2 gene that results in the expression of a TrkB protein including a mutation at one or more amino acid position(s) selected from the group of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or at least one point mutation in a NTRK3 gene that results in the expression of a TrkC protein including a mutation at one or more amino acid position(s) selected from the group of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810.

Also provided herein are methods of determining the likelihood that a subject having a cancer will have a positive response to treatment with a Trk inhibitor that include: (a) determining whether a cancer cell in a sample obtained from the subject has: at least one point mutation in a NTRK1 gene that results in the expression of a TrkA protein including a mutation at one or more amino acid position(s) selected from the group of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750; at least one point mutation in a NTRK2 gene that results in the expression of a TrkB protein including a mutation at one or more amino acid position(s) selected from the group of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or at least one point mutation in a NTRK3 gene that results in the expression of a TrkC protein including a mutation at one or more amino acid position(s) selected from the group of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810; and (b) determining that a subject having a cancer cell that has the at least one point mutation in a NTRK1 gene, the at least one point mutation in a NTRK2 gene, and/or the at least one point mutation in a NTRK3 gene, has an increased likelihood of having a positive response to treatment with a Trk inhibitor.

Also provided herein are methods of determining the likelihood that a subject having cancer will have a positive response to treatment with a Trk inhibitor that include: determining that a subject having a cancer cell that has: at least one point mutation in a NTRK1 gene that results in the expression of a TrkA protein including a mutation at one or more amino acid position(s) selected from the group of: 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750; at least one point mutation in a NTRK2 gene that results in the expression of a TrkB protein including a mutation at one or more amino acid position(s) selected from the group of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or at least one point mutation in a NTRK3 gene that results in the expression of a TrkC protein including a mutation at one or more amino acid position(s) selected from the group of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810, has an increased likelihood of having a positive response to treatment with a Trk inhibitor.

Also provided herein are methods of predicting the efficacy of a Trk inhibitor in a subject having cancer that include: (a) determining whether a cancer cell in a sample obtained from the subject has: at least one point mutation in a NTRK1 gene that results in the expression of a TrkA protein including a mutation at one or more amino acid position(s) selected from the group of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750; at least one point mutation in a NTRK2 gene that results in the expression of a TrkB protein including a mutation at one or more amino acid position(s) selected from the group of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or at least one point mutation in a NTRK3 gene that results in the expression of a TrkC protein including a mutation at one or more amino acid position(s) selected from the group of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810; and (b) determining that a Trk inhibitor is more likely to be effective in a subject having a cancer cell in a sample obtained from the subject that has the at least one point mutation in a NTRK1 gene, the at least one point mutation in a NTRK2 gene, and/or the at least one point mutation in a NTRK3 gene.

Also provided are methods of predicting the efficacy of a Trk inhibitor in a subject having cancer that include determining that a Trk inhibitor is more likely to be effective in a subject having a cancer cell in a sample obtained from the subject that has: at least one point mutation in a NTRK1 gene that results in the expression of a TrkA protein including a mutation at one or more amino acid position(s) selected from the group of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750; at least one point mutation in a NTRK2 gene that results in the expression of a TrkB protein including a mutation at one or more amino acid position(s) selected from the group of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or at least one point mutation in a NTRK3 gene that results in the expression of a TrkC protein including a mutation at one or more amino acid position(s) selected from the group of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810.

In some embodiments of any of the methods described herein, the cancer is selected from the group of: adenocarcinoma, adrenal gland cortical carcinoma, adrenal gland neuroblastoma, anus squamous cell carcinoma, appendix adenocarcinoma, bladder urothelial carcinoma, bile duct adenocarcinoma, bladder carcinoma, bladder urothelial carcinoma, bone chordoma, bone marrow leukemia lymphocytic chronic, bone marrow leukemia non-lymphocytic acute myelocytic, bone marrow lymph proliferative disease, bone marrow multiple myeloma, bone sarcoma, brain astrocytoma, brain glioblastoma, brain medulloblastoma, brain meningioma, brain oligodendroglioma, breast adenoid cystic carcinoma, breast carcinoma, breast ductal carcinoma in situ, breast invasive ductal carcinoma, breast invasive lobular carcinoma, breast metaplastic carcinoma, cervix neuroendocrine carcinoma, cervix squamous cell carcinoma, colon adenocarcinoma, colon carcinoid tumor, duodenum adenocarcinoma, endometrioid tunor, esophagus adenocarcinoma, esophagus and stomach carcinoma, eye intraocular melanoma, eye intraocular squamous cell carcinoma, eye lacrimal duct carcinoma, fallopian tube serous carcinoma, gallbladder adenocarcinoma, gallbladder glomus tumor, gastroesophageal junction adenocarcinoma, head and neck adenoid cystic carcinoma, head and neck carcinoma, head and neck neuroblastoma, head and neck squamous cell carcinoma, kidney chromophore carcinoma, kidney medullary carcinoma, kidney renal cell carcinoma, kidney renal papillary carcinoma, kidney sarcomatoid carcinoma, kidney urothelial carcinoma, kidney carcinoma, leukemia lymphocytic, leukemia lymphocytic chronic, liver cholangiocarcinoma, liver hepatocellular carcinoma, liver carcinoma, lung adenocarcinoma, lung adenosquamous carcinoma, lung atypical carcinoid, lung carcinosarcoma, lung large cell neuroendocrine carcinoma, lung non-small cell lung carcinoma, lung sarcoma, lung sarcomatoid carcinoma, lung small cell carcinoma, lung small cell undifferentiated carcinoma, lung squamous cell carcinoma, upper aerodigestive tract squamous cell carcinoma, upper aerodigestive tract carcinoma, lymph node lymphoma diffuse large B cell, lymph node lymphoma follicular lymphoma, lymph node lymphoma mediastinal B-cell, lymph node lymphoma plasmablastic lung adenocarcinoma, lymphoma follicular lymphoma, lymphoma, non-Hodgkins, nasopharynx and paranasal sinuses undifferentiated carcinoma, ovary carcinoma, ovary carcinosarcoma, ovary clear cell carcinoma, ovary epithelial carcinoma, ovary granulosa cell tumor, ovary serous carcinoma, pancreas carcinoma, pancreas ductal adenocarcinoma, pancreas neuroendocrine carcinoma, peritoneum mesothelioma, peritoneum serous carcinoma, placenta choriocarcinoma, pleura mesothelioma, prostate acinar adenocarcinoma, prostate carcinoma, rectum adenocarcinoma, rectum squamous cell carcinoma, skin adnexal carcinoma, skin basal cell carcinoma, skin melanoma, skin Merkel cell carcinoma, skin squamous cell carcinoma, small intestine adenocarcinoma, small intestine gastrointestinal stromal tumors (GISTs), large intestine/colon carcinoma, large intestine adenocarcinoma, soft tissue angiosarcoma, soft tissue Ewing sarcoma, soft tissue hemangioendothelioma, soft tissue inflammatory myofibroblastic tumor, soft tissue leiomyosarcoma, soft tissue liposarcoma, soft tissue neuroblastoma, soft tissue paraganglioma, soft tissue perivascular epitheliod cell tumor, soft tissue sarcoma, soft tissue synovial sarcoma, stomach adenocarcinoma, stomach adenocarcinoma diffuse-type, stomach adenocarcinoma intestinal type, stomach adenocarcinoma intestinal type, stomach leiomyosarcoma, thymus carcinoma, thymus thymoma lymphocytic, thyroid papillary carcinoma, unknown primary adenocarcinoma, unknown primary carcinoma, unknown primary malignant neoplasm, lymphoid neoplasm, unknown primary melanoma, unknown primary sarcomatoid carcinoma, unknown primary squamous cell carcinoma, unknown undifferentiated neuroendocrine carcinoma, unknown primary undifferentiated small cell carcinoma, uterus carcinosarcoma, uterus endometrial adenocarcinoma, uterus endometrial adenocarcinoma endometrioid, uterus endometrial adenocarcinoma papillary serous, and uterus leiomyosarcoma.

In some embodiments of any of the methods described herein, the subject is previously identified or diagnosed as having the cancer. In some embodiments of any of the methods described herein, the step of identifying a subject having a cancer cell that has the at least one point mutation in a NTRK1 gene, the at least one point mutation in a NTRK2 gene, and/or the at least one point mutation in a NTRK3 gene includes performing an assay to determine the presence of the at least one point mutation in a NTRK1 gene, the at least one point mutation in a NTRK2 gene, and/or the at least one point mutation in a NTRK3 gene, in a cancer cell in a sample from the subject. In some embodiments of any of the methods described herein, the assay is selected from the group of: denaturing gradient gel electrophoresis (DGGE), temperature gradient gel electrophoresis (TGGE), temperature gradient capillary electrophoresis, a single strand conformational polymorphism assay, a molecular beacon assay, a dynamic hybridization assay, a PCR-based assay, and denaturing high performance liquid chromatography. In some embodiments of any of the methods described herein, the assay includes sequencing a segment of the NTRK1 gene comprising the at least one point mutation, a segment of the NTRK2 gene comprising the at least one point mutation, and/or a segment of the NTRK3 gene comprising the at least one point mutation.

In some embodiments of any of the methods described herein, the Trk inhibitor a crystalline form of the compound of Formula I: 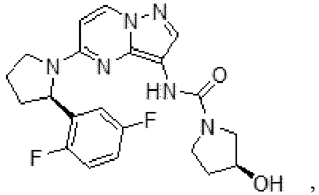 or a hydrogen sulfate salt thereof.

Some embodiments of any of the methods described herein further include: administering a therapeutically effective amount of the selected treatment to the identified subject. Some embodiments of any of the methods described herein further include: recording the selected treatment in the identified subject's clinical record (e.g., a computer readable medium). Some embodiments of any of the methods described herein further include: administering a therapeutically effective amount of a Trk inhibitor to a subject determined to have an increased likelihood of having a positive response to treatment with a Trk inhibitor.

Also provided herein are methods of determining a subject's risk for developing a cancer that include: (a) determining whether a cell in a sample obtained from the subject has: at least one point mutation in a NTRK1 gene that results in the expression of a TrkA protein including a mutation at one or more amino acid position(s) selected from the group of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750; at least one point mutation in a NTRK2 gene that results in the expression of a TrkB protein including a mutation at one or more amino acid position(s) selected from the group of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or at least one point mutation in a NTRK3 gene that results in the expression of a TrkC protein including a mutation at one or more amino acid position(s) selected from the group of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810; and (b) identifying a subject having a cell that has the at least one point mutation in a NTRK1 gene, the at least one point mutation in a NTRK2 gene, and/or the at least one point mutation in a NTRK3 gene as having an increased likelihood of developing a cancer.

Also provided herein are methods of determining a subject's risk for developing a cancer that include identifying a subject having a cell that has: at least one point mutation in a NTRK1 gene that results in the expression of a TrkA protein including a mutation at one or more amino acid position(s) selected from the group of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750; at least one point mutation in a NTRK2 gene that results in the expression of a TrkB protein including a mutation at one or more amino acid position(s) selected from the group of: 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or at least one point mutation in a NTRK3 gene that results in the expression of a TrkC protein including a mutation at one or more amino acid position(s) selected from the group of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810, as having an increased likelihood of developing a cancer.

Also provided herein are methods of assisting in the diagnosis of a cancer in a subject that include: (a) determining whether a cell in a sample obtained from the subject has: at least one point mutation in a NTRK1 gene that results in the expression of a TrkA protein including a mutation at one or more amino acid position(s) selected from the group of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750; at least one point mutation in a NTRK2 gene that results in the expression of a TrkB protein including a mutation at one or more amino acid position(s) selected from the group of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or at least one point mutation in a NTRK3 gene that results in the expression of a TrkC protein including a mutation at one or more amino acid position(s) selected from the group of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810; and (b) determining that a subject having a cell that has the at least one point mutation in a NTRK1 gene, the at least one point mutation in a NTRK2 gene, and/or the at least one point mutation in a NTRK3 gene, has an increased likelihood of having a cancer.

Also provided are methods of assisting in the diagnosis of a cancer in a subject that include determining that a subject having a cell that has: at least one point mutation in a NTRK1 gene that results in the expression of a TrkA protein including a mutation at one or more amino acid position(s) selected from the group of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750; at least one point mutation in a NTRK2 gene that results in the expression of a TrkB protein including a mutation at one or more amino acid position(s) selected from the group of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or at least one point mutation in a NTRK3 gene that results in the expression of a TrkC protein including a mutation at one or more amino acid position(s) selected from the group of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810, has an increased likelihood of having a cancer.

In some embodiments of any of the methods described herein, the subject is identified as having been exposed to a significant level of carcinogen(s). In some embodiments of any of the methods described herein, the subject is suspected of having a cancer. In some embodiments of any of the methods described herein, the subject has one or more symptoms of cancer. In some embodiments of any of the methods described herein, the cancer is selected from the group of: adenocarcinoma, adrenal gland cortical carcinoma, adrenal gland neuroblastoma, anus squamous cell carcinoma, appendix adenocarcinoma, bladder urothelial carcinoma, bile duct adenocarcinoma, bladder carcinoma, bladder urothelial carcinoma, bone chordoma, bone marrow leukemia lymphocytic chronic, bone marrow leukemia non-lymphocytic acute myelocytic, bone marrow lymph proliferative disease, bone marrow multiple myeloma, bone sarcoma, brain astrocytoma, brain glioblastoma, brain medulloblastoma, brain meningioma, brain oligodendroglioma, breast adenoid cystic carcinoma, breast carcinoma, breast ductal carcinoma in situ, breast invasive ductal carcinoma, breast invasive lobular carcinoma, breast metaplastic carcinoma, cervix neuroendocrine carcinoma, cervix squamous cell carcinoma, colon adenocarcinoma, colon carcinoid tumor, duodenum adenocarcinoma, endometrioid tumor, esophagus adenocarcinoma, esophagus and stomach carcinoma, eye intraocular melanoma, eye intraocular squamous cell carcinoma, eye lacrimal duct carcinoma, fallopian tube serous carcinoma, gallbladder adenocarcinoma, gallbladder glomus tumor, gastroesophageal junction adenocarcinoma, head and neck adenoid cystic carcinoma, head and neck carcinoma, head and neck neuroblastoma, head and neck squamous cell carcinoma, kidney chromophore carcinoma, kidney medullary carcinoma, kidney renal cell carcinoma, kidney renal papillary carcinoma, kidney sarcomatoid carcinoma, kidney urothelial carcinoma, kidney carcinoma, leukemia lymphocytic, leukemia lymphocytic chronic, liver cholangiocarcinoma, liver hepatocellular carcinoma, liver carcinoma, lung adenocarcinoma, lung adenosquamous carcinoma, lung atypical carcinoid, lung carcinosarcoma, lung large cell neuroendocrine carcinoma, lung non-small cell lung carcinoma, lung sarcoma, lung sarcomatoid carcinoma, lung small cell carcinoma, lung small cell undifferentiated carcinoma, lung squamous cell carcinoma, upper aerodigestive tract squamous cell carcinoma, upper aerodigestive tract carcinoma, lymph node lymphoma diffuse large B cell, lymph node lymphoma follicular lymphoma, lymph node lymphoma mediastinal B-cell, lymph node lymphoma plasmablastic lung adenocarcinoma, lymphoma follicular lymphoma, lymphoma, non-Hodgkins, nasopharynx and paranasal sinuses undifferentiated carcinoma, ovary carcinoma, ovary carcinosarcoma, ovary clear cell carcinoma, ovary epithelial carcinoma, ovary granulosa cell tumor, ovary serous carcinoma, pancreas carcinoma, pancreas ductal adenocarcinoma, pancreas neuroendocrine carcinoma, peritoneum mesothelioma, peritoneum serous carcinoma, placenta choriocarcinoma, pleura mesothelioma, prostate acinar adenocarcinoma, prostate carcinoma, rectum adenocarcinoma, rectum squamous cell carcinoma, skin adnexal carcinoma, skin basal cell carcinoma, skin melanoma, skin Merkel cell carcinoma, skin squamous cell carcinoma, small intestine adenocarcinoma, small intestine gastrointestinal stromal tumors (GISTs), large intestine/colon carcinoma, large intestine adenocarcinoma, soft tissue angiosarcoma, soft tissue Ewing sarcoma, soft tissue hemangioendothelioma, soft tissue inflammatory myofibroblastic tumor, soft tissue leiomyosarcoma, soft tissue liposarcoma, soft tissue neuroblastoma, soft tissue paraganglioma, soft tissue perivascular epitheliod cell tumor, soft tissue sarcoma, soft tissue synovial sarcoma, stomach adenocarcinoma, stomach adenocarcinoma diffuse-type, stomach adenocarcinoma intestinal type, stomach adenocarcinoma intestinal type, stomach leiomyosarcoma, thymus carcinoma, thymus thymoma lymphocytic, thyroid papillary carcinoma, unknown primary adenocarcinoma, unknown primary carcinoma, unknown primary malignant neoplasm, lymphoid neoplasm, unknown primary melanoma, unknown primary sarcomatoid carcinoma, unknown primary squamous cell carcinoma, unknown undifferentiated neuroendocrine carcinoma, unknown primary undifferentiated small cell carcinoma, uterus carcinosarcoma, uterus endometrial adenocarcinoma, uterus endometrial adenocarcinoma endometrioid, uterus endometrial adenocarcinoma papillary serous, and uterus leiomyosarcoma.

In some embodiments of any of the methods described herein, the step of determining whether a cell in a sample obtained from the subject has the at least one point mutation in a NTRK1 gene, the at least one point mutation in a NTRK2 gene, and/or the at least one point mutation in a NTRK3 gene, includes performing an assay to determine the presence of the at least one point mutation in a NTRK1 gene, the presence of the at least one point mutation in a NTRK2 gene, and/or the presence of the at least one point mutation in a NTRK3 gene, in a cell in the sample. In some embodiments of any of the methods described herein, the assay is selected from the group of: denaturing gradient gel electrophoresis (DGGE), temperature gradient gel electrophoresis (TGGE), temperature gradient capillary electrophoresis, a single strand conformational polymorphism assay, a molecular beacon assay, a dynamic hybridization assay, a PCR-based assay, denaturing high performance liquid chromatography. In some embodiments of any of the methods described herein, the assay includes sequencing a segment of the NTRK1 gene comprising the at least one point mutation, a segment of the NTRK2 gene comprising the at least one point mutation, and/or a segment of the NTRK3 gene comprising the at least one point mutation. Some embodiments of any of the methods described herein further include confirming the diagnosis of a cancer in a subject determined to have an increased likelihood of having a cancer.

In some embodiments of any of the methods described herein, the TrkA protein includes a mutation at one or more amino acid positions selected from the group of S241F, S241H, S241Y, R314G, R314H, R314L, R314P, N318S, G319S, S320F, V321M, I510T, V511M, L512F, L512R, S552R, A553T, R554P, R554Q, R554W, A636E, A636T, A636V, G637E, G637W, M638V, R649L, R649W, R654C, R654H, N655Y, D679N, D679Y, Y680H, T687I, M688I, L689M, P690H, R692C, R692H, P695S, P696L, E697K, E747K, R748L, R748Q, R748W, P749Q, R750C, R750H, and R750L; the TrkB protein includes a mutation at one or more amino acid position(s) selected from the group of M240I, N241D, E242K, R251G, R251K, I252V, S256L, S257F, D258N, I264M, A314E, A314G, A314V, L315F, G401A, G401E, G401R, T426I, G427S, R428Q, A440S, A440T, A440V, R598C, R598S, K599M, D600H, H602N, R603S, L664M, T665M, T665S, Q666L, Q666R, A677T, A678T, A678V, G679D, M680I, V689M, R691C, D692N, F746I, T747M, T748M, E749K, Q784H, G785V, R786Q, V787F, L788M, Q789E, G804E, C805R, and C805Y; and/or the TrkC protein includes a mutation at one or more amino acid position(s) selected from the group of V221I, R222Q, E223D, D242N, W243C, I244T, T269A, T269M, T270M, T270Q, T270V, V271L, V271M, E276D, D277E, D277G, D277N, T281I, T281P, T282M, T283A, T283K, T283M, S296I, S296R, V297I, V325M, R326C, R326G, R326H, R326L, R326P, N328S, P329N, P329S, P330Q, E344G, E344V, S345F, K346N, H349Y, V350E, E351D, Y353F, Q354K, D537E, D537Y, I538N, V539M, L540M, G545C, G545D, G550R, K551E, L560V, P562L, P562Q, P562R, P562T, K575E, D576N, P577S, P582Q, P582W, K583%, D584E, D584N, V601A, V601I, K602R, F603L, Y604F, Y604H, Y604N, C607F, G608C, G608E, G608S, D609G, D609H, D609N, D609V, G610R, P612A, P612L, P612S, P612T, D624Y, L625M, N626K, K627N, K627R, F628L, L629F, L629I, P634L, P634T, D635H, A636E, A636V, M637I, M637K, M637V, E650V, L651P, G652R, G652V, L653F, L653P, H677Y, R678Q, D679G, D679N, V687A, V687I, G688R, A689E, A689V, Y705N, S706I, T707M, D708N, P715L, P715S, S716Y, G717R, T730N, M731I, M731L, L732I, P738H, P738S, Y744F, R745P, R745W, K746R, K746T, G786C, G786S, R787C, R787H, R787S, P796L, P796S, D801N, Q808H, R809W, and E810K.

Also provided are kits that include one or more probes that each specifically hybridize to: a segment of a NTRK1 gene that encodes a mutation at one of amino acid positions 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750 in a TrkA protein; a segment of a NTRK2 gene that encodes a mutation at one of amino acid positions 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805 in TrkB protein; or a segment of a NTRK3 gene that encodes a mutation at one of amino acid positions 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810 in a TrkC protein.

In some embodiments of any of the kits provided herein, the kit includes one or more probes that each specifically hybridize to: a segment of a NTRK1 gene that encodes a mutation selected from the group consisting of S241F, S241H, S241Y, R314G, R314H, R314L, R314P, N318S, G319S, S320F, V321M, I510T, V511M, L512F, L512R, S552R, A553T, R554P, R554Q, R554W, A636E, A636T, A636V, G637E, G637W, M638V, R649L, R649W, R654C, R654H, N655Y, D679N, D679Y, Y680H, T687I, M688I, L689M, P690H, R692C, R692H, P695S, P696L, E697K, E747K, R748L, R748Q, R748W, P749Q, R750C, R750H, and R750L in a TrkA protein; a segment of a NTRK2 gene that encodes a mutation selected from the group consisting of M240I, N241D, E242K, R251G, R251K, I252V, S256L, S257F, D258N, I264M, A314E, A314G, A314V, L315F, G401A, G401E, G401R, T426I, G427S, R428Q, A440S, A440T, A440V, R598C, R598S, K599M, D600H, H602N, R603S, L664M, T665M, T665S, Q666L, Q666R, A677T, A678T, A678V, G679D, M680I, V689M, R691C, D692N, F746I, T747M, T748M, E749K, Q784H, G785V, R786Q, V787F, L788M, Q789E, G804E, C805R, and C805Y in TrkB protein; or a segment of a NTRK3 gene that encodes a mutation selected from the group consisting of V221I, R222Q, E223D, D242N, W243C, I244T, T269A, T269M, T270M, T270Q, T270V, V271L, V271M, E276D, D277E, D277G, D277N, T281I, T281P, T282M, T283A, T283K, T283M, S296I, S296R, V297I, V325M, R326C, R326G, R326H, R326L, R326P, N328S, P329N, P329S, P330Q, E344G, E344V, S345F, K346N, H349Y, V350E, E351D, Y353F, Q354K, D537E, D537Y, I538N, V539M, L540M, G545C, G545D, G550R, K551E, L560V, P562L, P562Q, P562R, P562T, K575E, D576N, P577S, P582Q, P582W, K583%, D584E, D584N, V601A, V601I, K602R, F603L, Y604F, Y604H, Y604N, C607F, G608C, G608E, G608S, D609G, D609H, D609N, D609V, G610R, P612A, P612L, P612S, P612T, D624Y, L625M, N626K, K627N, K627R, F628L, L629F, L629I, P634L, P634T, D635H, A636E, A636V, M637I, M637K, M637V, E650V, L651P, G652R, G652V, L653F, L653P, H677Y, R678Q, D679G, D679N, V687A, V687I, G688R, A689E, A689V, Y705N, S706I, T707M, D708N, P715L, P715S, S716Y, G717R, T730N, M731I, M731L, L732I, P738H, P738S, Y744F, R745P, R745W, K746R, K746T, G786C, G786S, R787C, R787H, R787S, P796L, P796S, D801N, Q808H, R809W, and E810K in a TrkC protein.

In some embodiments of any of the kits provided herein, the one or more probes are labeled with a detectable probe. In some embodiments of any of the kits provided herein, the one or more probes are covalently attached to a substrate. In some embodiments of any of the kits provided herein, the substrate is a film, a plate, or a bead.

As used herein, the word "a" before a noun represents one or more of the particular noun. For example, the phrase "a cell" represents "one or more cells."

The term "subject" means a vertebrate, including any member of the class mammalia, including humans, sports or pet animals, such as horse (e.g., race horse) or dog (e.g., race dogs), and higher primates. In preferred embodiments, the subject is a human.

The term "treating" or "positive response to treatment" means an improvement in the condition of a subject having a cancer, e.g., one or more of a decrease in the size of one or more tumor(s) in a subject, a decrease or no substantial change in the growth rate of one or more tumor(s) in a subject, a decrease in metastasis in a subject, and an increase in the period of remission for a subject (e.g., as compared to the one or more metric(s) in a subject having a similar cancer receiving no treatment or a different treatment, or as compared to the one or more metric(s) in the same subject prior to treatment). Additional metrics for assessing response to a treatment in a subject having a cancer are known in the art.

The term "point mutation" means a change in the nucleotide sequence of a gene that results in a single amino acid change in a protein encoded by the gene. For example, a point mutation in a gene can result in the deletion of a single amino acid in a protein encoded by the gene or can result in the substitution of an amino acid in a wildtype version of the encoded protein with a different amino acid. Non-limiting examples of point mutations in NTRK1, NTRK2, and NTRK3 genes are described herein.

The phrase "significant level of carcinogen" is meant a level of exposure to a carcigen that is known to increase (e.g., a statistically significant increase) the likelihood of a subject to develop a cancer (e.g., as compared to a subject that has not been exposed to the same level of exposure or has been exposed to a non-detectable amount of the carcinogen).

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

### DESCRIPTION OF DRAWINGS

Figure 1 is a diagram showing the position of the different point mutations detected in the NTRK3 gene (Summary) and the point mutations in NTRK3 gene that are associated with specific histologies (bottom nine rows).
Figure 2 is a diagram showing the position of all the different point mutations detected in the NTRK3 gene with confirmed expression above background (Summary) and the point mutations in the NTRK3 gene with confirmed expression above background that are associated with specific histologies (bottom nine rows).
Figure 3 is a graphic showing the position of a valine at amino acid position 689 or a methionine at amino acid position 689 relative to the asparagine at amino acid position 529 in TrkB protein.

### DETAILED DESCRIPTION

A variety of different NTRK1, NTRK2, and NTRK3 point mutations were discovered in biopsy samples from subjects having a variety of different cancers. In view of this discovery, provided herein are methods of treating a subject having a cancer (e.g., any of the cancers described herein) that include administering to a subject identified as having a cancer cell that has at least one point mutation in NTRK1, NTRK2, and/or NTRK3 (e.g., any of the point mutations in NTRK1, NTRK2, and/or NTRK3 described herein) a therapeutically effective amount of a Trk inhibitor (e.g., any of the Trk inhibitors described herein), methods of selecting a treatment including a therapeutically effective amount of a Trk inhibitor (e.g., any of the Trk inhibitors described herein) for a subject identified as having a cancer cell that has at least one point mutation in NTRK1, NTRK2, and/or NTRK (e.g., any of the point mutations in NTRK1, NTRK2, and/or NTKR3 described herein), methods of determining the likelihood that a subject having a cancer (e.g., any of the cancers described herein) will have a positive response to a treatment based upon whether the subject has a cancer cell that has at least one point mutation in NTRK1, NTRK2, and/or NTRK3 (e.g., any of the point mutations in NTRK1, NTRK2, and/or NTKR3 described herein), methods of predicting the efficacy of a Trk inhibitor (e.g., any of the Trk inhibitors described herein) in a subject having a cancer (e.g., any of the cancers described herein) based upon whether the subject has a cancer cell that has at least one point mutation in NTRK1, NTRK2, and/or NTRK3 (e.g., any of the point mutations in NTRK1, NTRK2, and/or NTRK3 described herein), methods of determining a subject's risk for developing a cancer (e.g., any of the cancers described herein) based upon whether the subject has a cell that has at least one point mutation in NTRK1, NTRK2, and/or NTRK3 (e.g., any of the point mutations in NTRK1, NTRK2, and/or NTRK3 described herein), and methods of assisting in the diagnosis of cancer (e.g., any of the cancers described herein) in a subject based upon whether the subject has a cell that has at least one point mutation in NTRK1, NTRK2, and/or NTRK3 (e.g., any of the point mutations in NTRK1, NTRK2, and/or NTRK3 described herein). As can be appreciated in the art, the various aspects described below can be used in any combination without limitation.

### Tropomyosin Receptor Kinases (Trks)

Three different NTRK genes have been implicated for a role in cancer (e.g., through discovery of chromosome translocations resulting in constitutively active Trk fusion proteins): NTRK1, NTRK2, and NTRK3. The NTRK1, NTRK2, and NTRK3 genes encode TrkA, TrkB, and TrkC, respectively. Non-limiting exemplary amino acid and cDNA sequences for wildtype TrkA, TrkB, and TrkC are provided below. The exemplary wildtype protein and cDNA sequences provided below can be used to identify a point mutation in a NTRK1, NTRK2, or NTRK3 gene or can be used to determine mutation in a TrkA, TrkB, or TrkC protein caused by a point mutation in a NTRK1, NTRK2, or NTRK3 gene, respectively. Additional wildtype protein and cDNA sequences for TrkA, TrkB, and TrkC are known in the art.
Wildtype Human TrkA Protein Isoform A (NP_002520) (SEQ ID NO: 1)
Wildtype Human TrkA cDNA Isoform A (NM_002529) (SEQ ID NO: 2)

Wildtype Human TrkA Protein Isoform B (NP_001007793) (SEQ ID NO: 3)
Wildtype Human TrkA cDNA Isoform B (NM_001007792) (SEQ ID NO: 4)

Wildtype Human TrkB Protein (NP_006171) (SEQ ID NO: 5)
Wildtype Human TrkB cDNA(NM_006180) (SEQ ID NO: 6)

Wildtype Human TrkC Protein (NP_001012338) (SEQ ID NO: 7)
Wildtype Human TrkC cDNA(NM_001012338) (SEQ ID NO: 8)

### NTRK Point Mutations

Different point mutations were discovered in NTRK1, NTRK2, and NTRK3 genes in biopsy samples from subjects having a variety of different cancers. A point mutation in a NTRK1, NTRK2, or NTRK3 gene can result, e.g., in a Trk protein (a TrkA, TrkB, and TrkC protein, respectively) that includes a substitution of an amino acid in a wildtype version of the Trk protein with a different amino acid. In other examples, a point mutation in a NTRK1, NTRK2, or NTRK3 gene can result, e.g., in a Trk protein (a TrkA, TrkB, and TrkC protein, respectively) with a deletion of an amino acid in a wildtype version of the Trk protein.

Non-limiting examples of the specific amino acid positions discovered to have mutations (e.g., substitutions or deletions) in TrkA, TrkB, or TrkC proteins in cancer cells having a NTRK1, NTRK2, or NTRK3 point mutation are listed below. Also listed below are the different specific amino acid mutations (e.g., substitutions or deletions) present in TrkA, TrkB, or TrkC proteins generated in cancer cells having a NTRK1, NTRK2, or NTRK3 point mutation, respectively.

Point mutations in NTRK1 gene were discovered to result in a TrkA protein that includes one or more (e.g., two, three, four, five, six, seven, eight, or nine) amino acid substitutions or deletions at amino acid positions: 3, 4, 5, 6, 7, 8, 10, 13, 15, 17, 18, 20, 22, 24, 25, 30, 31, 33, 34, 38, 39, 41, 42, 43, 49, 50, 52, 55, 56, 59, 62, 63, 66, 69, 71, 74, 79, 80, 85, 86, 88, 89, 90, 91, 92, 96, 97, 101, 104, 106, 107, 110, 112, 113, 115, 116, 117, 119, 126, 129, 132, 134, 138, 139, 142, 147, 149, 150, 155, 156, 157, 158, 161, 165, 166, 167, 169, 170, 171, 179, 185, 186, 189, 193, 195, 197, 198, 201, 202, 206, 208, 210, 211, 212, 214, 220, 221, 222, 223, 224, 225, 226, 228, 231, 233, 238, 239, 241, 243, 245, 246, 247, 248, 251, 252, 253, 258, 260, 261, 262, 263, 264, 266, 270, 273, 275, 277, 282, 287, 292, 293, 294, 296, 297, 298, 300, 302, 304, 306, 307, 309, 310, 311, 314, 318, 319, 320, 321, 323, 324, 326, 328, 329, 330, 335, 336, 337, 340, 341, 342, 344, 346, 347, 349, 357, 359, 360, 361, 366, 368, 371, 372, 374, 375, 379, 380, 381, 388, 389, 392, 393, 395, 397, 398, 402, 404, 406, 407, 408, 410, 411, 413, 415, 416, 417, 419, 421, 422, 425, 426, 432, 434, 440, 444, 447, 452, 453, 454, 455, 457, 460, 461, 465, 468, 471, 472, 475, 476, 477, 478, 479, 480, 484, 485, 486, 487, 488, 489, 494, 495, 500, 502, 503, 507, 508, 510, 511, 512, 515, 517, 518, 520, 522, 526, 527, 530, 533, 537, 539, 540, 541, 543, 547, 549, 550, 551, 552, 553, 554, 556, 559, 561, 566, 570, 573, 574, 575, 577, 578, 580, 583, 585, 587, 591, 593, 594, 595, 599, 602, 603, 606, 607, 609, 612, 614, 615, 616, 618, 620, 623, 626, 630, 631, 636, 637, 638, 639, 640, 641, 642, 644, 647, 649, 651, 654, 655, 657, 660, 661, 663, 664, 666, 671, 674, 677, 679, 680, 682, 683, 684, 686, 687, 688, 689, 690, 692, 695, 696, 697, 699, 702, 705, 706, 709, 710, 712, 715, 719, 723, 725, 728, 733, 734, 736, 741, 743, 744, 747, 748, 749, 750, 751, 753, 754, 755, 760, 761, 762, 763, 766, 768, 771, 772, 776, 777, 779, 780, 788, 790, and 791 (e.g., amino acid positions corresponding to those in wildtype sequence NP_002520 (SEQ ID NO: 1) or NP_001007793 (SEQ ID NO: 3)). Different specific amino acid substitutions or deletions present in TrkA protein generated in a cancer cell include one or more (e.g., two, three, four, five, six, seven, eight, or nine) of following: R3P, R3Q, G4A, A5T, A5V, R6W, R7S, G8E, A10E, A10T, V13I, W15C, A17T, T18M, G20D, W22R, L22Q, A24S, W25C, S30P, R31I, A33S, A33V, A34T, L38W, D38N, A39S, C41W, P42T, H43Q, R49G, R49P, R49Q, C50Y, R52L, R52Q, A55D, L56M, L59F, L62P, P63S, E66D, T69I, L71I, E74K, L79Q, Q80R, R85C, D86N, D86Y, R88K, R88S, G89S, L90M, L90del, G91R, E92K, L96V, T97I, S101C, S101N, S101R, R104H, V106M, A107V, A110V, H112Y, F113L, P115S, R116L, R116Q, R116W, L117P, R119C, R119H, R119P, A126D, A126P, A126T, S129F, W132F, W132R, T134N, L138H, S139F, E142K, G147E, P149A, P149H, L150P, A155V, L156Q, R157C, R157L, R157P, W158R, R161C, R161P, E165D, E165del, G166R, L167M, G169E, G169R, V170L, P171S, P171T, G179R, H185N, M186T, A189V, V193L, T195M, K197R, V198F, P201H, P201del, N202S, D206N, G208E, G208R, D210N, V211E, V211L, L212V, R214Q, R214W, R220W, G221D, G221V, L222Q, E223Q, Q224H, A225S, G226D, G226S, I228V, E231K, E233K, E233Q, V238M, M239I, S241F, S241H, S241Y, G243D, P245S, S246F, L247V, G248E, G248R, L251M, A252S, N253D, L258I, L258V, R260Q R260M, K261E, K261N, N262K, V263M, T264K, T264M, W266S, D270Q D270N, R273Q, R273W, E275A, S277F, V282I, S287I, T292M, A293V, V294A, M296K, H297Q, H298Q, C300R, C300Y, P302L, S304Y, D306E, G307A, P309S, A310E, A310S, P311L, R314G, R314H, R314L, R314P, N318S, G319S, S320F, V321M, N323S, E324D, E324K, S326R, I328V, F329V, T330A, P335L, A336E, A337P, A337T, T340I, V341M, R342Q, R342W, G344E, G344W, L346P, R347C, R347G, R347H, N349K, N349S, G357S, Y359C, T360M, L361R, P366L, P366R, P366S, P366T, G368C, S371F, A372S, A372T, I374N, M375I, M375V, M379T, D380G, N381S, E388D, E388K, D389Y, P392S, V393F, F395L, P397L, V398L, S402I, S402R, S404P, D406Y, P407L, P407R, V408G, K410N, K411N, K411del, E413K, E413Q, P415S, F416S, G417V, S419L, A421T, V422L, A425S, V426I, L432R, T434M, N440K, N440S, R444P, R444Q, R444W, K447M, K447N, K447T, R452C, R452G, P453L, P453Q, P453T, A454T, V455M, A457V, D460N, G461R, S465F, S465del, F468L, L471F, G472S, S475C, S475F, S475T, L476M, S477Y, S477_insS, P478L, T479I, E480K, E480Q, S484Y, G485R, L486I, Q487L, G488C, G488S, H489Q, H489Y, P494T, Q495R, A500T, V502A, H503N, H503Y, R507C, R507H, R508Q, R508W, I510T, V511M, L512F, L512R, E515K, G517R, E518K, A520T, G522W, L526F, L526P, A527T, H530Y, L533Q, D537E, D537N, M539L, M539R, L540Q, V541M, V543A, K547T, A549T, A549V, S550Y, E551D, E551V, S552R, A553T, R554P, R554Q, R554W, D556N, R559H, A561T, M566K, Q570R, V573M, R574C, R574H, F575L, G577S, V578I, T580I, R583C, R583L, L585R, M587T, Y591C, R593W, H594Q, G595R, R599H, R602Q, S603P, P606H, D607N, K609N, A612S, A612V, G614A, G614V, E615K, E615Q, D616H, D616N, A618V, G620C, G623C, Q626K, V630A, A631D, A636E, A636T, A636V, G637E, G637W, M638V, V639L, V639M, Y640C, L641M, A642S, A642V, L644M, V647G, V647L, R649L, R649W, L651M, R654C, R654H, N655Y, L657P, L657V, Q660L, G661E, V663E, V664I, I666T, M671T, D674E, D674N, S677N, D679N, D679Y, Y680H, R682C, R682H, R682S, V683G, G684E, R686G, R686H, T687I, M688I, L689M, P690H, R692C, R692H, P695S, P696L, E697K, I699V, R702C, R702H, R702L, R702S, T705S, T706K, D709N, D709Y, V710M, S712R, V715M, E719D, E719K, Y723C, K725M, K725T, W728R, N733S, T734M, A736E, A736T, T741M, G743R, R744C, R744H, E747K, R748L, R748Q, R748W, P749Q, R750C, R750H, R750L, A751D, P753Q, P754T, E755Q, M760I, M760V, R761Q, R761W, G762R, C763F, R766L, R766W, P768S, R771H, H772R, D776E, D776N, V777A, A779T, R780G, R780W, P788L, P788S, V790I, V790L, and Y791H (e.g., as compared to the wildtype sequence NP_002520 (SEQ ID NO: 1) or NP_001007793 (SEQ ID NO: 3), e.g., as shown in Tables 1 and 2).

Point mutations in NTRK2 gene were discovered to result in a TrkB protein that includes one or more amino acid substitutions or deletions at amino acid positions: 7, 9, 10, 14, 23, 26, 28, 29, 31, 34, 35, 37, 42, 45, 46, 47, 51, 53, 56, 57, 60, 65, 66, 69, 70, 72, 74, 75, 78, 80, 81, 82, 84, 88, 89, 97, 98, 100, 101, 105, 110, 113, 120, 122, 124, 125, 132, 133, 136, 138, 139, 146, 147, 158, 159, 166, 169, 172, 173, 175, 185, 187, 187, 191, 193, 195, 196, 198, 199, 202, 203, 207, 209, 209, 210, 221, 222, 223, 224, 225, 228, 230, 234, 240, 241, 242, 249, 251, 252, 254, 256, 257, 258, 261, 264, 266, 268, 272, 279, 280, 286, 289, 292, 293, 293, 294, 295, 296, 304, 310, 311, 314, 315, 319, 321, 326, 328, 331, 335, 341, 343, 349, 350, 354, 357, 358, 370, 373, 377, 379, 385, 386, 387, 388, 389, 390, 394, 395, 398, 401, 408, 410, 414, 416, 419, 423, 423, 426, 427, 428, 430, 432, 435, 440, 442, 446, 449, 452, 454, 455, 458, 460, 464, 475, 476, 480, 481, 482, 483, 484, 486, 496, 498, 501, 503, 514, 515, 517, 519, 521, 524, 528, 530, 539, 545, 547, 549, 552, 553, 558, 559, 561, 562, 563, 564, 566, 569, 574, 577, 578, 579, 580, 581, 582, 584, 589, 592, 595, 598, 599, 600, 602, 603, 608, 615, 615, 616, 618, 622, 624, 624, 625, 627, 629, 630, 632, 634, 638, 639, 646, 648, 649, 652, 653, 654, 656, 657, 658, 660, 662, 664, 665, 666, 668, 670, 671, 673, 674, 677, 678, 679, 680, 682, 684, 685, 689, 691, 692, 698, 698, 699, 700, 702, 706, 709, 710, 714, 715, 716, 725, 726, 727, 729, 736, 737, 741, 742, 744, 746, 747, 748, 749, 750, 752, 754, 755, 756, 758, 760, 761, 762, 766, 769, 773, 777, 779, 782, 783, 784, 785, 786, 787, 788, 789, 792, 793, 795, 797, 799, 802, 804, 805, 810, 812, 818, 821, 822, 825, 829, and 831 (e.g., amino acid positions corresponding to those in wildtype sequence NP_006171 (SEQ ID NO: 5)). Different specific amino acid substitutions or deletions present in TrkB protein generated in a cancer cell include one or more of following: W7R, G9E, G9V, P10H, R14W, V23A, V23M, W26R, A28D, A29T, A31T, T34A, T34R, S35F, K37R, R42Q, C45F, C45R, C45Y, S46R, D47N, G51D, V53A, P56L, P56S, R57S, P60H, P65H, P65T, E66D, T69P, T69S, E70K, F72L, A74S, N75K, R78K, E80Q, I81F, I82V, E84K, E88K, E88Q, A89T, T97A, I98V, D100N, S101F, F105L, A110E, K113R, I120N, I120V, F122I, R124Q, N125K, R132S, K133N, R136C, R136H, L138F, D139H, V146A, V146L, G147S, W158C, I159F, I159M, K166T, P169S, Q172K, D173Y, Y175H, P185L, A187E, A187S, I191T, N193S, G195A, L196F, S198T, A199T, A202D, A203S, T207I, E209D, E209K, E210V, A221V, G222D, D223H, P224S, V225I, M228T, W230L, N234Y, M240I, N241D, E242K, S249F, S249Y, R251G, R251K, I252V, N254S, S256L, S257F, D258N, G261R, I264M, C266S, C266Y, A268V, V272E, V279A, N280I, A286P, A286T, I289V, L292I, E293D, E293K, S294F, P295S, T296I, P304L, N310Δ (deletion), P311H, A314E, A314G, A314V, L315F, Y319C, G321V, E326D, K328Q, C331F, C331Y, H335L, E341K, E341V, H343D, D349Y, N350I, N350K, M354I, G357R, D358Y, D370Y, Q373L, H377Y, M379T, M379V, D385G, D386N, G387C, A388V, N389I, P390R, D394H, D394N, D394Y, V395Δ, E398K, G401A, G401E, G401R, G408R, T410N, S414I, E416G, S419F, T423I, T423S, T426I, G427S, R428Q, H430Y, S432L, A435V, A440S, A440T, A440V, V442L, V442M, C446Y, V449I, F452L, L454I, K455N, R458G, S460F, S460T, S460Y, M464V, V475A, K476E, K476I, G480D, V481I, G482R, G482V, P483T, A484T, V486F, V486I, P496R, P496S, H498N, H498Y, S501C, G503W, P514L, P514S, D515N, V517I, I519Δ, M521L, I524F, E528K, P530L, Q539H, F545V, Q547R, I549M, H552Q, N553S, R558K, E559D, E559K, G561S, E562K, G563R, G563V, A564T, G566E, F569L, Y574H, C577S, P578H, P578L, P578S, P578T, E579D, Q580P, D581N, K582T, L584F, T589S, D592A, D595E, R598C, R598S, K599M, D600H, H602N, R603S, L608M, H615L, H615Y, I616T, K618R, V622I, V624L, V624M, E625K, D627N, L629I, I630V, V632I, E634Q, H638L, G639R, G639V, R646M, H648Q, G649S, A652V, V653M, L654V, A656D, E657K, E657Q, G658D, P660L, P660T, T662M, L664M, T665M, T665S, Q666L, Q666R, Q668L, L670M, H671R, A673G, Q674H, A677T, A678T, A678V, G679D, M680I, Y682C, A684E, A684T, A684V, S685Y, V689M, R691C, D692N, C698R, C698W, L699P, V700F, E702D, V706M, G709R, D710Y, S714A, R715Q, R715W, D716N, V725G, G726C, G727D, T729S, M736I, P737T, I741N, I741V, M742L, R744K, F746I, T747M, T748M, E749K, S750N, V752I, S754T, L755M, G756W, V758E, V758L, V758M, W760R, E761D, E761Q, I762M, G766D, G766S, P769T, L773M, E777Q, I779M, I782M, T783I, Q784H, G785V, R786Q, V787F, L788M, Q789E, R792C, T793A, T793M, P795T, E797K, Y799N, M802L, G804E, C805R, C805Y, P810T, M812I, G818D, T821N, T821S, L822F, N825D, A829S, and P831L (e.g., compared to the wildtype sequence NP_006171 (SEQ ID NO: 5)).

Point mutations in NTRK3 gene were discovered to result in a TrkC protein that includes one or more amino acid substitutions or deletions at amino acid positions: 4, 5, 7, 8, 9, 14, 19, 21, 25, 27, 35, 36, 37, 39, 45, 46, 48, 49, 55, 63, 64, 67, 69, 71, 75, 76, 78, 82, 83, 85, 89, 90, 95, 96, 98, 99, 101, 111, 113, 114, 115, 116, 117, 119, 120, 121, 123, 124, 125, 126, 127, 130, 133, 134, 138, 140, 147, 148, 149, 152, 153, 154, 156, 157, 158, 159, 161, 163, 164, 165, 169, 171, 172, 174, 176, 178, 179, 184, 188, 189, 192, 194, 195, 196, 199, 200, 201, 202, 205, 208, 209, 210, 212, 215, 217, 218, 221, 222, 223, 227, 230, 232, 235, 239, 240, 242, 243, 244, 248, 249, 252, 253, 254, 255, 256, 260, 262, 266, 269, 270, 271, 273, 276, 277, 279, 281, 282, 283, 287, 289, 290, 292, 293, 294, 296, 297, 299, 301, 304, 305, 306, 308, 309, 312, 313, 314, 316, 320, 322, 323, 325, 326, 328, 329, 330, 332, 334, 336, 337, 339, 340, 343, 344, 345, 346, 349, 350, 351, 353, 354, 356, 357, 359, 361, 362, 364, 370, 372, 376, 378, 379, 380, 382, 384, 388, 389, 392, 393, 394, 396, 397, 398, 399, 401, 404, 405, 408, 411, 412, 415, 416, 417, 418, 421, 423, 425, 426, 429, 430, 431, 433, 435, 436, 437, 439, 448, 449, 450, 451, 452, 455, 457, 458, 459, 460, 461, 463, 464, 466, 467, 468, 469, 473, 474, 477, 478, 487, 488, 490, 491, 492, 494, 496, 497, 499, 501, 506, 507, 508, 509, 511, 512, 513, 514, 516, 518, , 519, 520, 521, 522, 526, 527, 529, 531, 533, 534, 535, 536, 537, 538, 539, 540, 542, 543, 545, 547, 550, 551, 560, 562, 565, 566, 567, 568, 569, 572, 575, 576, 577, 579, 581, 582, 583, 584, 586, 588, 590, 592, 595, 596, 597, 598, 599, 600, 601, 602, 603, 604, 605, 607, G608, 609, 610, 612, 615, 621, 623, 624, 625, 626, 627, 628, 629, 631, 632, 634, 635, 636, 637, 643, 644, 645, 647, 648, 649, 650, 651, 652, 653, 655, 656, 658, 660, 661, 663, 664, 665, 667, 668, 669, 672, 675, 677, 678, 679, 683, 685, 687, 688, 689, 693, 694, 695, 696, 697, 699, 700, 701, 702, 704, 705, 706, 707, 708, 710, 712, 714, 715, 716, 717, 719, 720, 723, 724, 726, 730, 731, 732, 735, 736, 738, 741, 744, 745, 746, 749, 751, 752, 753, 754, 755, 757, 759, 760, 762, 764, 766, 768, 772, 773, 777, 778, 781, 782, 783, 784, 786, 787, 789, 790, 791, 791, 792, 793, 796, 801, 805, 806, 807, 808, 809, 810, 812, 813, 814, 815, 819, 822, 824, 825, 826, 827, 828, 830, 832, 833, 834, and 836 (e.g., amino acid positions corresponding to those in wildtype sequence NP_001012338 (SEQ ID NO: 7)). Different specific amino acid substitutions or deletions present in TrkC protein generated in a cancer cell include one or more of following: S4C, S4F, L5I, P7L, P7R, A8D, K9E, K9N, R14P, G19E, V21F, V21I, Y25C, G27A, N35S, C36W, V37A, S39R, C45W, R46P, R46W, P48L, D49G, P55S, G63W, N64K, G67E, A69T, I71V, D75G, D75N, I76T, R78K, R78S, S82F, I83V, I85M, R89C, R89H, R89S, S90N, N95S, A96S, A96T, D98G, D98N, M99I, L101I, K111N, S113T, G114E, L115F, L115P, L115R, R116Q, R116W, S117N, Q119H, Q119K, P120H, R121G, R121K, F123L, A124V, K125E, K125N, N126K, P127H, R130C, R130H, N133H, L134Q, R138Q, R138W, T140N, F147L, Q148H, T149M, T149R, L152I, R153Q, E154K, Q156H, Q156R, L157M, E158K, Q159H, Q159K, F161I, N163T, C164G, C164S, S165N, R169S, M171L, Q172H, W174L, E176K, G178E, G178V, E179K, S184C, S184N, S184R, Y188C, Y188F, Y188H, C189F, A192T, G194D, S195C, S195F, Q196K, L199H, L199P, L199V, F200V, R201C, M202I, S205G, D208E, D208N, L209I, L209P, L209R, L209V, P210S, I212M, S215T, V217A, V217I, N218S, V221I, R222Q, E223D, A227T, T230S, N232S, G235E, G235R, P239H, P239S, P239T, D240G, D240H, D242N, W243C, I244T, L248M, Q249H, N252S, N252T, T253N, H254Q, Q255H, T256N, W260R, N262S, I266V, T269A, T269M, L270M, L270Q, L270V, V271L, V271M, V273M, V273Δ, E276D, D277E, D277G, D277N, G279D, T281I, T281P, L282M, T283A, T283K, T283M, E287D, E287Q, V289A, V290A, M292I, M292V, S293R, N294T, S296I, S296R, V297I, L299Δ, V301F, P304L, P304S, P304T, P305Q, P305R, P305S, P305T, R306C, R306H, R306P, V308L, S309I, E312K, E312Q, P313T, E314A, E314D, E314Q, R316C, R316H, C320F, E322A, E322K, E322Q, F323L, V325M, R326C, R326G, R326H, R326L, R326P, N328S, P329N, P329S, P330Q, T332M, H334Q, L336R, H337R, G339K, Q340H, Q340K, R343L, E344G, E344V, S345F, K346N, H349Y, V350E, E351D, Y353F, Q354K, G356E, G356R, G356Y, E357D, S359F, G361N, G361S, C362F, L364F, H370N, N372K, Y376N, L378V, I379V, A380P, A380V, N382H, N382I, N382T, L384M, N388K, Q389E, Q389H, N392S, G393D, G393S, H394Q, L396I, K397N, E398D, E398K, P399L, P401Q, P401S, T404M, T404S, D405N, D405V, I408M, D411E, D411N, E412K, P415H, P415S, T416I, P417L, P418H, V421L, H423Q, P425S, E426K, T429I, F430V, G431V, G431W, S433F, A435E, V436A, V436F, G437E, A439P, V448A, L449P, F450L, V451I, M452I, M452K, M452L, M452V, K455N, K455R, G457C, G457V, R458P, R459G, R459W, S460T, K461R, G463R, G463V, M464I, G466C, P467H, P467S, V468L, V468M, A469D, G473C, E474G, S477L, A478G, G487C, G487S, I488T, T490K, T490M, P491H, S492L, L494M, A496E, A496V, G497R, G497V, G497W, D499N, V501L, T506A, T506S, R507C, R507H, R507P, I508T, P509L, P509S, I511T, E512K, N513I, N513K, P514H, P514S, Y516F, R518C, R518H, Q519E, Q519L, G520E, H521N, N522K, P526A, P526Q, D527E, Y529N, Q531R, I533F, I533L, K534E, K534R, R535M, R536I, R536T, D537E, D537Y, I538N, V539M, L540M, R542L, R542Δ, E543D, G545C, G545D, G547E, G547V, G550R, K551E, L560V, P562Δ, P562L, P562Q, P562R, P562T, D565H, K566N, M567T, L568F, V569L, K572N, K575E, D576N, P577S, L579M, A581D, R582Q, R582W, K583T, D584E, D584N, Q586K, Q586L, E588Q, E590D, E590K, L592I, L595P, Q596K, H597N, H597Q, E598G, H599L, H599Y, I600V, V601A, V601I, K602R, F603L, Y604F, Y604H, Y604N, G605V, C607F, G608C, G608E, G608S, D609G, D609H, D609N, D609V, G610R, P612A, P612L, P612S, P612T, M615I, K621N, G623E, D624Y, L625M, N626K, K627N, K627R, F628L, L629F, L629I, A631V, H632N, H632Y, P634L, P634T, D635H, A636E, A636V, M637I, M637K, M637V, Q643E, Q643H, P644T, R645C, R645L, R645S, A647D, A647I, K648N, G649S, G649V, E650V, L651P, G652R, G652V, L653F, L653P, Q655K, Q655Δ, M656R, H658N, H658Y, A660T, S661G, I663V, A664P, A664S, S665L, M667I,M667L, V668M, Y669C, Y669S, S672Y, F675S, H677Y, R678Q, D679G, D679N, R683S, C685F, V687A, V687I, G688R, A689E, A689V, V693L, K694N, I695F, I695T, G696R, G696W, D697N, G699S, M700T, S701F, R702I, V704F, Y705N, S706I, T707M, D708N, Y710C, Y710H, L712F, L712P, N714S, P715L, P715S, S716Y, G717R, D719N, F720I, F720L, W723R, C724F, V726L, T730N, M731I, M731L, L732I, R735C, R735H, R735S, W736C, P738H, P738S, S741C, S741I, Y744F, R745P, R745W, K746R, K746T, T749K, S751N, D752N, V753L, W754C, W754L, S755R, G757E, G757R, G757W, I759M,L760F, E762D, E762K, F764I, Y766F, K768E, K768R, F772L, Q773K, T777M, E778K, E778V, E781K, C782R, C782S, I783N, T784S, G786C, G786S, R787C, R787H, R787S, L789F, E790V, R791Q, R791W, P792H, R793L, R793Q, P796L, P796S, D801N, G805R,G805W, C806S, W807G, Q808H, R809W, E810K, Q812H, Q813E, Q813K, R814Q, L815M, E819K, K822R, L824F, H825R, H825Y, A826G, A826S, A826V, L827F, G828E, G828W, A830D, P832A, P832R, P832T, I833V, Y834C, Y834N, D836E, and D836N (e.g., compared to the wildtype sequence NP_001012338). In some biopsy samples, mutation in the NTRK3 gene results in a TrkC protein lacking amino acids 548 to 562 in the wildtype TrkC protein (e.g., as compared to NP_001012338 (SEQ ID NO: 7)).

As one skilled in the art can appreciate, the specific substitutions listed above are exemplary. For example, when a naturally-occurring amino acid at an amino acid position is substituted with a different amino acid, it is understood that an amino acid having a chemically-related amino acid side chain may also be substituted (and detected in a cancer cell). Amino acids that have chemically-related amino acid side chains are listed in Table A.

**Table A. Chemically Related Amino Acid Side Chains**

| | |
|---|---|
| Positively-Charged Side Chains | Lysine, Arginine, Histidine |
| Negatively-Charged Side Chains | Glutamate and Aspartate |
| Nonpolar and/or Aliphatic Side Groups | Glycine, Alanine, Valine, Leucine, Isoleucine, and Proline |
| Polar, Uncharged Side Groups | Serine, Threonine, Cysteine, Methionine, Asparagine, Glutamine |
| Aromatic Side Chains | Phenylalanine, Tyrosine, and Tryptophan |

Any of the point mutations described herein may result in, e.g., increased the catalytic activity of a TrkA, TrkB, or TrkC kinase. Any of the point mutations described herein may result in, e.g., a decrease in the auto-inhibited conformation of a Trk kinase (e.g., a TrkA, TrkB, or TrkC kinase). Any of the point mutations described herein may result in, e.g., an increase in the activated conformation of a Trk kinase (e.g., a TrkA, TrkB, or TrkC kinase).

### Isolating Genomic DNA from a Biopsy Sample

Methods of isolating genomic DNA from biopsy sample are well known in the art. For example, a number of commercially available kits can be used to isolate genomic DNA from a sample containing mammalian cells (e.g., a biopsy sample). Non-limiting examples of commercially available kits for the isolation of genomic DNA from a sample containing mammalian cells include: ChargeSwitch® gDNA Tissue Kit (Life Technologies), Genomic DNA Isolation Kit (Norgen Biotek Corp., Ontario, Canada), QIAmp DNA FFPE (Qiagen), QIAsymphony DSP DNA kits (Qiagen), REPLI-g Mini Kit (Qiagen), Generation Capture Plate Kit (Qiagen), QI Amp 96 DNA Blood Kit (Qiagen), QIAmp DNA Mini kit (Qiagen), Biosprint 15 DNA Bloot Kit (Qiagen), Biosprint 96 DNA Blood Kit (Qiagen), MagAttract DNA Mini M48 Kit (Qiagen), QIAmp DNA Blood BioRobot 9604 Kit (Qiagen), QIAmp DNA Investigator Kit (Qiagen), QIAmp DNA Micro Kit, Xtreme DNA Isolation Kit (Isohelix; Harrietsham, Kent, UK), DDK DNA Isolation Kit (Isohelix), and XtraClean DNA kit (Isohelix). Genomic DNA can be isolated from a sample (e.g., a biopsy sample) using these and other commercially available genomic DNA isolation kits by following the manufacturer's instructions.

An exemplary method for isolating genomic DNA from a sample (e.g., a biopsy sample) include the steps of: lysing mammalian cells present in the sample, precipitating proteins in the lysate, removing the supernatant, precipitating genomic DNA out of the supernatant, washing the genomic DNA pellet with ethanol, and rehydrating the genomic DNA pellet in a pharmaceutically acceptable buffer (e.g., sterile or filtered water, or a buffered solution).

### Assays for Determining the Presence of a Point Mutation

Some of the methods provided herein include a step of performing an assay to determine the presence of at least one (e.g., at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, or at least twenty) point mutation in a NTRK1, NTRK2, and/or NTRK3 gene (e.g., any of the point mutations in NTRK1, NTRK2, and/or NTKR3 described herein) in a cell (e.g., cancer cell) in a sample from the subject (e.g., a biopsy sample).

A variety of assays for determining the presence of one or more point mutations in a cell (e.g., a cancer cell) are known in the art. Non-limiting examples of such assays (which can be used in any of the methods described herein) include: denaturing gradient gel electrophoresis (DGGE) (Nollau et al., Clin. Chem. 43:1114-1128, 1997), temperature gradient gel electrophoresis (TGGE) (Nollau et al., Clin. Chem. 43:1114-1128, 1997), temperature gradient capillary electrophoresis, single strand conformational polymorphism assays (see, e.g., Tahira et al., Human Mutat. 26:69-77, 2005), molecular beacon assays (see, e.g., Totowa, NJ, Vol. 212, pp. 111-128, 2003), dynamic hybridization (see, e.g., Howell et al., Nature Biotechnol. 17:87-88, 1999), PCR-based assays (e.g., tetraprimer ARMS-PCR (see, e.g., Zhang et al., Plos One 8:e62126, 2013), real-time PCR, allele-specific PCR (see, e.g., Gaudet et al., Methods Mol. Biol. 578:415-424, 2009), and TaqMan Assay Genotyping (see, e.g., Woodward, Methods Mol. Biol. 1145:67-74, 2014, and TaqMan®OpenArray® Genotyping Plates from Life Technologies)), Flap endonuclease assays (also called Invader assays) (see, e.g., Olivier et al., Mutat. Res. 573:103-110, 2005), oligonucleotide ligation assays (see, e.g., Bruse et al., Biotechniques 45:559-571, 2008), or, denaturing high performance liquid chromatography (see, e.g., Yu et al., J. Clin. Pathol. 58:479-485, 2005), high-resolution melting of an amplified sequence containing the point mutation (see, e.g., Wittwer et al., Clinical Chemistry 49:853-860, 2003), or sequencing (e.g., Maxam-Gilbert sequencing, chain-termination methods, shotgun sequencing, bridge PCR, and next-generation sequencing methods (e.g., massively parallel signature sequencing, polony sequencing, 454 pyrosequencing, Illumina (Solexa) sequencing, SOLiD sequencing, Ion Torrent semiconductor sequence, DNA nanoball sequencing, heliscope single molecule sequencing, and single molecule real-time sequencing)). Additional details and a summary of various next-generation sequencing methods are described in Koboldt et al., Cell 155:27-38, 2013.

In some embodiments, the assay used to determine the presence of the at least one point mutation in NTRK1, NTRK2, and/or NTRK3 includes a PCR assay (e.g., a real-time PCR-assay, e.g., a real-time PCR-based genotyping assay) (with or without a prior pre-amplification step). In some embodiments of any of the methods described herein the assay used to determine the presence of at least one point mutation in NTRK1, NTRK2, and/or NTRK3 is performed using TaqMan®-based sequencing (e.g., TaqMan®-based OpenArray® sequencing, e.g., high throughput TaqMan®-based Open Array® sequencing) (with or without a prior pre-amplification step). Methods for designing primers for use in the assays described herein are well-known in the art. For example, several vendors provide free software for designing forward and reverse primers for use in any of the assays described herein. A forward or reverse primer for use in any of the assays described herein can contain at least 10 (e.g., 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides). In some examples, a forward or reverse primer used in any of the assays described herein can include a label (e.g., any of the exemplary labels described herein) or can include a contiguous tag sequence (e.g., between about 5 nucleotides and about 25 nucleotides, between about 10 nucleotides and about 25 nucleotides, between about 10 nucleotides and 20 nucleotides, between about 5 nucleotides and about 20 nucleotides) that does not hybridize to a sequence within the subject's genome (e.g., the human genome).

In some embodiments, the assay includes the use of one or more probes (e.g., detectably labeled probes) that specifically hybridize to one or more segments of a NTRK1, NTRK2, and/or NTRK3 gene that include a point mutation (e.g., any of the point mutations in NTRK1, NTRK2, and/or NTRk3 described herein). For example, the one or more probes can have 9, 10, 11, 12, 13, 14, 15, 16, 17, 18, 19, 20, 21, 22, 23, 24, 25, 26, 27, 28, 29, 30, 31, 32, 33, 34, or 35 nucleotides. Additional description of the probes that can be used in exemplary assays are described herein.

### Subjects

In various embodiments of the methods described herein, the subject can be previously identified or diagnosed as having a cancer (e.g., any of the cancers described herein). A subject can, e.g., be previously identified as having a cancer cell that has at least one (e.g., two, three, four, five, six, seven, eight, nine, or ten) point mutation in a NTRK1, NTRK2, and/or NTRK3 gene (e.g., any of the NTRK1, NTRK2, and/or NTRK3 point mutations described herein). For example, a subject can be previously identified as having at least one (e.g., two, three, four, five, six, seven, eight, nine, or ten) point mutation in a NTRK2 gene that results in the expression of a TrkB protein including a mutation at one or more (e.g., two, three, four, or five) amino acid position(s) selected from the group consisting of: 240, 241, 242, 264, 314, 315, 401, 426, 427, 428, 440, and 689 (e.g., a TrkB protein including one or more (e.g., two, three, four, five, six, seven, eight, nine, or ten) of M240I, N241D, E242K, I264M, A314E, A314G, A314V, L315F, G401A, G401E, G401R, T426I, G427S, R428Q, A440S, A440T, A440V, and V689M).

In the methods of predicting a subject's risk of developing a cancer and the methods of assisting in the diagnosis of a cancer, the subject can be an undiagnosed subject, the subject can be identified as having been exposed to a significant level of carcinogen(s), the subject can be suspected of having a cancer (e.g., any of the cancers described herein), the subject can present with one or more (e.g., two, three, four, or five) symptoms of cancer (e.g., any of the symptoms of cancer described herein), and/or the subject is known to an elevated risk of developing a cancer (e.g., a family history of cancer).

### Cancer

Methods of treating a cancer are provided herein. Point mutations in NTRK1, NTRK2, and/or NTRK3 were detected in biopsy samples obtained from subjects having a variety of different cancers including, but not limited to: adenocarcinoma, adrenal gland cortical carcinoma, adrenal gland neuroblastoma, anus squamous cell carcinoma, appendix adenocarcinoma, bladder urothelial carcinoma, bile duct adenocarcinoma, bladder carcinoma, bladder urothelial carcinoma, bone chordoma, bone marrow leukemia lymphocytic chronic, bone marrow leukemia non-lymphocytic acute myelocytic, bone marrow lymph proliferative disease, bone marrow multiple myeloma, bone sarcoma, brain astrocytoma, brain glioblastoma, brain medulloblastoma, brain meningioma, brain oligodendroglioma, breast adenoid cystic carcinoma, breast carcinoma, breast ductal carcinoma in situ, breast invasive ductal carcinoma, breast invasive lobular carcinoma, breast metaplastic carcinoma, cervix neuroendocrine carcinoma, cervix squamous cell carcinoma, colon adenocarcinoma, colon carcinoid tumor, duodenum adenocarcinoma, endometrioid tumor, esophagus adenocarcinoma, esophagus and stomach carcinoma, eye intraocular melanoma, eye intraocular squamous cell carcinoma, eye lacrimal duct carcinoma, fallopian tube serous carcinoma, gallbladder adenocarcinoma, gallbladder glomus tumor, gastroesophageal junction adenocarcinoma, head and neck adenoid cystic carcinoma, head and neck carcinoma, head and neck neuroblastoma, head and neck squamous cell carcinoma, kidney chromophore carcinoma, kidney medullary carcinoma, kidney renal cell carcinoma, kidney renal papillary carcinoma, kidney sarcomatoid carcinoma, kidney urothelial carcinoma, kidney carcinoma, leukemia lymphocytic, leukemia lymphocytic chronic, liver cholangiocarcinoma, liver hepatocellular carcinoma, liver carcinoma, lung adenocarcinoma, lung adenosquamous carcinoma, lung atypical carcinoid, lung carcinosarcoma, lung large cell neuroendocrine carcinoma, lung non-small cell lung carcinoma, lung sarcoma, lung sarcomatoid carcinoma, lung small cell carcinoma, lung small cell undifferentiated carcinoma, lung squamous cell carcinoma, upper aerodigestive tract squamous cell carcinoma, upper aerodigestive tract carcinoma, lymph node lymphoma diffuse large B cell, lymph node lymphoma follicular lymphoma, lymph node lymphoma mediastinal B-cell, lymph node lymphoma plasmablastic lung adenocarcinoma, lymphoma follicular lymphoma, lymphoma, non-Hodgkins, nasopharynx and paranasal sinuses undifferentiated carcinoma, ovary carcinoma, ovary carcinosarcoma, ovary clear cell carcinoma, ovary epithelial carcinoma, ovary granulosa cell tumor, ovary serous carcinoma, pancreas carcinoma, pancreas ductal adenocarcinoma, pancreas neuroendocrine carcinoma, peritoneum mesothelioma, peritoneum serous carcinoma, placenta choriocarcinoma, pleura mesothelioma, prostate acinar adenocarcinoma, prostate carcinoma, rectum adenocarcinoma, rectum squamous cell carcinoma, skin adnexal carcinoma, skin basal cell carcinoma, skin melanoma, skin Merkel cell carcinoma, skin squamous cell carcinoma, small intestine adenocarcinoma, small intestine gastrointestinal stromal tumors (GISTs), large intestine/colon carcinoma, large intestine adenocarcinoma, soft tissue angiosarcoma, soft tissue Ewing sarcoma, soft tissue hemangioendothelioma, soft tissue inflammatory myofibroblastic tumor, soft tissue leiomyosarcoma, soft tissue liposarcoma, soft tissue neuroblastoma, soft tissue paraganglioma, soft tissue perivascular epitheliod cell tumor, soft tissue sarcoma, soft tissue synovial sarcoma, stomach adenocarcinoma, stomach adenocarcinoma diffuse-type, stomach adenocarcinoma intestinal type, stomach adenocarcinoma intestinal type, stomach leiomyosarcoma, thymus carcinoma, thymus thymoma lymphocytic, thyroid papillary carcinoma, unknown primary adenocarcinoma, unknown primary carcinoma, unknown primary malignant neoplasm, lymphoid neoplasm, unknown primary melanoma, unknown primary sarcomatoid carcinoma, unknown primary squamous cell carcinoma, unknown undifferentiated neuroendocrine carcinoma, unknown primary undifferentiated small cell carcinoma, uterus carcinosarcoma, uterus endometrial adenocarcinoma, uterus endometrial adenocarcinoma endometrioid, uterus endometrial adenocarcinoma papillary serous, and uterus leiomyosarcoma. See, e.g., Tables 1-4.

Additional examples of cancers include: acute lymphoblastic leukemia, acute myeloid leukemia, adrenocortical carcinoma, anal cancer, appendix cancer, astrocytoma, atypical teratoid/rhabdoid tumor, basal cell carcinoma, B-cell cancer, bile duct cancer, bladder cancer, bone cancer (e.g., osteosarcoma, malignant fibrous histiocytoma, and Ewing sarcoma), brain cancer (e.g., astrocytoma, brain and spinal cord tumor, brain stem glioma, central nervous system atypical teratoid/rhabdoid tumor, central nervous system embryonal tumors, central nervous system germ cell tumors, craniopharyngioma, and ependymoma), breast cancer, bronchogenic carcinoma, bronchus cancer, cancer of hematological tissues, cancer of the oral cavity or pharynx, carcinoid tumor, cervical cancer, childhood cancers, chordoma, chronic lymphocytic leukemia, chronic myelogenous leukemia, chronic myeloproliferative neoplasms, colon cancer, colorectal cancer, craniopharyngioma, cutaneous T-cell lymphoma, ductal carcinoma in situ, embryonal tumor, endrometrial cancer, ependymoma, esophageal cancer, esthesioneuroblastoma, Ewing sarcoma, extracranial germ cell tumor, extragonadal germ cell tumor, extrahepatic bile duct cancer, eye cancer (e.g., intraocular melanoma and retinoblastoma), fallopian tube cancer, fibrosarcoma, fibrous histiocytoma of bone, osteosarcoma, gallbladder cancer, gastric cancer, gastrointestinal carcinoid tumor, gastrointestinal stromal tumor (GIST), germ cell tumor, gestational trophoblastic disease, glioblastoma (e.g., glioblastoma multiforme), glioma (e.g., lower-grade glioma), hairy cell leukemia, head and neck cancer, heart cancer, histiocytosis, Hodgkin lymphoma, hypopharyngeal cancer, inflammatory myofibroblastic tumors, intrahepatic cholangiocarcinoma, intraocular melanoma, islet cell tumor, kidney cancer (e.g., renal cell cancer or Wilms tumor), kidney carcinoma, Langerhans cell histiocytosis, large cell neuroendocrine cancer, laryngeal cancer, leukemia (e.g., acute lymophoblastic leukemia, acute myeloid leukemia, chronic lymphocytic leukemia, chronic myelogenous leukemia, and hairy cell leukemia), lip and oral cavity cancer, liver cancer, liver carcinoma, lung cancer (e.g., lung adenocarcinoma), lymphoma (e.g., Burkitt lymphoma, cutaneous T-cell lymphoma, Hodgkin lymphoma, non-Hodgkin lymphoma, and primary central nervous system lymphoma), malignant fibrous histiocytoma of bone and osteosarcoma, medulloblastoma, melanoma, Merkel cell carcinoma, mesothelioma, mouth cancer, mouth carcinoma, multiple myeloma, myelodysplastic syndromes, nasal cavity and paranasal sinus cancer, nasopharyngeal cancer, neoplasm (e.g., a melanocystic neoplasm or lymphoid neoplasm), nephroma, neuroblastoma, non-Hodgkin's lymphoma, non-small cell lung cancer, oral cancer, oral cavity cancer, oropharyngeal cancer, osteosarcoma, ovarian cancer, pancreatic cancer, papillary thyroid carcinoma, paraganglioma, parathyroid cancer, pediatric glioma, penile cancer, pharyngeal cancer, pheochromocytoma, pilocytic astrocytoma, pituitary tumor, plasma cell neoplasm, primary peritoneal cancer, prostate cancer, rectum carcinoma, renal cell cancer, retinoblastoma, salivary gland cancer, sarcoma (e.g., Ewing sarcoma, osteosarcoma, rhabdomyosarcoma, soft tissue sarcoma, uterine sarcoma, and undifferentiated sarcoma), secretory breast carcinoma, Sezary syndrome, skin cancer (e.g., melanoma and Merkel cell carcinoma), small bowel cancer, small cell lung cancer, small intestine cancer, large intestine/colon carcinoma, large intestine adenocarcinoma, soft tissue sarcoma, Spitz nevi, Spitz tumors, spitzoid melanoma, stomach cancer, squamous cell carcinoma, squamous neck cancer, testicular cancer, throat cancer, thymoma and thymic carcinoma, thyroid carcinoma, urethral cancer, uterine cancer, uterine corpus endometrioid carcinoma, urinary bladder cancer, urinary tract carcinoma, vaginal cancer, vulvar cancer, and Wilms tumor.

Methods of diagnosing a cancer (e.g., any of the cancers described herein) are known in the art. For example, a health care professional (e.g., a physician) can diagnose a subject as having a cancer by observing one or more symptoms of a cancer in the subject. Non-limiting examples of symptoms of a cancer include fever, fatigue, pain, hyperpigmentation, jaundice, erythema, pruritis, excessive hair growth, long-term constipation, diarrhea, change in the size of stool, pain when urinating, blood in urine, change in bladder function, sore that do not heal, white patches inside the mouth or on tongue, unusual bleeding or discharge, indigestion, trouble swallowing, changes in warts, moles, or freckles, nagging cough, hoarseness, lump or area of thickening that can be felt under skin, weight changes, trouble breathing, discomfort after eating, persistent, unexplained muscle or joint pain, persistent, unexplained fevers and night sweats, and unexplained bruising. The diagnosis of a cancer by a health care profession (e.g., a physician) can also include performing laboratory tests (e.g., urine or blood tests, e.g., complete blood count), imaging tests (e.g., computerized tomography (CT), bone scan, magnetic resonance imaging (MRI), positron emission tomography (PET) scan, ultrasound, and X-ray), and obtaining and/or examining a biopsy sample from the subject.

Exemplary methods of assisting in the diagnosis of a cancer in a subject and methods of predicting a subject's risk of developing a cancer are provided herein.

### Trk Inhibitors

A variety of Trk inhibitors are known in the art. Non-limiting examples of Trk inhibitors are described below.

Non-limiting examples of Trk inhibitors are described in U.S. Patent No. 8,513,263 and International Publication No. WO 2010/048314 both of which are incorporated by reference in their entireties herein, and include a compound of Formula I: 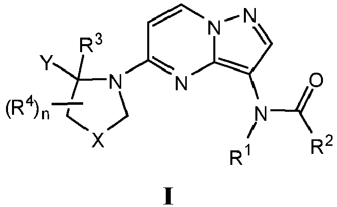 or a pharmaceutically acceptable salt thereof, wherein:
R¹ is H or (1-6C alkyl);
R² is NR^{b}R^{c}, (1-4C)alkyl, (1-4C)fluoroalkyl, CF₃, (1-4C)hydroxyalkyl, -(1-4C alkyl)hetAr¹, -(1-4C alkyl)NH₂, -(1-4C alkyl)NH(1-4C alkyl), -(1-4C alkyl)N(1-4C alkyl)₂, hetAr², hetCyc¹, hetCyc², phenyl which is optionally substituted with NHSO₂(1-4C alkyl), or (3-6C)^{e}cycloalkyl which is optionally substituted with (1-4C alkyl), CN, OH, OMe, NH₂, NHMe, N(CH₃)₂, F, CF₃, CO₂(1-4C alkyl), CO₂H, C(=O)NR^{e}R^{f} or C(=O)OR^{g};
R^{b} is H or (1-6C alkyl);
R^{c} is H, (1-4C)alkyl, (1-4C)hydroxyalkyl, hetAr³, or phenyl, wherein said phenyl is optionally substituted with one or more substituents independently selected from halogen, CN, CF₃ and -O(1-4C alkyl),
or NR^{b}R^{c} forms a 4 membered heterocyclic ring having a ring nitrogen atom wherein said heterocyclic ring is optionally substituted with one or more substituents independently selected from halogen, OH, (1-4C alkyl), (1-4 C)alkoxy, -OC(=O)(1-4C alkyl), NH₂, - NHC(=O)O(1-4C alkyl) and (1-4C)hydroxyalkyl,
or NR^{b}R^{c} forms a 5-6 membered heterocyclic ring having a ring heteroatom which is nitrogen and optionally having a second ring heteroatom or group selected from N, O and SO₂, wherein the heterocyclic ring is optionally substituted with one or more substituents independently selected from OH, halogen, CF₃, (1-4C)alkyl, CO₂(1-4C alkyl), CO₂H, NH₂, NHC(=O)O(1-4C alkyl) and oxo,
or NR^{b}R^{c} forms a 7-8 membered bridged heterocyclic ring having a ring nitrogen atom and optionally having a second ring heteroatom selected from N and O, wherein said ring is optionally substituted with CO₂(1-4C alkyl);
hetAr¹ is a 5-membered heteroaryl ring having 1-3 ring nitrogen atoms;
hetAr² is 5-6 membered heteroaryl ring having at least one nitrogen ring atom and optionally having a second ring heteroatom independently selected from N and S, wherein said heteroaryl ring is optionally substituted with one or more substituents independently selected from (1-4C alkyl), halogen, -(1-4 C)alkoxy, and NH(1-4C alkyl);
hetCyc¹ is a carbon-linked 4-6 membered azacyclic ring optionally substituted with one or more substituents independently selected from (1-4C alkyl), and CO₂(1-4C alkyl);
hetCyc² is a pyridinone or pyridazinone ring which is optionally substituted with a substituent selected from (1-4C)alkyl;
hetAr³ is a 5-6 membered heteroaryl ring having 1-2 ring heteroatoms independently selected from N and O and optionally substituted with one or more substituents independently selected from (1-4C)alkyl;
R^{e} is H or (1-4C)alkyl;
R^{f} is H, (1-4C)alkyl, or (3-6C)cycloalkyl;
or NR^{e}R^{f} forms a 5-6-membered azacyclic ring optionally having an additional ring heteroatom selected from N and O, wherein the azacyclic ring is optionally substituted with OH;
R^{g} is H or (1-6C)alkyl;
Y is (i) phenyl optionally substituted with one or more substituents independently selected from halogen, (1-4C)alkoxy, CF₃ and CHF₂, or (ii) a 5-6 membered heteroaryl ring having a ring heteroatom selected from N and S, wherein said heteroaryl ring is optionally substituted with one or more halogen atoms;
X is null, -CH₂-, -CH₂CH₂-, -CH₂O- or -CH₂NR^{d}- ;
R^{d} is H or (1-4C alkyl);
R³ is H or (1-4C alkyl);
each R⁴ is independently selected from halogen, (1-4C)alkyl, OH, (1-4C)alkoxy, NH₂, NH(1-4C alkyl) and CH₂OH; and
n is 0, 1, 2, 3, 4, 5 or 6.

For example, a Trk inhibitor can include one or more compounds selected from the group consisting of:
(R)-N-(5-(2-(2,5-difluorophenyl)pyrrolidin-1-yl)pyrazolo[1,5-a]pyrimidin-3-yl)-3-hydroxyazetidine-1-carboxamide;
N-(5-(2-(3-fluorophenyl)-2-methylpyrrolidin-1-yl)pyrazolo[1,5-a]pyrimidin-3-yl)-3-hydroxyazetidine-1-carboxamide;
(R)-1-(5-(2-(2,5-difluorophenyl)pyrrolidin-5-difluorophenyl)pyrrolidin-1-yl)pyrazolo[1,5-a]pyrimidin-3-yl)-3-phenylurea;
(R)-N-(5-(2-(2-(difluoromethyl)-5-fluorophenyl)pyrrolidin-1-yl)pyrazolo[1,5-a]pyrimidin-3-yl)-3-hydroxyazetidine-1-carboxamide;
(R)-N-(5-(2-(3-fluorophenyl)pyrrolidin-1-yl)pyrazolo[1,5-a]pyrimidin-3-yl)-1-methyl-6-oxo-1,6-dihydropyridazine-3-carboxamide;
(S)-N-(5-((R)-2-(2,5-difluorophenyl)pyrrolidin-1-yl)pyrazolo[1,5-a]pyrimidin-3-yl)-3-hydroxypyrrolidine-1-carboxamide;
(3R,4R)-N-(5-((R)-2-(2,5-difluorophenyl)pyrrolidin-1-yl)pyrazolo[1,5-a]pyrimidin-3-yl)-3,4-dihydroxypyrrolidine-1-carboxamide;
(S)-N-(5-((R)-2-(2-chloro-5-fluorophenyl)pyrrolidin-1-yl)pyrazolo[1,5-a]pyrimidin-3-yl)-3-methylpiperazine-1-carboxamide;
(R)-N-(5-(2-(2,5-difluorophenyl)pyrrolidin-1-yl)pyrazolo[1,5-a]pyrimidin-3-yl)-3-hydroxy-3-methylazetidine-1-carboxamide;
(R)-N-(5-(2-(3-fluorophenyl)pyrrolidin-1-yl)pyrazolo[1,5-a]pyrimidin-3-yl)-3-hydroxyazetidine-1-carboxamide; and
(R)-1-(4-chlorophenyl)-3-(5-(2-(2,5-difluorophenyl)pyrrolidin-1-yl)pyrazolo[1,5-a]pyrimidin-3 -yl)urea,
or a pharmaceutically acceptable salt thereof.

In some embodiments, a Trk inhibitor can be (S)-N-(5-((R)-2-(2,5-difluorophenyl)pyrrolidin-1-yl)pyrazolo[1,5-a]pyrimidin-3-yl)-3-hydroxypyrrolidine-1-carboxamide sulfate. For example, a Trk inhibitor can be a polymorph such as those described in U.S. Publication No. 2016/0137654 and International Publication No. WO 2016/077841, both of which are incorporated by reference in their entireties herein. Additional disclosure relating to Trk inhibitors can be found, for example, in U.S. Provisional Application Nos. 62/329,653, 62/329,561, and 62/338,359, all of which are incorporated by reference in their entireties.

Additional examples of Trk inhibitors are the macrocyclic compounds described in U.S. Patent No. 8,933,084 and International Publication No. WO 2011/146336, both of which are herein incorporated by reference in their entireties. For example, Trk inhibitors include compounds of Formula I: 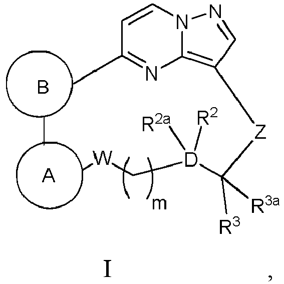 or a pharmaceutically acceptable salt thereof, wherein:
ring A is selected from rings A-I, A-2, and A-3 having the structures: 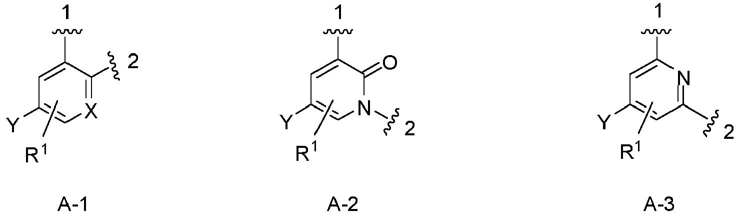 wherein the wavy line labeled 1 indicates the point of attachment of ring A to ring B and the wavy line labeled 2 indicates the point of attachment of ring A to W;
   X is N or CH;
   Y is H or F;
   R¹ is H, (1-3C)alkoxy, or halogen;
ring B is selected from rings B-1 and B-2 having the structures: 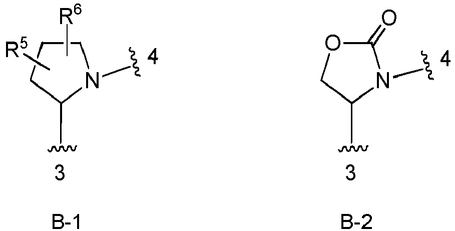 wherein the wavy line labeled 3 indicates the point of attachment to ring A and the wavy line labeled 4 indicates the point of attachment to the pyrazolo[1,5-a]pyrimidine ring of Formula I;
   W is O, NH, or CH₂, wherein when ring A is A-2, then W is CH₂;
   m is 0, 1, or 2;
   D is carbon, R² and R^{2a} are independently H, F, (1-3 C)alkyl or OH (provided that R² and R^{2a} are not both OH), and R³ and R^{3a} are independently H, (1-3 C)alkyl or hydroxy(1-3 C)alkyl, or
   D is carbon or nitrogen, R² and R³ are absent, and R^{2a} and R^{3a} together with the atoms to which they are attached form a 5-6 membered heteroaryl ring having 1-2 ring heteroatoms;
   Z is *-NR^{4a}C(=O)-, *-ONHC(=O)-, *-NR^{4b}CH₂- or *-OC(=O)-, wherein the asterisk indicates the point of attachment of Z to the carbon bearing R³;
   R^{4a} is H, (1-6C)alkyl, fluoro(I-6C)alkyl, difluoro(I-6C)alkyl, trifluoro(I-6C)alkyl, hydroxy(1-6C alkyl), or dihydroxy(2-6C alkyl);
   R^{4b} is H, (1-6C)alkyl, fluoro(1-6C)alkyl, difluoro(1-6C)alkyl, trifluoro(1-6C)alkyl, hydroxy(1-6C alkyl), dihydroxy(2-6C alkyl), (1-6C alkyl)C(O)-, (3-6C cycloalkyl)C(O)-, Ar¹C(O)-, HOCH₂C(O)-, (1-6C alkyl)sulfonyl, (3-6C cycloalkyl)sulfonyl, Ar²(SO₂)-, HO₂CCH₂-, or (1-6C alkyl)NH(CO)-;
   Ar¹ is phenyl optionally substituted with one or more substituents independently selected from halogen, (1-6C)alkyl, and (1-6C)alkoxy;
   Ar² is phenyl optionally substituted with one or more substituents independently selected from halogen, (1-6C)alkyl, and (1-6C)alkoxy; and
   R⁵ and R⁶ are independently H, halogen, OH, (1-6C)alkyl, or hydroxy(1-6C)alkyl.

For example, a Trk inhibitor can include one or more compounds selected from the group consisting of:
(6R)-9-fluoro-13-oxa-2,11, 17,21,22,25-hexaazapentacyclo[17.5.2.0^{2,6}.0^{7,12}.0^{22,26}]hexacosa-1(25),7,9,11,19(26),20,23-heptaen-18-one;
(6R)-9-fluoro-15-hydroxy-13-oxa-2,11, 17,21,22,25-hexaazapentacyclo[17.5.2.0^{2,6}.0^{7,12}.0^{22,26}] hexacosa-1(25),7,9,11,19(26),20,23-heptaen-18-one;
(6*R*,15*R*)-9-fluoro-15-hydroxy-13-oxa-2,11,17,21,22,25-hexaazapentacyclo-[17.5.2. 0^{2,6}.0^{7,12}.0^{22,26}] hexacosa-1(25),7,9,11,19(26),20,23-heptaen-18-one;
(6*R*)-9-fluoro-13-oxa-2,11, 16,20,21,24-hexaazapentacyclo[16.5.2.0^{2,6}.0^{7,12}.0^{21,25}]pentacosa-1(24),7,9,11,18(25),19,22-heptaen-17-one;
(6*R*)-9-fluoro-13-oxa-2, 11, 18,22,23,26-,hexaazapentacyclo[18.5.2.0^{2,6}.0^{7,12}.0^{23,27}]heptacosa-1(26)7,9,11,20(27),21,24-heptaen-19-one;
(6*R*,13,*S*)-9-fluoro-13-methyl-2, 11, 15, 19,20,23-hexaazapentacyclo [15.5.2.1^{7,11}.0^{2,6}.0^{20,24}]pentacosa-1(23),7,9,17(24),18,21-hexaene-16,25-dione;
(6*R*)-9-fluoro-2, 11, 13, 16,20,21,24-heptaazapentacyclo[16.5.2.0^{2,6}.0^{7,12}.0^{21,25}]pentacosa-1(24,7,9,11,18(25),19,22-heptaen-17-one;
(6*R*)-9-fluoro-2,11, 13, 17,21,22,25-heptaazapentacyclo[17.5.2.0^{2,6}.0^{7,12}.0^{22,26}]hexacosa-1(25)7, 9, 11, 19(26),20,23-heptaen-18-one;
(6*R*)-9-fluoro-17-methyl-13-oxa-2, 11, 17,21,22,25-hexaazapentacyclo[17.5.2.0^{2,6}.0^{7,12}.0^{22,26}] hexacosa-1(25), 7,9,11,19(26),20,23-heptaen-18-one;
(6R)-9,15,15-trifluoro-13-oxa-2,11,17,21,22,25-hexaazapentacyclo[17.5.2.0^{2,6}.0^{7,12}.0^{22,26}]hexacosa-1(25),7,9,11,19(26),20,23-heptaen-18-one;
(6*R*)-9-fluoro-2,11,16,20,21,24-hexaazapentacyclo[16.5.2.0^{2,6}.0^{7,12}.0^{21,25}]pentacosa-1(24),7,9,11,18(25),19,22-heptaen-17-one;
(6*R*)-9-fluoro-15-methyl-2, 11, 16,20,21,24-hexaazapentacyclo[16.5.2.0^{2,6}.0^{7,12}.0^{21,25}]pentacosa-1(24),7,9,11,18(25),19,22-heptaen-17-one;
(6R)-9-fluoro-(15R)-methyl-2, 11, 16,20,21,24-hexaazapentacyclo[16.5.2.0^{2,6}.0^{7,12}.0^{21,25}]pentacosa-1(24),7,9,11,18(25),19,22-heptaen-17-one;
(6*R*)-9-fluoro-15-methyl-2, 11, 16,20,21,24-hexaazapentacyclo[16.5.2.0^{2,6}.0^{7,12}.0^{21,25}]pentacosa-1(24),7,9,11,18(25),19,22-heptaen-17-one;
(6*R*)-9-fluoro-15,15-dimethyl-13-oxa-2,11, 17,21,22,25-hexaazapentacyclo [17.5.2.0^{2,6}.0^{7,12}.0^{22,26}]hexacosa-1(25),7,9, 11, 19(26),20,23-heptaen-18-one; and
(6*R*)-9-fluoro-15,15-dimethyl-2, 11, 16,20,21,24-hexaazapentacyclo[16.5.2.0^{2,6}.0^{7,12}.0^{21,25}]pentacosa-1(24),7,9,11,18(25),19,22-heptaen-17-one;
or a pharmaceutically acceptable salt thereof.

Additional examples of Trk inhibitors are the substituted pyrazolo[1,5-a] pyrimidine compounds described in U.S. Patent No. 8,791,123 and International Publication No. WO 2011/006074, both of which are herein incorporated by reference in their entireties. For example, Trk inhibitors that can include compounds of Formula I: 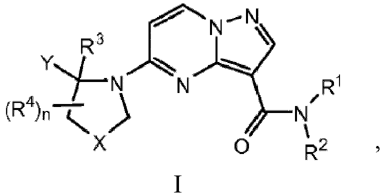 or a pharmaceutically acceptable salt thereof, wherein:
R¹ is H or (1-6C alkyl);
R² is H, (1-6C)alkyl, -(1-6C)fluoroalkyl, -(1-6C)difluoroalkyl, -(1-6C)trifluoroalkyl, -(1-6C)chloroalkyl, -(2-6C)chlorofluoroalkyl, -(2-6C)difluorochloroalkyl, -(2-6C)chlorohydroxyalkyl, -(1-6C)hydroxyalkyl, -(2-6C)dihydroxyalkyl, -(1-6C alkyl)CN, -(1-6C alkyl)SO₂NH₂, -(1-6C alkyl)NHSO₂(1-3C alkyl), -(1-6C alkyl)NH₂, -(1-6C alkyl)NH(1-4C alkyl), -(1-6C alkyl)N(1-4C alkyl)₂, -(1-6C alkyl)NHC(=O)O(1-4C alkyl), -(1-6C alkyl)hetCyc¹, -(1-6C alkyl)hetAr¹, hetAr², hetCyc², -O(1-6C alkyl) which is optionally substituted with halogen, OH or (1-4C)alkoxy, -O(3-6C cycloalkyl), Cyc¹, -(1-6C alkyl)(3-6C cycloalkyl), -(1-6C alkyl)(1-4C alkoxy), -(1-6C hydroxyalkyl)(1-4C alkoxy), a bridged 7 -membered cycloalkyl ring optionally substituted with (1-6C)hydroxyalkyl, or a bridged 7-8 membered heterocyclic ring having 1-2 ring nitrogen atoms;
or NR¹R² forms a 4-6 membered azacyclic ring optionally substituted with one or more substituents independently selected from (1-6C)alkyl, OH, CO₂H, (1-3C alkyl)CO₂H, -O(1-6C alkyl), and (1-6C)hydroxyalkyl;
hetCyc¹ is a 5-6 membered heterocyclic ring having 1-2 ring heteroatoms independently selected from N and O, wherein hetCyc¹ is optionally substituted with oxo, OH, halogen, or (1-6C)alkyl;
hetCyc² is a 6 membered carbon-linked heterocyclic ring having 1-2 ring heteroatoms independently selected from N and O, wherein hetCyc² is optionally substituted with F, SO₂NH₂, SO₂(1-3C alkyl), or halogen;
hetAr¹ is a 5-membered heteroaryl ring having 1-2 ring heteroatoms independently selected from N and O and optionally substituted with (1-4C)alkyl;
hetAr² is a 5-6 membered heteroaryl ring having 1-2 ring nitrogen atoms and optionally substituted with one or more substituents independently selected from (1-4C)alkyl, (3-6C)cycloalkyl, halogen, and OH;
Cyc¹ is a 3-6 membered cycloalkyl ring which is optionally substituted with one or more substituents independently selected from -(1-4C alkyl), -OH, -OMe, -CO₂H, -(1-4C alkyl)OH, halogen, and CF₃;
Y is (i) phenyl optionally substituted with one or more substituents independently selected from halogen, (1-4C)alkoxy, -CF₃, -CHF₂, -O(1-4C alkyl)hetCyc³, -(1-4C alkyl)hetCyc³, -O(1-4C alkyl)O(1-3C alkyl) and -O(3-6C dihydroxyalkyl), or (ii) a 5-6 membered heteroaryl ring having a ring heteroatom selected from N and S, wherein the heteroaryl ring is optionally substituted with one or more substituents independently selected from halogen, -O(1-4C alkyl), (1-4C)alkyl, and NH₂, or (iii) a pyrid-2-on-3-yl ring optionally substituted with one or more substituents independently selected from halogen and (1-4C)alkyl;
hetCyc³ is a 5-6 membered heterocyclic ring having 1-2 ring heteroatoms independently selected from N and O and optionally substituted with (1-6C)alkyl;
X is -CH₂-, -CH₂CH₂-, -CH₂O-, or -CH₂NR^{d}-;
R^{d} is H or -(1-4C alkyl);
R³ is H or -(1-4C alkyl);
each R⁴ is independently selected from halogen, -(1-4C)alkyl, -OH, -(1-4C)alkoxy, -NH2, -NH(1-4C alkyl), and -CH₂OH; and
n is 0, 1, 2, 3, 4, 5, or 6.

For example, a Trk inhibitor can include one or more compounds selected from the group consisting of:
(R)-5-(2-(2,5-difluorophenyl)pyrrolidin-1-yl)-N-(pyridin-2-yl)pyrazolo[1,5-a]pyrimidine-3-carboxamide;
(R)-5-(2-(2,5-difluorophenyl)pyrrolidin-1-yl)-N-(2-morpholinoethyl)pyrazolo[1,5-a]pyrimidine-3-carboxamide;
N-((2S)-bicyclo[2.2.1]heptan-2-yl)-5-((R)-2-(2,5-difluorophenyl)pyrrolidin-1-yl)pyrazolo[1,5-a]pyrimidine-3-carboxamide;
(R)-5-(2-(2,5-difluorophenyl)pyrrolidin-1-yl)-N-(2-(2-oxoimidazolidin-1-yl)ethyl)pyrazole[l,5-a]pyrimidine-3-carboxamide;
5-((R)-2-(2,5-difluorophenyl)pyrrolidin-1-yl)-N-((R)-2,3-dihydroxypropyl)pyrazolo[1,5-a]pyrimidine-3-carboxamide;
(R)-N-cyclopropyl-5-(2-(5-fluoropyridin-3-yl)pyrrolidin-1-yl)pyrazolo[1,5-a]pyrimidine-3 -carboxamide;
(R)-N-tert-butyl-5-(2-(5-fluoropyridin-3-yl)pyrrolidin-1-yl)pyrazolo[1,5-a]pyrimidine-3 -carboxamide;
(R)-5-(2-(2,5-difluorophenyl)pyrrolidin-1-yl)pyrazolo[1,5-a]pyrimidine-3-carboxamide;
(R)-5-(2-(5-fluoropyridin-3-yl)pyrrolidin-1-yl)-N-(1-methylcyclobutyl)pyrazolo[1,5-a]pyrimidine-3-carboxamide; and
5-((R)-2-(5-fluoropyridin-3-yl)pyrrolidin-1-yl)-N-((S)-1,1,1-trifluoropropan-2-yl)pyrazolo[1,5-a]pyrimidine-3-carboxamide;
or a pharmaceutically acceptable salt thereof.

Additional examples of Trk inhibitors are the substituted imidazo[1,2-b]pyridazine compounds described in U.S. Patent No. 8,450,322 and International Publication No. WO 2010/033941, both of which are herein incorporated by reference in their entireties. For example, Trk inhibitors can include compounds of Formula I: 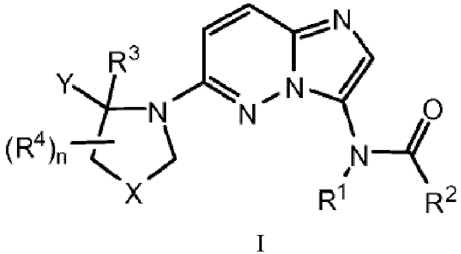 or a pharmaceutically acceptable salt thereof, wherein:
R¹ is H or (1-6C alkyl);
R² is NR^{b}R^{c}, (1-4C)alkyl, (1-4C)fluoroalkyl, CF3, (1-4C)hydroxyalkyl, -(1-4C alkyl)hetAr¹, -(1-4C alkyl)NH(1-4C alkyl), hetAr², hetCyc¹, hetCyc², phenyl which is optionally substituted with NHSO₂(1-4C alkyl), or (3-6C)cycloalkyl which is optionally substituted with (1-4C alkyl), CN, OH, CF3, CO₂(1-4C alkyl) or CO₂H;
R^{b} is H or (1-6C alkyl);
R^{c} is H, (1-4C)alkyl, (1-4C)hydroxyalkyl, hetAr³, or phenyl, wherein said phenyl IS optionally substituted with one or more substituents independently selected from halogen, CN, CF3 and -O(1-4C alkyl),
or NR^{b}R^{c} forms a 4 membered heterocyclic ring having a ring nitrogen atom, wherein said heterocyclic ring is optionally substituted with one or more substituents independently selected from halogen, OH, (1-4C alkyl), (1-4 C)alkoxy, -OC(=O)(1-4C alkyl), NH₂, -NHC(=O)O(1-4C alkyl), and (1-4C)hydroxyalkyl,
or NR^{b}R^{c} forms a 5-6 membered heterocyclic ring having a ring heteroatom which is nitrogen and optionally having a second ring heteroatom or group selected from N, O, and SO₂, wherein the heterocyclic ring is optionally substituted with one or more substituents independently selected from OH, halogen, CF3, (1-4C)alkyl, CO₂(1-4C alkyl), CO₂H, NH₂, NHC(=O)O(1-4C alkyl), and oxo,
or NR^{b}R^{c} forms a 7-8 membered bridged heterocyclic ring having 1-2 ring nitrogen atoms and optionally substituted with CO₂(1-4C alkyl);
hetAr¹ is a 5-membered heteroaryl ring having 1-3 ring nitrogen atoms;
hetAr² is 5-6 membered heteroaryl ring having at least one nitrogen ring atom and optionally having a second ring heteroatom independently selected from N and S, wherein said heteroaryl ring is optionally substituted with one or more substituents independently selected from (1-4C alkyl), halogen, -(1-4 C)alkoxy, and NH(1-4C alkyl);
hetCyc¹ is a carbon-linked 4-6 membered azacyclic ring optionally substituted with one or more substituents independently selected from (1-4C alkyl), CO₂H and CO₂(1-4C alkyl);
hetCyc² is a pyridinone or pyridazinone ring substituted with a substituent selected from (1-4C)alkyl;
hetAr³ is a 5-6 membered heteroaryl ring having 1-2 ring heteroatoms independently selected from N and O and optionally substituted with one or more substituents independently selected from (1-4C)alkyl;
Y is a phenyl ring optionally substituted with one or more substituents independently selected from halogen, (1-4C)alkoxy, CF₃ and CHF₂, or a 5-6 membered heteroaryl ring having a ring heteroatom selected from N and S;
X is null, -CH₂-, -CH₂CH₂-, -CH₂O-, or -CH₂NR^{d}- ;
R^{d} is H or (1-4C alkyl);
R³ is H or (1-4C alkyl);
each R⁴ is independently selected from halogen, (1-4C)alkyl, OH, (1-4 C)alkoxy, NH₂, NH(1-4C alkyl), and CH₂OH; and
n is 0, 1, 2, 3, 4, 5, or 6.

Additional Trk inhibitors can be found in U.S. Publication No. 2015/0166564 and WO 2012/158413, both of which are incorporated by reference in their entireties herein. For example, a Trk inhibitor can be a compound of Formula I: 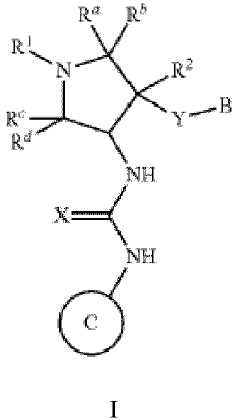 or stereoisomers, tautomers, or pharmaceutically acceptable salts, solvates or prodrugs thereof, wherein:
the Y-B moiety and the NH-C(=X)-NH moiety are in the trans configuration;
R^{a}, R^{b}, R^{c} and R^{d} are independently selected from H and (1-3C)alkyl;
X is O, S or NH;
R¹ is (1-3C alkoxy)(1-6C)alkyl, (trifluoromethoxy)(1-6C)alkyl, (1-3C sulfanyl)(1-6C)alkyl, monofluoro(1-6C)alkyl, difluoro(1-6C)alkyl, trifluoro(1-6C)alkyl, tetrafluoro(2-6C)alkyl, pentafluoro(2-6C)alkyl, cyano(1-6C)alkyl, aminocarbonyl(1-6C)alkyl, hydroxy(1-6C)alkyl, dihydroxy(2-6C)alkyl, (1-6C)alkyl, (1-3Calkylamino)(1-3C)alkyl, (1-4C alkoxycarbonyl)(1-6C)alkyl, amino(1-6C)alkyl, hydroxy(1-3C alkoxy)(1-6C)alkyl, di(1-3C alkoxy)(1-6C)alkyl, (1-3C alkoxy)trifluoro(1-6C)alkyl, hydroxytrifluoro(1-6C)alkyl, (1-4C alkoxycarbonyl)(1-3C alkoxy)(1-6C)alkyl, hydroxycarbonyl(1-3C alkoxy)(1-6C)alkyl, hetar⁵(CH₂)₀₋₁, or Ar⁵(CH₂)₀₋₁;
R² is H, F, or OH;
Y is a bond, -O- or -OCH₂-;
B is Ar¹, hetAr¹, 1-6C alkyl or (1-6C)alkoxy;
Ar¹ is phenyl optionally substituted with one or more substituents independently selected from halogen, CF₃, CF₃O-, (1-4C)alkoxy, hydroxy(1-4C)alkyl, (1-6C)alkyl and CN;
hetAr¹ is a 5-6 membered heteroaryl having 1-3 ring heteroatoms independently selected from N, S and O, and optionally substituted with 1-2 groups independently selected form (1-6C)alkyl, halogen, OH, CF₃, NH₂ and hydroxy(1-2C)alkyl;
Ring C is formula C-1, C-2, or C-3 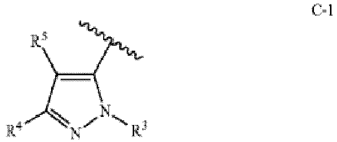 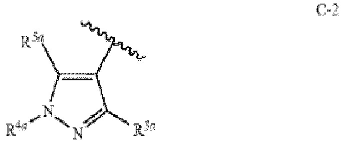 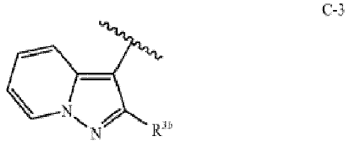
   R³ is H, (1-6C)alkyl, hydroxy(1-6C)alkyl, Ar², hetCyc¹, (3-7C)cycloalkyl, or hetAr²;
   Ar² is phenyl optionally substituted with one or more groups independently selected from halogen, (1-6C)alkyl and hydroxymethyl;
   hetCyc¹ is a 5-6-membered saturated or partially unsaturated heterocyclic ring having 1-2 ring heteroatoms independently selected from N and O;
   hetAr² is a 5-6 membered heteroaryl ring having 1-3 ring heteroatoms independently selected from N, O and S and optionally substituted with one or more groups independently selected from (1-6C)alkyl and halogen;
   R⁴ is H, OH, (1-6C)alkyl, monofluoro(1-6C)alkyl, difluoro(1-6C)alkyl, trifluoro(1-6C)alkyl, tetrafluoro(2-6C)alkyl, pentafluoro(2-6C)alkyl, cyano(1-6C)alkyl, hydroxy(1-6C)alkyl, dihydroxy(2-6C)alkyl, (1-3C alkoxy)(1-6C)alkyl, amino(1-6C)alkyl, aminocarbonyl(1-6C)alkyl, (1-3C)alkylsulfonamido(1-6C)alkyl, sulfamido(1-6C)alkyl, hydroxylcarbonyl(1-6C)alkyl, hetAr³(1-6C)alkyl, Ar³(1-6C)alkyl, (1-6C)alkoxy, monofluoro(1-6C)alkoxy, difluoro(1-6C)alkoxy trifluoro(1-6C)alkoxy, tetrafluoro(2-6C)alkoxy, pentafluoro(2-6C)alkoxy cyano(1-6C)alkoxy, hydroxy(1-6C)alkoxy, dihydroxy(2-6C)alkoxy, amino(2-6C)alkoxy, aminocarbonyl(1-6C)alkoxy, hydroxycarbonyl(1-6C)alkoxy, hetCyc²(1-6C)alkoxy, hetAr³(1-6C)alkoxy, Ar³(1-6C)alkoxy, (1-4C alkoxy)(1-6C)alkoxy, (1-3C alkylsulfonyl)(1-6C)alkoxy, (3-6C)cycloalkyl [optionally substituted with F, OH, (1-6C alkyl), (1-6C) alkoxy, or (1-3C alkoxy)(1-6C)alkyl], hetAr⁴, Ar⁴, hetCyc²(O)CH₂-, (1-4C alkoxycarbonyl)(1-6C)alkoxy, hydroxycarbonyl(1-6C)alkoxy, aminocarbonyl(1-6C)alkoxy, hetCyc²C(=O)(1-6C)alkoxy, hydroxy(1-3C alkoxy)(1-6C)alkoxy, hydroxytrifluoro(1-6C)alkoxy, (1-3C)alkylsulfonamido(1-6C)alkoxy, (1-3C)alkylamido(1-6C)alkoxy, di(1-3C alkyl)aminocarboxy, hetCyc²C(=O)O-, hydroxydifluoro(1-6C)alkyl, (1-4C alkylcarboxy)(1-6C)alkyl, (1-6C)alkoxycarbonyl, hydroxycarbonyl, aminocarbonyl, (1-3C alkoxy)amino-carbonyl, hetCyc³, halogen, CN, trifluoromethylsulfonyl, N-(1-3C alkyl)pyridinonyl, N-(1-3C trifluoroalkyl)pyridinonyl, (1-4C alkylsiloxy)(1-6C)alkoxy, isoindoline-1,3-dionyl(1-6C)alkoxy or N-(1-3C alkyl)oxadiazolonyl;
   hetCyc² is a 4-6 membered heterocyclic ring having 1-2 ring heteroatoms independently selected from N and O and optionally substituted with 1-2 groups independently selected from (1-6C)alkyl, (1-4C alkylcarboxy)(1-6C)alkyl, and (1-6C)acyl;
   hetCyc³ is a 4-7 membered heterocycle having 1-2 ring heteroatoms independently selected from N and O and optionally substituted with one or more substituents independently selected from F, CN, CF₃, (1-6C)alkyl, hydroxy(1-6C)alkyl, (1-3C alkoxy)(1-6C)alkyl, (1-6C)acyl-, (1-6C)alkylsulfonyl, trifluoromethylsulfonyl and (1-4C alkoxy)carbonyl;
   hetAr³ is a 5-membered heteroaryl ring having 1-3 ring atoms independently selected from N, O and S and optionally substituted with (1-6C)alkyl;
   Ar³ is phenyl optionally substituted with (1-4C)alkoxy;
   hetAr⁴ is a 5-6 membered heteroaryl ring having 1-3 ring heteroatoms independently selected from N, S and O and optionally substituted with 1-2 substituents independently selected from (1-6C)alkyl, halogen, CN, hydroxy(1-6C)alkyl, trifluoro(1-6C)alkyl, (3-6C)cycloalkyl, (3-6C cycloalkyl)CH₂- (3-6C cycloalkyl)C(=O)-, (1-3C alkoxy)(1-6C)alkyl, (1-6C)alkoxy, (1-6C)alkylsulfonyl, NH₂, (1-6C alkyl)amino, di(1-6C alkyl)amino, (1-3C trifluoroalkoxy), (1-3C)trifluoroalkyl, and methoxybenzyl; or a 9-10 membered bicyclic heteroaryl having 1-3 ring nitrogen atoms;
   Ar⁴ is phenyl optionally substituted with one or more groups independently selected from (1-6C)alkyl, halogen, CN, CF₃, CF₃O-, (1-6C)alkoxy, (1-6Calkyl)OC(=O)-, aminocarbonyl, (1-6C)alkylthio, hydroxy(1-6C)alkyl, (1-6C alkyl)SO₂-, HOC(=O)- and (1-3C alkoxy)(1-3C alkyl)OC(=O)-;
   R⁵ is H, (1-6C)alkyl, monofluoro(1-6C)alkyl, difluoro(1-6C)alkyl, trifluoro(1-6C)alkyl, tetrafluoro(2-6C)alkyl, pentafluoro(2-6C)alkyl, halogen, CN, (1-4C)alkoxy, hydroxy(1-4C)alkyl, (1-3C alkoxy)(1-4C)alkyl, (1-4C alkyl)OC(=O)-, (1-6C)alkylthio, phenyl [optionally substituted with one or more groups independently selected from halogen, (1-6C)alkyl and (1-6C)alkoxy], (3-4C)cycloalkyl, amino, aminocarbonyl, or trifluoro(1-3C alky)amido; or
   R⁴ and R⁵ together with the atoms to which they are attached form a 5-6 membered saturated, partially unsaturated or unsaturated carbocyclic ring optionally substituted with one or more substituents independently selected from (1-6C)alkyl, or
   R⁴ and R⁵ together with the atoms to which they are attached form 5-6 membered saturated, partially unsaturated or unsaturated heterocyclic ring having a ring heteroatom selected from N, O or S, wherein said heterocyclic ring is optionally substituted with one or two substituents independently selected from (1-6C alkyl)C(=O)O-, (1-6)acyl, (1-6C)alkyl and oxo, and said sulfur ring atom is optionally oxidized to S(=O) or SO₂;
   hetAr⁵ is a 5-6 membered heteroaryl ring having 1-3 ring heteroatoms independently selected from N, O or S, wherein the ring is optionally substituted with one or more substituents independently selected from halogen, (1-6C)alkyl, (1-6C)alkoxy and CF₃;
   Ar⁵ is phenyl optionally substituted with one or more groups independently selected from halogen, (1-6C)alkyl, (1-6C)alkoxy, CF₃O-, (1-4C)alkoxycarbonyl and aminocarbonyl;
   R^{3a} is hydrogen, halogen, (1-6C)alkyl, trifluoro(1-6C)alkyl, (3-6C)cycloalkyl, phenyl optionally substituted with one or more substituents independently selected from halogen, (1-6C)alkyl and hydroxymethyl, or a 5-6 membered heteroaryl ring having 1-3 ring heteroatoms independently selected from N, O and S and optionally substituted with one or more groups independently selected from (1-6C)alkyl and halogen;
   R^{3b} is hydrogen, (1-6C)alkyl, trifluoro(1-6C)alkyl, (3-6C)cycloalkyl, phenyl optionally substituted with one or more substituents independently selected from halogen, (1-6C)alkyl and hydroxymethyl, or a 5-6 membered heteroaryl ring having 1-3 ring heteroatoms independently selected from N, O and S and optionally substituted with one or more groups independently selected from (1-6C)alkyl and halogen;
   R^{4a} is hydrogen, (1-6C)alkyl, trifluoro(1-6C)alkyl, phenyl [optionally substituted with one or more groups independently selected from (1-6C)alkyl, halogen, CN, CF₃, CF₃O-, (1-6C)alkoxy, (1-6Calkyl)OC(=O)-, aminocarbonyl, (1-6C)alkylthio, hydroxy(1-6C)alkyl, (1-6C alkyl)SO₂-, HOC(=O)- and (1-3C alkoxy)(1-3C alkyl)OC(=O)-], or a 5-6 membered heteroaryl ring having 1-3 ring heteroatoms independently selected from N, S and O and optionally substituted with 1-2 substituents independently selected from (1-6C)alkyl, hydroxy(1-6C)alkyl, trifluoro(1-6C)alkyl, (3-6C)cycloalkyl, (3-6C cycloalkyl)CH₂- (3-6C cycloalkyl)C(=O)-, (1-3C alkoxy)(1-6C)alkyl, (1-6C)alkoxy, (1-6C)alkylsulfonyl, NH₂, (1-6C alkyl)amino, di(1-6C alkyl)amino, (1-3C trifluoroalkoxy)(1-3C)trifluoroalkyl, and methoxybenzyl; and
   R^{5a} is hydrogen, halogen, (1-6C)alkyl, trifluoro(1-6C)alkyl, (3-6C)cycloalkyl, phenyl optionally substituted with one or more substituents independently selected from halogen, (1-6C)alkyl and hydroxymethyl, or a 5-6 membered heteroaryl ring having 1-3 ring heteroatoms independently selected from N, O and S and optionally substituted with one or more groups independently selected from (1-6C)alkyl and halogen.

Further examples of Trk inhibitors can be found in International Publication No. WO 2014078454, which is incorporated by reference in its entirety herein. For example, a Trk inhibitor can be a compound of Formula I: 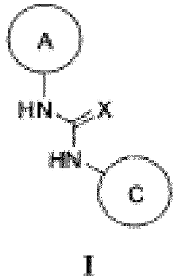 or stereoisomers, tautomers, or pharmaceutically acceptable salts, or solvates thereof, wherein:
X is O, S, NH or N-CN;
Ring A is formula A-1 or A-2 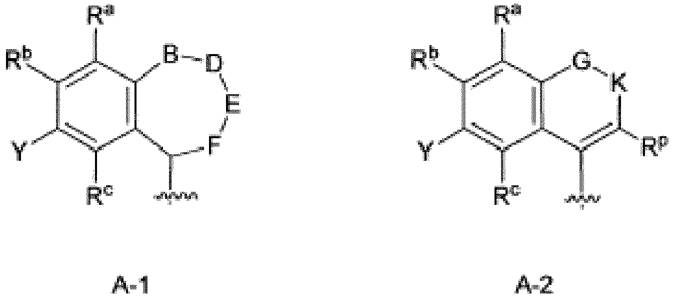
   Y is H, halogen, (1-3C alkoxy)(1-6C)alkyl, (1-6C)alkyl [optionally substituted with 1-5 fluoros], cyano(1-6C)alkyl, hydroxy(1-6C)alkyl, dihydroxy(2-6C)alkyl, aminocarbonyl(1-6C)alkyl, (1-6C)alkoxy [optionally substituted with 1-5 fluoros], CN, aminocarbonyl or (1-4C alkoxy)carbonyl;
   R^{a}, R^{b} and R^{c} are independently selected from H, halogen, (1-3C)alkyl, (1-3C)alkoxy and CN;
   B is NR¹, O, a bond, CR^{d}R^{e}, S or SO₂;
   D is NR¹, O, a bond, CR^{f}R⁸, S or SO₂;
   E is NR¹, O, a bond, or CR^{h}R\ S or SO₂;
   F is CR^{j}R^{k};
   provided that the ring formed by B, D, E, and F together with the atoms to which they are attached contains at least five atoms and zero or one of B, D or E is NR¹ or O;
   G is CR^{m}Rⁿ;
   K is NR¹; R¹ is (1-6C)alkyl [optionally substituted with one to five fluoros], (1-6C)cycloalkyl [optionally substituted with one to five fluoros], (1-3C alkoxy)(2-6C)alkyl [optionally substituted with one to five fluoros], (1-6C)alkylC(=O)- or (1-6C alkoxy)C=O-;
   R^{d}, R^{e}, R^{f}, R^{g}, R^{h}, R\ R^{j} and R^{k} are independently H, OH, (1-6C)alkyl [optionally substituted with one to five fluoros], (3-6C)cycloalkyl [optionally substituted with one to five fluoros], (1-3C alkoxy)(2-6C)alkyl [optionally substituted with one to five fluoros], hydroxy(2- 6C)alkyl [optionally substituted with one to five fluoros], (2-6C)cyanoalkyl, (1-6C)alkoxy [optionally substituted with one to five fluoros], or (1-3C alkoxy)(2-6C)alkoxy [optionally substituted with one to five fluoros], or one of a pair of R^{d} and R^{e}, or R^{f} and R⁸, or R^{h} and R¹, or R* and R^{k}, together with the carbon atom to which they are attached form a (3-6C)cycloalkyl, oxetanyl or azetidinyl ring, or one of a pair of R^{d} and R^{e}, or R^{f} and R⁸, or R^{h} and R¹, or R^{j} and R^{k} form an oxo group, and wherein only one of R^{d} and R^{e} can be OH and neither is OH if B is connected to a heteroatom, and only one of R^{f} and R⁸ can be OH and neither is OH if D is connected to a heteroatom, and only one of R^{h} and R' can be OH and neither is OH if E is connected to a heteroatom, and only one of R^{j} and R^{k} can be OH and neither is OH if F is connected to a heteroatom;
   R^{m} is H, (1-3C)alkyl [optionally substituted with 1-5 fluoros], cyclopropyl or cyclobutyl, and
   R" is H or (1-3C)alkyl [optionally substituted with 1-5 fluoros], or
   R^{m} and Rⁿ together form an oxo group;
   R^{p} is H, (1-6C)alkyl [optionally substituted with one to five fluoros], (3-6C)cycloalkyl [optionally substituted with one to five fluoros], (1-3C alkoxy)(2-6C)alkyl [optionally substituted with one to five fluoros], hydroxy(2-6C)alkyl [optionally substituted with one to five fluoros], or (2-6C)cyanoalkyl;
Ring C is formula C-1 or C-2 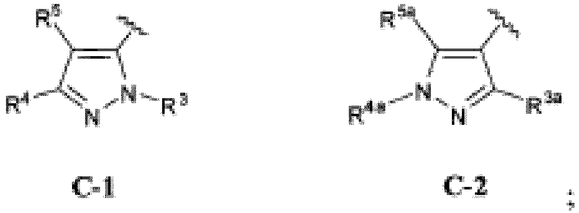
   R³ is (1-6C)alkyl, hydroxy(1-6C)alkyl, Ar², hetCyc¹, (3-7C)cycloalkyl, or hetAr²;
   Ar² is phenyl optionally substituted with one or more groups independently selected from halogen and (1-6C)alkyl; hetCyc¹ is a 5-6-membered saturated or partially unsaturated heterocyclic ring having 1- 2 ring heteroatoms independently selected from N and O;
   hetAr² is a 5-6 membered heteroaryl ring having 1-3 ring heteroatoms independently selected from N, O and S and optionally substituted with one or more groups independently selected from (1-6C)alkyl and halogen;
   R⁴ is OH, (1-6C)alkyl, monofluoro(1-6C)alkyl, difluoro(1-6C)alkyl, trifluoro(1-6C)alkyl, tetrafluro(2-6C)alkyl, pentafluro(2-6C)alkyl, cyano(1-6C)alkyl, hydroxy(1-6C)alkyl, dihydroxy(2-6C)alkyl, (1-3C alkoxy)(1-6C)alkyl, amino(1-6C)alkyl, aminocarbonyl(1-6C)alkyl, (1-3C)alkylsulfonamido(1-6C)alkyl, sulfamido(1-6C)alkyl, hydroxycarbonyl(1 -6C)alkyl, hetAr³(1-6C)alkyl, Ar³(1-6C)alkyl, (1-6C)alkoxy, monofluoro(1-6C)alkoxy, difluoro(1-6C)alkoxy, trifluoro(1-6C)alkoxy, tetrafluoro(2-6C)alkoxy, pentafluoro(2-6C)alkoxy, cyano(1-6C)alkoxy, hydroxy(1-6C)alkoxy, dihydroxy(2-6C)alkoxy, amino(2-6C)alkoxy, hydroxyl- carbonyl(1-6C)alkoxy, hetCyc²(1-6C)alkoxy, hetAr³(1-6C)alkoxy, Ar³(1-6C)alkoxy, (1-4C alkoxy)(1-6C)alkoxy, (1-3C alkylsulfonyl)(1-6C)alkoxy, (3-6C)cycloalkyl [optionally substituted with F, OH, (1-6C alkyl), (1-6C) alkoxy, or (1-3C alkoxy)(1-6C)alkyl], hetAr⁴, hetAr⁴-O-, Ar⁴, hetCyc²(O)CH₂-, (1-4C alkoxycarbonyl)(1-6C)alkoxy, hydroxycarbonyl(1-6C)alkoxy, aminocarbonyl(1-6C)alkoxy, hetCyC²C(=O)(1-6C)alkoxy, hydroxy(1-3C alkoxy)(1-6C)alkoxy, hydroxytrifluoro(1-6C)alkoxy, (1-3C)alkylsulfonamido(1-6C)alkoxy, (1-3C)alkylamido(1-6C)alkoxy, di(1-3C alkyl)amino-carboxy, hetCyc²C(=O)0-, hydroxydifluoro(1-6C)alkyl, (1-4C alkylcarboxy)(1-6C)alkyl, (1-6C)alkoxycarbonyl, hydroxylcarbonyl, aminocarbonyl, (1-3C alkoxy)aminocarbonyl, hetCyc³, halogen, CN, trifluoromethylsulfonyl, N-(1-3C alkyl)oxadiazolonyl, hetAr⁵ or hetCyc⁴-0-;
   hetCyc is a 4-6 membered heterocyclic ring having 1-2 ring heteroatoms independently selected from N and O and optionally substituted with 1-2 groups independently selected from (1-6C)alkyl, (1-4C alkylcarboxy)(1-6C)alkyl, and (1-6C)acyl;
   hetCyc³ is a 4-7 membered heterocycle having 1-2 ring heteroatoms independently selected from N and O and optionally substituted with one or more substituents independently selected from F, CN, (1-6C)alkyl, trifluoro(1-6C)alkyl, hydroxy(1-6C)alkyl, (1-3C alkoxy)(1-6C)alkyl, (1-6C)acyl-, (1-6C)alkylsulfonyl, trifluoromethylsulfonyl and (1-4C alkoxy)carbonyl; hetAr³ is a 5 -membered heteroaryl ring having 1-3 ring atoms independently selected from N, O and S and optionally substituted with (1-6C)alkyl;
   Ar³ is phenyl optionally substituted with (1-4C)alkoxy;
   hetAr⁴ is a 5-6 membered heteroaryl ring having 1-3 ring heteroatoms independently selected from N, S and O and optionally substituted with one or more substituents independently selected from (1-6C)alkyl, halogen, CN, hydroxy(1-6C)alkyl, trifluoro(1-6C)alkyl, difluoro(1-6C)alkyl, fluoro(1-6C)alkyl, (3-6C)cycloalkyl, (3-6C cycloalkyl)CH₂-(3-6C cycloalkyl)C(=O)-, (1-3C alkoxy)(1-6C)alkyl, (1-6C)alkoxy, (1-6C)alkylsulfonyl, NH₂, (1-6C alkyl)amino, di(1-6C alkyl)amino, (1-3C trifluoroalkoxy), fluoro(1-6C alkyl)amino, difluoro(1-6C alkyl)amino, trifluoro(1-6C alkyl)amino, and (3-4C cycloalkyl)amino;
hetAr⁵ is a group selected from the structures: 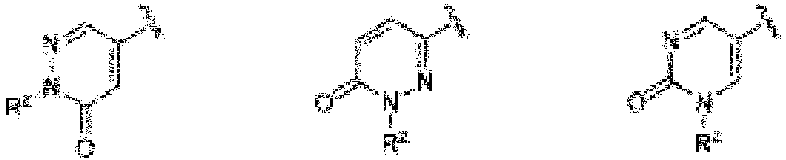 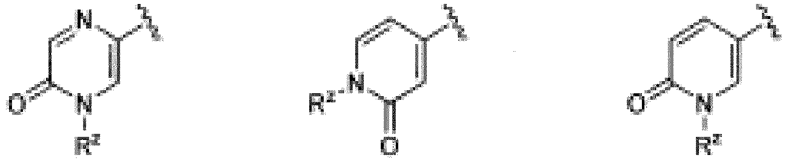
   where R^{z} is (3-4C)cycloalkyl or (1-3C)alkyl (optionally substituted with 1-3 fluoros), wherein each of said hetAr⁵ groups is optionally further substituted with one or more groups independently selected from F and (1-3C)alkyl optionally substituted with 1-3 fluoros;
   hetCyc⁴ is a 7-8 membered bridged heterocycle having a ring nitrogen atom and optionally substituted with one or more groups independently selected from (1-6C)alkyl and halogen;
   Ar⁴ is phenyl optionally substituted with one or more groups independently selected from (1-6C)alkyl, halogen, CN, CF₃, CF₃O-, (1-6C)alkoxy, (1-6Calkyl)OC(=O)-, aminocarbonyl, (1-6C)alkylthio, hydroxy(1-6C)alkyl, (1-6C alkyl)SO₂-, HOC(=O)- and (1-3C alkoxy)(1-3C alkyl)OC(=O)-;
   R⁵ is (1-6C)alkyl, monofluoro(1-6C)alkyl, difluoro(1-6C)alkyl, trifluoro(1-6C)alkyl, tetrafluoro(2-6C)alkyl, pentafluoro(2-6C)alkyl, halogen, CN, (1-4C)alkoxy, hydroxy(1-4C)alkyl, (1-3C alkoxy)(1-4C)alkyl, (1-4C alkyl)OC(=O)-, (1-6C)alkylthio, (3-4C)cycloalkyl, amino, aminocarbonyl, trifluoro(1-3C alkyl)amido, or phenyl (optionally substituted with one or more groups independently selected from halogen, (1-6C)alkyl and (1-6C)alkoxy); or R⁴ and R⁵ together with the atoms to which they are attached form a 5-6 membered saturated, partially unsaturated or unsaturated carbocyclic ring optionally substituted with one or more substituents independently selected from (1-6C)alkyl, or R⁴ and R⁵ together with the atoms to which they are attached form 5-6 membered saturated, partially unsaturated or unsaturated heterocyclic ring having a ring heteroatom selected from N, O or S, wherein said heterocyclic ring is optionally substituted with one or two substituents independently selected from (1-6C alkyl)C(=O)O-, (1-6C)acyl, (1-6C)alkyl and oxo, and said sulfur ring atom is optionally oxidized to S(=O) or SO₂;
   R^{3a} is halogen, (1-6C)alkyl, trifluoro(1-6C)alkyl, (3-6C)cycloalkyl, phenyl optionally substituted with one or more substituents independently selected from halogen and (1-6C)alkyl, or a 5-6 membered heteroaryl ring having 1-3 ring heteroatoms independently selected from N, O and S and optionally substituted with one or more groups independently selected from (1- 6C)alkyl and halogen;
   R^{4a} is hydrogen, (1-6C)alkyl, trifluoro(1-6C)alkyl, phenyl [optionally substituted with one or more groups independently selected from (1-6C)alkyl, halogen, CN, CF₃, CF₃0-, (1-6C)alkoxy, (1-6Calkyl)OC(=O)-, aminocarbonyl, (1-6C)alkylthio, hydroxy(1-6C)alkyl, (1-6C alkyl)SO₂-, HOC(=O)- and (1-3C alkoxy)(1-3C alkyl)OC(=O)-], or a 5-6 membered heteroaryl ring having 1-3 ring heteroatoms independently selected from N, S and O and optionally substituted with 1-2 substituents independently selected from (1-6C)alkyl, hydroxy(1-6C)alkyl, trifluoro(1-6C)alkyl, (3-6C)cycloalkyl, (3-6C cycloalkyl)CH₂- (3-6C cycloalkyl)C(=O)-, (1-3C alkoxy)(1-6C)alkyl, (1-6C)alkoxy, (1-6C)alkylsulfonyl, NH₂, (1-6C alkyl)amino, di(1-6C alkyl)amino and (1-3C trifluoroalkoxy)(1-3C)trifluoroalkyl; and
   R^{5a} is halogen, (1-6C)alkyl, trifluoro(1-6C)alkyl, (3-6C)cycloalkyl, phenyl optionally substituted with one or more substituents independently selected from halogen and (1-6C)alkyl, or a 5-6 membered heteroaryl ring having 1-3 ring heteroatoms independently selected from N, O and S and optionally substituted with one or more groups independently selected from (1- 6C)alkyl and halogen.

Further examples of Trk inhibitors can be found in International Publication No. WO 2014078417, which is incorporated by reference in its entirety herein. For example, a Trk inhibitor can be a compound of Formula I: 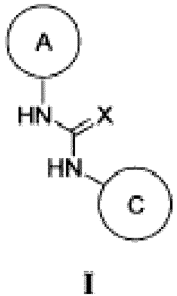 or stereoisomers, tautomers, or pharmaceutically acceptable salts, solvates or prodrugs thereof, wherein:
X is O, S, NH or N-CN;
Ring A is 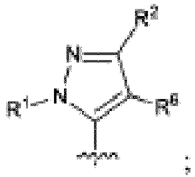
   R¹ is phenyl optionally substituted with one or more substituents independently selected from halogen and (1-3C)alkyl;
   R² is (1-3C)alkyl [optionally substituted with 1 to 5 fluoros] or (3-4C)cycloalkyl [optionally substituted with one or two fluoros];
   R⁶ is H or CH₃;
Ring C is formula C-1 or C-2 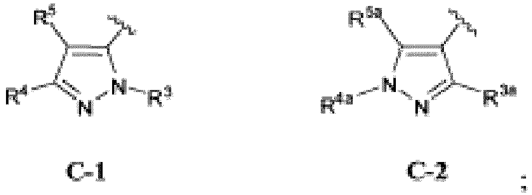
R³ is (1-6C)alkyl, hydroxy(1-6C)alkyl, Ar², hetCyc¹, (3-7C)cycloalkyl, or hetAr²;
Ar² is phenyl optionally substituted with one or more substituents independently selected from halogen and (1-6C)alkyl;
hetCyc¹ is a 5-6-membered saturated or partially unsaturated heterocyclic ring having 1-2 ring heteroatoms independently selected from N and O;
hetAr² is a 5-6 membered heteroaryl ring having 1-3 ring heteroatoms independently selected from N, O and S and optionally substituted with one or more substituents independently selected from (1-6C)alkyl and halogen; R⁴ is hetAr⁴, hetAr⁵ or hydroxy(1-6C)alkoxy;
hetAr⁴ is a 5-6 membered heteroaryl ring having 1-3 ring heteroatoms independently selected from N, S and O and substituted with one or more substituents independently selected from (1-6C)alkyl, halogen, CN, hydroxy(1-6C)alkyl, trifluoro(1-6C)alkyl, difluoro(1-6C)alkyl, fluoro(1-6C)alkyl, (3-6C)cycloalkyl, (3-6C cycloalkyl)CH₂- (3-6C cycloalkyl)C(=O)-, (1-3C alkoxy)(1-6C)alkyl, (1-6C)alkoxy, (1-6C)alkylsulfonyl, NH₂, (1- 6C alkyl)amino, di(1-6C alkyl)amino, (1-3C trifluoroalkoxy), fluoro(1-6C alkyl)amino, difluoro(1-6C alkyl)amino, trifluoro(1-6C alkyl)amino, and (3-4C cycloalkyl)amino;
hetAr⁵ is a group selected from the structures: 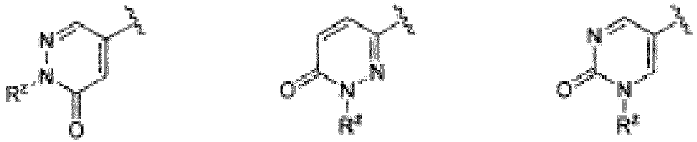 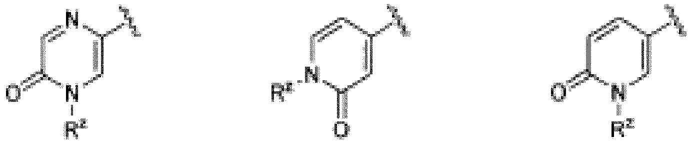
   where R^{z} is (3-4C)cycloalkyl or (1-3C)alkyl (optionally substituted with 1-3 fluoros), wherein each of said hetAr⁵ groups is optionally further substituted with one or more substituents independently selected from F and (1-3C)alkyl optionally substituted with 1-3 fluoros;
   R⁵ is (1-6C)alkyl, monofiuoro(1-6C)alkyl, difluoro(1-6C)alkyl, trifluoro(1-6C)alkyl, tetrafluoro(2-6C)alkyl, pentafluoro(2-6C)alkyl, halogen, CN, (1-4C)alkoxy, hydroxy(1-4C)alkyl, (1-3C alkoxy)(1-4C)alkyl, (1-4C alkyl)OC(=O)-, (1-6C)alkylthio, (3- 4C)cycloalkyl, amino, aminocarbonyl, trifluoro(1-3C alkyl)amido, or phenyl (optionally substituted with one or more substituents independently selected from halogen, (1-6C)alkyl and (1-6C)alkoxy); or
   R⁴ and R⁵ together with the atoms to which they are attached form a 5-6 membered saturated, partially unsaturated or unsaturated carbocyclic ring optionally substituted with one or more substituents independently selected from (1-6C)alkyl, or
   R⁴ and R⁵ together with the atoms to which they are attached form 5-6 membered saturated, partially unsaturated or unsaturated heterocyclic ring having a ring heteroatom selected from N, O or S, wherein said heterocyclic ring is optionally substituted with one or two substituents independently selected from (1-6C alkyl)C(=O)O-, (1-6C)acyl, (1-6C)alkyl and oxo, and said sulfur ring atom is optionally oxidized to S(=O) or SO₂; R is hydrogen, halogen, (1-6C)alkyl, trifluoro(1-6C)alkyl, (3-6C)cycloalkyl, phenyl optionally substituted with one or more substituents independently selected from halogen and (1-6C)alkyl, or a 5-6 membered heteroaryl ring having 1-3 ring heteroatoms independently selected from N, O and S and optionally substituted with one or more substituents independently selected from (1-6C)alkyl and halogen;
   R^{4a} is hydrogen, (1-6C)alkyl, trifluoro(1-6C)alkyl, phenyl [optionally substituted with one or more substituents independently selected from (1-6C)alkyl, halogen, CN, CF₃, CF3O-, (1-6C)alkoxy, (1-6Calkyl)OC(=O)-, aminocarbonyl, (1-6C)a!kylthio, hydroxy(1-6C)alkyl, (1-6C alkyl)SO₂-, HOC(=O)- and (1-3C alkoxy)(1-3C alkyl)OC(=O)-], or a 5-6 membered heteroaryl ring having 1-3 ring heteroatoms independently selected from N, S and O and optionally substituted with 1-2 substituents independently selected from (1-6C)alkyl, hydroxy(1-6C)alkyl, trifluoro(1-6C)alkyl, (3-6C)cycloalkyl, (3-6C cycloalkyl)CH₂- (3-6C cycloalkyl)C(=O)-, (1-3C alkoxy)(1-6C)alkyl, (1-6C)alkoxy, (1-6C)alkylsulfonyl, NH₂, (1- 6C alkyl)amino, di(1-6C alkyl)amino and (1-3C trifluoroalkoxy)(1-3C)trifluoroalkyl; and
   R^{5a} is hydrogen, halogen, (1-6C)alkyl, trifluoro(1-6C)alkyl, (3-6C)cycloalkyl, phenyl optionally substituted with one or more substituents independently selected from halogen and (1-6C)alkyl, or a 5-6 membered heteroaryl ring having 1-3 ring heteroatoms independently selected from N, O and S and optionally substituted with one or more substituents independently selected from (1-6C)alkyl and halogen.

Additional examples of Trk inhibitors can be found in International Publication No. WO 2014078408, which is incorporated by reference in its entirety herein. For example, a Trk inhibitor can be a compound of Formula I: 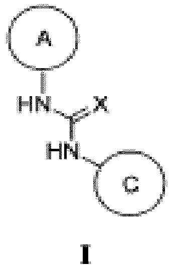 or stereoisomers, tautomers, or pharmaceutically acceptable salts, solvates or prodrugs thereof, wherein:
X is O, S, NH or -N-CN;
Ring A is formula A-1 A-2, A-3 or A-4 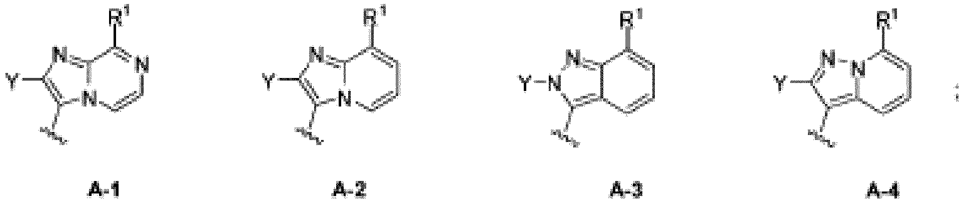
   R¹ is H, halogen, (1-3C)alkyl [optionally substituted with 1-5 fluoros], (1- 3C)alkoxy [optionally substituted with 1-5 fluoros], or (3-5C)cycloalkyl;
   Y is Ar¹ or hetAr¹;
   Ar¹ is phenyl optionally substituted with one or more substituents independently selected from halogen, (1-3C)alkyl [optionally substituted with 1-5 fluoros], and (1-3C)alkoxy [optionally substituted with 1-5 fluoros];
   hetAr¹ is pyridyl optionally substituted with one or more substituents independently selected from halogen, (1-3C)alkyl [optionally substituted with 1-5 fluoros], and (1-3C)alkoxy [optionally substituted with 1-5 fluoros];
Ring C is formula C-1 or C-2 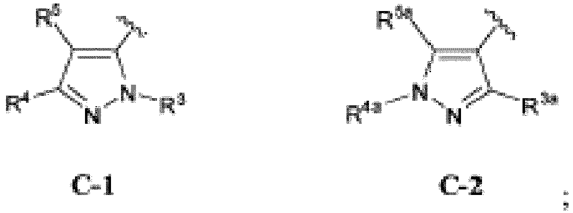
   R³ is (1-6C)alkyl, hydroxy(1-6C)alkyl, Ar², hetCyc¹, (3-7C)cycloalkyl, or hetAr²; Ar is phenyl optionally substituted with one or more substituents independently selected from halogen and (1-6C)alkyl; hetCyc¹ is a 5-6-membered saturated or partially unsaturated heterocyclic ring having 1-2 ring heteroatoms independently selected from N and O;
   hetAr² is a 5-6 membered heteroaryl ring having 1-3 ring heteroatoms independently selected from N, O and S and optionally substituted with one or more substituents independently selected from (1-6C)alkyl and halogen;
   R⁴ is OH, (1-6C)alkyl, monofluoro(1-6C)alkyl, difluoro(1-6C)alkyl, trifluoro(1-6C)alkyl, tetrafluro(2-6C)alkyl, pentafluro(2-6C)alkyl, cyano(1-6C)alkyl, hydroxy(1-6C)alkyl, dihydroxy(2-6C)alkyl, (1-3C alkoxy)(1-6C)alkyl, amino(1-6C)alkyl, aminocarbonyl(1-6C)alkyl, (1-3C)alkylsulfonamido(1-6C)alkyl, sulfamido(1-6C)alkyl, hydroxycarbonyl(1-6C)alkyl, hetAr³(1-6C)alkyl, Ar³(1-6C)alkyl, (1-6C)alkoxy, monofluoro(1-6C)alkoxy, difluoro(1-6C)alkoxy, trifluoro(1-6C)alkoxy, tetrafluoro(2- 6C)alkoxy, pentafluoro(2-6C)alkoxy, cyano(1-6C)alkoxy, hydroxy(1-6C)alkoxy, dihydroxy(2-6C)alkoxy, amino(2-6C)alkoxy, hydroxyl-carbonyl(1-6C)alkoxy, hetCyc²(1-6C)alkoxy, hetAr³(1-6C)alkoxy, Ar³(1-6C)alkoxy, (1-4C alkoxy)(1-6C)alkoxy, (1-3C alkylsulfonyl)(16C)alkoxy, (3-6C)cycloalkyl [optionally substituted with F, OH, (1-6C alkyl), (1-6C) alkoxy, or (1-3C alkoxy)(1-6C)alkyl], hetAr⁴, hetAr⁴-O-, Ar⁴, hetCyc²(O)CH₂-, (1-4C alkoxycarbonyl)(1-6C)alkoxy, hydroxycarbonyl(1-6C)alkoxy, aminocarbonyl(1-6C)alkoxy, hetCyc²C(=O)(1-6C)alkoxy, hydroxy(1-3C alkoxy)(1-6C)alkoxy, hydroxytrifluoro(1-6C)alkoxy, (1-3C)alkylsulfonamido(l-6C)alkoxy, (1-3C)alkylamido(1-6C)alkoxy, di(1-3C alkyl)amino-carboxy, hetCyc²C(=O)0-, hydroxydifluoro(1-6C)alkyl, (1-4C alkylcarboxy)(1-6C)alkyl, (1-6C)alkoxycarbonyl, hydroxylcarbonyl, aminocarbonyl, (1-3C alkoxy)aminocarbonyl, hetCyc³, halogen, CN, trifluoromethylsulfonyl, N-(1-3C alkyl)oxadiazolonyl, or hetAr⁵;
   hetCyc is a 4-6 membered heterocyclic ring having 1-2 ring heteroatoms independently selected from N and O and optionally substituted with 1-2 groups independently selected from (1-6C)alkyl, (1-4C alkylcarboxy)(1-6C)alkyl, and (1- 6C)acyl;
   hetCyc is a 4-7 membered heterocycle having 1-2 ring heteroatoms independently selected from N and O and optionally substituted with one or more substituents independently selected from F, CN, (1-6C)alkyl, trifluoro(1-6C)alkyl, hydroxy(1-6C)alkyl, (1-3C alkoxy)(1-6C)alkyl, (1-6C)acyl-, (1-6C)alkylsulfonyl, trifluoromethylsulfonyl and (1-4C alkoxy)carbonyl;
   hetAr³ is a 5-membered heteroaryl ring having 1-3 ring atoms independently selected from N, O and S and optionally substituted with (1-6C)alkyl; AT³ is phenyl optionally substituted with (1-4C)alkoxy;
   hetAr⁴ is a 5-6 membered heteroaryl ring having 1-3 ring heteroatoms independently selected from N, S and O and optionally substituted with one or more substituents independently selected from (1-6C)alkyl, halogen, CN, hydroxy(1-6C)alkyl, trifluoro(1-6C)alkyl, difluoro(1-6C)alkyl, fluoro(1-6C)alkyl, (3-6C)cycloalkyl, (3-6C cycloalkyl)CH₂- (3-6C cycloalkyl)C(=O)-, (1-3C alkoxy)(1-6C)alkyl, (1-6C)alkoxy, (1- 6C)alkylsulfonyl, NH₂, (1-6C alkyl)amino, di(1-6C alkyl)amino, (1-3C trifluoroalkoxy), fluoro(1-6C alkyl)amino, difluoro(1-6C alkyl)amino, trifluoro(1-6C alkyl)amino, and (3- 4C cycloalkyl)amino;
   hetAr⁵ is a group selected from the structures: 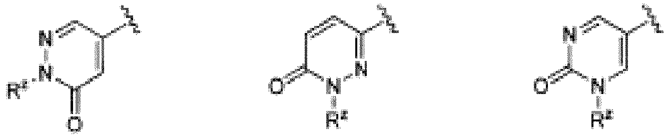 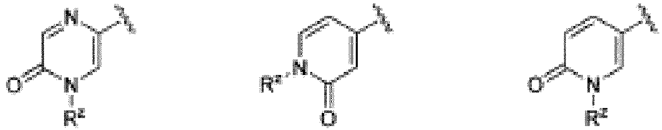
      where R^{z} is (3-4C)cycloalkyl or (1-3C)alkyl (optionally substituted with 1-3 fluoros), wherein each of said hetAr⁵ groups is optionally further substituted with one or more substituents independently selected from F and (1-3C)alkyl optionally substituted with 1-3 fluoros;
      Ar⁴ is phenyl optionally substituted with one or more substituents independently selected from (1-6C)alkyl, halogen, CN, CF₃, CF₃0-, (1-6C)alkoxy, (1-6Calkyl)OC(=O)-, aminocarbonyl, (1-6C)alkylthio, hydroxy(1-6C)alkyl, (1-6C alkyl)SO₂-, HOC(=O)- and (1-3C alkoxy)(1-3C alkyl)OC(=O)-;
      R⁵ is (1-6C)alkyl, monofluoro(1-6C)alkyl, difluoro(1-6C)alkyl, trifluoro(1-6C)alkyl, tetrafluoro(2-6C)alkyl, pentafluoro(2-6C)alkyl, halogen, CN, (1-4C)alkoxy, hydroxy(1-4C)alkyl, (1-3C alkoxy)(1-4C)alkyl, (1-4C alkyl)OC(=O)-, (1-6C)alkylthio, (3-4C)cycloalkyl, amino, aminocarbonyl, trifluoro(1-3C alkyl)amido, or phenyl (optionally substituted with one or more substituents independently selected from halogen, (1-6C)alkyl and (1-6C)alkoxy); or
      R⁴ and R⁵ together with the atoms to which they are attached form a 5-6 membered saturated, partially unsaturated or unsaturated carbocyclic ring optionally substituted with one or more substituents independently selected from (1-6C)alkyl, or R⁴ and R⁵ together with the atoms to which they are attached form 5-6 membered saturated, partially unsaturated or unsaturated heterocyclic ring having a ring heteroatom selected from N, O or S, wherein said heterocyclic ring is optionally substituted with one or two substituents independently selected from (1-6C alkyl)C(=O)O-, (1-6C)acyl, (1- 6C)alkyl and oxo, and said sulfur ring atom is optionally oxidized to S(=O) or SO₂;
      R^{3a} is hydrogen, halogen, (1-6C)alkyl, trifluoro(1-6C)alkyl, (3-6C)cycloalkyl, phenyl optionally substituted with one or more substituents independently selected from halogen and (1-6C)alkyl, or a 5-6 membered heteroaryl ring having 1-3 ring heteroatoms independently selected from N, O and S and optionally substituted with one or more substituents independently selected from (1-6C)alkyl and halogen;
      R^{4a} is hydrogen, (1-6C)alkyl, trifluoro(1-6C)alkyl, phenyl [optionally substituted with one or more substituents independently selected from (1-6C)alkyl, halogen, CN, CF₃, CF₃0-, (1-6C)alkoxy, (1-6Calkyl)OC(=O)-, aminocarbonyl, (1-6C)alkylthio, hydroxy(1-6C)alkyl, (1-6C alkyl)SO₂-, HOC(=O)- and (1-3C alkoxy)(1-3C alkyl)OC(=O)-], or a 5-6 membered heteroaryl ring having 1-3 ring heteroatoms independently selected from N, S and O and optionally substituted with 1-2 substituents independently selected from (1-6C)alkyl, hydroxy(1-6C)alkyl, trifluoro(1-6C)alkyl, (3- 6C)cycloalkyl, (3-6C cycloalkyl)CH₂- (3-6C cycloalkyl)C(=O)-, (1-3C alkoxy)(1-6C)alkyl, (1-6C)alkoxy, (1-6C)alkylsulfonyl, NH₂, (1-6C alkyl)amino, di(1-6C alkyl)amino and (1-3C trifluoroalkoxy)(1-3C)trifluoroalkyl; and
      R^{5a} is hydrogen, halogen, (1-6C)alkyl, trifluoro(1-6C)alkyl, (3-6C)cycloalkyl, phenyl optionally substituted with one or more substituents independently selected from halogen and (1-6C)alkyl, or a 5-6 membered heteroaryl ring having 1-3 ring heteroatoms independently selected from N, O and S and optionally substituted with one or more substituents independently selected from (1-6C)alkyl and halogen.

Further examples of Trk inhibitors can be found in International Publication No. WO 2014078378, which is incorporated by reference in its entirety herein. For example, a Trk inhibitor can be a compound of Formula I: 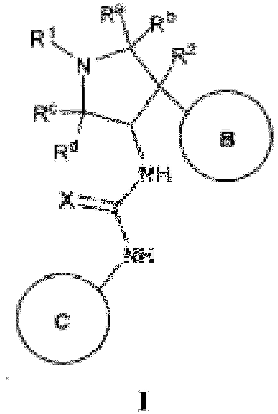 or stereoisomers, tautomers, or pharmaceutically acceptable salts, solvates or prodrugs thereof, wherein:
Ring B and the NH-C(=X)-NH moiety are in the trans configuration;
R^{a}, R^{b}, R^{c} and R^{d} are independently selected from H and (1-3C)alkyl,
or R^{c} and R^{d} are independently selected from H and (1-3C)alkyl, and R^{a} and R^{b} together with the atom to which they are attached form a cyclopropyl ring;
X is O, S, NH or N-CN;
R¹ is (1-3C alkoxy)(1-6C)alkyl, (trifluoromethoxy)(1-6C)alkyl, (1-3C sulfanyl)(1-6C)alkyl, monofluoro(1-6C)alkyl, difluoro(1-6C)alkyl, trifluoro(1-6C)alkyl, tetrafluoro(2-6C)alkyl, pentafluro(2-6C)alkyl, cyano(1-6C)alkyl, aminocarbonyl(1-6C)alkyl, hydroxy(1-6C)alkyl, dihydroxy(2-6C)alkyl, (1-6C)alkyl, (1-3Calkylamino)(1-3C)alkyl, (1-4C alkoxycarbonyl)(1-6C)alkyl, amino(1-6C)alkyl, hydroxy(1-3C alkoxy)(1-6C)alkyl, di(1-3C alkoxy)(1-6C)alkyl, (1-3C alkoxy)trifluoro(1-6C)alkyl, hydroxytrifluoro(1-6C)alkyl, (1-4C alkoxycarbonyl)(1-3C alkoxy)(1-6C)alkyl, or hydroxycarbonyl(1-3C alkoxy)(1-6C)alkyl;
R² is H, F, or OH;
Ring B is Ar¹ or hetAr¹;
Ar¹ is phenyl optionally substituted with one or more substituents independently selected from halogen, CF₃, CF₃0-, (1-4C)alkoxy, hydroxy(1-4C)alkyl, (1-6C)alkyl and CN; hetAr¹ is a 5-6 membered heteroaryl having 1-3 ring heteroatoms independently selected from N, S and O, and optionally substituted with one or more groups independently selected from (1-6C)alkyl, halogen, OH, CF₃, NH₂ and hydroxy(1-2C)alkyl;
Ring C is selected from formulas C-1 through C-13: 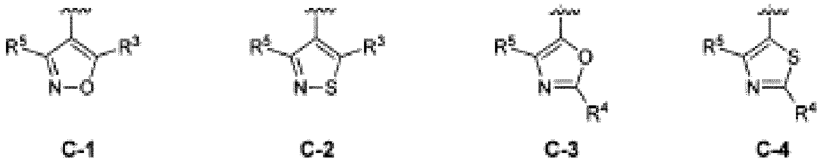 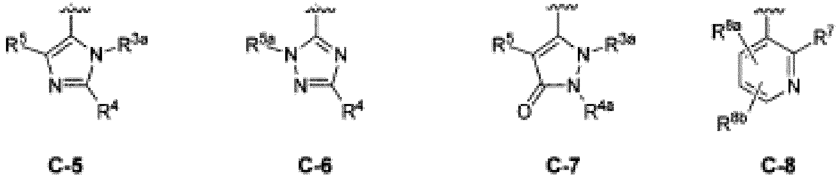 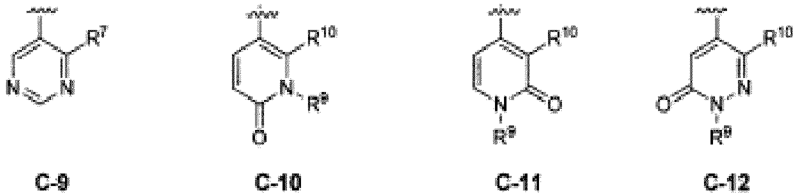 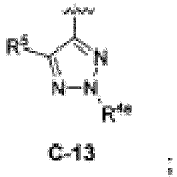
R is H, NH₂, CN, halogen, (1-3C)alkyl [optionally substituted with 1 to 3 fluoros],
H₂NC(=O)-, (1-3Calkyl)NHC(=O)-, di(1-3Calkyl)NHC(=OK hydroxy(1-3C)alkyl, CH3OCH2CH2, (3-4C)cycloalkyl or (1-3C)alkoxy;
R^{3a} is H, (1-3C)alkyl, CF3CH2CH2, HCF2CH2CH2, H2FCCH2CH2, CF3CH2, HOCH₂CH₂, MeOCH₂CH₂, or (3-4C)cycloalkyl;
R⁴ is H, OH, (1-6C)alkyl [optionally substituted with 1-5 fluoros], cyano(1-6C)alkyl, hydroxy(1-6C)alkyl, dihydroxy(2-6C)alkyl, (1-3C alkoxy)(1-6C)alkyl, amino(1-6C)alkyl, aminocarbonyl(1-6C)alkyl, (1-3C)alkylsulfonamido(1-6C)alkyl, sulfamido(1-6C)alkyl, hydroxycarbonyl(1-6C)alkyl, hetAr³(1-6C)alkyl, Ar³(1-6C)alkyl, (1-6C)alkoxy [optionally substituted with 1-5 fluoros], cyano(1-6C)alkoxy, hydroxy(1-6C)alkoxy, dihydroxy(2-6C)alkoxy, amino(2-6C)alkoxy, hydroxyl-carbonyl(1-6C)alkoxy, hetCyc (1-6C)alkoxy, hetAr³(1-6C)alkoxy, Ar³(1-6C)alkoxy, (1-4C alkoxy)(1-6C)alkoxy, (1-3C alkylsulfonyl)(1-6C)alkoxy, (3-6C)cycloalkyl [optionally substituted with F, OH, (1-6C alkyl), (1-6C) alkoxy, or (1-3C alkoxy)(1-6C)alkyl], hetAr⁴, hetAr⁴-O-, Ar⁴, hetCyc²(O)CH₂-, (1-4C alkoxycarbonyl)(1-6C)alkoxy, hydroxycarbonyl(1-6C)alkoxy, aminocarbonyl(1-6C)alkoxy, hetCyc²C(=O)(1-6C)alkoxy, hydroxy(1-3C alkoxy)(1-6C)alkoxy, hydroxytrifluoro(1-6C)alkoxy, (1-3C)alkylsulfonamido(1-6C)alkoxy, (1-3C)alkylamido(1-6C)alkoxy, di(1-3C alkyl)amino-carboxy, hetCyc C(=O)0-, hydroxydifluoro(1-6C)alkyl, (1-4C alkylcarboxy)(1- 6C)alkyl, (1-6C)alkoxycarbonyl, hydroxylcarbonyl, aminocarbonyl, (1-3C alkoxy)aminocarbonyl, hetCyc , halogen, CN, trifluoromethylsulfonyl, N-(1-3C alkyl)oxadiazolonyl, or hetAr⁵;
R^{4a} is H, (1-6C)alkyl, CF₃CH₂CH₂, HCF₂CH₂CH₂, H₂FCCH₂CH₂, CF₃CH₂,, cyano(1-6C)alkyl, hydroxy(1-6C)alkyl, dihydroxy(2-6C)alkyl, (1-3C alkoxy)(1-6C)alkyl, amino(1-6C)alkyl, aminocarbonyl(1-6C)alkyl, (1-3C)alkylsulfonamido(1-6C)alkyl, sulfamido(1-6C)alkyl, hydroxycarbonyl(1-6C)alkyl, hetAr³(1-6C)alkyl, Ar³(1-6C)alkyl, (3-6C)cycloalkyl [optionally substituted with F, OH, (1-6C alkyl), (1-6C) alkoxy, or (1-3 C alkoxy)(1-6C)alkyl], hetAr⁴, Ar⁴, hydroxydifluoro(1-6C)alkyl, (1-4C alkylcarboxy)(1-6C)alkyl, hetCyc³, N-(1-3C alkyl)oxadiazolonyl, or hetAr⁵;
hetCyc is a 4-6 membered heterocyclic ring having 1-2 ring heteroatoms independently selected from N and O and optionally substituted with 1-2 groups independently selected from (1-6C)alkyl, (1-4C alkylcarboxy)(1-6C)alkyl, and (1-6C)acyl;
hetCyc is a 4-7 membered heterocycle having 1-2 ring heteroatoms independently selected from N and O and optionally substituted with one or more substituents independently selected from F, CN, (1-6C)alkyl, trifluoro(1-6C)alkyl, hydroxy(1-6C)alkyl, (1-3C alkoxy)(1- 6C)alkyl, (1-6C)acyl-, (1-6C)alkylsulfonyl, trifluoromethylsulfonyl and (1-4C alkoxy)carbonyl;
hetAr is a 5-membered heteroaryl ring having 1-3 ring atoms independently selected from N, O and S and optionally substituted with (1-6C)alkyl;
Ar is phenyl optionally substituted with (1-4C)alkoxy;
hetAr⁴ is independently a 5-6 membered heteroaryl ring having 1-3 ring heteroatoms independently selected from N, S and O and optionally substituted with one or more substituents independently selected from (1-6C)alkyl [optionally substituted with 1-3 fluoros], halogen, CN, hydroxy(1-6C)alkyl, (3-6C)cycloalkyl, (3-6C cycloalkyl)CH₂- (3-6C cycloalkyl)C(=O)-, (1-3C alkoxy)(1-6C)alkyl, (1-6C)alkoxy, (1-6C)alkylsulfonyl, NH₂, (1-6C alkyl)amino, di(1-6C alkyl)amino, (1-3C trifluoroalkoxy), fluoro(1-6C alkyl)amino, difluoro(1-6C alkyl)amino, trifluoro(1-6C alkyl)amino, and (3-4C cycloalkyl)amino;
hetAr⁵ is a group selected from the structures: 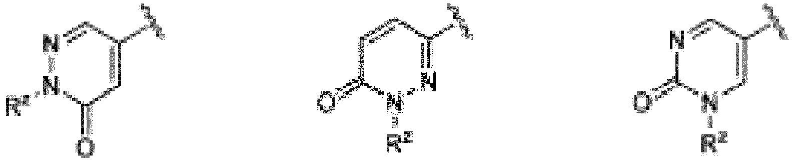 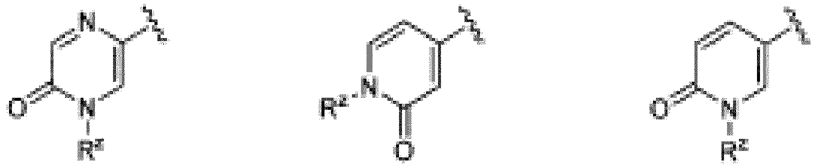
where R^{z} is (3-4C)cycloalkyl or (1-3C)alkyl (optionally substituted with 1-3 fluoros), wherein each of said hetAr⁵ groups is optionally further substituted with one or more groups independently selected from F and (1-3C)alkyl optionally substituted with 1-3 fluoros;
Ar⁴ is phenyl optionally substituted with one or more groups independently selected from (1-6C)alkyl, halogen, CN, CF₃, CF₃O-, (1-6C)alkoxy, (1-6Calkyl)OC(=O)-, aminocarbonyl, (1-6C)alkylthio, hydroxy(1-6C)alkyl, (1-6C alkyl)SO₂-, HOC(=O)- and (1-3C alkoxy)(1-3C alkyl)OC(=O)-;
R⁵ is H, (1-6C)alkyl [optionally substituted with 1-5 fluoros], halogen, CN, (1-4C)alkoxy, hydroxy(1-4C)alkyl, (1-3C alkoxy)(1-4C)alkyl, (1-4C alkyl)OC(=O)-, (1-6C)alkylthio, (3-4C)cycloalkyl, amino, aminocarbonyl, trifluoro(1-3C alkyl)amido, or phenyl [optionally substituted with one or more groups independently selected from halogen, (1-6C)alkyl and (1-6C)alkoxy];
R^{5a} is H, (1-6C)alkyl, CF₃CH₂CH₂, HCF₂CH₂CH₂, H₂FCCH₂CH₂, CF₃CH₂, hydroxy(1-4C)alkyl, (1-3C alkoxy)(1-4C)alkyl, (3-4C)cycloalkyl, or phenyl [optionally substituted with one or more groups independently selected from halogen, (1-6C)alkyl and (1-6C)alkoxy];
R is (1-6C)alkyl, (3-6C)cycloalkyl, or phenyl [optionally substituted with one or more groups independently selected from halogen, (1-6C)alkyl, (1-6C)alkoxy, (3- 4C)cycloalkyl, amino, aminocarbonyl, and trifluoro(1-3C)alkylamido];
R^{8a} and R^{8b} are independently H, halogen, CN, NH₂, (1-6C)alkyl [optionally substituted with 1-5 fluoros], (1-6C)alkoxy, (1-3C alkoxy)(1-6C)alkyl, (1-3C alkoxy)(1-6C)alkoxy, (1-6C alkyl)sulfonyl, (3-6C cycloalkyl)sulfonyl, (3-4C)cycloalkyl, amino, (1-6Calkyl)NH-, phenyl [optionally substituted with (1-6C alkyl)SO₂-] or hetAr⁴, wherein only one of R^{8a} and R^{8b} can be phenyl [optionally substituted with (1-6C alkyl)SO₂-] or hetAr⁴;
R⁹ is H, (1-6C)alkyl, CF₃CH₂-, CF₃CH₂CH₂-, (1-3Calkoxy)(1-6C)alkyl or (3-4C)cycloalkyl; and
R¹⁰ is (3-6C)cycloalkyl or phenyl [optionally substituted with one or more substituents independently selected from halogen, (1-6C)alkyl, (1-6C)alkoxy, (3-4C)cycloalkyl, amino, aminocarbonyl and trifluoro(1-3C alkyl)amido].

Additional examples of Trk inhibitors can be found in International Publication No. WO 2014078372, which is incorporated by reference in its entirety herein. For example, a Trk inhibitor can be a compound of Formula I: 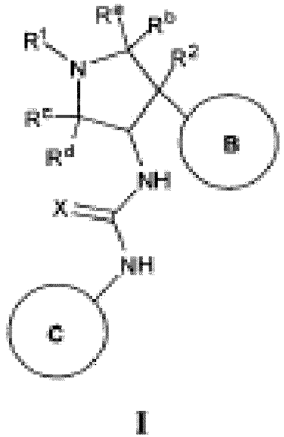 or stereoisomers, tautomers, or pharmaceutically acceptable salts, solvates or prodrugs thereof, wherein:
Ring B and the NH-C(=X)-NH moiety are in the trans configuration;
R^{a}, R^{b}, R^{o} and R^{d} are independently selected from H and (1-3C)alkyl,
or R^{c} and R^{d} are independently selected from H and (1-3C)alkyl, and R^{a} and R^{b} together with the atom to which they are attached form a cyclopropyl ring;
X is O, S, NH or N-CN;
R¹ is (1-3C alkoxy)(1-6C)alkyl, (trifluoromethoxy)(1-6C)alkyl, (1-3C sulfanyl)(1-6C)alkyl, monofiuoro(1-6C)alkyl, difluoro(1-6C)alkyl, trifluoro(1-6C)alkyl, tetrafluoro(2-6C)alkyl, pentafluro(2-6C)alkyl, cyano(1-6C)alkyl, aminocarbonyl(1-6C)alkyl, hydroxy(1-6C)alkyl, dihydroxy(2-6C)alkyl, (1-6C)alkyl, (1-3C)alkylamino(1-3C)alkyl, (1-4C) alkoxycarbonyl(1-6C)alkyl, amino(1-6C)alkyl, hydroxy(1-3C alkoxy)(1-6C)alkyl, di(1-3C alkoxy)(1-6C)alkyl, (1-3C alkoxy)trifluoro(1-6C)alkyl, hydroxytrifluoro(1-6C)alkyl, (1-4C alkoxycarbonyl)(1-3C alkoxy)(1-6C)alkyl, or hydroxycarbonyl(1-3C alkoxy)(1-6C)alkyl;
R² is H, F, or OH;
Ring B is Ar¹ or hetAr¹;
Ar¹ is phenyl optionally substituted with one or more substituents independently selected from halogen, CF₃, CF₃0-, (1-4C)alkoxy, hydroxy(1-4C)alkyl, (1-6C)alkyl and CN; hetAr¹ is a 5-6 membered heteroaryl having 1-3 ring heteroatoms independently selected from N, S and O, and optionally substituted with one or more groups independently selected from (1-6C)alkyl, halogen, OH, CF₃, NH₂ and hydroxy(1-2C)alkyl;
Ring C is selected from formulas C-1 through C-9 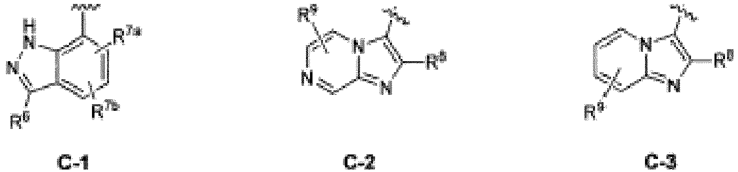 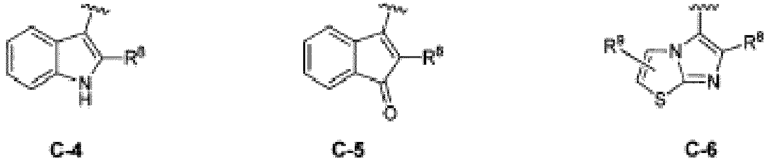 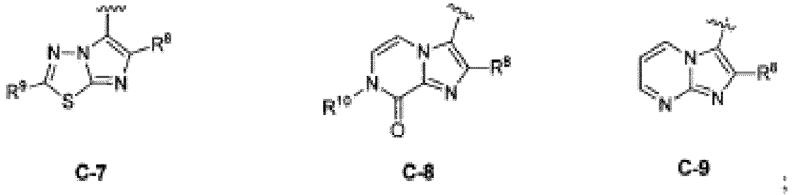
R is H, halogen, or phenyl [optionally substituted with one or more substituents independently selected from halogen and (1-3C)alkyl];
R^{7a} and R^{7b} are independently H, (1-6C)alkyl, or phenyl [optionally substituted with one or more substituents independently selected from halogen and (1-3C)alkyl], wherein only one of R^{7a} and R^{7b} can be phenyl optionally substituted with one or more substituents independently selected from halogen and (1-3C)alkyl;
R⁸ is phenyl optionally substituted with one or more substituents independently selected from halogen, (1-3C)alkyl and (3-6C)cycloalkyl;
R⁹ is H, halogen, (1-6C)alkyl [optionally substituted with one to five fluoros] or (1-6C)alkoxy; and
R¹⁰ is H or (1-6C)alkyl.

Further examples of Trk inhibitors can be found in International Publication No. WO 2014078331, which is incorporated by reference in its entirety herein. For example, a Trk inhibitor can be a compound of Formula I-C: 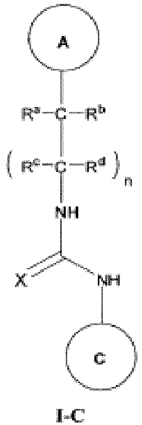 or stereoisomers, tautomers, or pharmaceutically acceptable salts, solvates or prodrugs thereof, wherein:
X is O, S, NH or N-CN;
Ring A is formula A-1 or A-2 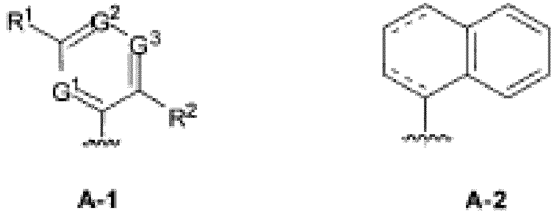 wherein the dashed lines are optional double bonds;
n is 0 or 1 when Ring A is formula A-1, and n is 0 when Ring A is formula A-2;
G¹, G² and G³ are independently CR^{X} or N, wherein no more than 2 of G¹, G² and G³ can be N;
each R^{x} is independently H, halogen, (1-4C)alkyl or (1-4C)alkoxy;
R¹ is H, halogen, (1-3C)alkoxy(1-3C)alkyl (optionally substituted with 1-5 fluoros), (1-3C alkyl)sulfanyl(1-3C)alkyl (optionally substituted with 1-5 fluoros), (1-3C)alkyl (optionally substituted with 1-5 fluoros), (1-3C)alkoxy (optionally substituted with 1-5 fluoros), (1-3C alkyl)sulfanyl (optionally substituted with 1-5 fluoros), cyano(1-3C)alkyl (optionally substituted with 1-5 fluoros), hydroxy(1-3C)alkyl (optionally substituted with 1-5 fluoros), (1-4C)alkyl (optionally substituted with 1-5 fluoros), CH₃CH₂NR^{y}, CF₃CH₂NR^{y}, HCF₂CH₂NR^{y}, H₂CFCH₂NR^{y}, CH₃NR^{y}CH₂, R^{y}R^{y}NCH₂CH₂, R^{y}R^{y}NCH₂CFH, or
R^{y}R^{y}NCH₂CF₂;
each R^{y} is independently H or methyl;
when n is 0, R is selected from the group consisting of H, halogen, (1-6C)alkyl [optionally substituted with 1-5 fluoros], (1-6C)alkoxy [optionally substituted with 1-5 fluoros], (1-3C alkoxy)(1-4C)alkyl, (3-6C cycloalkyl)CH₂0-, amino(1-3C)alkyl,
CF₃CH₂NHCH₂, HCF₂CH₂NHCH₂, a C5-C8 bridged cycloalkyl, hetCyc³, hetCyc^{a}CH₂, Cyc^{a}, hetAr¹ and Ar¹, and
when n is 1, R is selected from the group consisting of H, halogen, CF₃, F₂CH, FCH₂, methyl and methoxy.
hetCyc³ is a 4-6 membered heterocyclic ring having a ring heteroatom selected from N, O and S and optionally substituted with 1-3 groups independently selected from OH, F, (1-6C)alkoxy or (1-6C)alkyl [optionally substituted with 1-3 fluoros];
Cyc^{a} is a (3-6C)cycloalkyl optionally substituted with (1-4C)alkoxy, (1-4C)alkyl, F or OH;
hetAr¹ is a 5-6 membered heteroaryl having 1-3 ring heteroatoms independently selected from N, S and O, and optionally substituted with 1 -2 groups independently selected from (1-6C)alkyl, halogen, OH, CF₃, NH₂ and hydroxy(1-2C)alkyl;
Ar¹ is phenyl optionally substituted with one or more substituents independently selected from halogen, CF₃, CF₃0-, (1-4C)alkoxy, (1-4C)sulfanyl, hydroxy(1-4C)alkyl, (1-6C)alkyl and CN;
R^{a} is H, (1-3C)alkyl, cyclopropyl, cyclobutyl, or CF₃, and
R^{b} is H, methyl or ethyl,
or R^{a} and R^{b} together with the carbon atom to which they are attached form a 3-6 membered cycloalkyl ring;
R^{c} is H, methyl or ethyl
R^{d} is CF₃CH₂CH₂, phenyl or phenylCH₂- wherein each phenyl ring is optionally substituted with one or more substituents independently selected from halogen, methoxy and methoxymethyl;
Ring C is formula C-1 or C-2 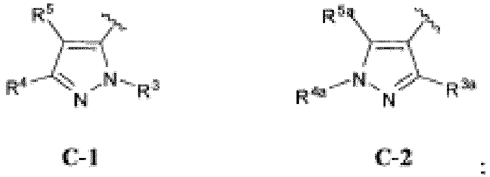
R³ is (1-6C)alkyl, hydroxy(1-6C)alkyl, Ar², hetCyc¹, (3-7C)cycloalkyl, a C5-C8 bridged cycloalkyl, or hetAr²;
Ar² is phenyl optionally substituted with one or more groups independently selected from halogen and (1-6C)alkyl;
hetCyc¹ is a 5-6-membered saturated or partially unsaturated heterocyclic ring having 1-2 ring heteroatoms independently selected from N and O;
hetAr is a 5-6 membered heteroaryl ring having 1-3 ring heteroatoms independently selected from N, O and S and optionally substituted with one or more groups independently selected from (1-6C)alkyl and halogen;
R⁴ is OH, (1-6C)alkyl, monofluoro(1-6C)alkyl, difluoro(1-6C)alkyl, trifluoro(1-6C)alkyl, tetrafluro(2-6C)alkyl, pentafluro(2-6C)alkyl, cyano(1-6C)alkyl, hydroxy(1-6C)alkyl, dihydroxy(2-6C)alkyl, (1-3C alkoxy)(1-6C)alkyl, amino(1-6C)alkyl, aminocarbonyl(l -6C)alkyl, (1 -3C)alkylsulfonamido(1 -6C)alkyl, sulfamido(l -6C)alkyl, hydroxycarbonyl(1-6C)alkyl, hetAr³(1-6C)alkyl, Ar³(1-6C)alkyl, (1-6C)alkoxy, monofluoro(1-6C)alkoxy, difluoro(1-6C)alkoxy, trifluoro(1-6C)alkoxy, tetrafluoro(2- 6C)alkoxy, pentafluoro(2-6C)alkoxy, cyano(I-6C)alkoxy, hydroxy(1-6C)alkoxy, dihydroxy(2-6C)alkoxy, amino(2-6C)alkoxy, hydroxyl-carbonyl(1-6C)alkoxy, hetCyc²(1- 6C)alkoxy, hetAr³(1-6C)alkoxy, Ar³(1-6C)alkoxy, (1-4C alkoxy)(1-6C)alkoxy, (1-3C alkylsulfonyl)(1-6C)alkoxy, (3-6C)cycloalkyl [optionally substituted with F, OH, (1-6C alkyl), (1-6C) alkoxy, or (1-3C alkoxy)(1-6C)alkyl], hetAr⁴, hetAr⁴-0-, Ar⁴, hetCyc²(O)CH₂-, (1-4C alkoxycarbonyl)(1-6C)alkoxy, hydroxycarbonyl(1-6C)alkoxy, aminocarbonyl(1- 6C)alkoxy, hetCyc²C(=O)(1-6C)alkoxy, hydroxy(1-3C alkoxy)(1-6C)alkoxy, hydroxytrifluoro(1-6C)alkoxy, (1-3C)alkylsulfonamido(1-6C)alkoxy, (1-3C)alkylamido(1-6C)alkoxy, di(1-3C alkyl)amino-carboxy, hetCyc²C(=O)0-, hydroxydifluoro(1-6C)alkyl, (1-4C alkylcarboxy)(1-6C)alkyl, (1-6C)alkoxycarbonyl, hydroxylcarbonyl, aminocarbonyl, (1-3C alkoxy)aminocarbonyl, hetCyc³, halogen, CN, trifluoromethylsulfonyl, N-(1-3C alkyl)oxadiazolonyl, hetAr⁵, Ar⁴-0-, hetCyc⁴-0-, Cyc'-O-, or aminohydroxy(1-6C)alkoxy; hetCyc² is a 4-6 membered heterocyclic ring having 1 -2 ring heteroatoms independently selected from N and O and optionally substituted with 1-2 groups independently selected from (1-6C)alkyl, 1-4C alkoxy)carbonyl, (1-6C)acyl, halogen and oxo;
hetCyc is a 4-7 membered heterocycle having 1 -2 ring heteroatoms independently selected from N and O and optionally substituted with one or more substituents
independently selected from F, CN, (1-6C)alkyl, trifluoro(1-6C)alkyl, hydroxy(1-6C)alkyl, (1-3C alkoxy)(1-6C)alkyl, (1-6C)acyl-, (1-6C)alkylsulfonyl, trifluoromethylsulfonyl and (1- 4C alkoxy)carbonyl;
hetCyc⁴ is a 5-8 membered monocyclic, spirocyclic or bridged heterocycle having a ring nitrogen atom and optionally substituted with one or more groups independently selected from (1-6C)alkyl and halogen;
Cyc¹ is a 3-6 membered carbocycle optionally substituted with an amino group; hetAr³ is a 5-membered heteroaryl ring having 1-3 ring atoms independently selected from N, O and S and optionally substituted with (1-6C)alkyl;
Ar is phenyl optionally substituted with (1-4C)alkoxy;
hetAr⁴ is a 5-6 membered heteroaryl ring having 1-3 ring heteroatoms independently selected from N, S and O and optionally substituted with one or more substituents independently selected from (1-6C)alkyl, halogen, CN, hydroxy(1-6C)alkyl, trifluoro(1-6C)alkyl, difluoro(1-6C)alkyl, fluoro(1-6C)alkyl, (3-6C)cycloalkyl, (3-6C cycloalkyl)CH₂- (3-6C cycloalkyl)C(=O)-, (1-3C alkoxy)(1-6C)alkyl, (1-6C)alkoxy, (1-6C)alkylsulfonyl, NH₂, (1-6C alkyl)amino, di(1-6C alkyl)amino, (1-3C trifluoroalkoxy), fluoro(1-6C alkyl)amino, difluoro(1-6C alkyl)amino, trifluoro(1-6C alkyl)amino, and (3-4C cycloalkyl)amino;
hetAr⁵ is a group selected from the structures: 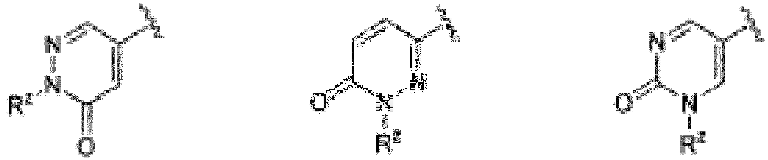 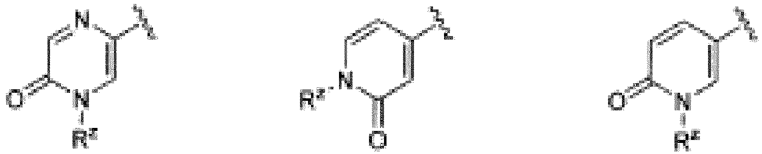
   where R^{z} is (3-4C)cycloalkyl or (1-3C)alkyl (optionally substituted with 1-3 fluoros), wherein each of said hetAr⁵ groups is optionally further substituted with one or more groups independently selected from F and (1-3C)alkyl optionally substituted with 1-3 fluoros;
   Ar⁴ is phenyl optionally substituted with one or more groups independently selected from (1-6C)alkyl, halogen, CN, CF₃, CF₃0-, (1-6C)alkoxy, (1-6Calkyl)OC(=O)-, aminocarbonyl, (1-6C)alkylthio, hydroxy(1-6C)alkyl, (1-6C alkyl)SO₂-, HOC(=O)- and (1- 3C alkoxy)(1-3C alkyl)OC(=O)-;
   R⁵ is (1-6C)alkyl, monofluoro(1-6C)alkyl, difluoro(1-6C)alkyl, trifluoro(1-6C)alkyl, tetrafluoro(2-6C)alkyl, pentafluoro(2-6C)alkyl, halogen, CN, (1-4C)alkoxy, hydroxy(1-4C)alkyl, (1-3C alkoxy)(1-4C)alkyl, (1-4C alkyl)OC(=O)-, (1-6C)alkylthio, (3- 4C)cycloalkyl, amino, aminocarbonyl, trifluoro(1-3C alkyl)amido, or phenyl (optionally substituted with one or more groups independently selected from halogen, (1-6C)alkyl and (1-6C)alkoxy); or
   R⁴ and R⁵ together with the atoms to which they are attached form a 5-6 membered saturated, partially unsaturated or unsaturated carbocyclic ring optionally substituted with one or more substituents independently selected from (1-6C)alkyl, or
   R⁴ and R⁵ together with the atoms to which they are attached form 5-6 membered saturated, partially unsaturated or unsaturated heterocyclic ring having a ring heteroatom selected from N, O or S, wherein said heterocyclic ring is optionally substituted with one or two substituents independently selected from (1-6C alkyl)C(=O)0-, (1-6C)acyl, (1-6C)alkyl and oxo, and said sulfur ring atom is optionally oxidized to S(=O) or SO₂;
   R^{3a} is halogen, (1-6C)alkyl, trifluoro(1-6C)alkyl, (3-6C)cycloalkyl, phenyl optionally substituted with one or more substituents independently selected from halogen and (1-6C)alkyl, or a 5-6 membered heteroaryl ring having 1-3 ring heteroatoms independently selected from N, O and S and optionally substituted with one or more groups independently selected from (1-6C)alkyl and halogen;
   R^{4a} is hydrogen, (1-6C)alkyl, trifluoro(1-6C)alkyl, phenyl [optionally substituted with one or more groups independently selected from (1-6C)alkyl, halogen, CN, CF₃, CF₃0-, (1-6C)alkoxy, (1-6Calkyl)OC(=O)-, aminocarbonyl, (1-6C)alkylthio, hydroxy(1-6C)alkyl, (1-6C alkyl)SO₂-, HOC(=O)- and (1-3C alkoxy)(1-3C alkyl)OC(=O)-], or a 5-6 membered heteroaryl ring having 1-3 ring heteroatoms independently selected from N, S and O and optionally substituted with 1-2 substituents independently selected from (1-6C)alkyl, hydroxy(1-6C)alkyl, trifluoro(1-6C)alkyl, (3-6C)cycloalkyl, (3-6C cycloalkyl)CH₂- (3-6C cycloalkyl)C(=O)-, (1-3C alkoxy)(1-6C)alkyl, (1-6C)alkoxy, (1-6C)alkylsulfonyl, NH₂, (1- 6C alkyl)amino, di(1-6C alkyl)amino and (1-3C trifluoroalkoxy)(1-3C)trifluoroalkyl; and
   R^{5a} is halogen, (1-6C)alkyl, trifluoro(1-6C)alkyl, (3-6C)cycloalkyl, phenyl optionally substituted with one or more substituents independently selected from halogen and (1-6C)alkyl, or a 5-6 membered heteroaryl ring having 1-3 ring heteroatoms independently selected from N, O and S and optionally substituted with one or more groups independently selected from (1-6C)alkyl and halogen.

Additional examples of Trk inhibitors can be found in International Publication No. WO 2014078328, which is incorporated by reference in its entirety herein. For example, a Trk inhibitor can be a compound of Formula I-1: 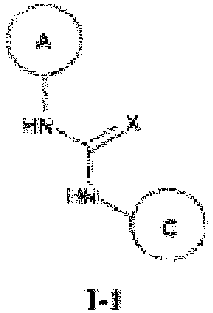 or stereoisomers, tautomers, or pharmaceutically acceptable salts, solvates or prodrugs thereof, wherein:
Ring A is selected from formulas A-1, A-2, A-3 or A-4: 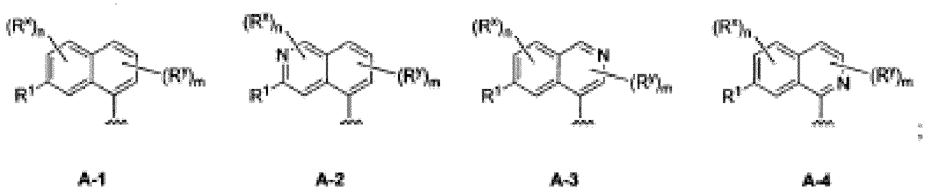
   R¹ is H, halogen, (1-3C)alkoxy(1-3C)alkyl [optionally substituted with 1-5 fluoros], (1-3C alkyl)sulfanyl(1-3C)alkyl [optionally substituted with 1-5 fluoros], (1-3C)alkoxy [optionally substituted with 1-5 fluoros], (1-3C alkyl)sulfanyl [optionally substituted with 1-5 fluoros], cyano(1-3C)alkyl [optionally substituted with 1-5 fluoros], hydroxy(1-3C)alkyl [optionally substituted with 1-5 fluoros], (1-4C)alkyl [optionally substituted with 1-5 fluoros], CH₃CH₂NR^{a}, CF₃CH₂NR^{a}, HCF₂CH₂NR^{a}, H₂CFCH₂NR^{a}, CH₃NR^{a}CH₂, R^^CHzCHs or R^^CHzCFz;
   each R^{a} is independently H or methyl;
   R^{x} and R^{y} are independently selected from H, halogen, (1-3C)alkyl [optionally substituted with 1-5 fluoros] or (1-3C)alkoxy [optionally substituted with 1-5 fluoros];
   n is 0, 1 or 2;
   m is 0, 1 or 2;
   X is O, S, NH or N-CN;
Ring C is formula C-1 or C-2 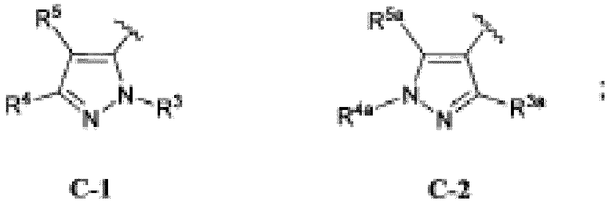
   R³ is (1-6C)alkyl, hydroxy(1-6C)alkyl, Ar², hetCyc¹, (3-7C)cycloalkyl, or hetAr²;
   Ar is phenyl optionally substituted with one or more groups independently selected from halogen and (1-6C)alkyl;
   hetCyc¹ is a 5-6-membered saturated or partially unsaturated heterocyclic ring having 1-2 ring heteroatoms independently selected from N and O;
   hetAr is a 5-6 membered heteroaryl ring having 1-3 ring heteroatoms independently selected from N, O and S and optionally substituted with one or more groups independently selected from (1-6C)alkyl and halogen;
   R⁴ is OH, (1-6C)alkyl, monofluoro(1-6C)alkyl, difluoro(1-6C)alkyl, trifluoro(1-6C)alkyl, tetrafluro(2-6C)alkyl, pentafluro(2-6C)alkyl, cyano(1-6C)alkyl, hydroxy(1-6C)alkyl, dihydroxy(2-6C)alkyl, (1-3C alkoxy)(1-6C)alkyl, amino(1-6C)alkyl, aminocarbonyl(1 -6C)alkyl, (1-3C)alkylsulfonamido(1-6C)alkyl, sulfamido(1-6C)alkyl, hydroxycarbonyl(1-6C)alkyl, hetAr³(1-6C)alkyl, Ar³(1-6C)alkyl, (1-6C)alkoxy, monofluoro(1-6C)alkoxy, difluoro(1-6C)alkoxy trifluoro(1-6C)alkoxy, tetrafluoro(2- 6C)alkoxy, pentafluoro(2-6C)alkoxy, cyano(1-6C)alkoxy, hydroxy(1-6C)alkoxy, dihydroxy(2-6C)alkoxy, amino(2-6C)alkoxy, hydroxyl-carbonyl(1-6C)alkoxy, hetCyc²(1- 6C)alkoxy, hetAr³(1-6C)alkoxy, Ar³(1-6C)alkoxy, (1-4C alkoxy)(1-6C)alkoxy, (1-3C alkylsulfonyl)(1-6C)alkoxy, (3-6C)cycloalkyl [optionally substituted with F, OH, (1-6C alkyl), (1-6C) alkoxy, or (1-3C alkoxy)(1-6C)alkyl], hetAr⁴, hetAr⁴-0-, Ar⁴, hetCyc²(O)CH₂-, (1-4C alkoxycarbonyl)(1-6C)alkoxy, hydroxycarbonyl(I-6C)alkoxy, aminocarbonyl(1- 6C)alkoxy, hetCyc²C(=O)(1-6C)alkoxy, hydroxy(1-3C alkoxy)(1-6C)alkoxy, hydroxytrifluoro(1-6C)alkoxy, (1-3C)alkylsulfonamido(1-6C)alkoxy, (1-3C)alkylamido(1-6C)alkoxy, di(1-3C alkyl)amino-carboxy, hetCyc²C(=O)0-, hydroxydifluoro(1-6C)alkyl, (1- 4C alkylcarboxy)(1-6C)alkyl, (1-6C)alkoxycarbonyl, hydroxylcarbonyl, aminocarbonyl, (1- 3C alkoxy)aminocarbonyl, hetCyc³, halogen, CN, trifluoromethylsulfonyl, N-(1-3C alkyl)oxadiazolonyl, or hetAr⁵;
   hetCyc is a 4-6 membered heterocyclic ring having 1-2 ring heteroatoms independently selected from N and O and optionally substituted with 1-2 groups independently selected from (1-6C)alkyl, (1-4C alkylcarboxy)(1-6C)alkyl, and (1-6C)acyl; hetCyc is a 4-7 membered heterocycle having 1-2 ring heteroatoms independently selected from N and O and optionally substituted with one or more substituents independently selected from F, CN, (1-6C)alkyl, trifluoro(1-6C)alkyl, hydroxy(1-6C)aikyl, (1-3C alkoxy)(1-6C)alkyl, (1-6C)acyl-, (1-6C)alkylsulfonyl, trifluoromethylsulfonyl and (1- 4C alkoxy)carbonyl; hetAr³ is a 5-membered heteroaryl ring having 1-3 ring atoms independently selected from N, O and S and optionally substituted with (1-6C)alkyl;
   Ar³ is phenyl optionally substituted with (1-4C)alkoxy;
   hetAr⁴ is a 5-6 membered heteroaryl ring having 1-3 ring heteroatoms independently selected from N, S and O and optionally substituted with one or more substituents independently selected from (1-6C)alkyl, halogen, CN, hydroxy(1-6C)alkyl, trifluoro(1-6C)alkyl, difluoro(1-6C)alkyl, fluoro(1-6C)alkyl, (3-6C)cycloalkyl, (3-6C cycloalkyl)CH₂- (3-6C cycloalkyl)C(=O)-, (1-3C alkoxy)(1-6C)alkyl, (1-6C)alkoxy, (1-6C)alkylsulfonyl, NH₂, (1-6C alkyl)amino, di(1-6C alkyl)amino, (1-3C trifluoroalkoxy), fluoro(1-6C alkyl)amino, difluoro(1-6C alkyl)amino, trifluoro(1-6C alkyl)amino, and (3-4C cycloalkyl)amino;
   hetAr⁵ is a group selected from the structures: 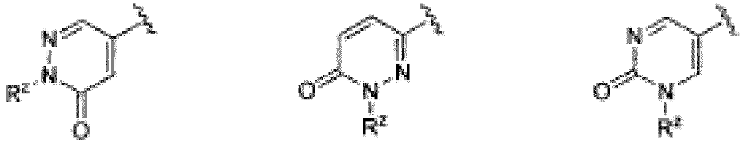 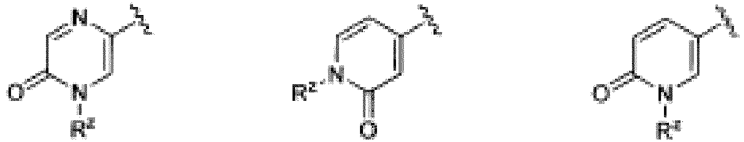
   where R^{z} is (3-4C)cycloalkyl or (1-3C)alkyl (optionally substituted with 1-3 fluoros), wherein each of said hetAr⁵ groups is optionally further substituted with one or more groups independently selected from F and (1-3C)alkyl optionally substituted with 1-3 fluoros;
   Ar⁴ is phenyl optionally substituted with one or more groups independently selected from (1-6C)alkyl, halogen, CN, CF₃, CF₃0-, (1-6C)alkoxy, (1-6Calkyl)OC(=O)-, aminocarbonyl, (1-6C)alkylthio, hydroxy(1-6C)alkyl, (1-6C alkyl)SO₂-, HOC(=O)- and (1- 3C alkoxy)(1-3C alkyl)OC(=O)-;
   R⁵ is (1-6C)alkyl, monofluoro(1-6C)alkyl, difluoro(1-6C)alkyl, trifluoro(1-6C)alkyl, tetrafluoro(2-6C)alkyl, pentafluoro(2-6C)alkyl, halogen, CN, (1-4C)alkoxy, hydroxy(1-4C)alkyl, (1-3C alkoxy)(1-4C)alkyl, (1-4C alkyl)OC(=O)-, (1-6C)alkylthio, (3- 4C)cycloalkyl, amino, aminocarbonyl, trifluoro(1-3C alkyl)amido, or phenyl (optionally substituted with one or more groups independently selected from halogen, (1-6C)alkyl and (1-6C)alkoxy); or
   R⁴ and R⁵ together with the atoms to which they are attached form a 5-6 membered saturated, partially unsaturated or unsaturated carbocyclic ring optionally substituted with one or more substituents independently selected from (1-6C)alkyl, or R⁴ and R⁵ together with the atoms to which they are attached form 5-6 membered saturated, partially unsaturated or unsaturated heterocyclic ring having a ring heteroatom selected from N, O or S, wherein said heterocyclic ring is optionally substituted with one or two substituents independently selected from (1-6C alkyl)C(-0)0-, (1-6C)acyl, (1-6C)alkyl and oxo, and said sulfur ring atom is optionally oxidized to S(=O) or SO₂;
   R^{3a} is halogen, (1-6C)alkyl, trifluoro(1-6C)alkyl, (3-6C)cycloalkyl, phenyl optionally substituted with one or more substituents independently selected from halogen and (1-6C)alkyl, or a 5-6 membered heteroaryl ring having 1-3 ring heteroatoms independently selected from N, O and S and optionally substituted with one or more groups independently selected from (1-6C)alkyl and halogen;
   R^{4a} is (1-6C)alkyl, trifluoro(1-6C)alkyl, phenyl [optionally substituted with one or more groups independently selected from (1-6C)alkyl, halogen, CN, CF₃, CF₃O-, (1-6C)alkoxy, (1-6Calkyl)OC(=O)-, aminocarbonyl, (1-6C)alkylthio, hydroxy(1-6C)alkyl, (1-6C alkyl)SO₂-, HOC(=O)- and (1-3C alkoxy)(1-3C alkyl)OC(=O)-], or a 5-6 membered heteroaryl ring having 1-3 ring heteroatoms independently selected from N, S and O and optionally substituted with 1-2 substituents independently selected from (1-6C)alkyl, hydroxy(1-6C)alkyl, trifluoro(1-6C)alkyl, (3-6C)cycloalkyl, (3-6C cycloalkyl)CH₂- (3-6C cycloalkyl)C(=O)-, (1-3C alkoxy)(1-6C)alkyl, (1-6C)alkoxy, (1-6C)alkylsulfonyl, NH₂, (1- 6C alkyl)amino, di(1-6C alkyl)amino and (1-3C trifluoroalkoxy)(1-3C)trifluoroalkyl; and
   R^{5a} is halogen, (1-6C)alkyl, trifluoro(1-6C)alkyl, (3-6C)cycloalkyl, phenyl optionally substituted with one or more substituents independently selected from halogen and (1-6C)alkyl, or a 5-6 membered heteroaryl ring having 1-3 ring heteroatoms independently selected from N, O and S and optionally substituted with one or more groups independently selected from (1-6C)alkyl and halogen.

Further examples of Trk inhibitors can be found in International Publication No. WO 2014078325, which is incorporated by reference in its entirety herein. For example, a Trk inhibitor can be a compound of Formula I: 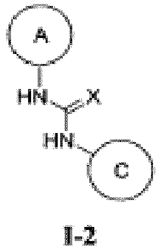 or a stereoisomer, tautomer, or pharmaceutically acceptable salt, solvate or prodrug thereof, wherein:
Ring A is formula A-1, A-2, A-3, A-4, A-5 or A-6 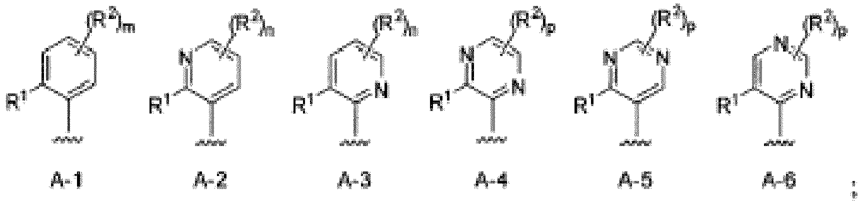
m is 0, 1, 2, 3 or 4;
n is 0, 1, 2 or 3;
p is 0, 1 or 2 ;
R¹ is formula R¹-1, R¹-2 or R¹-3 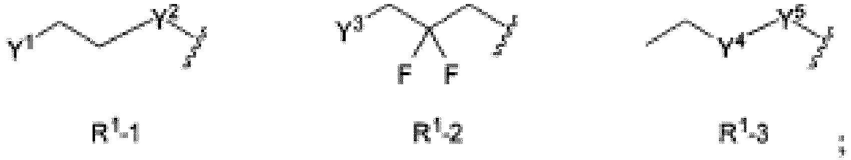
Y¹ is CH₃CH₂-, CF₃CH₂-, CH₃0-, F₃CO-, F₂CHO-, FCH₂0-, CH₃S-, F₃CS-, F₂CHS-, or FCH₂S-;
Y² is O, S, NH, MeN- or CH₂;
Y³ is CH3O-, CH3S-, MeNH- or Me₂N-;
Y⁴ is CH₂ and Y⁵ is S or O, or Y⁴ is S or O and Y⁵ is CH₂;
R² is halogen, (1-3C)alkyl (optionally substituted with 1-3 fluoros), (1-3C)alkoxy (optionally substituted with 1-3 fluoros), CH₃OCH₂- (optionally substituted with 1-3 fluoros), (1-3C alkyl)sulfanyl, di(1-3C)alkylamino, cyclopropyl, cyclobutyl or azetidinyl, wherein each of said cyclopropyl, cyclobutyl and azetidinyl is optionally substituted with 1 to 2 fluoros;
X is O, S, NH or N-CN;
Ring C is formula C-1 or C-2 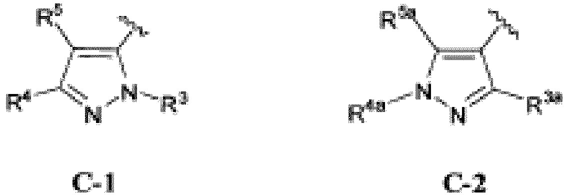
R³ is (1-6C)alkyl, hydroxy(1-6C)alkyl, Ar², hetCyc¹, (3-7C)cycloalkyl, or hetAr²;
Ar² is phenyl optionally substituted with one or more groups independently selected from halogen and (1-6C)alkyl;
hetCyc¹ is a 5-6-membered saturated or partially unsaturated heterocyclic ring having 1-2 ring heteroatoms independently selected from N and O;
hetAr² is a 5-6 membered heteroaryl ring having 1-3 ring heteroatoms independently selected from N, O and S and optionally substituted with one or more groups independently selected from (1-6C)alkyl and halogen;
R⁴ is H, OH, (1-6C)alkyl, monofluoro(1-6C)alkyl, difluoro(1-6C)alkyl, trifluoro(1-6C)alkyl, tetrafluro(2-6C)alkyl, pentafluro(2-6C)alkyl, cyano(1-6C)alkyl, hydroxy(1-6C)alkyl, dihydroxy(2-6C)alkyl, (1-3C alkoxy)(1-6C)alkyl, amino(1-6C)alkyl, aminocarbonyl(1-6C)alkyl, (1 -3C)alkylsulfonamido(1 -6C)alkyl, sulfamido(1 -6C)alkyl, hydroxycarbonyl(1-6C)alkyl, hetAr³(1-6C)alkyl, Ar³(1-6C)alkyl, (1-6C)alkoxy, monofluoro(1-6C)alkoxy, difluoro(1-6C)alkoxy trifluoro(1-6C)alkoxy, tetrafluoro(2- 6C)alkoxy, pentafluoro(2-6C)alkoxy cyano(I-6C)alkoxy, hydroxy(1-6C)alkoxy, dihydroxy(2-6C)alkoxy, amino(2-6C)alkoxy, hydroxyl-carbonyl(1-6C)alkoxy, hetCyc²(1 - 6C)alkoxy, hetAr³(1-6C)alkoxy, Ar³(1-6C)alkoxy, (1-4C alkoxy)(1-6C)alkoxy, (1-3C alkylsulfonyl)(1-6C)alkoxy, (3-6C)cycloalkyl [optionally substituted with F, OH, (1-6C alkyl), (1-6C) alkoxy, or (1-3C alkoxy)(1-6C)alkyl], hetAr⁴, hetAr⁴-0-, Ar⁴, hetCyc²(O)CH₂-, (1-4C alkoxycarbonyl)(1-6C)alkoxy, hydroxycarbonyl(I-6C)alkoxy, aminocarbonyl(1-6C)alkoxy, hetCyc²C(=O)(1-6C)alkoxy, hydroxy(1-3C alkoxy)(1- 6C)alkoxy, hydroxytrifluoro(1-6C)alkoxy, (1-3C)alkylsulfonamido(1-6C)alkoxy, (1- 3C)alkylamido(1-6C)alkoxy, di(1-3C alkyl)amino-carboxy, hetCyc C(=O)0-, hydroxydifluoro(1-6C)alkyl, (1-4C alkylcarboxy)(1-6C)alkyl, (1-6C)alkoxycarbonyl, hydroxylcarbonyl, aminocarbonyl, (1-3C alkoxy)aminocarbonyl, hetCyc, halogen, CN, trifluoromethylsulfonyl, N-(1-3C alkyl)oxadiazolonyl, or hetAr⁵;
hetCyc² is a 4-6 membered heterocyclic ring having 1-2 ring heteroatoms independently selected from N and O and optionally substituted with 1-2 groups independently selected from (1-6C)alkyl, (1-4C alkylcarboxy)(1-6C)alkyl, (1-6C)acyl and halogen;
hetCyc³ is a 4-7 membered heterocycle having 1-2 ring heteroatoms independently selected from N and O and optionally substituted with one or more substituents independently selected from F, CN, (1-6C)alkyl, trifluoro(1-6C)alkyl, hydroxy(1-6C)alkyl, (1-3C alkoxy)(1-6C)alkyl, (1-6C)acyl-, (1-6C)alkylsulfonyl, trifluoromethylsulfonyl and (1-4C alkoxy)carbonyl;
hetAr³ is a 5 -membered heteroaryl ring having 1-3 ring atoms independently selected from N, O and S and optionally substituted with (1-6C)alkyl;
Ar is phenyl optionally substituted with (1-4C)alkoxy;
hetAr⁴ is a 5-6 membered heteroaryl ring having 1-3 ring heteroatoms independently selected from N, S and O and optionally substituted with one or more substituents independently selected from (1-6C)alkyl, halogen, CN, hydroxy(1-6C)alkyl, trifluoro(1-6C)alkyl, difluoro(1-6C)alkyl, fluoro(1-6C)alkyl, (3-6C)cycloalkyl, (3-6C cycloalkyl)CH₂- (3-6C cycloalkyl)C(=O)-, (1-3C alkoxy)(1-6C)alkyl, (1-6C)alkoxy, (1- 6C)alkylsulfonyl, NH₂, (1-6C alkyl)amino, di(1-6C alkyl)amino, (1-3C trifluoroalkoxy), fluoro(1-6C alkyl)amino, difluoro(1-6C alkyl)amino, trifluoro(1-6C alkyl)amino, and (3- 4C cycloalkyl)amino;
hetAr⁵ is a group selected from the structures: 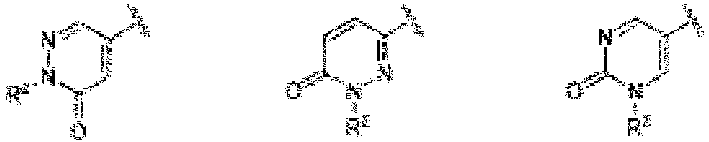 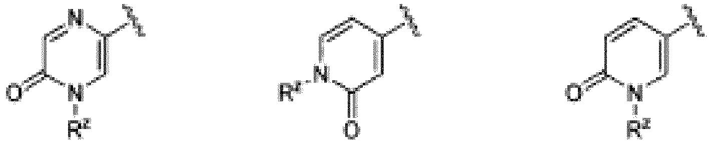
where R^{z} is (3-4C)cycloalkyl or (1-3C)alkyl (optionally substituted with 1-3 fluoros), wherein each of said hetAr⁵ groups is optionally further substituted with one or more groups independently selected from F and (1-3C)alkyl optionally substituted with 1- 3 fluoros; AT⁴ is phenyl optionally substituted with one or more groups independently selected from (1-6C)alkyl, halogen, CN, CF₃, CF₃0-, (1-6C)alkoxy, (1-6Calkyl)OC(=O)-, aminocarbonyl, (1-6C)alkylthio, hydroxy(1-6C)alkyl, (1-6C alkyl)SO₂-, HOC(=O)- and (1-3C alkoxy)(1-3C alkyl)OC(=O)-;
R⁵ is (1-6C)alkyl, monofluoro(1-6C)alkyl, difluoro(1-6C)alkyl, trifluoro(1- 6C)alkyl, tetrafluoro(2-6C)alkyl, pentafluoro(2-6C)alkyl, halogen, CN, (1-4C)alkoxy, hydroxy(1-4C)alkyl, (1-3C alkoxy)(1-4C)alkyl, (1-4C alkyl)OC(=O)-, (1-6C)alkylthio, (3-4C)cycloalkyl, amino, aminocarbonyl, trifluoro(1-3C alkyl)amido, or phenyl (optionally substituted with one or more groups independently selected from halogen, (1- 6C)alkyl and (1-6C)alkoxy); or
R⁴ and R⁵ together with the atoms to which they are attached form a 5-6 membered saturated, partially unsaturated or unsaturated carbocyclic ring optionally substituted with one or more substituents independently selected from (1-6C)alkyl, or
R⁴ and R⁵ together with the atoms to which they are attached form 5-6 membered saturated, partially unsaturated or unsaturated heterocyclic ring having a ring heteroatom selected from N, O or S, wherein said heterocyclic ring is optionally substituted with one or two substituents independently selected from (1-6C alkyl)C(=O)0-, (1-6C)acyl, (1- 6C)alkyl and oxo, and said sulfur ring atom is optionally oxidized to S(=O) or SO₂;
R^{3a} is halogen, (1-6C)alkyl, trifluoro(1-6C)alkyl, (3-6C)cycloalkyl, phenyl optionally substituted with one or more substituents independently selected from halogen and (1-6C)alkyl, or a 5-6 membered heteroaryl ring having 1-3 ring heteroatoms independently selected from N, O and S and optionally substituted with one or more groups independently selected from (1-6C)alkyl and halogen;
R^{4a} is (1-6C)alkyl, trifluoro(1-6C)alkyl, phenyl [optionally substituted with one or more groups independently selected from (1-6C)alkyl, halogen, CN, CF₃, CF₃0-, (1-6C)alkoxy, (1-6Calkyl)OC(=O)-, aminocarbonyl, (1-6C)alkylthio, hydroxy(1-6C)alkyl, (1-6C alkyl)SO₂-, HOC(=O)- and (1-3C alkoxy)(1-3C alkyl)OC(=O)-], or a 5-6 membered heteroaryl ring having 1-3 ring heteroatoms independently selected from N, S and O and optionally substituted with 1-2 substituents independently selected from (1- 6C)alkyl, hydroxy(1-6C)alkyl, trifluoro(1-6C)alkyl, (3-6C)cycloalkyl, (3-6C cycloalkyl)CH₂- (3-6C cycloalkyl)C(=O)-, (1-3C alkoxy)(1-6C)alkyl, (1-6C)alkoxy, (1- 6C)alkylsulfonyl, NH₂, (1-6C alkyl)amino, di(1-6C alkyl)amino and (1-3C trifluoroalkoxy(1-3 C)trifluoroalkyl; and R^{5a} is (1-6C)alkyl, trifluoro(1-6C)alkyl, (3-6C)cycloalkyl, phenyl optionally substituted with one or more substituents independently selected from halogen and (1-6C)alkyl, or a 5-6 membered heteroaryl ring having 1-3 ring heteroatoms independently selected from N, O and S and optionally substituted with one or more groups independently selected from (1-6C)alkyl and halogen.

Additional examples of Trk inhibitors can be found in International Publication No. WO 2014078323, which is incorporated by reference in its entirety herein. For example, a Trk inhibitor can be a compound of Formula I: 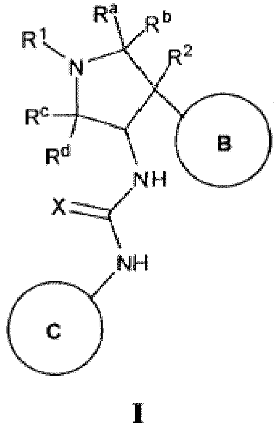 or stereoisomers, tautomers, or pharmaceutically acceptable salts, solvates or prodrugs thereof, wherein:
Ring B and the NH-C(=X)-NH moiety are in the trans configuration;
R^{a}, R^{b}, R^{c} and R^{d} are independently selected from H and (1-3C)alkyl,
or R^{o} and R^{d} are independently selected from H and (1-3C)alkyl, and R^{a} and R^{b} together with the atom to which they are attached form a cyclopropyl ring;
X is O, S, NH, or N-CN;
R¹ is (1-3C alkoxy)(1-6C)alkyl, (trifluoromethoxy)(1-6C)alkyl, (1-3C sulfanyl)(1-6C)alkyl, monofluoro(1-6C)alkyl, difluoro(1-6C)alkyl, trifluoro(1-6C)alkyl, tetrafluoro(2-6C)alkyl, pentafluro(2-6C)alkyl, cyano(1-6C)alkyl, aminocarbonyl(1-6C)alkyl, hydroxy(1-6C)alkyl, dihydroxy(2-6C)alkyl, (1-6C)alkyl, (1-3Calkylamino)(1-3C)alkyl, (1-4C alkoxycarbonyl)(1-6C)alkyl, amino(1-6C)alkyl, hydroxy(1-3C alkoxy)(1-6C)alkyl, di(1-3C alkoxy)(1-6C)alkyl, (1-3C alkoxy)trifluoro(1-6C)alkyl, hydroxytrifluoro(1-6C)alkyl, (1-4C alkoxycarbonyl)(1-3C alkoxy)(1-6C)alkyl or hydroxycarbonyl(1-3C alkoxy)(1-6C)alkyl;
R² is H, F, or OH;
Ring B is Ar¹ or hetAr¹;
Ar¹ is phenyl optionally substituted with one or more substituents independently selected from halogen, CF₃, CF₃0-, (1-4C)alkoxy, hydroxy(1-4C)alkyl, (1-6C)alkyl and CN; hetAr¹ is a 5-6 membered heteroaryl having 1-3 ring heteroatoms independently selected from N, S and O, and optionally substituted with one or more substituents independently selected from (1-6C)alkyl, halogen, OH, CF₃, NH₂ and hydroxy(1-2C)alkyl;
Ring C is 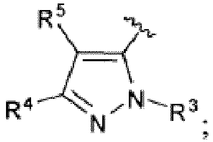
R³ is H, (1-6C)alkyl, hydroxy(1-6C)alkyl, Ar², hetCyc¹, (3-7C)cycloalkyl, hetAr², or a C5-C8 bridged carbocyclic ring;
Ar² is phenyl optionally substituted with one or more substituents independently selected from halogen and (1-6C)alkyl;
hetCyc¹ is a 5-6-membered saturated or partially unsaturated heterocyclic ring having 1-2 ring heteroatoms independently selected from N and O;
hetAr² is a 5-6 membered heteroaryl ring having 1-3 ring heteroatoms independently selected from N, O and S and optionally substituted with one or more substituents independently selected from (1-6C)alkyl and halogen;
R⁴ is selected fro -6C alkyl)SO₂-, (1-6C alkyl)C(=O)- and from the structures: 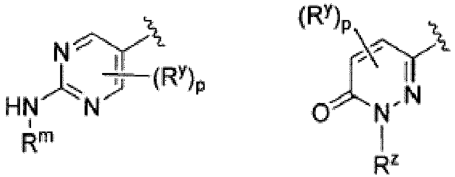 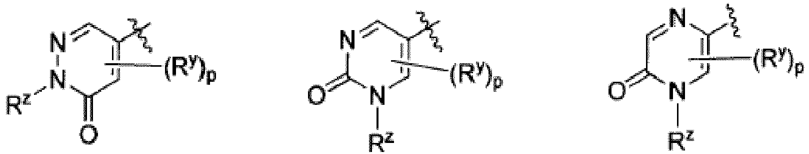 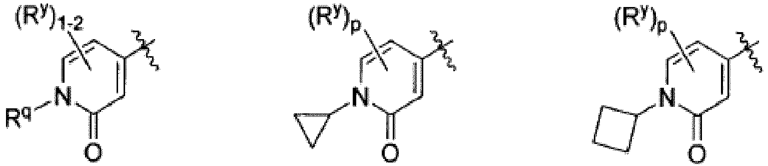 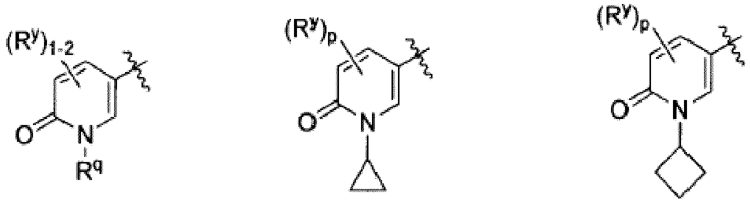 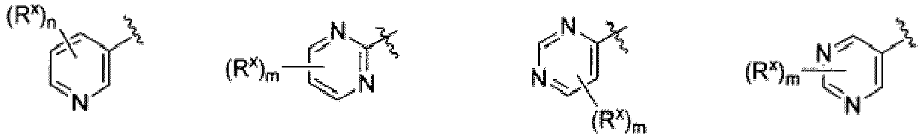 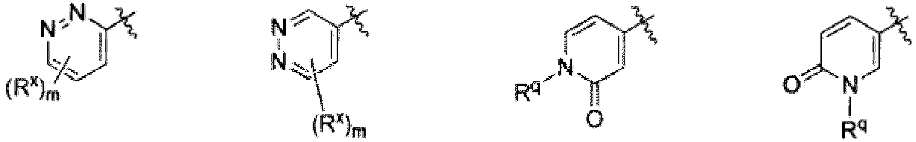 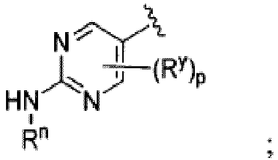
R^{m} is (1-3C)alkyl substituted with 1-3 fluoros, or (3-4C)cycloalkyl;
Rⁿ is (1-3C)alkyl;
R^{q} is (1-3C)alkyl optionally substituted with 1-3 fluoros;
R^{x} is (1-6C)alkyl, halogen, CN, hydroxy(1-6C)alkyl, trifiuoro(1-6C)alkyl, (3-6C)cycloalkyl, (3-6C cycloalkyl)CH₂-, (3-6C cycloalkyl)C(=O)-, (1-3C alkoxy)(1-6C)alkyl, (1-6C)alkoxy, (1-6C)alkylsulfonyl, NH₂, (1-6C alkyl)amino, di(1-6C alkyl)amino, trifluoro(1-3C)alkoxy or trifluoro(1-6C)alkyl;
n is 0, 1, 2, 3 or 4;
m is 0, 1, 2 or 3;
R^{y} is F or (1-3C)alkyl optionally substituted with 1-3 fluoros;
p is 0, 1 or 2;
R^{z} is (3-4C)cycloalkyl, or (1-3C)alkyl optionally substituted with 1-3 fluoros; and R⁵ is H, (1-6C)alkyl, monofluoro(1-6C)alkyl, difluoro(1-6C)alkyl, trifluoro(1- 6C)alkyl, tetrafluoro(2-6C)alkyl, pentafluoro(2-6C)alkyl, halogen, CN, (1-4C)alkoxy, hydroxy(1-4C)alkyl, (1-3C alkoxy)(1-4C)alkyl, (1-4C alkyl)OC(=O)-, (1-6C)alkylsulfanyl, phenyl [optionally substituted with one or more substituents independently selected from halogen, (1-6C)alkyl and (1-6C)alkoxy], (3-4C)cycloalkyl, amino, aminocarbonyl, or trifluoro(1-3C alkyl)amido.

Additional examples of Trk inhibitors can be found in International Publication No. WO 2014078322, which is incorporated by reference in its entirety herein. For example, a Trk inhibitor can be a compound of Formula I: 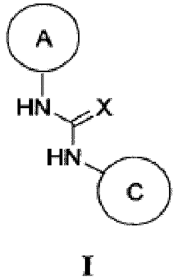 or stereoisomers, tautomers, or pharmaceutically acceptable salts, solvates or prodrugs thereof, wherein:
X is O, S, NH or N-CN;
Ring A is 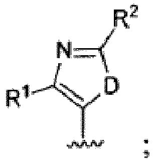
   D is O or S;
   R¹ is phenyl optionally substituted with one or more substituents independently selected from halogen and (1-3C)alkyl;
   R is (1-6C)alkyl [optionally substituted with 1 to 5 fluoros] or (3-6C)cycloalkyl [optionally substituted with one or two fluoros];
Ring C is formula C-1 or C-2 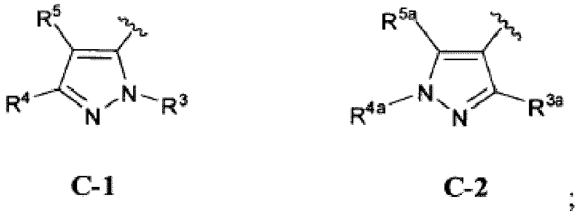
   R³ is (1-6C)alkyl, hydroxy(1-6C)alkyl, Ar², hetCyc¹, (3-7C)cycloalkyl, or hetAr²;
   Ar is phenyl optionally substituted with one or more groups independently selected from halogen and (1-6C)alkyl;
   hetCyc¹ is a 5-6-membered saturated or partially unsaturated heterocyclic ring having 1-2 ring heteroatoms independently selected from N and O; hetAr² is a 5-6 membered heteroaryl ring having 1-3 ring heteroatoms independently selected from N, O and S and optionally substituted with one or more groups independently selected from (1-6C)alkyl and halogen;
   R⁴ is H, OH, (1-6C)alkyl, monofluoro(1-6C)alkyl, difluoro(1-6C)alkyl, trifluoro(1-6C)alkyl, tetrafluro(2-6C)alkyl, pentafluro(2-6C)alkyl, cyano(1-6C)alkyl, hydroxy(1-6C)alkyl, dihydroxy(2-6C)alkyl, (1-3C alkoxy)(1-6C)alkyl, amino(1-6C)alkyl, aminocarbonyl(1-6C)alkyl, (1-3C)alkylsulfonamido(1-6C)alkyl, sulfamido(1-6C)alkyl, hydroxycarbonyl(1-6C)alkyl, hetAr³(1-6C)alkyl, Ar³(1-6C)alkyl, (1-6C)alkoxy, monofluoro(1-6C)alkoxy, difluoro(1-6C)alkoxy, trifluoro(1-6C)alkoxy, tetrafluoro(2- 6C)alkoxy, pentafluoro(2-6C)alkoxy, cyano(I-6C)alkoxy, hydroxy(1-6C)alkoxy, dihydroxy(2-6C)alkoxy, amino(2-6C)alkoxy, hydroxyl-carbonyl(1-6C)alkoxy, hetCyc²(1- 6C)alkoxy, hetAr³(1-6C)alkoxy, Ar³(1-6C)alkoxy, (1-4C alkoxy)(1-6C)alkoxy, (1-3C alkylsulfonyl)(1-6C)alkoxy, (3-6C)cycloalkyl [optionally substituted with F, OH, (1-6C alkyl), (1-6C) alkoxy, or (1-3C alkoxy)(1-6C)alkyl], hetAr⁴, hetAr⁴-0-, Ar⁴, hetCyc²(O)CH₂-, (1-4C alkoxycarbonyl)(1-6C)alkoxy, hydroxycarbonyl(I-6C)alkoxy, aminocarbonyl(1- 6C)alkoxy, hetCyc²C(=O)(1-6C)alkoxy, hydroxy(1-3C alkoxy)(1-6C)alkoxy, hydroxytrifluoro(1-6C)alkoxy, (1-3 C)alkylsulfonamido(1-6C)alkoxy, (1-3C)alkylamido(1-6C)alkoxy, di(1-3C alkyl)amino-carboxy, hetCyc²C(=O)0-, hydroxydifluoro(1-6C)alkyl, (1-4C alkylcarboxy)(1-6C)alkyl, (1-6C)alkoxycarbonyl, hydroxylcarbonyl, aminocarbonyl, (1-3C alkoxy)aminocarbonyl, hetCyc , halogen, CN, trifluoromethylsulfonyl, N-(1-3C alkyl)oxadiazolonyl, or hetAr⁵;
   hetCyc² is a 4-6 membered heterocyclic ring having 1-2 ring heteroatoms independently selected from N and O and optionally substituted with 1-2 groups independently selected from (1-6C)alkyl, (1-4C alkylcarboxy)(1-6C)alkyl, and (1-6C)acyl; hetCyc is a 4-7 membered heterocycle having 1-2 ring heteroatoms independently selected from N and O and optionally substituted with one or more substituents independently selected from F, CN, (1-6C)alkyl, trifluoro(1-6C)alkyl, hydroxy(1-6C)alkyl, (1-3C alkoxy)(1-6C)alkyl, (1-6C)acyl-, (1-6C)alkylsulfonyl, trifluoromethylsulfonyl and (1- 4C alkoxy)carbonyl;
   hetAr is a 5 -membered heteroaryl ring having 1-3 ring atoms independently selected from N, O and S and optionally substituted with (1-6C)alkyl;
   Ar is phenyl optionally substituted with (1-4C)alkoxy;
   hetAr⁴ is a 5-6 membered heteroaryl ring having 1-3 ring heteroatoms independently selected from N, S and O and optionally substituted with one or more substituents independently selected from (1-6C)alkyl, halogen, CN, hydroxy(1-6C)alkyl, trifluoro(1-6C)alkyl, difluoro(1-6C)alkyl, fluoro(1-6C)alkyl, (3-6C)cycloalkyl, (3-6C cycloalkyl)CH₂- (3-6C cycloalkyl)C(O)-, (1-3C alkoxy)(l -6C)alkyl, (1-6C)alkoxy, (1-6C)alkylsulfonyl, NH₂, (1-6C alkyl)amino, di(1-6C alkyl)amino, (1-3C trifluoroalkoxy), fluoro(1-6C alkyl)amino, difluoro(1-6C alkyl)amino, trifluoro(1-6C alkyl)amino, and (3-4C cycloalkyl)amino;
   hetAr⁵ is a group selected from the structures: 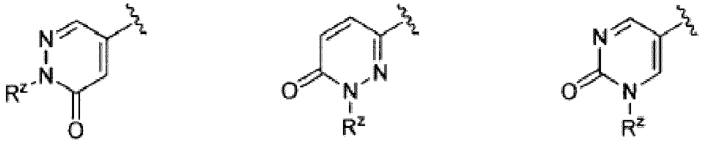 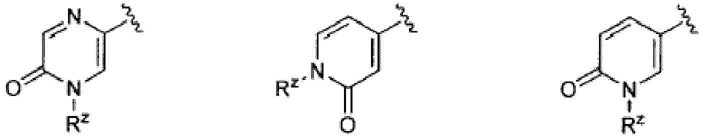
   where R^{z} is (3-4C)cycloalkyl or (1-3C)alkyl (optionally substituted with 1-3 fluoros), wherein each of said hetAr⁵ groups is optionally further substituted with one or more groups independently selected from F and (1-3C)alkyl optionally substituted with 1-3 fluoros;
   Ar⁴ is phenyl optionally substituted with one or more groups independently selected from (1-6C)alkyl, halogen, CN, CF₃, CF₃0-, (1-6C)alkoxy, (1-6C alkyl)OC(O)-, aminocarbonyl, (1-6C)alkylthio, hydroxy(1-6C)alkyl, (1-6C alkyl)SO₂-, HOC(O)- and (1- 3C alkoxy)(1-3C alkyl)OC(=O)-;
   R⁵ is (1-6C)alkyl, monofluoro(1-6C)alkyl, difluoro(1-6C)alkyl, trifluoro(1-6C)alkyl, tetrafluoro(2-6C)alkyl, pentafluoro(2-6C)alkyl, halogen, CN, (1-4C)alkoxy, hydroxy(1-4C)alkyl, (1-3C alkoxy)(1-4C)alkyl, (1-4C alkyl)OC(=O)-, (1-6C)alkylthio, (3- 4C)cycloalkyl, amino, aminocarbonyl, trifluoro(1-3C alkyl)amido, or phenyl (optionally substituted with one or more groups independently selected from halogen, (1-6C)alkyl and (1-6C)alkoxy); or
   R⁴ and R⁵ together with the atoms to which they are attached form a 5-6 membered saturated, partially unsaturated or unsaturated carbocyclic ring optionally substituted with one or more substituents independently selected from (1-6C)alkyl, or
   R⁴ and R⁵ together with the atoms to which they are attached form 5-6 membered saturated, partially unsaturated or unsaturated heterocyclic ring having a ring heteroatom selected from N, O or S, wherein said heterocyclic ring is optionally substituted with one or two substituents independently selected from (1-6C alkyl)C(=O)O-, (l-6C)acyl, (1-6C)alkyl and oxo, and said sulfur ring atom is optionally oxidized to S(=O) or SO₂; ^{3a} is hydrogen, halogen, (1-6C)alkyl, trifluoro(1-6C)alkyl, (3-6C)cycloalkyl, phenyl optionally substituted with one or more substituents independently selected from halogen and (1-6C)alkyl, or a 5-6 membered heteroaryl ring having 1-3 ring heteroatoms independently selected from N, O and S and optionally substituted with one or more groups independently selected from (1-6C)alkyl and halogen;
   R^{4a} is hydrogen, (1-6C)alkyl, trifluoro(1-6C)alkyl, phenyl [optionally substituted with one or more groups independently selected from (1-6C)alkyl, halogen, CN, CF₃, CF₃0-, (1-6C)alkoxy, (1-6Calkyl)OC(=O)-, aminocarbonyl, (1-6C)alkylthio, hydroxy(1-6C)alkyl, (1-6C alkyl)SO₂-, HOC(=O)- and (1-3C alkoxy)(1-3C alkyl)OC(=O)-], or a 5-6 membered heteroaryl ring having 1-3 ring heteroatoms independently selected from N, S and O and optionally substituted with 1-2 substituents independently selected from (1-6C)alkyl, hydroxy(1-6C)alkyl, trifluoro(1-6C)alkyl, (3-6C)cycloalkyl, (3-6C cycloalkyl)CH₂- (3-6C cycloalkyl)C(=O)-, (1-3C alkoxy)(1-6C)alkyl, (1-6C)alkoxy, (1-6C)alkylsulfonyl, NH₂, (1- 6C alkyl)amino, di(1-6C alkyl)amino and (1-3C trifluoroalkoxy)(1-3C)trifluoroalkyl; and
   R^{5a} is hydrogen, halogen, (1-6C)alkyl, trifluoro(1-6C)alkyl, (3-6C)cycloalkyl, phenyl optionally substituted with one or more substituents independently selected from halogen and (1-6C)alkyl, or a 5-6 membered heteroaryl ring having 1-3 ring heteroatoms independently selected from N, O and S and optionally substituted with one or more groups independently selected from (1-6C)alkyl and halogen.

Further examples of Trk inhibitors can be found in International Publication No. WO 2015175788, which is incorporated by reference in its entirety herein. For example, a Trk inhibitor can be a compound 1-((3S,4R)-4-(3-fluorophenyl)-1-(2-methoxyethyl)pyrrolidin-3-yl)-3-(4-methyl-3-(2-methylpyrimidin-5-yl)-1-phenyl-1H-pyrazol-5-yl)urea, or a pharmaceutically acceptable salt thereof. In some embodiments, the compound is a chloride salt.

Exemplary Trk inhibitors include AR-772, AR-786, AR-256, and AR-618.

Non-limiting examples of Trk inhibitors can be found in U.S. Patent No. 8,299,057 and International Publication No. WO 2009/013126 both of which are incorporated by reference in their entireties. For example, a Trk inhibitor can be a compound of Formula (I): 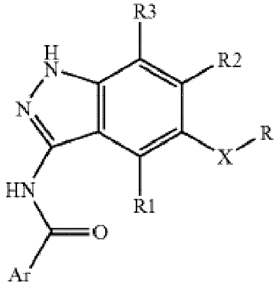 wherein:

X is -CH₂-, -CH(OH)-, -CH(OR')- or -C(R'R")-,

wherein:
R' is C₁-C₆ alkyl and R" is hydrogen;
Ar is phenyl, pyrazolyl or pyridyl optionally substituted with one or more substituents independently selected from halogen, nitro, COR4, OR7, NR5R6, NHSO₂R10, a straight or branched C₁-C₆ alkyl optionally substituted by a heterocyclyl, in its turn optionally substituted by a straight or branched C₁-C₆ alkyl or an heterocyclylalkyl, or
a heterocyclyl optionally substituted by a straight or branched C₁-C₆ alkyl, in its turn optionally substituted by a heterocyclyl or a C₁-C₆ alkoxycarbonyl, or a C₁-C₆ dialkylamino:
   R4 is NR5R6, or a heterocyclyl, optionally further substituted by a straight or branched C₁-C₆ alkyl, heterocyclylalkyl, heterocyclyl or a C₁-C₆ dialkylamino;
   R5 and R6 are independently hydrogen, R8R9N-C₂-C₆ alkyl, R8O-C₂-C₆alkyl, a straight or branched C₁-C₆ alkyl optionally further substituted by C₁-C₆ alkoxy, C₁-C₆ dialkylamino, halogen, phenyl, hydroxyl or heterocyclyl in its turn optionally substituted by alkyl, C₃-C₆ cycloalkyl optionally substituted by hydroxyl or trifluoro C₁-C₆ alkyl, heterocyclyl optionally substituted by C₁-C₆ alkyl in its turn optionally substituted by halogen or heterocyclyl, C₁-C₆alkoxycarbonyl, C₁-C₆ dialkylamino, heterocyclyl, or phenyl,
   or R5 and R6, taken together with the nitrogen atom to which they are bonded, may form a heterocyclyl group optionally substituted by a straight or branched C₁-C₆ alkyl, in its turn optionally substituted by a heterocyclyl or a C₁-C₆ alkoxycarbonyl, a C₁-C₆ dialkylamino or a heterocyclyl;
   R7 is straight or branched C₁-C₆ alkyl, optionally substituted by C₁-C₆dialkylamino or heterocyclyl in its turn substituted by C₁-C₆ alkyl;
   R8 and R9 are independently an optionally further substituted straight or branched C₁-C₆ alkyl;
   R10 is an optionally further substituted straight or branched C₁-C₆ alkyl;
   R is phenyl or pyridyl optionally substituted halogen or straight or branched C₁-C₆ alkyl;
   R1, R2 and R3 are hydrogen;
   or optical isomers, tautomers or pharmaceutically acceptable salt thereof.

For example, a Trk inhibitor can be entrectinib (N-[5-(3,5difluoro-benzyl)-1H-indazol-3-yl]-4-(4-methyl-piperazin-1-yl)-2-(tetrahydro-pyran-4-ylamino)-benzamide), or a pharmaceutically acceptable salt thereof. For example, a Trk inhibitor can be a polymorph such as those described in U.S. Publication No. 2015/0051222 or International Publication No. WO 2013/174876, both of which are incorporated by reference in their entireties herein. In some embodiments, a Trk inhibitor can be any disclosed in U.S. Publication No. 2015/0283132, International Publication No. WO 2015/124697, U.S. Patent No. 8,946,226, International Publication No. WO 2010/012733, U.S. Patent No. 8,912,194, and International Publication No. WO 2010/058006, all of which are incorporated by reference in their entireties herein.

Additional examples of Trk inhibitors can be found in International Publication No. WO 2015/017533, which is incorporated by reference in its entirety herein.

Further examples of Trk inhibitors can be found in International Publication No. WO 2015/112806, which is incorporated by reference in its entirety herein. For example, a Trk inhibitor can be a compound of Formula (I-A): 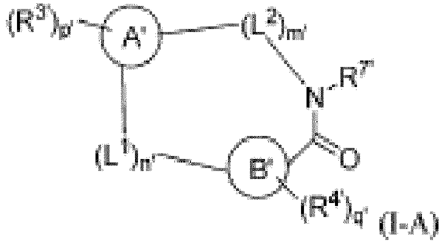 or a pharmaceutically acceptable salt thereof, wherein:
Ring A' and Ring B' are each independently a monocyclic or bicyclic aryl or heteroaryl; wherein one of Ring A' and Ring B' is a monocyclic aryl or heteroaryl and the other is a bicyclic heteroaryl; and at least one of Ring A' and Ring B' comprises at least one nitrogen ring member;
each L¹ and L² is independently -C(R^{1'})(R^{2'})-, -O-, -N(R^{k'})-, -S-, -S(O)- or -S(O)₂; each R¹ and R² are independently H, deuterium, halogen, C₁₋₆alkyl, C₂₋₆alkenyl, C₂₋₆alkynyl, C₃-₆cycloalkyl, 3- to 7-membered heterocycloalkyl, C₆-₁₀ aryl, or mono- or bicyclic heteroaryl, -OR^{a'}, -OC(O)R^{a'}, -OC(O)NR^{a'} R^{b'}, -OS(O)R^{a'}, -OS(O)₂R^{a'}, -SR^{a'}, -S(O)R^{a'},-S(O)₂R^{a'}, -S(O)NR^{a'}R^{b'}, -S(O)₂NR^{a'}R^{b'}, -OS(O)NR^{a'}R^{b'}, -OS(O)₂NR^{a'}R^{b'}, -NR^{a'}R^{b'},-NR^{a'}C(O)R^{b'}, -NR^{a'}C(O)OR^{b'}, -NR^{a'}C(O)NR^{a'}R^{b'}, -NR^{a'}S(O)R^{b'}, -NR^{a'}S(O)₂R^{b'},-NR^{a'}S(O)NR^{a'}R^{b'}, -NR^{a'}S(O)₂NR^{a'}R^{b'}, -C(O)R^{a'}, -C(O)OR^{a'}, -C(O)NR^{a'}R^{b'}, -PR^{a'}R^{b'},-P(O)R^{a'}R^{b'}, -P(O)₂R^{a'}R^{b'}, -P(O)NR^{a'}R^{b'}, -P(O)₂NR^{a'}R^{b'}, -P(O)OR^{a'}, -P(O)₂OR^{a'}, -CN, or -N0₂, or R^{1'} and R^{2'} taken together with the carbon or carbons to which they are attached form a C₃₋₆cycloalkyl or a 4- to 6-membered heterocycloalkyl, wherein each hydrogen atom in C₁₋₆alkyl, C₂₋₆alkenyl, C₂-₆alkynyl, C₃-₆cycloalkyl, 3- to 7-membered heterocycloalkyl, C₆₋₁₀ aryl, mono- or bicyclic heteroaryl, 4- to 6-membered heterocycloalkyl is independently optionally substituted by deuterium, halogen, C₁₋₆alkyl, C₁₋₆haloalkyl, -OR^{e'}, -OC(O)R^{e'},-OC(O)NR^{e'}R^{f'}, -OS(O)R^{e'}, -OS(O)₂R^{e'}, -OS(O)NR^{e'}R^{f'}, -OS(O)₂NR^{e}R^{f}, -SR^{e'}, -S(O)R^{e'},-S(O)₂R^{e'}, -S(O)NR^{e'}R^{f'}, -S(O)₂NR^{e'}R^{f'}, -NR^{e'}R^{f'}, -NR^{e'}C(O)R^{f'}, -NR^{e'}C(O)OR^{f'},-NR^{e'}C(O)NR^{e'}R^{f'}, -NR^{e'}S(O)R^{f'}, -NR^{e'}S(O)₂R^{f'}, -NR^{e'}S(O)NR^{e'}R^{f'}, -NR^{e'}S(O)₂NR^{e'}R^{f'},-C(O)R^{e''}, -C(O)OR^{e'}, -C(O)NR^{e'}R^{f'}, -PR^{e'}R^{f'}, -P(O)R^{e}R^{f'}, -P(O)₂R^{e'}R^{f'}, -P(O)NR^{e'}R^{f'},-P(O)₂NR^{e'}R^{f'}, -P(O)OR^{e'}, -P(O)₂OR^{e'}, -CN, or -NO₂;
each R^{k'} is independently H, deuterium, C₁₋₆alkyl, C₂-₆alkenyl, C₂₋₆alkynyl, C₃₋₆cycloalkyl, 3- to 7-membered heterocycloalkyl, C₆₋₁₀ aryl, or mono- or bicyclic heteroaryl, wherein each hydrogen atom in C₁₋₆alkyl, C₂-₆alkenyl, C₂-₆alkynyl, C₃₋₆cycloalkyl, 3- to 7-membered heterocycloalkyl, C₆₋₁₀ aryl, or mono- or bicyclic heteroaryl is independently optionally substituted by deuterium, halogen, C₁₋₆alkyl, C₁₋₆haloalkyl, -Or^{e'}, -OC(O)R^{e'},-OC(O)NR^{e'}R^{f'}, -OS(O)R^{e'}, -OS(O)₂R^{e'}, -OS(O)NR^{e'}R^{f'}, -OS(O)₂NR^{e'}R^{f'}, -SR^{e'}, -S(O)R^{e'},-S(O)₂R^{e'}, -S(O)NR^{e'}R^{f'}, -S(O)₂NR^{e'}R^{f'}, -NR^{e'}R^{f'}, -NR^{e'}C(O)R^{f'}, -NR^{e'}C(O)OR^{f'},-NR^{e'}C(O)NR^{e'}R^{f'}, -NR^{e'}S(O)R^{f'}, -NR^{e'}S(O)₂R^{f'}, -NR^{e'}S(O)NR^{e'}R^{f'}, -NR^{e'}S(O)₂NR^{e'}R^{f'},-C(O)R^{e'}, -C(O)OR^{e'}, -C(O)NR^{e'}R^{f'}, -PR^{e'}R^{f'}, -P(O)R^{e'}R^{f'}, -P(O)₂R^{e'}R^{f'}, -P(O)NR^{e'}R^{f'},-P(O)₂NR^{e'}R^{f'}, -P(O)OR^{e'}, -P(O)₂OR^{e'}, -CN, or -NO₂;
each R^{3'} and R^{4'} is independently deuterium, halogen, -OR^{c'}, -OC(O)R^{c'},-OC(O)NR^{c'}R^{d'}, -OC(=N)NR^{c'}R^{d'}, -OS(O)R^{c'}, -OS(O)₂R^{c'}, -OS(O)NR^{c'}R^{d'}, -OS(O)₂NR^{c'}R^{d'},-SR^{c'}, -S(O)R^{c'}, -S(O)₂R^{c'}, -S(O)NR^{c'}R^{d'}, -S(O)₂NR^{c'}R^{d'}, -NR^{c'}R^{d'}, -NR^{c'}C(O)R^{d'},-NR^{c'}C(O)OR^{d'}, -NR^{c'}C(O)NR^{c'}R^{d'}, -NR^{c'}C(=N)NR^{c'}R^{d'}, -NR^{c'}S(O)R^{d'}, -NR^{c'}S(O)₂R^{d'},-NR^{c'}S(O)NR^{c'}R^{d'}, -NR^{c'}S(O)₂NR^{C'}R^{d'}, -C(O)R^{c'}, -C(O)OR^{c'}, -C(O)NR^{c'}R^{d'}, -C(=N)NR^{c'}R^{d'},-PR^{c'}R^{d'}, -P(O)P^{c'}R^{d'}, -P(O)₂R^{c'}R^{d'}, -P(O)NR^{c'}R^{d'}, -P(O)₂NR^{c'}R^{d'}, -P(O)OR^{c'}, -P(O)₂OR^{c'}, -CN, -NO₂, C₁₋₆alkyl, C₂-₆alkenyl, C₂-₆alkynyl, C₃₋₆cycloalkyl, 3- to 7-membered heterocycloalkyl, C₆₋₁₀ aryl, or mono- or bicyclic heteroaryl, or any two R^{3'} groups or any two R^{4'} groups taken together with the ring to which they are attached form a C₅₋₈cycloalkyl or a 5- to 8-membered heterocycloalkyl, wherein each hydrogen atom in C₁₋₆alkyl, C₂₋₆alkenyl, C₂₋₆alkynyl, C₃₋₆cycloalkyl, 3- to 7-membered heterocycloalkyl, C₆₋₁₀ aryl, mono- or bicyclic heteroaryl C₅₋₈cycloalkyl or a 5- to 8-membered heterocycloalkyl is independently optionally substituted by deuterium, halogen, C₁₋₆alkyl, C₁₋₆haloalkyl, -OR^{e'}, -OC(O)R^{e'}, -OC(O)NR^{e'}R^{f'}, -OS(O)R^{e'},-OS(O)₂R^{e'}, -OS(O)NR^{e'}R^{f'}, -OS(O)₂NR^{e'}R^{f'}, -SR^{e'}, -S(O)R^{e'}, -S(O)₂R^{e'}, -S(O)NR^{e'}R^{f'},-S(O)₂NR^{e'}R^{f'}, -NR^{e'}R^{f'}, -NR^{e'}C(O)R^{f'}, -NR^{e'}C(O)OR^{f'}, -NR^{e'}C(O)NR^{e'}R^{f'}, -NR^{e'}S(O)R^{f'},-NR^{e'}S(O)₂R^{f'}, -NR^{e'}S(O)NR^{e'}R^{f'}, -NR^{e'}S(O)₂NR^{e'}R^{f'}, -C(O)R^{e'}, -C(O)OR^{e'}, -C(O)NR^{e'}R^{f'},-PR^{e'}R^{f'}, -P(O)R^{e'}R^{f'}, -P(O)₂R^{e'}R^{f'}, -P(O)NR^{e'}R^{f'}, -P(O)₂NR^{e'}R^{f'}, -P(O)OR^{E'}, -P(O)₂OR^{e'}, -CN, or -NO₂;
R^{7'} is H, deuterium, C₁₋₆alkyl, C₂-₆alkenyl, C₂-₆alkynyl, C₃-₆cycloalkyl, 3- to 7-membered heterocycloalkyl, C₆₋₁₀ aryl, or mono- or bicyclic heteroaryl, wherein each hydrogen atom in C₁₋₆alkyl, C₂₋₆alkenyl, C₂-₆alkynyl, C₃₋₆cycloalkyl, 3- to 7-membered heterocycloalkyl, C₆₋₁₀ aryl, or mono- or bicyclic heteroaryl is independently optionally substituted by deuterium, halogen, -OR^{i'}, -OC(O)R^{i'}, -OC(O)NRⁱ' R^{j'}, -OS(O)R^{i'}, -OS(O)₂R^{i'},-OS(O)NR^{i'}R^{i'}, -OS(O)₂NR^{i'}R^{j'}, -SR^{i'}, -S(O)R^{i'}, -S(O)₂R^{i'}, -S(O)NR^{i'}R^{j'}, -S(O)₂NR^{i'}R^{j'}, -NR^{i'}R^{j'}, -NR^{i'}C(O)R^{j'}, -NR^{i'}C(O)OR^{j'}, -NR^{i'}C(O)NR^{i'}R^{j'}, -NR^{i'}S(O)R^{j'}, -NR^{i'}S(O)₂R^{j'}, -NR^{i'}S(O)NR^{i'}R^{j'}, -NR^{i'}S(O)₂NR^{i'}R^{j'}, -C(O)R^{i'}, -C(O)OR^{i'}, -C(O)NR^{i'}R^{j'}, -PR^{i'}R^{j'}, -P(O)R^{i'}R^{j'}, -P(O)₂R^{i'}R^{j'},-P(O)NR^{i'}R^{j'}, -P(O)₂NR^{i'}R^{j'}, -P(O)OR^{i'}, -P(O)₂OR^{i'}, -CN, or -NO₂;
each R^{a'}, R^{b'}, R^{c'}, R^{d'}, R^{e'}, R^{f'}, R^{i'} and R^{j'} is independently selected from the group consisting of H, deuterium, C₁₋₆alkyl, C₂₋₆alkenyl, C₂₋₆alkynyl, C₃-₆cycloalkyl, 3- to 7-membered heterocycloalkyl, C₆₋₁₀ aryl, and heteroaryl;
m' is 2, 3, 4, or 5;
n' is 2, 3, or 4;
p' is 0, 1, 2, 3, or 4; and
q' is 0, 1, 2, 3, or 4; or a pharmaceutically acceptable salt thereof. Exemplary Trk inhibitors include TPX-0005.

A Trk inhibitor can be one found in U.S. Patent No. 9,187,489 and International Publication No. WO 2013/183578, both of which are incorporated by reference in their entireties herein. Exemplary Trk inhibitors include PLX7486 and DS-6051.

Non-limiting examples of Trk inhibitors can be found in U.S. Publication No. 2015/0306086 and International Publication No. WO 2013/074518, both of which are incorporated by reference in their entireties herein. Exemplary Trk inhibitors include TSR-011.

Further examples of Trk inhibitors can be found in U.S. Patent No. 8,637,516, International Publication No. WO 2012/034091, U.S. Patent No. 9,102,671, International Publication No. WO 2012/116217, U.S. Publication No. 2010/0297115, International Publication No. WO 2009/053442, U.S. Patent No. 8,642,035, International Publication No. WO 2009092049, U.S. Patent No. 8,691,221, International Publication No. WO2006131952, all of which are incorporated by reference in their entireties herein. Exemplary Trk inhibitors include GNF-4256, described in Cancer Chemother. Pharmacol. 75(1):131-141, 2015; and GNF-5837 (N-[3-[[2,3-dihydro-2-oxo-3-(1H-pyrrol-2-ylmethylene)-1H-indol-6-yl]amino]-4-methylphenyl]-N'-[2-fluoro-5-(trifluoromethyl)phenyl]-urea), described in ACSMed. Chem. Lett. 3(2):140-145, 2012, each of which is incorporated by reference in its entirety herein.

Additional examples of Trk inhibitors include those disclosed in U.S. Publication No. 2010/0152219, U.S. Patent No. 8,114,989, and International Publication No. WO 2006/123113, all of which are incorporated by reference in their entireties herein. Exemplary Trk inhibitors include AZ623, described in Cancer 117(6):1321-1391, 2011; AZD6918, described in Cancer Biol. Ther. 16(3):477-483, 2015; AZ64, described in Cancer Chemother. Pharmacol. 70:477-486, 2012; AZ-23 ((S)-5-Chloro-N2-(1-(5-fluoropyridin-2-yl)ethyl)-N4-(5-isopropoxy-1H-pyrazol-3-yl)pyrimidine-2,4-diamine), described in Mol. Cancer Ther. 8:1818-1827, 2009; and AZD7451; each of which is incorporated by reference in its entirety.

A Trk inhibitor can include those described in U.S. Patent Nos. 7,615,383; 7,384,632; 6,153,189; 6,027,927; 6,025,166; 5,910,574; 5,877,016; and 5,844,092, each of which is incorporated by reference in its entirety.

Further examples of Trk inhibitors include CEP-751, described in Int. J. Cancer 72:672-679, 1997; CT327, described in Acta Derm. Venereol. 95:542-548, 2015; compounds described in International Publication No. WO 2012/034095; compounds described in U.S. Patent No. 8,673,347 and International Publication No. WO 2007/022999; compounds described in U.S. Patent No. 8,338,417; compounds described in International Publication No. WO 2016/027754; compounds described in U.S. Patent No. 9,242,977; compounds described in U.S. Publication No. 2016/0000783; sunitinib (N-(2-diethylaminoethyl)-5-[(Z)-(5-fluoro-2-oxo-1H-indol-3-ylidene)methyl]-2,4-dimethyl-1H-pyrrole-3-carboxamide), as described in PLoS One 9:e95628, 2014; compounds described in International Publication No. WO 2011/133637; compounds described in U.S. Patent No. 8,637,256; compounds described in Expert. Opin. Ther. Pat. 24(7):731-744, 2014; compounds described in Expert Opin. Ther. Pat. 19(3):305-319, 2009; (R)-2-phenylpyrrolidine substituted imadizopyridazines, e.g., (4-((5-chloro-4-(methylamino)-7H-pyrrolo[2,3-d]pyrimidin-2-yl)amino)-3-methoxyphenyl)(morpholino)methanone as described in ACS Med. Chem. Lett. 6(5):562-567, 2015; GTx-186 and others, as described in *PLoS One* 8(12):e83380, 2013; K252a ((9S-(9α,10β,12α))-2,3,9,10,11,12-hexahydro-10-hydroxy-10-(methoxycarbonyl)-9-methyl-9,12-epoxy-1H-diindolo[1,2,3-fg:3',2',1'-kl]pyrrolo[3,4-i][1,6]benzodiazocin-1-one), as described in Mol. Cell Biochem. 339(1-2):201-213, 2010; 4-aminopyrazolylpyrimidines, e.g., AZ-23 (((S)-5-chloro-N2-(1-(5-fluoropyridin-2-yl)ethyl)-N4-(5-isopropoxy-1H-pyrazol-3-yl)pyrimidine-2,4-diamine)), as described in J. Med. Chem. 51(15):4672-4684, 2008; PHA-739358 (danusertib), as described *in* Mol. Cancer Ther. 6:3158, 2007; Gö 6976 (5,6,7,13-tetrahydro-13-methyl-5-oxo-12H-indolo[2,3-a]pyrrolo[3,4-c]carbazole-12-propanenitrile), as described in J. Neurochem. 72:919-924, 1999; GW441756 ((3Z)-3-[(1-methylindol-3-yl)methylidene]-1H-pyrrolo[3,2-b]pyridin-2-one), as described in *IJAE* 115:117, 2010; milciclib (PHA-848125AC), described in *J. Carcinog.* 12:22, 2013; AG-879 ((2E)-3-[3,5-Bis(1,1-dimethylethyl)-4-hydroxyphenyl]-2-cyano-2-propenethioamide); altiratinib (N-(4-((2-(cyclopropanecarboxamido)pyridin-4-yl)oxy)-2,5-difluorophenyl)-N-(4-fluorophenyl)cyclopropane-1,1-dicarboxamide); cabozantinib (N-(4-((6,7-Dimethoxyquinolin-4-yl)oxy)phenyl)-N'-(4-fluorophenyl)cyclopropane-1,1-dicarboxamide); lestaurtinib ((5S,6S,8R)-6-Hydroxy-6-(hydroxymethyl)-5-methyl-7,8,14,15-tetrahydro-5H-16-oxa-4b,8a,14-triaza-5,8-methanodibenzo[b,h]cycloocta[jkl]cyclopenta[e]-as-indacen-13(6H)-one); dovatinib (4-amino-5-fluoro-3-[6-(4-methylpiperazin-1-yl)-1H-benzimidazol-2-yl]quinolin-2(1H)-one mono 2-hydroxypropanoate hydrate); sitravatinib (N-(3-fluoro-4-((2-(5-(((2-methoxyethyl)amino)methyl)pyridin-2-yl)thieno[3,2-b]pyridin-7-yl)oxy)phenyl)-N-(4-fluorophenyl)cyclopropane-1,1-dicarboxamide); ONO-5390556; regorafenib (4-[4-({[4-Chloro-3-(trifluoromethyl)phenyl]carbamoyl}amino)-3-fluorophenoxy]-N-methylpyridine-2-carboxamide hydrate); VSR-902A; all of the references above are incorporated by reference in their entireties herein.

The ability of a Trk inhibitor to act as a TrkA, TrkB, and/or Trk C inhibitor may be tested using the assays described in Examples A and B in U.S. Patent No. 8,513,263, which is incorporated herein by reference.

### Methods of Treating a Subject having a Cancer

Provided herein are methods of treating a subject having a cancer (e.g., any of the cancers described herein) that include identifying a subject having a cancer cell that has at least one (e.g., two, three, four, five, six, seven, eight, nine, ten, eleven, or twelve) point mutation in a NTRK1, NTRK2, and/or NTRK3 gene that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), and administering to the identified subject a therapeutically effective amount of a Trk inhibitor (e.g., any of the Trk inhibitors described herein). Also provided are methods of treating a subject having a cancer (e.g., any of the cancers described herein) that include identifying a subject having a cancer cell that has at least one (e.g., one, two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M), and administering to the identified subject a therapeutically effective amount of a Trk inhibitor (e.g., any of the Trk inhibitors described herein).

Also provided are methods of treating a subject having a cancer (e.g., any of the cancers described herein) and identified as having a cancer cell that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein) that include administering to the identified subject a therapeutically effective amount of a Trk inhibitor (e.g., any of the Trk inhibitors described herein). Also provided are methods of treating a subject having a cancer (e.g., any of the cancers described herein) and identified as having a cancer cell that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M) that include administering to the identified subject a therapeutically effective amount of a Trk inhibitor (e.g., any of the Trk inhibitors described herein).

In some examples, the Trk inhibitor is administered orally, subcutaneously, intraperitoneally, intravenously, or intramuscularly. The Trk inhibitor can be administered in one or more doses comprising between about 1 mg and about 250 mg, between about 1 mg and about 200 mg, between about 1 mg and about 180 mg, between about 1 mg and about 160 mg, between about 1 mg and about 140 mg, between about 1 mg and about 120 mg, between about 1 mg and about 100 mg, between about 1 mg and about 80 mg, between about 1 mg and about 60 mg, between about 1 mg and about 40 mg, between about 1 mg and about 40 mg, between about 10 mg and about 200 mg, between about 10 mg and about 180 mg, between about 10 mg and about 160 mg, between about 10 mg and about 140 mg, between about 10 mg and about 120 mg, between about 10 mg and about 100 mg, between about 10 mg and about 80 mg, between about 10 mg and about 60 mg, between about 10 mg and about 40 mg, between about 10 mg and about 20 mg, between about 20 mg and about 200 mg, between about 20 mg and about 180 mg, between about 20 mg and about 160 mg, between about 20 mg and about 140 mg, between about 20 mg and about 120 mg, between about 20 mg and about 100 mg, between about 20 mg and about 80 mg, between about 20 mg and about 60 mg, between about 20 mg and about 40 mg, between about 40 mg and about 200 mg, between about 40 mg and about 180 mg, between about 40 mg and about 160 mg, between about 40 mg and about 140 mg, between about 40 mg and about 120 mg, between about 40 mg and about 100 mg, between about 40 mg and about 80 mg, between about 40 mg and about 60 mg, between about 60 mg and about 200 mg, between about 60 mg and about 180 mg, between about 60 mg and about 140 mg, between about 60 mg and about 120 mg, between about 60 mg and about 100 mg, between about 60 mg and about 80 mg, between about 80 mg and about 200 mg, between about 80 mg and about 180 mg, between about 80 mg and about 160 mg, between about 80 mg and about 140 mg, between about 80 mg and about 120 mg, between about 80 mg and about 100 mg, between about 90 mg and about 110 mg, between about 95 mg and about 105 mg, between about 100 mg and about 200 mg, between about 100 mg and about 180 mg, between about 100 mg and about 160 mg, between about 100 mg and about 140 mg, between about 100 mg and about 120 mg, between about 120 mg and about 200 mg, between about 120 mg and about 180 mg, between about 120 mg and about 160 mg, between about 120 mg and about 140 mg, between about 140 mg and about 200 mg, between about 140 mg and about 180 mg, between about 140 mg and about 160 mg, between about 160 mg and about 200 mg, between about 160 mg and about 200 mg, between about 160 mg and about 180 mg, or between about 180 mg and about 200 mg. The appropriate dose of a Trk inhibitor to be administered to a subject can be determined by a medical professional, e.g., based upon one or more of the subject's mass, the subject's condition, subject's gender, and the other diseases that the subject may have.

Multiple doses of a Trk inhibitor (e.g., any of the doses described herein) can be administered once every six months, once every five months, once every four months, once every three months, once every two months, once every six weeks, once a month, once every three weeks, once every two weeks, once a week, twice a week, three times a week, four times a week, three times a week, every other day, once a day, twice a day, or three times a day. The Trk inhibitor can be self-administered (e.g., by the subject having a cancer) or can be administered by a health care professional (e.g., a physician, a nurse, a physician's assistance, or a pharmacist). In some examples, the subject is hospitalized or treated on in inpatient basis. In other examples, the subject is treated on an outpatient basis.

The cancer can be any of the exemplary cancers described herein. In some embodiments, the subject has previously been identified or diagnosed as having a cancer. In some examples, the subject has previously been administered a treatment for cancer, and the treatment for cancer has been unsuccessful (e.g., high toxicity in the subject or no positive response to the previously administered treatment for cancer).

Some examples of these methods further include recording in the subject's clinical record (e.g., a computer readable medium) that the subject should be administered a treatment comprising a therapeutically effective amount of a Trk inhibitor in the future.

In some examples, the step of identifying a subject having a cancer cell that has the at least one point mutation (e.g., any of the point mutations described herein) in a NTRK1, NTRK2, and/or NTRK3 gene that results in the expression of a TrkA, TrkB, and/or TrkC protein including a mutation at one or more amino acid position(s), comprises performing an assay to determine the presence of the at least one point mutation in a NTRK1, NTRK2, and/or NTRK3 gene in a cancer cell in a sample (e.g., a biopsy sample) from the subject. Any of the assays described herein can be used to determine the presence of the at least one point mutation in a NTRK1, NTRK2, and/or NTRK3 gene. In addition, any of the kits provided herein can be used in an assay to determine the presence of the at least one point mutation in a NTRK1, NTRK2, and/or NTRK3 gene. In some examples, the assay includes sequencing a segment of a NTRK1, NTRK2, and/or NTRK3 gene including the at least one point mutation.

### Methods of Selecting a Treatment for a Subject

Also provided herein are methods of selecting a treatment for a subject having a cancer (e.g., any of the cancers described herein) that include identifying a subject having a cancer cell that has at least one (e.g., two, three, four, five, six, seven, eight, nine, ten, eleven, or twelve) point mutation in a NTRK1, NTRK2, and/or NTRK3 gene that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), and selecting a treatment comprising a therapeutically effective amount of a Trk inhibitor (e.g., any of the Trk inhibitors described herein) for the identified subject. Also provided are methods of selecting a treatment for a subject having a cancer (e.g., any of the cancers described herein) that include identifying a subject having a cancer cell that has at least one (e.g., one, two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M), and selecting a treatment comprising a therapeutically effective amount of a Trk inhibitor (e.g., any of the Trk inhibitors described herein) for the identified subject.

Also provided are methods of selecting a treatment for a subject having a cancer (e.g., any of the cancers described herein) that include selecting a treatment comprising a therapeutically effective amount of a Trk inhibitor (e.g., any of the Trk inhibitors described herein) for a subject identified as having a cancer cell that has at least one (e.g., two, three, four, five, six, seven, eight, nine, ten, eleven, or twelve) point mutation in a NTRK1, NTRK2, and/or NTRK3 gene that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein). Also provided are methods of selecting a treatment for a subject having a cancer (e.g., any of the Trk inhibitors described herein) that include selecting a treatment comprising a therapeutically effective amount of a Trk inhibitor (e.g., any of the Trk inhibitors described herein) for a subject identified as having a cancer cell that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M).

Some examples of these methods further include administering the selected treatment to the identified subject (e.g., using any of the Trk inhibitors, any of the routes of administration, any of the doses, and/or any of the frequencies of administration described herein). In some examples, the selected treatment is self-administered. In other examples, the selected treatment is administered by a medical professional (e.g., any of the medical professionals described herein). Some examples of these methods further include recording the selected treatment in the identified subject's clinical record (e.g., a computer readable medium).

The cancer can be any of the exemplary cancers described herein. In some embodiments, the subject has previously been identified or diagnosed as having a cancer. In some examples, the subject has previously been administered a treatment for cancer, and the treatment for cancer has been unsuccessful (e.g., high toxicity in the subject or no positive response to the previously administered treatment for cancer).

In some examples, the step of identifying a subject having a cancer cell that has the at least one point mutation (e.g., any of the point mutations described herein) in a NTRK1, NTRK2, and/or NTRK3 gene that results in the expression of a TrkA, TrkB, and/or TrkC protein including a mutation at one or more amino acid position(s), comprises performing an assay to determine the presence of the at least one point mutation in a NTRK1, NTRK2, and/or NTRK3 gene in a cancer cell in a sample (e.g., a biopsy sample) from the subject. Any of the assays described herein can be used to determine the presence of the at least one point mutation in a NTRK1, NTRK2, and/or NTRK3 gene. In addition, any of the kits provided herein can be used in an assay to determine the presence of the at least one point mutation in a NTRK1, NTRK2, and/or NTRK3 gene. In some examples, the assay includes sequencing a segment of a NTRK1, NTRK2, and/or NTRK3 gene including the at least one point mutation.

### Methods of Selecting a Subject for Treatment with a Trk Inhibitor

Also provided are methods of selecting a subject having a cancer for treatment with a Trk inhibitor that include identifying a subject having a cancer (e.g., any of the cancers described herein) and having a cancer cell that has at least one (e.g., two, three, four, five, six, seven, eight, nine, ten, eleven, or twelve) point mutation in a NTRK1, NTRK2, and/or NTRK3 gene that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), and selecting the identified subject for treatment with a Trk inhibitor (e.g., any of the Trk inhibitors described herein). Also provided are methods of selecting a subject having a cancer for treatment with a Trk inhibitor that include identifying a subject having a cancer (e.g., any of the cancers described herein) and having a cancer cell that has at least one (e.g., one, two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M), and selecting the identified subject for treatment with a Trk inhibitor (e.g., any of the Trk inhibitors described herein).

Also provided are methods of selecting a subject having a cancer for treatment with a Trk inhibitor that include selecting a subject having cancer (e.g., any of the cancers described herein) and identified as having a cancer cell that has at least one (e.g., two, three, four, five, six, seven, eight, nine, ten, eleven, or twelve) point mutation in a NTRK1, NTRK2, and/or NTRK3 gene that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein) for treatment with a Trk inhibitor (e.g., any of the Trk inhibitors described herein). Also provided are methods of selecting a subject having a cancer for treatment with a Trk inhibitor that include selecting a subject having a cancer (e.g., any of the cancers described herein) and identified as having a cancer cell that has at least one (e.g., one, two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M) for treatment with a Trk inhibitor (e.g., any of the Trk inhibitors described herein).

Some examples of these methods further include administering a therapeutically effective amount of a Trk inhibitor (e.g., using any of the Trk inhibitors, any of the routes of administration, any of the doses, and/or any of the frequencies of administration described herein) to the selected subject. In some examples, the Trk inhibitor is self-administered. In other examples, the Trk inhibitor is administered to the selected subject by a medical professional. In some examples, the selected subject is hospitalized. In other examples, the subject is administered the Trk inhibitor on an outpatient basis. Some methods further include recording in the subject's clinical record (e.g., a computer readable medium) that the subject is selected for treatment with a Trk inhibitor.

The cancer can be any of the exemplary cancers described herein. In some embodiments, the subject has previously been identified or diagnosed as having a cancer. In some examples, the subject has previously been administered a treatment for cancer, and the treatment for cancer has been unsuccessful (e.g., high toxicity in the subject or no positive response to the previously administered treatment for cancer).

In some examples, the step of identifying a subject having a cancer cell that has the at least one point mutation (e.g., any of the point mutations described herein) in a NTRK1, NTRK2, and/or NTRK3 gene that results in the expression of a TrkA, TrkB, and/or TrkC protein including a mutation at one or more amino acid position(s), comprises performing an assay to determine the presence of the at least one point mutation in a NTRK1, NTRK2, and/or NTRK3 gene in a cancer cell in a sample (e.g., a biopsy sample) from the subject. Any of the assays described herein can be used to determine the presence of the at least one point mutation in a NTRK1, NTRK2, and/or NTRK3 gene. In addition, any of the kits provided herein can be used in an assay to determine the presence of the at least one point mutation in a NTRK1, NTRK2, and/or NTRK3 gene. In some examples, the assay includes sequencing a segment of a NTRK1, NTRK2, and/or NTRK3 gene including the at least one point mutation.

### Methods of Determining the Likelihood that a Subject will have a Positive Response to a Trk Inhibitor

Also provided are methods of determining the likelihood that a subject having a cancer (e.g., any of the cancers described herein) will have a positive response to treatment with a Trk inhibitor (e.g., any of the Trk inhibitors described herein) that include determining whether a cancer cell in a sample obtained from the subject has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), and determining that a subject having a cancer cell that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTKR3 point mutations) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein) has an increased likelihood of having a positive response to treatment with a Trk inhibitor (e.g., as compared to a subject having a cancer cell that does not have a point mutation in a NTRK1, NTRK2, and/or NTRK3 gene that results in the expression of a TrkA, TrkB, and/or TrkC protein comprising one or more mutations (e.g., any of the mutations in TrkA, TrkB, and/or TrkC described herein)). Also provided are methods of determining the likelihood that a subject having a cancer (e.g., any of the cancers described herein) will have a positive response to treatment with a Trk inhibitor (e.g., any of the Trk inhibitors described herein) that include determining whether a cancer cell in a sample obtained from the subject has at least one (e.g., one, two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M) and determining that a subject having a cancer cell that has at least one (e.g., one, two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M) has an increased likelihood of having a positive response to treatment with a Trk inhibitor (e.g., as compared to a subject having a cancer cell that does not have a point mutation in a NTRK2 gene that results in the expression of a TrkB protein comprising a mutation at one or more amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M)).

Also provided are methods of determining the likelihood that a subject having cancer (e.g., any of the cancers described herein) will have a positive response to a treatment with a Trk inhibitor (e.g., any of the Trk inhibitors described herein) that include determining that a subject having a cancer cell that has at least one (e.g., two, three, four, five, six, seven, eight, nine, ten, eleven, or twelve) point mutation in a NTRK1, NTRK2, and/or NTRK3 gene (e.g., that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein) has an increased likelihood of having a positive response to treatment with a Trk inhibitor (e.g., as compared to a subject having a cancer cell that does not have at least one point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more mutations (e.g., any of the mutations in TrkA, TrkB, and/or TrkC described herein)). Also provided are methods of determining the likelihood that a subject having cancer (e.g., any of the cancers described herein) will have a positive response to a treatment with a Trk inhibitor (e.g., any of the Trk inhibitors described herein) that include determining that a subject having a cancer cell that has at least one (e.g., one, two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M) has an increased likelihood of having a positive response to treatment with a Trk inhibitor (e.g., as compared to a subject having a cancer cell that does not have at least one point mutation in a NTRK2 gene that results in the expression of a TrkB protein comprising a mutation at one or more amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M)).

Some examples of these methods include administering a therapeutically effective amount of a Trk inhibitor (e.g., any of the Trk inhibitors described herein) to a subject determined to have an increased likelihood of having a positive response to treatment with a Trk inhibitor (e.g., using any of the Trk inhibitors, any of the routes of administration, any of the doses, and/or any of the frequencies of administration described herein). In some examples, the Trk inhibitor is self-administered. In other examples, the Trk inhibitor is administered to the selected subject by a medical professional. In some examples, the selected subject is hospitalized. In other examples, the subject is administered the Trk inhibitor on an outpatient basis. Some methods further include recording in the subject's clinical record (e.g., a computer readable medium) that the subject has an increased likelihood of having a positive response to treatment with a Trk inhibitor.

The cancer can be any of the exemplary cancers described herein. In some embodiments, the subject has previously been identified or diagnosed as having a cancer. In some examples, the subject has previously been administered a treatment for cancer, and the treatment for cancer has been unsuccessful (e.g., high toxicity in the subject or no positive response to the previously administered treatment for cancer).

In some examples, the step of determining whether a cancer cell in a sample obtained from the subject has at least one (e.g., two, three, four, five, six, seven, eight, nine, ten, eleven, or twelve) point mutation in a NTRK1, NTRK2, and/or NTRK3 gene that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), comprises performing an assay to determine the presence of the at least one point mutation in a NTRK1, NTRK2, and/or NTRK3 gene in a cancer cell in the sample (e.g., a biopsy sample) from the subject. Any of the assays described herein can be used to determine the presence of the at least one point mutation in a NTRK1, NTRK2, and/or NTRK3 gene. In addition, any of the kits provided herein can be used in an assay to determine the presence of the at least one point mutation in a NTRK1, NTRK2, and/or NTRK3 gene. In some examples, the assay includes sequencing a segment of a NTRK1, NTRK2, and/or NTRK3 gene including the at least one point mutation.

### Methods of Predicting the Efficacy of Treatment with a Trk inhibitor in a Subject

Also provided are methods of predicting the efficacy of a Trk inhibitor (e.g., any of the Trk inhibitors described herein) in a subject having cancer (e.g., any of the cancers described herein) that include determining whether a cancer cell in a sample obtained from the subject has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), and determining that a Trk inhibitor is more likely to be effective in a subject having a cancer cell in a sample obtained from the subject that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein) (e.g., as compared to a subject having a cancer cell in a sample obtained from the subject that does not have a point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more mutations (e.g., any of the mutations in TrkA, TrkB, and/or TrkC described herein)).

Also provided are methods of predicting the efficacy of a Trk inhibitor (e.g., any of the Trk inhibitors described herein) in a subject having cancer (e.g., any of the cancers described herein) that include determining whether a cancer cell in a sample obtained from the subject has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M), and determining that a Trk inhibitor is more likely to be effective in a subject having a cancer cell in a sample obtained from the subject that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M) (e.g., as compared to a subject having a cancer cell in a sample obtained from the subject that does not have a point mutation in a NTRK3 gene that results in the expression of a TrkB protein comprising a mutation at one or more amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M)).

Also provided are methods of predicting the efficacy of a Trk inhibitor in a subject having a cancer (e.g., any of the cancers described herein) that include determining that a Trk inhibitor (e.g., any of the Trk inhibitors described herein) is more likely to be effective in a subject having a cancer cell in a sample obtained from the subject that has at least one point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein) (e.g., as compared to a subject having a cancer cell in a sample obtained from the subject that does not have a point mutation in a NTRK1, NTRK2, and/or NTRK3 gene (e.g., any of the NTRK1, NTRK2, and/or NTKR3 point mutations described herein) that results in the expression of a TrkA, TrkB, and/or TrkC protein comprising one or more mutations (e.g., any of the mutations in TrkA, TrkB, and/or TrkC described herein)).

Also provided are methods of predicting the efficacy of a Trk inhibitor in a subject having a cancer (e.g., any of the cancers described herein) that include determining that a Trk inhibitor (e.g., any of the Trk inhibitors described herein) is more likely to be effective in a subject having a cancer cell in a sample obtained from the subject that has at least one point mutation in a NTRK2 gene that results in the expression of a TrkB protein comprising a mutation at one or more amino acid position(s) selected from the group consisting of: 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M) (e.g., as compared to a subject having a cancer cell in a sample obtained from the subject that does not have a mutation at one or more amino acid position(s) selected from the group consisting of: 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M)).

Some methods further include recording in the subject's clinical record (e.g., a computer readable medium) the predicted efficacy of a Trk inhibitor in the subject having a cancer. Some examples of these methods further include selecting a treatment for the subject based on the predicted efficacy of a Trk inhibitor in the subject. Some examples further include administering the selected treatment to the subject (e.g., using any of the Trk inhibitors, any of the routes of administration, any of the doses, and/or any of the frequencies of administration described herein).

The cancer can be any of the exemplary cancers described herein. In some embodiments, the subject has previously been identified or diagnosed as having a cancer. In some examples, the subject has previously been administered a treatment for cancer, and the treatment for cancer has been unsuccessful (e.g., high toxicity in the subject or no positive response to the previously administered treatment for cancer).

In some examples, the step of determining whether a cancer cell in a sample obtained from the subject has at least one (e.g., two, three, four, five, six, seven, eight, nine, ten, eleven, or twelve) point mutation in a NTRK1, NTRK2, and/or NTRK3 gene that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), comprises performing an assay to determine the presence of the at least one point mutation in a NTRK1, NTRK2, and/or NTRK3 gene in a cancer cell in the sample (e.g., a biopsy sample) from the subject. Any of the assays described herein can be used to determine the presence of the at least one point mutation in a NTRK1, NTRK2, and/or NTRK3 gene. In addition, any of the kits provided herein can be used in an assay to determine the presence of the at least one point mutation in a NTRK1, NTRK2, and/or NTRK3 gene. In some examples, the assay includes sequencing a segment of a NTRK1, NTRK2, and/or NTRK3 gene including the at least one point mutation.

### Methods of Predicting the Risk of Developing a Cancer

Also provided are methods of determining a subject's risk for developing a cancer (e.g., any of the cancers described herein) that include determining whether a cell in a sample obtained from the subject has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), and identifying a subject having a cell that has at least one point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein) as having an increased likelihood of developing a cancer (e.g., as compared to a subject not having a point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more mutations (e.g., any of the mutations in TrkA, TrkB, and/or TrkC described herein)).

Also provided are methods of identifying a determining a subject's risk for developing a cancer (e.g., any of the cancers described herein) that include determining whether a cell in a sample obtained from the subject has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M), and identifying a subject having a cell that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M) as having an increased likelihood of developing a cancer (e.g., as compared to a subject not having a point mutation in a NTRK2 gene that results in the expression of a TrkB protein comprising a mutation at one or more amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M)).

Also provided are methods of determining a subject's risk for developing a cancer (e.g., any of the cancers described herein) that include identifying a subject having a cell that has at least one (e.g., two, three, four, five, six, seven, eight, nine, ten, eleven, or twelve) point mutation(s) in a NTRK1, NTRK2, and/or NTRK3 gene (e.g., any of the NTRK1, NTRK2, and/or NTRK point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein) as having an increased likelihood of developing a cancer (e.g., as compared to a subject having a cell that does not have a point mutation(s) in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more mutations (e.g., any of the mutations in TrkA, TrkB, and/or TrkC described herein)).

Also provided are methods of determining a subject's risk for developing a cancer (e.g., any of the cancers described herein) that include identifying a subject having a cell that has at least one (e.g., two, three, four, five, six, seven, eight, nine, ten, eleven, or twelve) point mutation(s) in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M), as having an increased likelihood of developing a cancer (e.g., as compared to a subject having a cell that does not have a point mutation(s) in a NTRK2 gene that results in the expression of a TrkB protein comprising a mutation at one or more amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M)).

Some methods further include recording in the subject's clinical record (e.g., a computer readable medium) the subject's risk of developing a cancer. The cancer can be any of the exemplary cancers described herein.

In some examples, the subject is identified as having been exposed to a significant level of carcinogen(s) (e.g., tobacco smoke, UVB radiation, and gamma irradiation). In some examples, the subject is suspected of having cancer, presents with one or more symptoms of cancer (e.g., any of the symptoms of cancer described herein), and/or has a family history of cancer.

In some examples, the step of determining whether a cancer cell in a sample obtained from the subject has at least one (e.g., two, three, four, five, six, seven, eight, nine, ten, eleven, or twelve) point mutation in a NTRK1, NTRK2, and/or NTRK3 gene that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), comprises performing an assay to determine the presence of the at least one point mutation in a NTRK1, NTRK2, and/or NTRK3 gene in a cancer cell in the sample (e.g., a biopsy sample) from the subject. Any of the assays described herein can be used to determine the presence of the at least one point mutation in a NTRK1, NTRK2, and/or NTRK3 gene. In addition, any of the kits provided herein can be used in an assay to determine the presence of the at least one point mutation in a NTRK1, NTRK2, and/or NTRK3 gene. In some examples, the assay includes sequencing a segment of a NTRK1, NTRK2, and/or NTRK3 gene including the at least one point mutation.

### Methods of Assisting in the Diagnosis of Cancer in a Subject

Also provided herein are methods of assisting in the diagnosis of a cancer (e.g., any of the cancers described herein) that include determining whether a cell in a sample obtained from the subject has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), and determining that a subject having a cell that has at least one (e.g., two, three, four, five, six, seven, eight, nine, ten, eleven, or twelve) point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein) has an increased risk likelihood of having a cancer (e.g., as compared to a subject not having a point mutation in a NTRK1, NTRK2, and/or NTRK3 gene (e.g., any of the NTRK1, NTRK2, and/or NTRK point mutations described herein) that results in the expression of a TrkA, TrkB, and/or TrkC protein comprising one or more mutations (e.g., any of the mutations in TrkA, TrkB, and/or TrkC described herein)).

Also provided herein are methods of assisting in the diagnosis of a cancer (e.g., any of the cancers described herein) that include determining whether a cell in a sample obtained from the subject has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M), and determining that a subject having a cell that has at least one (e.g., two, three, four, five, six, seven, eight, nine, ten, eleven, or twelve) point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M) has an increased risk likelihood of having a cancer (e.g., as compared to a subject not having a point mutation in a NTRK2 gene that results in the expression of a TrkB protein comprising a mutation at one or more amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M)).

Also provided are methods of assisting in the diagnosis of a cancer (e.g., any of the cancers described herein) in a subject that include determining that a subject having a cell that has at least one point mutation in a NTRK1, NTRK2, and/or NTRK3 gene (e.g., any of the NTRK1, NTRK2, and/or NTRK3 point mutations described herein) that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein) has an increased likelihood of having a cancer (e.g., as compared to a subject having a cell that does not have a point mutation in a NTRK1, NTRK2, and/or NTRK3 gene that results in the expression of a TrkA, TrkB, and/or TrkC protein comprising one or more mutations (e.g., any of the mutations in TrkA, TrkB, and/or TrkC described herein)).

Also provided are methods of assisting in the diagnosis of a cancer (e.g., any of the cancers described herein) in a subject that include determining that a subject having a cell that has at least one point mutation in a NTRK2 gene that results in the expression of a TrkB protein comprising a mutation at one or more (e.g., two, three, four, five, six, seven, eight, nine, ten, eleven, or twelve) amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M) has an increased likelihood of having a cancer (e.g., as compared to a subject having a cell that does not have a point mutation in a NTRK2 gene that results in the expression of a TrkB protein comprising a mutation at one or more amino acid position(s) selected from the group consisting of 240, 241, 242, 264, 314, 315, 401, 426, 427, 428, 440, and 689 (e.g., one or more of the mutations of M240I, N241D, E242K, I264M, A314E, A314G, A314V, L315F, G401A, G401E, G401R, T426I, G427S, R428Q, A440S, A440T, A440V, and V689M)).

Some embodiments further include confirming the diagnosis of a cancer in a subject determined to have an increased likelihood of having a cancer. Confirming the diagnosis of a cancer in a subject can include, e.g., performing additional laboratory tests (e.g., urine or blood tests, e.g., complete blood count), imaging tests (e.g., computerized tomography (CT), bone scan, magnetic resonance imaging (MRI), positron emission tomography (PET) scan, ultrasound, and X-ray), and/or physical examination to determine the presence of one or more symptoms of a cancer in the subject.

Some methods further include recording in the subject's clinical record (e.g., a computer readable medium) the subject's likelihood of having a cancer. The cancer can be any of the exemplary cancers described herein.

In some examples, the subject is identified as having been exposed to a significant level of carcinogen(s) (e.g., tobacco smoke, UVB radiation, and gamma irradiation). In some examples, the subject is suspected of having cancer, presents with one or more symptoms of cancer (e.g., any of the symptoms of cancer described herein), and/or has a family history of cancer.

In some examples, the step of determining whether a cancer cell in a sample obtained from the subject has at least one (e.g., two, three, four, five, six, seven, eight, nine, ten, eleven, or twelve) point mutation in a NTRK1, NTRK2, and/or NTRK3 gene that results in the expression of a TrkA, TrkB, and/or TrkC protein comprising one or more (e.g., two, three, four, five, six, seven, eight, nine, ten, eleven, or twelve) mutations (e.g., any of the mutations in TrkA, TrkB, and/or TrkC described herein), comprises performing an assay to determine the presence of the at least one point mutation in a NTRK1, NTRK2, and/or NTRK3 gene in a cancer cell in the sample (e.g., a biopsy sample) from the subject. Any of the assays described herein can be used to determine the presence of the at least one point mutation in a NTRK1, NTRK2, and/or NTRK3 gene. In addition, any of the kits provided herein can be used in an assay to determine the presence of the at least one point mutation in a NTRK1, NTRK2, and/or NTRK3 gene. In some examples, the assay includes sequencing a segment of a NTRK1, NTRK2, and/or NTRK3 gene including the at least one point mutation.

### Kits

Also provided herein are kits that include one or more (e.g., two, three, four, five, six, seven, eight, nine, ten, eleven, twelve, thirteen, fourteen, fifteen, sixteen, seventeen, or eighteen) probes that specifically hybridize to a segment of a NTRK1, NTRK2, and/or NTRK3 gene that comprises one of the point mutations described herein (e.g., any of the point mutations in NTRK1, NTRK2, and/or NTRK3 described herein). For example, the kits provided herein can include one or more probes that specifically hybridize to a segment of a NTRK2 gene that encodes a mutation at one of: amino acid positions 240, 241, 242, 264, 314, 315, 401, 426, 427, 428, 440, and 689 in TrkB protein (e.g., encodes a mutation selected from the group of: M240I, N241D, E242K, I264M, A314E, A314G, A314V, L315F, G401A, G401E, G401R, T426I, G427S, R428Q, A440S, A440T, A440V, and V689M in a TrkB protein).

Each of the one or more probes can have a length of 8, 9, 10, 11, 12, 13, 14, 15, 16, 17, 18, 19, 20, 21, 22, 23, 24, 25, 26, 27, 28, 29, 30, 31, 32, 33, 34, or 35 nucleotides. In some embodiments, the one or more probes include a detectable label (e.g., a fluorophore, a quencher, a radioisotope, or a metal). In some embodiments, the one or more probes can be covalently attached to a substrate (e.g., a film, a plate, or a bead).

### Numbered Paragraphs

The following numbered paragraphs, which are not the Claims, further describe the invention:
1. A method of treating a subject having a cancer, the method comprising:
   (a) identifying a subject having a cancer cell that has:
      at least one point mutation in a NTRK1 gene that results in the expression of a TrkA protein comprising a mutation at one or more amino acid position(s) selected from the group consisting of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750;
      at least one point mutation in a NTRK2 gene that results in the expression of a TrkB protein comprising a mutation at one or more amino acid position(s) selected from the group consisting of240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or
      at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810; and
   (b) administering to the identified subject a therapeutically effective amount of a Trk inhibitor.
2. A method of treating a subject identified as having a cancer cell that has: at least one point mutation in a NTRK1 gene that results in the expression of a TrkA protein comprising a mutation at one or more amino acid position(s) selected from the group consisting of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750; at least one point mutation in a NTRK2 gene that results in the expression of a TrkB protein comprising a mutation at one or more amino acid position(s) selected from the group consisting of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810, the method comprising administering to the subject a therapeutically effective amount of a Trk inhibitor.
3. A method of selecting a treatment for a subject having a cancer, the method comprising:
   (a) identifying a subject having a cancer cell that has:
      at least one point mutation in a NTRK1 gene that results in the expression of a TrkA protein comprising a mutation at one or more amino acid position(s) selected from the group consisting of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750;
      at least one point mutation in a NTRK2 gene that results in the expression of a TrkB protein comprising a mutation at one or more amino acid position(s) selected from the group consisting of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or
      at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810; and
   (b) selecting a treatment comprising a therapeutically effective amount of a Trk inhibitor for the identified subject.
4. A method of selecting a treatment for a subject having a cancer, the method comprising:
   selecting a treatment comprising a therapeutically effective amount of a Trk inhibitor for a subject identified as having a cancer cell that has:
   at least one point mutation in a NTRK1 gene that results in the expression of a TrkA protein comprising a mutation at one or more amino acid position(s) selected from the group consisting of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750;
   at least one point mutation in a NTRK2 gene that results in the expression of a TrkB protein comprising a mutation at one or more amino acid position(s) selected from the group consisting of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or
   at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810.
5. A method of determining the likelihood that a subject having a cancer will have a positive response to treatment with a Trk inhibitor, the method comprising:
   (a) determining whether a cancer cell in a sample obtained from the subject has:
      at least one point mutation in a NTRK1 gene that results in the expression of a TrkA protein comprising a mutation at one or more amino acid position(s) selected from the group consisting of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750;
      at least one point mutation in a NTRK2 gene that results in the expression of a TrkB protein comprising a mutation at one or more amino acid position(s) selected from the group consisting of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or
      at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810; and
   (b) determining that a subject having a cancer cell that has the at least one point mutation in a NTRK1 gene, the at least one point mutation in a NTRK2 gene, and/or the at least one point mutation in a NTRK3 gene, has an increased likelihood of having a positive response to treatment with a Trk inhibitor.
6. A method of determining the likelihood that a subject having cancer will have a positive response to treatment with a Trk inhibitor, the method comprising:
   determining that a subject having a cancer cell that has:
      at least one point mutation in a NTRK1 gene that results in the expression of a TrkA protein comprising a mutation at one or more amino acid position(s) selected from the group consisting of: 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750;
      at least one point mutation in a NTRK2 gene that results in the expression of a TrkB protein comprising a mutation at one or more amino acid position(s) selected from the group consisting of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or
      at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810,
   has an increased likelihood of having a positive response to treatment with a Trk inhibitor.
7. A method of predicting the efficacy of a Trk inhibitor in a subject having cancer, the method comprising:
   (a) determining whether a cancer cell in a sample obtained from the subject has:
      at least one point mutation in a NTRK1 gene that results in the expression of a TrkA protein comprising a mutation at one or more amino acid position(s) selected from the group consisting of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750;
      at least one point mutation in a NTRK2 gene that results in the expression of a TrkB protein comprising a mutation at one or more amino acid position(s) selected from the group consisting of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or
      at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810; and
   (b) determining that a Trk inhibitor is more likely to be effective in a subject having a cancer cell in a sample obtained from the subject that has the at least one point mutation in a NTRK1 gene, the at least one point mutation in a NTRK2 gene, and/or the at least one point mutation in a NTRK3 gene.
8. A method of predicting the efficacy of a Trk inhibitor in a subject having cancer, the method comprising:
   determining that a Trk inhibitor is more likely to be effective in a subject having a cancer cell in a sample obtained from the subject that has:
   at least one point mutation in a NTRK1 gene that results in the expression of a TrkA protein comprising a mutation at one or more amino acid position(s) selected from the group consisting of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750;
   at least one point mutation in a NTRK2 gene that results in the expression of a TrkB protein comprising a mutation at one or more amino acid position(s) selected from the group consisting of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or
   at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810.
9. The method of any one of paragraphs 1-8, wherein the cancer is selected from the group consisting of: adenocarcinoma, adrenal gland cortical carcinoma, adrenal gland neuroblastoma, anus squamous cell carcinoma, appendix adenocarcinoma, bladder urothelial carcinoma, bile duct adenocarcinoma, bladder carcinoma, bladder urothelial carcinoma, bone chordoma, bone marrow leukemia lymphocytic chronic, bone marrow leukemia non-lymphocytic acute myelocytic, bone marrow lymph proliferative disease, bone marrow multiple myeloma, bone sarcoma, brain astrocytoma, brain glioblastoma, brain medulloblastoma, brain meningioma, brain oligodendroglioma, breast adenoid cystic carcinoma, breast carcinoma, breast ductal carcinoma in situ, breast invasive ductal carcinoma, breast invasive lobular carcinoma, breast metaplastic carcinoma, cervix neuroendocrine carcinoma, cervix squamous cell carcinoma, colon adenocarcinoma, colon carcinoid tumor, duodenum adenocarcinoma, endometrioid tunor, esophagus adenocarcinoma, esophagus and stomach carcinoma, eye intraocular melanoma, eye intraocular squamous cell carcinoma, eye lacrimal duct carcinoma, fallopian tube serous carcinoma, gallbladder adenocarcinoma, gallbladder glomus tumor, gastroesophageal junction adenocarcinoma, head and neck adenoid cystic carcinoma, head and neck carcinoma, head and neck neuroblastoma, head and neck squamous cell carcinoma, kidney chromophore carcinoma, kidney medullary carcinoma, kidney renal cell carcinoma, kidney renal papillary carcinoma, kidney sarcomatoid carcinoma, kidney urothelial carcinoma, kidney carcinoma, leukemia lymphocytic, leukemia lymphocytic chronic, liver cholangiocarcinoma, liver hepatocellular carcinoma, liver carcinoma, lung adenocarcinoma, lung adenosquamous carcinoma, lung atypical carcinoid, lung carcinosarcoma, lung large cell neuroendocrine carcinoma, lung non-small cell lung carcinoma, lung sarcoma, lung sarcomatoid carcinoma, lung small cell carcinoma, lung small cell undifferentiated carcinoma, lung squamous cell carcinoma, upper aerodigestive tract squamous cell carcinoma, upper aerodigestive tract carcinoma, lymph node lymphoma diffuse large B cell, lymph node lymphoma follicular lymphoma, lymph node lymphoma mediastinal B-cell, lymph node lymphoma plasmablastic lung adenocarcinoma, lymphoma follicular lymphoma, lymphoma, non-Hodgkins, nasopharynx and paranasal sinuses undifferentiated carcinoma, ovary carcinoma, ovary carcinosarcoma, ovary clear cell carcinoma, ovary epithelial carcinoma, ovary granulosa cell tumor, ovary serous carcinoma, pancreas carcinoma, pancreas ductal adenocarcinoma, pancreas neuroendocrine carcinoma, peritoneum mesothelioma, peritoneum serous carcinoma, placenta choriocarcinoma, pleura mesothelioma, prostate acinar adenocarcinoma, prostate carcinoma, rectum adenocarcinoma, rectum squamous cell carcinoma, skin adnexal carcinoma, skin basal cell carcinoma, skin melanoma, skin Merkel cell carcinoma, skin squamous cell carcinoma, small intestine adenocarcinoma, small intestine gastrointestinal stromal tumors (GISTs), large intestine/colon carcinoma, large intestine adenocarcinoma, soft tissue angiosarcoma, soft tissue Ewing sarcoma, soft tissue hemangioendothelioma, soft tissue inflammatory myofibroblastic tumor, soft tissue leiomyosarcoma, soft tissue liposarcoma, soft tissue neuroblastoma, soft tissue paraganglioma, soft tissue perivascular epitheliod cell tumor, soft tissue sarcoma, soft tissue synovial sarcoma, stomach adenocarcinoma, stomach adenocarcinoma diffuse-type, stomach adenocarcinoma intestinal type, stomach adenocarcinoma intestinal type, stomach leiomyosarcoma, thymus carcinoma, thymus thymoma lymphocytic, thyroid papillary carcinoma, unknown primary adenocarcinoma, unknown primary carcinoma, unknown primary malignant neoplasm, lymphoid neoplasm, unknown primary melanoma, unknown primary sarcomatoid carcinoma, unknown primary squamous cell carcinoma, unknown undifferentiated neuroendocrine carcinoma, unknown primary undifferentiated small cell carcinoma, uterus carcinosarcoma, uterus endometrial adenocarcinoma, uterus endometrial adenocarcinoma endometrioid, uterus endometrial adenocarcinoma papillary serous, and uterus leiomyosarcoma.
10. The method of any one of paragraphs 1-9, wherein the subject is previously identified or diagnosed as having the cancer.
11. The method of any one of paragraphs 1, 3, 5, and 7, wherein the step of identifying a subject having a cancer cell that has the at least one point mutation in a NTRK1 gene, the at least one point mutation in a NTRK2 gene, and/or the at least one point mutation in a NTRK3 gene comprises performing an assay to determine the presence of the at least one point mutation in a NTRK1 gene, the at least one point mutation in a NTRK2 gene, and/or the at least one point mutation in a NTRK3 gene, in a cancer cell in a sample from the subject.
12. The method of paragraph 11, wherein the assay is selected from the group consisting of: denaturing gradient gel electrophoresis (DGGE), temperature gradient gel electrophoresis (TGGE), temperature gradient capillary electrophoresis, a single strand conformational polymorphism assay, a molecular beacon assay, a dynamic hybridization assay, a PCR-based assay, and denaturing high performance liquid chromatography.
13. The method of paragraph 11, wherein the assay comprises sequencing a segment of the NTRK1 gene comprising the at least one point mutation, a segment of the NTRK2 gene comprising the at least one point mutation, and/or a segment of the NTRK3 gene comprising the at least one point mutation.
14. The method of any one of paragraphs 1-13, wherein the Trk inhibitor a crystalline form of the compound of Formula I: 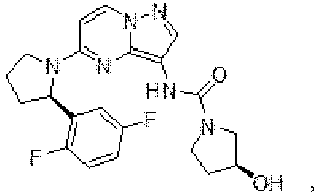 or a hydrogen sulfate salt thereof.
15. The method of paragraph 3 or 4, further comprising:
   administering a therapeutically effective amount of the selected treatment to the identified subject.
16. The method of paragraph 3, 4, and 15, further comprising:
   recording the selected treatment in the identified subject's clinical record.
17. The method of paragraph 16, wherein the subject's clinical record is a computer readable medium.
18. The method of paragraph 5 or 6, further comprising:
   administering a therapeutically effective amount of a Trk inhibitor to a subject determined to have an increased likelihood of having a positive response to treatment with a Trk inhibitor.
19. A method of determining a subject's risk for developing a cancer, the method comprising:
   (a) determining whether a cell in a sample obtained from the subject has:
      at least one point mutation in a NTRK1 gene that results in the expression of a TrkA protein comprising a mutation at one or more amino acid position(s) selected from the group consisting of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750;
      at least one point mutation in a NTRK2 gene that results in the expression of a TrkB protein comprising a mutation at one or more amino acid position(s) selected from the group consisting of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or
      at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810; and
   (b) identifying a subject having a cell that has the at least one point mutation in a NTRK1 gene, the at least one point mutation in a NTRK2 gene, and/or the at least one point mutation in a NTRK3 gene as having an increased likelihood of developing a cancer.
20. A method of determining a subject's risk for developing a cancer, the method comprising:
   identifying a subject having a cell that has:
      at least one point mutation in a NTRK1 gene that results in the expression of a TrkA protein comprising a mutation at one or more amino acid position(s) selected from the group consisting of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750;
      at least one point mutation in a NTRK2 gene that results in the expression of a TrkB protein comprising a mutation at one or more amino acid position(s) selected from the group consisting of: 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or
      at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810;
   as having an increased likelihood of developing a cancer.
21. A method of assisting in the diagnosis of a cancer in a subject, the method comprising:
   (a) determining whether a cell in a sample obtained from the subject has:
      at least one point mutation in a NTRK1 gene that results in the expression of a TrkA protein comprising a mutation at one or more amino acid position(s) selected from the group consisting of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750;
      at least one point mutation in a NTRK2 gene that results in the expression of a TrkB protein comprising a mutation at one or more amino acid position(s) selected from the group consisting of 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or
      at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810; and
   (b) determining that a subject having a cell that has the at least one point mutation in a NTRK1 gene, the at least one point mutation in a NTRK2 gene, and/or the at least one point mutation in a NTRK3 gene, has an increased likelihood of having a cancer.
22. A method of assisting in the diagnosis of a cancer in a subject, the method comprising:
   determining that a subject having a cell that has:
      at least one point mutation in a NTRK1 gene that results in the expression of a TrkA protein comprising a mutation at one or more amino acid position(s) selected from the group consisting of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750;
      at least one point mutation in a NTRK2 gene that results in the expression of a TrkB protein comprising a mutation at one or more amino acid position(s) selected from the group consisting of240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805; and/or
      at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810,
   has an increased likelihood of having a cancer.
23. The method of any one of paragraphs 19-22, wherein the subject is identified as having been exposed to a significant level of carcinogen(s).
24. The method of any one of paragraphs 19-22, wherein the subject is suspected of having a cancer.
25. The methods of any one of paragraphs 19-22, wherein the subject has one or more symptoms of cancer.
26. The method of any one of paragraphs 19-25, wherein the cancer is selected from the group consisting of: adenocarcinoma, adrenal gland cortical carcinoma, adrenal gland neuroblastoma, anus squamous cell carcinoma, appendix adenocarcinoma, bladder urothelial carcinoma, bile duct adenocarcinoma, bladder carcinoma, bladder urothelial carcinoma, bone chordoma, bone marrow leukemia lymphocytic chronic, bone marrow leukemia non-lymphocytic acute myelocytic, bone marrow lymph proliferative disease, bone marrow multiple myeloma, bone sarcoma, brain astrocytoma, brain glioblastoma, brain medulloblastoma, brain meningioma, brain oligodendroglioma, breast adenoid cystic carcinoma, breast carcinoma, breast ductal carcinoma in situ, breast invasive ductal carcinoma, breast invasive lobular carcinoma, breast metaplastic carcinoma, cervix neuroendocrine carcinoma, cervix squamous cell carcinoma, colon adenocarcinoma, colon carcinoid tumor, duodenum adenocarcinoma, endometrioid tumor, esophagus adenocarcinoma, esophagus and stomach carcinoma, eye intraocular melanoma, eye intraocular squamous cell carcinoma, eye lacrimal duct carcinoma, fallopian tube serous carcinoma, gallbladder adenocarcinoma, gallbladder glomus tumor, gastroesophageal junction adenocarcinoma, head and neck adenoid cystic carcinoma, head and neck carcinoma, head and neck neuroblastoma, head and neck squamous cell carcinoma, kidney chromophore carcinoma, kidney medullary carcinoma, kidney renal cell carcinoma, kidney renal papillary carcinoma, kidney sarcomatoid carcinoma, kidney urothelial carcinoma, kidney carcinoma, leukemia lymphocytic, leukemia lymphocytic chronic, liver cholangiocarcinoma, liver hepatocellular carcinoma, liver carcinoma, lung adenocarcinoma, lung adenosquamous carcinoma, lung atypical carcinoid, lung carcinosarcoma, lung large cell neuroendocrine carcinoma, lung non-small cell lung carcinoma, lung sarcoma, lung sarcomatoid carcinoma, lung small cell carcinoma, lung small cell undifferentiated carcinoma, lung squamous cell carcinoma, upper aerodigestive tract squamous cell carcinoma, upper aerodigestive tract carcinoma, lymph node lymphoma diffuse large B cell, lymph node lymphoma follicular lymphoma, lymph node lymphoma mediastinal B-cell, lymph node lymphoma plasmablastic lung adenocarcinoma, lymphoma follicular lymphoma, lymphoma, non-Hodgkins, nasopharynx and paranasal sinuses undifferentiated carcinoma, ovary carcinoma, ovary carcinosarcoma, ovary clear cell carcinoma, ovary epithelial carcinoma, ovary granulosa cell tumor, ovary serous carcinoma, pancreas carcinoma, pancreas ductal adenocarcinoma, pancreas neuroendocrine carcinoma, peritoneum mesothelioma, peritoneum serous carcinoma, placenta choriocarcinoma, pleura mesothelioma, prostate acinar adenocarcinoma, prostate carcinoma, rectum adenocarcinoma, rectum squamous cell carcinoma, skin adnexal carcinoma, skin basal cell carcinoma, skin melanoma, skin Merkel cell carcinoma, skin squamous cell carcinoma, small intestine adenocarcinoma, small intestine gastrointestinal stromal tumors (GISTs), large intestine/colon carcinoma, large intestine adenocarcinoma, soft tissue angiosarcoma, soft tissue Ewing sarcoma, soft tissue hemangioendothelioma, soft tissue inflammatory myofibroblastic tumor, soft tissue leiomyosarcoma, soft tissue liposarcoma, soft tissue neuroblastoma, soft tissue paraganglioma, soft tissue perivascular epitheliod cell tumor, soft tissue sarcoma, soft tissue synovial sarcoma, stomach adenocarcinoma, stomach adenocarcinoma diffuse-type, stomach adenocarcinoma intestinal type, stomach adenocarcinoma intestinal type, stomach leiomyosarcoma, thymus carcinoma, thymus thymoma lymphocytic, thyroid papillary carcinoma, unknown primary adenocarcinoma, unknown primary carcinoma, unknown primary malignant neoplasm, lymphoid neoplasm, unknown primary melanoma, unknown primary sarcomatoid carcinoma, unknown primary squamous cell carcinoma, unknown undifferentiated neuroendocrine carcinoma, unknown primary undifferentiated small cell carcinoma, uterus carcinosarcoma, uterus endometrial adenocarcinoma, uterus endometrial adenocarcinoma endometrioid, uterus endometrial adenocarcinoma papillary serous, and uterus leiomyosarcoma.
27. The method of paragraph 21 or 23, wherein the step of determining whether a cell in a sample obtained from the subject has the at least one point mutation in a NTRK1 gene, the at least one point mutation in a NTRK2 gene, and/or the at least one point mutation in a NTRK3 gene, comprises performing an assay to determine the presence of the at least one point mutation in a NTRK1 gene, the presence of the at least one point mutation in a NTRK2 gene, and/or the presence of the at least one point mutation in a NTRK3 gene, in a cell in the sample.
28. The method of paragraph 27, wherein the assay is selected from the group consisting of: denaturing gradient gel electrophoresis (DGGE), temperature gradient gel electrophoresis (TGGE), temperature gradient capillary electrophoresis, a single strand conformational polymorphism assay, a molecular beacon assay, a dynamic hybridization assay, a PCR-based assay, denaturing high performance liquid chromatography.
29. The method of paragraph 27, wherein the assay comprises sequencing a segment of the NTRK1 gene comprising the at least one point mutation, a segment of the NTRK2 gene comprising the at least one point mutation, and/or a segment of the NTRK3 gene comprising the at least one point mutation.
30. The method of paragraph 22, further comprising confirming the diagnosis of a cancer in a subject determined to have an increased likelihood of having a cancer.
31. The method of any one of paragraphs 1-30, wherein:
   the TrkA protein comprises a mutation at one or more amino acid positions selected from the group consisting of S241F, S241H, S241Y, R314G, R314H, R314L, R314P, N318S, G319S, S320F, V321M, I510T, V511M, L512F, L512R, S552R, A553T, R554P, R554Q, R554W, A636E, A636T, A636V, G637E, G637W, M638V, R649L, R649W, R654C, R654H, N655Y, D679N, D679Y, Y680H, T687I, M688I, L689M, P690H, R692C, R692H, P695S, P696L, E697K, E747K, R748L, R748Q, R748W, P749Q, R750C, R750H, and R750L;
   the TrkB protein comprises a mutation at one or more amino acid position(s) selected from the group consisting of M240I, N241D, E242K, R251G, R251K, I252V, S256L, S257F, D258N, I264M, A314E, A314G, A314V, L315F, G401A, G401E, G401R, T426I, G427S, R428Q, A440S, A440T, A440V, R598C, R598S, K599M, D600H, H602N, R603S, L664M, T665M, T665S, Q666L, Q666R, A677T, A678T, A678V, G679D, M680I, V689M, R691C, D692N, F746I, T747M, T748M, E749K, Q784H, G785V, R786Q, V787F, L788M, Q789E, G804E, C805R, and C805Y; and/or
   the TrkC protein comprises a mutation at one or more amino acid position(s) selected from the group consisting of V221I, R222Q, E223D, D242N, W243C, I244T, T269A, T269M, T270M, T270Q, T270V, V271L, V271M, E276D, D277E, D277G, D277N, T281I, T281P, T282M, T283A, T283K, T283M, S296I, S296R, V297I, V325M, R326C, R326G, R326H, R326L, R326P, N328S, P329N, P329S, P330Q, E344G, E344V, S345F, K346N, H349Y, V350E, E351D, Y353F, Q354K, D537E, D537Y, I538N, V539M, L540M, G545C, G545D, G550R, K551E, L560V, P562L, P562Q, P562R, P562T, K575E, D576N, P577S, P582Q, P582W, K583%, D584E, D584N, V601A, V601I, K602R, F603L, Y604F, Y604H, Y604N, C607F, G608C, G608E, G608S, D609G, D609H, D609N, D609V, G610R, P612A, P612L, P612S, P612T, D624Y, L625M, N626K, K627N, K627R, F628L, L629F, L629I, P634L, P634T, D635H, A636E, A636V, M637I, M637K, M637V, E650V, L651P, G652R, G652V, L653F, L653P, H677Y, R678Q, D679G, D679N, V687A, V687I, G688R, A689E, A689V, Y705N, S706I, T707M, D708N, P715L, P715S, S716Y, G717R, T730N, M731I, M731L, L732I, P738H, P738S, Y744F, R745P, R745W, K746R, K746T, G786C, G786S, R787C, R787H, R787S, P796L, P796S, D801N, Q808H, R809W, and E810K.
32. A kit comprising:
   one or more probes that each specifically hybridize to:
   a segment of a NTRK1 gene that encodes a mutation at one of amino acid positions 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750 in a TrkA protein;
   a segment of a NTRK2 gene that encodes a mutation at one of amino acid positions 240, 241, 242, 251, 252, 256, 257, 258, 264, 314, 315, 401, 426, 427, 428, 440, 598, 599, 600, 602, 603, 664, 665, 666, 677, 678, 679, 680, 689, 691, 692, 746, 747, 748, 749, 784, 785, 786, 787, 788, 789, 804, and 805 in TrkB protein; or
   a segment of a NTRK3 gene that encodes a mutation at one of amino acid positions 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 608, 609, 610, 612, 624, 625, 626, 627, 628, 629, 634, 635, 636, 637, 650, 651, 652, 653, 677, 678, 679, 687, 688, 689, 705, 706, 707, 708, 715, 716, 717, 730, 731, 732, 738, 744, 745, 746, 786, 787, 796, 801, 808, 809, and 810 in a TrkC protein.
33. The kit of paragraph 32, wherein the kit comprises one or more probes that each specifically hybridize to:
   a segment of a NTRK1 gene that encodes a mutation selected from the group consisting of S241F, S241H, S241Y, R314G, R314H, R314L, R314P, N318S, G319S, S320F, V321M, I510T, V511M, L512F, L512R, S552R, A553T, R554P, R554Q, R554W, A636E, A636T, A636V, G637E, G637W, M638V, R649L, R649W, R654C, R654H, N655Y, D679N, D679Y, Y680H, T687I, M688I, L689M, P690H, R692C, R692H, P695S, P696L, E697K, E747K, R748L, R748Q, R748W, P749Q, R750C, R750H, and R750L in a TrkA protein;
   a segment of a NTRK2 gene that encodes a mutation selected from the group consisting of M240I, N241D, E242K, R251G, R251K, I252V, S256L, S257F, D258N, I264M, A314E, A314G, A314V, L315F, G401A, G401E, G401R, T426I, G427S, R428Q, A440S, A440T, A440V, R598C, R598S, K599M, D600H, H602N, R603S, L664M, T665M, T665S, Q666L, Q666R, A677T, A678T, A678V, G679D, M680I, V689M, R691C, D692N, F746I, T747M, T748M, E749K, Q784H, G785V, R786Q, V787F, L788M, Q789E, G804E, C805R, and C805Y in TrkB protein; or
   a segment of a NTRK3 gene that encodes a mutation selected from the group consisting of V221I, R222Q, E223D, D242N, W243C, I244T, T269A, T269M, T270M, T270Q, T270V, V271L, V271M, E276D, D277E, D277G, D277N, T281I, T281P, T282M, T283A, T283K, T283M, S296I, S296R, V297I, V325M, R326C, R326G, R326H, R326L, R326P, N328S, P329N, P329S, P330Q, E344G, E344V, S345F, K346N, H349Y, V350E, E351D, Y353F, Q354K, D537E, D537Y, I538N, V539M, L540M, G545C, G545D, G550R, K551E, L560V, P562L, P562Q, P562R, P562T, K575E, D576N, P577S, P582Q, P582W, K583%, D584E, D584N, V601A, V601I, K602R, F603L, Y604F, Y604H, Y604N, C607F, G608C, G608E, G608S, D609G, D609H, D609N, D609V, G610R, P612A, P612L, P612S, P612T, D624Y, L625M, N626K, K627N, K627R, F628L, L629F, L629I, P634L, P634T, D635H, A636E, A636V, M637I, M637K, M637V, E650V, L651P, G652R, G652V, L653F, L653P, H677Y, R678Q, D679G, D679N, V687A, V687I, G688R, A689E, A689V, Y705N, S706I, T707M, D708N, P715L, P715S, S716Y, G717R, T730N, M731I, M731L, L732I, P738H, P738S, Y744F, R745P, R745W, K746R, K746T, G786C, G786S, R787C, R787H, R787S, P796L, P796S, D801N, Q808H, R809W, and E810K in a TrkC protein.
34. The kit of paragraph 32 or 33, wherein the one or more probes are labeled with a detectable probe.
35. The kit of paragraphs 32-34, wherein the one or more probes are covalently attached to a substrate.
36. The kit of paragraph 35, wherein the substrate is a film, a plate, or a bead.

The invention is further described in the following examples, which do not limit the scope of the invention described in the claims.

### EXAMPLES

### Example 1. Identification of TRK Point Mutations in Cancer Biopsy Samples

A set of experiments were performed to identify point mutations in NTRK1, NTRK2, and NTRK3 genes in biopsy samples from patients having a variety of different cancers.

### Methods

One thousand eight hundred and twenty three mutations in NTRK1, NTRK2, and NTRK3 genes were identified in next-generation sequencing data from 42,155 tumor biopsy samples. Mutations that were synonymous, lead to loss of a canonical stop codon, cause truncation of the kinase domain, or are mapped to an alternative transcript that does not contain a full kinase domain were filtered out. All mutations within the given, preceding, and subsequent codons were clustered together. Duplicate clusters were then removed.

The mutation clusters were ranked for follow-up by combining several component scores into a single score via multiplication. The hotspot score captures the prevalence of the mutation cluster (size of the cluster/size of the largest cluster). The domain score captures the functional relevance of the protein region harboring the culture to oncogenesis (kinase domain = 1, Ig-like domains = 0.9, leucine-rich repeats = 0.3, and none = 0.05). The co-alteration score captures the likelihood that the mutation cluster contains drivers based on the presence of co-occurring mutations in other oncogenes. The exac score captures the rarity of the mutations in a large collection of germline samples, as a measure of relevance to oncogenesis. The convervation score captures how conserved the region of the protein is across placental mammals, as a measure of functional relevance.

Expression filtering was also used, after clustering, to confirm that at least one mutation in a given cluster be associated with expression of the relevant NTRK gene above background.

Structural modeling was performed by mapping specific amino acid residues onto a TrkB protein crystal structure (PDB entry 4ASZ) and a judgment was made on activation potential based on the structure and knowledge of kinase regulation.

### Results

Point mutations in each of NTRK1, NTRK2, and NTRK3 were detected in biopsy samples from different patients having a variety of different cancers. Table 1 lists the point mutations identified in NTRK1, Table 2 lists the point mutations identified in NTRK2, Table 3 lists the point mutations identified in NTRK2 that have been confirmed to be expressed in cancer cells in the biopsy sample, and Table 4 lists the point mutations identified in NTRK3.

**Table 1. Detected TrkA Protein Mutations Resulting from NTRK1 Point Mutations Detected in Cancer Biopsy Samples**

| Mutated Samples | Mutations Ref.Transcript/Protein | Domain | Hotspot Score | Domain Score | co-Alteration Score | Exac Score | Conservation Score | Total Score | Disease |
|---|---|---|---|---|---|---|---|---|---|
| 4 | R3P | | 0.2000 | 0.0500 | 1.0000 | 1.0000 | 0.4693 | 0.0047 | Breast Carcinoma; Lung Squamous Cell Carcinoma; Unknown Primary Carcinoma |
| | R3Q | | | | | | | | |
| | G4A | | | | | | | | |
| | NM_002529 | | | | | | | | |
| | NP_002520 | | | | | | | | |
| 8 | A5T | | 0.4000 | 0.0500 | 0.0000 | 0.9016 | 0.3090 | 0.0000 | Colon Adenocarcinoma; Lung Adenocarcinoma; Skin Melanoma |
| | A5V | | | | | | | | |
| | NM_001007792 | | | | | | | | |
| | NP_001007793 | | | | | | | | |
| 3 | R6W | | 0.1500 | 0.0500 | 1.0000 | 0.0000 | 0.6577 | 0.0000 | Prostate Carcinoma; Uterus Leiomyosarcoma; Peritoneum Mesothelioma |
| | R7S | | | | | | | | |
| | G8E | | | | | | | | |
| | NM_002529 | | | | | | | | |
| | NP_002520 | | | | | | | | |
| 3 | A10E | | 0.1500 | 0.0500 | 1.0000 | 1.0000 | 0.0000 | 0.0000 | Colon Adenocarcinoma; Lung Squamous Cell Carcinoma |
| | A10T | | | | | | | | |
| | NM_001007792 | | | | | | | | |
| | NP_001007793 | | | | | | | | |
| 1 | V13I | | 0.0500 | 0.0500 | 1.0000 | 0.9836 | 0.4192 | 0.0000 | Lung Adenocarcinoma |
| | NM_001007792 | | | | | | | | |
| | NP_001007793 | | | | | | | | |
| 1 | W15C | | 0.0500 | 0.0500 | 1.0000 | 1.0000 | 0.9187 | 0.0023 | Lung Squamous Cell Carcinoma |
| | NM_002529 | | | | | | | | |
| | NP_002520 | | | | | | | | |
| 1 | A17T | | 0.0500 | 0.0500 | 1.0000 | 1.0000 | 0.0000 | 0.0000 | Unknown Primary Carcinoma |
| | NM_002529 | | | | | | | | |
| | NP_002520 | | | | | | | | |
| 1 | T18M | | 0.0500 | 0.0500 | 1.0000 | 1.0000 | 0.9970 | 0.0022 | Liver Hepatocellular Carcinoma |
| | NM_001007792 | | | | | | | | |
| | NP_001007793 | | | | | | | | |
| 1 | G20D | | 0.0500 | 0.0500 | 1.0000 | 0.9672 | 0.9968 | 0.0000 | Kidney Renal Cell Carcinoma |
| | NM_002529 | | | | | | | | |
| | NP_002520 | | | | | | | | |
| 1 | W22R | | 0.0500 | 0.0500 | 1.0000 | 1.0000 | 0.9909 | 0.0022 | Liver Hepatocellular Carcinoma |
| | NM_001007792 | | | | | | | | |
| | NP_001007793 | | | | | | | | |
| 4 | L22Q | | 0.2000 | 0.0500 | 1.0000 | 0.9344 | 0.9903 | 0.0000 | Lung Adenocarcinoma; Kidney Renal Cell Carcinoma; Bone Chordoma |
| | NM_002529 | | | | | | | | |
| | NP_002520 | | | | | | | | |
| 4 | A24S | | 0.2000 | 0.0500 | 1.0000 | 1.0000 | 0.7401 | 0.0000 | Colon Adenocarcinoma; Lung Adenocarcinoma; Stomach Adenocarcinoma |
| | W25C | | | | | | | | |
| | NM_002529 | | | | | | | | |
| | NP_002520 | | | | | | | | |
| 1 | S30P | | 0.0500 | 0.0500 | 1.0000 | 1.0000 | 0.9903 | 0.0000 | Breast Carcinoma |
| | NM_002529 | | | | | | | | |
| | NP_002520 | | | | | | | | |
| 1 | R311 | | 0.0500 | 0.0500 | 1.0000 | 1.0000 | 0.9463 | 0.0021 | Lung Adenocarcinoma |
| | NM_001007792 | | | | | | | | |
| | NP_001007793 | | | | | | | | |
| 3 | A33S | | 0.1500 | 0.0500 | 1.0000 | 0.9672 | 0.7928 | 0.0010 | Colon Adenocarcinoma; Breast Invasive Ductal Carcinoma; Soft Tissue Paraganglioma |
| | A33V | | | | | | | | |
| | A34T | | | | | | | | |
| | NM_002529 | | | | | | | | |
| | NP_002520 | | | | | | | | |
| 1 | L38W | | 0.0500 | 0.0500 | 1.0000 | 1.0000 | 0.9909 | 0.0022 | Kidney Renal Papillary Carcinoma |
| | NM_001007792 | | | | | | | | |
| | NP_001007793 | | | | | | | | |
| 2 | D38N | | 0.1000 | 0.0500 | 1.0000 | 0.9836 | 0.8478 | 0.0011 | Bladder Urothelial Carcinoma; Duodenum Adenocarcinoma |
| | A39S | | | | | | | | |
| | NM_002529 | | | | | | | | |
| | NP_002520 | | | | | | | | |
| 3 | C41W | | 0.1500 | 0.0500 | 0.0000 | 1.0000 | 0.7805 | 0.0000 | Lung Adenocarcinoma; Lung Small Cell Undifferentiated Carcinoma; Uterus Endometrial Adenocarcinoma |
| | P42T | | | | | | | | |
| | H43Q | | | | | | | | |
| | NM_002529 | | | | | | | | |
| | NP_002520 | | | | | | | | |
| 7 | R49G | | 0.3500 | 0.0500 | 1.0000 | 0.9180 | 0.6369 | 0.0000 | Breast Carcinoma; Unknown Primary Adenocarcinoma; Uterus Endometrial Adenocarcinoma Endometrioid |
| | R49P | | | | | | | | |
| | R49Q | | | | | | | | |
| | C50Y | | | | | | | | |
| | NM_002529 | | | | | | | | |
| | NP_002520 | | | | | | | | |
| 2 | R52L | | 0.1000 | 0.0500 | 1.0000 | 1.0000 | 0.0000 | 0.0000 | Unknown Primary Melanoma; Ovary Epithelial Carcinoma |
| | R52Q | | | | | | | | |
| | NM_002529 | | | | | | | | |
| | NP_002520 | | | | | | | | |
| 2 | A55D | | 0.1000 | 0.0500 | 1.0000 | 1.0000 | 0.8122 | 0.0000 | Pancreas Ductal Adenocarcinoma; Lymphoma Non-Hodgkins |
| | L56M | | | | | | | | |
| | NM_002529 | | | | | | | | |
| | NP_002520 | | | | | | | | |
| 1 | L59F | | 0.0500 | 0.0500 | 1.0000 | 1.0000 | 0.7258 | 0.0002 | Unknown Primary Adenocarcinoma |
| | NM_002529 | | | | | | | | |
| | NP_002520 | | | | | | | | |
| 6 | L62P | | 0.3000 | 0.0500 | 1.0000 | 0.8361 | 0.9893 | 0.0000 | Lung Adenocarcinoma; Colon Adenocarcinoma; Prostate Acinar Adenocarcinoma |
| | P63S | | | | | | | | |
| | NM_002529 | | | | | | | | |
| | NP_002520 | | | | | | | | |
| 1 | E66D | | 0.0500 | 0.0500 | 1.0000 | 1.0000 | 0.9958 | 0.0025 | Unknown Primary Carcinoma |
| | NM_002529 | | | | | | | | |
| | NP_002520 | | | | | | | | |
| 1 | T69I | | 0.0500 | 0.0500 | 1.0000 | 1.0000 | 0.9289 | 0.0002 | Lung Adenocarcinoma |
| | NM_002529 | | | | | | | | |
| | NP_002520 | | | | | | | | |
| 1 | L71I | | 0.0500 | 0.0500 | 1.0000 | 1.0000 | 0.9348 | 0.0023 | Lung Adenocarcinoma |
| | NM_002529 | | | | | | | | |
| | NP_002520 | | | | | | | | |
| 3 | E74K | | 0.1500 | 0.0500 | 1.0000 | 0.9836 | 0.9959 | 0.0024 | Colon Adenocarcinoma; Unknown Primary Melanoma; Kidney Sarcomatoid Carcinoma |
| | NM_002529 | | | | | | | | |
| | NP_002520 | | | | | | | | |
| 5 | L79Q | | 0.2500 | 0.0500 | 1.0000 | 0.8033 | 0.7826 | 0.0000 | Lung Adenocarcinoma; Ovary Serous Carcinoma; Bone Marrow Multiple Myeloma |
| | Q80R | | | | | | | | |
| | NM_002529 | | | | | | | | |
| | NP_002520 | | | | | | | | |
| 6 | R85C | | 0.3000 | 0.0500 | 0.1667 | 0.9016 | 0.1407 | 0.0002 | Soft Tissue Sarcoma; Lung Adenocarcinoma; Lung Non-Small Cell Lung Carcinoma |
| | D86N | | | | | | | | |
| | D86Y | | | | | | | | |
| | NM_002529 | | | | | | | | |
| | NP_002520 | | | | | | | | |
| 5 | R88K | | 0.2500 | 0.0500 | 1.0000 | 0.9508 | 0.8021 | 0.0055 | Lung Adenocarcinoma; Colon Adenocarcinoma; Unknown Primary Adenocarcinoma |
| | R88S | | | | | | | | |
| | G89S | | | | | | | | |
| | L90M | | | | | | | | |
| | L90del | | | | | | | | |
| | NM_002529 | | | | | | | | |
| | NP_002520 | | | | | | | | |
| 4 | G89S | LRR 1 | 0.2000 | 0.3000 | 1.0000 | 0.9180 | 0.7915 | 0.0418 | Lung Adenocarcinoma; Colon Adenocarcinoma; Unknown Primary Melanoma |
| | L90M | | | | | | | | |
| | L90del | | | | | | | | |
| | G91R | | | | | | | | |
| | NM_002529 | | | | | | | | |
| | NP_002520 | | | | | | | | |
| 4 | L90M | LRR 1 | 0.2000 | 0.3000 | 1.0000 | 0.9672 | 0.6363 | 0.0350 | Lung Adenocarcinoma; Colon Adenocarcinoma; Unknown Primary Undifferentiated Neuroendocrine Carcinoma |
| | L90del | | | | | | | | |
| | G91R | | | | | | | | |
| | E92K | | | | | | | | |
| | NM_002529 | | | | | | | | |
| | NP_002520 | | | | | | | | |
| 2 | L96V | LRR 1 | 0.1000 | 0.3000 | 1.0000 | 1.0000 | 0.9615 | 0.0268 | Breast Ductal Carcinoma in Situ; Unknown Primary Melanoma |
| | T97I | | | | | | | | |
| | NM_002529 | | | | | | | | |
| | NP_002520 | | | | | | | | |
| 3 | S101C | LRR 1 | 0.1500 | 0.3000 | 1.0000 | 0.9836 | 0.9908 | 0.0185 | Kidney Renal Cell Carcinoma; Unknown Primary Adenocarcinoma; Unknown Primary Undifferentiated Neuroendocrine Carcinoma |
| | S101N | | | | | | | | |
| | S101R | | | | | | | | |
| | NM_002529 | | | | | | | | |
| | NP_002520 | | | | | | | | |
| 1 | R104H | LRR 1 | 0.0500 | 0.3000 | 1.0000 | 0.9180 | 0.0000 | 0.0000 | Pleura Mesothelioma |
| | NM_002529 | | | | | | | | |
| | NP_002520 | | | | | | | | |
| 3 | V106M | LRR 1 | 0.1500 | 0.3000 | 1.0000 | 0.8033 | 0.9958 | 0.0335 | Ovary Serous Carcinoma; Lung Non-Small Cell Lung Carcinoma; Unknown Primary Squamous Cell Carcinoma |
| | A107V | | | | | | | | |
| | NM_002529 | | | | | | | | |
| | NP_002520 | | | | | | | | |
| 1 | A110V | LRR 1 | 0.0500 | 0.3000 | 1.0000 | 1.0000 | 0.9427 | 0.0126 | Skin Melanoma |
| | NM_002529 | | | | | | | | |
| | NP_002520 | | | | | | | | |
| 2 | H112Y | LRR 1 | 0.1000 | 0.3000 | 1.0000 | 1.0000 | 0.9309 | 0.0250 | Bone Marrow Multiple Myeloma; Lung Small Cell Carcinoma |
| | F113L | | | | | | | | |
| | NM_002529 | | | | | | | | |
| | NP_002520 | | | | | | | | |
| 5 | P115S | LRR 2 | 0.2500 | 0.3000 | 0.6000 | 0.9672 | 0.7729 | 0.0168 | Lung Adenocarcinoma; Colon Adenocarcinoma; Lung Squamous Cell Carcinoma |
| | R116L | | | | | | | | |
| | R116Q | | | | | | | | |
| | R116W | | | | | | | | |
| | L117P | | | | | | | | |
| | NM_002529 | | | | | | | | |
| | NP_002520 | | | | | | | | |
| 7 | R119C | LRR 2 | 0.3500 | 0.3000 | 0.0000 | 0.8525 | 0.1038 | 0.0000 | Breast Invasive Ductal Carcinoma; Lung Adenocarcinoma; Colon Adenocarcinoma |
| | R119H | | | | | | | | |
| | R119P | | | | | | | | |
| | NM_002529 | | | | | | | | |
| | NP_002520 | | | | | | | | |
| 5 | A126D | LRR 2 | 0.2500 | 0.3000 | 0.4000 | 1.0000 | 0.8130 | 0.0000 | Lung Adenocarcinoma; Colon Adenocarcinoma; Bone Marrow Multiple Myeloma |
| | A126P | | | | | | | | |
| | A126T | | | | | | | | |
| | NM_002529 | | | | | | | | |
| | NP_002520 | | | | | | | | |
| 1 | S129F | LRR 2 | 0.0500 | 0.3000 | 0.0000 | 1.0000 | 0.9955 | 0.0000 | Breast Carcinoma |
| | NM_002529 | | | | | | | | |
| | NP_002520 | | | | | | | | |
| 3 | W132F | LRR 2 | 0.1500 | 0.3000 | 1.0000 | 0.9672 | 0.9902 | 0.0000 | Lung Small Cell Undifferentiated Carcinoma; Unknown Primary Malignant Neoplasm; Unknown Primary Squamous Cell Carcinoma |
| | W132R | | | | | | | | |
| | NM_002529 | | | | | | | | |
| | NP_002520 | | | | | | | | |
| 1 | T134N | LRR 2 | 0.0500 | 0.3000 | 1.0000 | 0.9344 | 0.9955 | 0.0000 | Pleura Mesothelioma |
| | NM_002529 | | | | | | | | |
| | NP_002520 | | | | | | | | |
| 3 | L138H | | 0.1500 | 0.0500 | 0.3333 | 1.0000 | 0.9928 | 0.0025 | Lung Adenocarcinoma; Breast Carcinoma; Head and Neck Carcinoma |
| | S139F | | | | | | | | |
| | NM_002529 | | | | | | | | |
| | NP_002520 | | | | | | | | |
| 4 | E142K | | 0.2000 | 0.0500 | 1.0000 | 0.9344 | 0.9231 | 0.0000 | Breast Invasive Ductal Carcinoma; Brain Glioblastoma; Appendix Adenocarcinoma |
| | NM_002529 | | | | | | | | |
| | NP_002520 | | | | | | | | |
| 1 | G147E | | 0.0500 | 0.0500 | 1.0000 | 1.0000 | 0.9350 | 0.0023 | Unknown Primary Melanoma |
| | NM_002529 | | | | | | | | |
| | NP_002520 | | | | | | | | |
| 4 | P149A | LRRCT | 0.2000 | 0.3000 | 1.0000 | 0.9016 | 0.9577 | 0.0000 | Colon Adenocarcinoma; Stomach Adenocarcinoma; Gastroesophageal Junction Adenocarcinoma |
| | P149H | | | | | | | | |
| | L150P | | | | | | | | |
| | NM_002529 | | | | | | | | |
| | NP_002520 | | | | | | | | |
| 5 | A155V | LRRCT | 0.2500 | 0.3000 | 0.6000 | 0.9180 | 0.1806 | 0.0000 | Colon Adenocarcinoma; Pancreas Ductal Adenocarcinoma; Gastroesophageal Junction Adenocarcinoma |
| | L156Q | | | | | | | | |
| | R157C | | | | | | | | |
| | R157L | | | | | | | | |
| | R157P | | | | | | | | |
| | NM_002529 | | | | | | | | |
| | NP_002520 | | | | | | | | |
| 5 | L156Q | LRRCT | 0.2500 | 0.3000 | 1.0000 | 0.9180 | 0.1924 | 0.0000 | Brain Glioblastoma; Pancreas Ductal Adenocarcinoma; Gastroesophageal Junction Adenocarcinoma |
| | R157C | | | | | | | | |
| | R157L | | | | | | | | |
| | R157P | | | | | | | | |
| | W158R | | | | | | | | |
| | NM_002529 | | | | | | | | |
| | NP_002520 | | | | | | | | |
| 2 | R161C | LRRCT | 0.1000 | 0.3000 | 1.0000 | 0.9836 | 0.8626 | 0.0177 | Stomach Adenocarcinoma Intestinal Type; Placenta Choriocarcinoma |
| | R161P | | | | | | | | |
| | NM_002529 | | | | | | | | |
| | NP_002520 | | | | | | | | |
| 5 | E165D | LRRCT | 0.2500 | 0.3000 | 0.4000 | 0.9672 | 0.7651 | 0.0136 | Colon Adenocarcinoma; Skin Melanoma; Lung Small Cell Undifferentiated Carcinoma |
| | E165del | | | | | | | | |
| | G166R | | | | | | | | |
| | L167M | | | | | | | | |
| | NM_002529 | | | | | | | | |
| | NP_002520 | | | | | | | | |
| 7 | G169E | LRRCT | 0.3500 | 0.3000 | 0.0000 | 0.0000 | 0.7231 | 0.0000 | Colon Adenocarcinoma; Head and Neck Squamous Cell Carcinoma; Skin Melanoma |
| | G169R | | | | | | | | |
| | V170L | | | | | | | | |
| | P171S | | | | | | | | |
| | P171T | | | | | | | | |
| | NM_002529 | | | | | | | | |
| | NP_002520 | | | | | | | | |
| 1 | G179R | LRRCT | 0.0500 | 0.3000 | 1.0000 | 1.0000 | 0.7376 | 0.0111 | Skin Basal Cell Carcinoma Breast Carcinoma; Lung Non-Small Cell Lung Carcinoma; Unknown Primary Adenocarcinoma |
| | NM_002529 | | | | | | | | |
| | NP_002520 | | | | | | | | |
| 3 | H185N | LRRCT | 0.1500 | 0.3000 | 1.0000 | 0.9508 | 0.8078 | 0.0115 | |
| | M186T | | | | | | | | |
| | NM_002529 | | | | | | | | |
| | NP_002520 | | | | | | | | |
| 1 | A189V | LRRCT | 0.0500 | 0.3000 | 1.0000 | 1.0000 | 0.6980 | 0.0052 | Soft Tissue Inflammatory Myofibroblastic Tumor |
| | NM_002529 | | | | | | | | |
| | NP_002520 | | | | | | | | |
| 1 | V193L | LRRCT | 0.0500 | 0.3000 | 0.0000 | 1.0000 | 0.3208 | 0.0000 | Lung Adenocarcinoma |
| | NM_002529 | | | | | | | | |
| | NP_002520 | | | | | | | | |
| 1 | T195M | Ig-like C2-type 1 | 0.0500 | 0.9000 | 1.0000 | 0.9016 | 0.0000 | 0.0000 | Lymph Node Lymphoma Plasmablastic |
| | NM_002529 | | | | | | | | |
| | NP_002520 | | | | | | | | |
| 3 | K197R | Ig-like C2-type 1 | 0.1500 | 0.9000 | 1.0000 | 0.9836 | 0.2979 | 0.0000 | Lung Adenocarcinoma; Unknown Primary Melanoma; Ovary Carcinosarcoma |
| | V198F | | | | | | | | |
| | NM_002529 | | | | | | | | |
| | NP_002520 | | | | | | | | |
| 14 | P201H | Ig-like C2-type 1 | 0.7000 | 0.9000 | 1.0000 | 0.7869 | 0.9556 | 0.0000 | Lung Adenocarcinoma; Breast Carcinoma; Brain Glioblastoma |
| | P201del | | | | | | | | |
| | N202S | | | | | | | | |
| | NM_002529 | | | | | | | | |
| | NP_002520 | | | | | | | | |
| 1 | D206N | Ig-like C2-type 1 | 0.0500 | 0.9000 | 1.0000 | 1.0000 | 0.3748 | 0.0169 | Skin Melanoma |
| | NM_002529 | | | | | | | | |
| | NP_002520 | | | | | | | | |
| 2 | G208E | Ig-like C2-type 1 | 0.1000 | 0.9000 | 0.0000 | 1.0000 | 0.9963 | 0.0000 | Lung Adenocarcinoma; Brain Glioblastoma |
| | G208R | | | | | | | | |
| | NM_002529 | | | | | | | | |
| | NP_002520 | | | | | | | | |
| 9 | D210N | Ig-like C2-type 1 | 0.4500 | 0.9000 | 1.0000 | 0.9180 | 0.0000 | 0.0000 | Breast Carcinoma; Bone Marrow Multiple Myeloma; Skin Melanoma |
| | V211E | | | | | | | | |
| | V211L | | | | | | | | |
| | L212V | | | | | | | | |
| | NM_002529 | | | | | | | | |
| | NP_002520 | | | | | | | | |
| 10 | R214Q | Ig-like C2-type 1 | 0.5000 | 0.9000 | 1.0000 | 0.7377 | 0.0000 | 0.0000 | Breast Carcinoma; Lung Adenocarcinoma; Ovary Serous Carcinoma |
| | R214W | | | | | | | | |
| | NM_002529 | | | | | | | | |
| | NP_002520 | | | | | | | | |
| 8 | R220W | Ig-like C2-type 1 | 0.4000 | 0.9000 | 1.0000 | 0.9344 | 0.2129 | 0.0000 | Lung Adenocarcinoma; Breast Carcinoma; Breast Invasive Ductal Carcinoma |
| | G221D | | | | | | | | |
| | G221V | | | | | | | | |
| | L222Q | | | | | | | | |
| | NM_002529 | | | | | | | | |
| | NP_002520 | | | | | | | | |
| 6 | G221D | Ig-like C2-type 1 | 0.3000 | 0.9000 | 1.0000 | 1.0000 | 0.0000 | 0.0000 | Lung Adenocarcinoma; Breast Carcinoma; Breast Invasive Ductal Carcinoma |
| | G221V | | | | | | | | |
| | L222Q | | | | | | | | |
| | E223Q | | | | | | | | |
| | NM_002529 | | | | | | | | |
| | NP_002520 | | | | | | | | |
| 3 | L222Q | Ig-like C2-type 1 | 0.1500 | 0.9000 | 1.0000 | 1.0000 | 0.0518 | 0.0023 | Lung Adenocarcinoma |
| | E223Q | | | | | | | | |
| | Q224H | | | | | | | | |
| | NM_002529 | | | | | | | | |
| | NP_002520 | | | | | | | | |
| 3 | E223Q | Ig-like C2-type 1 | 0.1500 | 0.9000 | 1.0000 | 1.0000 | 0.0801 | 0.0108 | Lung Adenocarcinoma; Soft Tissue Neuroblastoma |
| | Q224H | | | | | | | | |
| | A225S | | | | | | | | |
| | NM_002529 | | | | | | | | |
| | NP_002520 | | | | | | | | |
| 5 | Q224H | Ig-like C2-type 1 | 0.2500 | 0.9000 | 1.0000 | 0.9672 | 0.5966 | 0.0216 | Lung Adenocarcinoma; Lung Non-Small Cell Lung Carcinoma; Head and Neck Carcinoma |
| | A225S | | | | | | | | |
| | G226D | | | | | | | | |
| | G226S | | | | | | | | |
| | NM_002529 | | | | | | | | |
| | NP_002520 | | | | | | | | |
| 1 | I228V | Ig-like C2-type 1 | 0.0500 | 0.9000 | 1.0000 | 1.0000 | 0.8524 | 0.0000 | Lung Adenocarcinoma |
| | NM_002529 | | | | | | | | |
| | NP_002520 | | | | | | | | |
| 1 | E231K | Ig-like C2-type 1 | 0.0500 | 0.9000 | 1.0000 | 1.0000 | 0.9963 | 0.0448 | Skin Basal Cell Carcinoma |
| | NM_002529 | | | | | | | | |
| | NP_002520 | | | | | | | | |
| 2 | E233K | Ig-like C2-type 1 | 0.1000 | 0.9000 | 1.0000 | 1.0000 | 0.9963 | 0.0897 | Breast Carcinoma; Lung Squamous Cell Carcinoma |
| | E233Q | | | | | | | | |
| | NM_002529 | | | | | | | | |
| | NP_002520 | | | | | | | | |
| 2 | V238M | Ig-like C2-type 1 | 0.1000 | 0.9000 | 1.0000 | 1.0000 | 0.9288 | 0.0836 | Head and Neck Squamous Cell Carcinoma; Skin Melanoma |
| | M239I | | | | | | | | |
| | NM_002529 | | | | | | | | |
| | NP_002520 | | | | | | | | |
| 3 | S241F | Ig-like C2-type 1 | 0.1500 | 0.9000 | 1.0000 | 1.0000 | 0.8094 | 0.1093 | Ovary Serous Carcinoma; Lung Non-Small Cell Lung Carcinoma |
| | S241H | | | | | | | | |
| | S241Y | | | | | | | | |
| | NM_002529 | | | | | | | | |
| | NP_002520 | | | | | | | | |
| 1 | G243D | Ig-like C2-type 1 | 0.0500 | 0.9000 | 1.0000 | 1.0000 | 0.0000 | 0.0000 | Breast Carcinoma |
| | NM_002529 | | | | | | | | |
| | NP_002520 | | | | | | | | |
| 5 | P245S | Ig-like C2-type 1 | 0.2500 | 0.9000 | 1.0000 | 1.0000 | 0.6902 | 0.0000 | Brain Glioblastoma; Unknown Primary Melanoma; Rectum Adenocarcinoma |
| | S246F | | | | | | | | |
| | L247V | | | | | | | | |
| | NM_002529 | | | | | | | | |
| | NP_002520 | | | | | | | | |
| 8 | S246F | Ig-like C2-type 1 | 0.4000 | 0.9000 | 1.0000 | 1.0000 | 0.1783 | 0.0120 | Unknown Primary Melanoma; Breast Carcinoma; Ovary Serous Carcinoma |
| | L247V | | | | | | | | |
| | G248E | | | | | | | | |
| | G248R | | | | | | | | |
| | NM_002529 | | | | | | | | |
| | NP_002520 | | | | | | | | |
| 3 | L251M | Ig-like C2-type 1 | 0.1500 | 0.9000 | 1.0000 | 1.0000 | 0.9120 | 0.0205 | Breast Invasive Ductal Carcinoma; Unknown Primary Adenocarcinoma; Lung Small Cell Undifferentiated Carcinoma |
| | A252S | | | | | | | | |
| | N253D | | | | | | | | |
| | NM_002529 | | | | | | | | |
| | NP_002520 | | | | | | | | |
| 2 | L258I | Ig-like C2-type 1 | 0.1000 | 0.9000 | 1.0000 | 1.0000 | 0.9963 | 0.0673 | Lung Adenocarcinoma; Uterus Endometrial Adenocarcinoma |
| | L258V | | | | | | | | |
| | NM_002529 | | | | | | | | |
| | NP_002520 | | | | | | | | |
| 5 | R260G | Ig-like C2-type 1 | 0.2500 | 0.9000 | 1.0000 | 0.9836 | 0.4621 | 0.0748 | Lung Adenocarcinoma; Skin Melanoma; Uterus Endometrial Adenocarcinoma Endometrioid |
| | R260M | | | | | | | | |
| | K261E | | | | | | | | |
| | K261N | | | | | | | | |
| | N262K | | | | | | | | |
| | NM_002529 | | | | | | | | |
| | NP_002520 | | | | | | | | |
| 4 | K261E | Ig-like C2-type 1 | 0.2000 | 0.9000 | 1.0000 | 0.9344 | 0.4844 | 0.0567 | Skin Melanoma; Uterus Endometrial Adenocarcinoma Endometrioid; Lung Large Cell Neuroendocrine Carcinoma |
| | K261N | | | | | | | | |
| | N262K | | | | | | | | |
| | V263M | | | | | | | | |
| | NM_002529 | | | | | | | | |
| | NP_002520 | | | | | | | | |
| 6 | N262K | Ig-like C2-type 1 | 0.3000 | 0.9000 | 0.1667 | 0.9344 | 0.6661 | 0.0172 | Lung Adenocarcinoma; Ovary Serous Carcinoma; Lung Non-Small Cell Lung Carcinoma |
| | V263M | | | | | | | | |
| | T264K | | | | | | | | |
| | T264M | | | | | | | | |
| | NM_002529 | | | | | | | | |
| | NP_002520 | | | | | | | | |
| 1 | W266S | Ig-like C2-type 1 | 0.0500 | 0.9000 | 0.0000 | 1.0000 | 0.9963 | 0.0000 | Lung Adenocarcinoma |
| | NM_002529 | | | | | | | | |
| | NP_002520 | | | | | | | | |
| 4 | D270G | Ig-like C2-type 1 | 0.2000 | 0.9000 | 1.0000 | 0.9016 | 0.9504 | 0.0000 | Lung Adenocarcinoma; Breast Carcinoma; Skin Melanoma |
| | D270N | | | | | | | | |
| | NM_002529 | | | | | | | | |
| | NP_002520 | | | | | | | | |
| 3 | R273Q | Ig-like C2-type 1 | 0.1500 | 0.9000 | 1.0000 | 0.8197 | 0.9564 | 0.0000 | Lung Adenocarcinoma; Bone Marrow Leukemia Lymphocytic Chronic; Uterus Endometrial Adenocarcinoma Papillary Serous |
| | R273W | | | | | | | | |
| | NM_002529 | | | | | | | | |
| | NP_002520 | | | | | | | | |
| 5 | E275A | Ig-like C2-type 1 | 0.2500 | 0.9000 | 1.0000 | 0.6393 | 0.9893 | 0.0000 | Breast Invasive Ductal Carcinoma; Ovary Serous Carcinoma; Soft Tissue Sarcoma |
| | NM_002529 | | | | | | | | |
| | NP_002520 | | | | | | | | |
| 1 | S277F | Ig-like C2-type 1 | 0.0500 | 0.9000 | 1.0000 | 1.0000 | 0.9974 | 0.0449 | Skin Squamous Cell Carcinoma |
| | NM_002529 | | | | | | | | |
| | NP_ 002520 | | | | | | | | |
| 2 | V282I | Ig-like C2-type 1 | 0.1000 | 0.9000 | 0.0000 | 0.9836 | 0.9613 | 0.0000 | Lung Adenocarcinoma; Brain Glioblastoma |
| | NM_002529 | | | | | | | | |
| | NP_002520 | | | | | | | | |
| 1 | S287I | | 0.0500 | 0.0500 | 1.0000 | 1.0000 | 0.9672 | 0.0012 | Cervix Neuroendocrine Carcinoma |
| | NM_002529 | | | | | | | | |
| | NP_002520 | | | | | | | | |
| 6 | T292M | | 0.3000 | 0.0500 | 1.0000 | 0.9016 | 0.3317 | 0.0000 | Lung Adenocarcinoma; Breast Carcinoma; Lung Squamous Cell Carcinoma |
| | A293V | | | | | | | | |
| | V294A | | | | | | | | |
| | NM_002529 | | | | | | | | |
| | NP_002520 | | | | | | | | |
| 3 | M296K | | 0.1500 | 0.0500 | 0.0000 | 1.0000 | 0.0000 | 0.0000 | Breast Ductal Carcinoma in Situ; Lung Non-Small Cell Lung Carcinoma; Lung Large Cell Neuroendocrine Carcinoma |
| | H297Q | | | | | | | | |
| | H298Q | | | | | | | | |
| | NM_002529 | | | | | | | | |
| | NP_002520 | | | | | | | | |
| 2 | C300R | Ig-like C2-type 2 | 0.1000 | 0.9000 | 1.0000 | 0.9672 | 0.9973 | 0.0000 | Small Intestine Adenocarcinoma; Soft Tissue Myoepithelial Carcinoma |
| | C300Y | | | | | | | | |
| | NM_002529 | | | | | | | | |
| | NP_002520 | | | | | | | | |
| 1 | P302L | Ig-like C2-type 2 | 0.0500 | 0.9000 | 1.0000 | 1.0000 | 0.9989 | 0.0000 | Breast Invasive Ductal Carcinoma |
| | NM_002529 | | | | | | | | |
| | NP_002520 | | | | | | | | |
| 2 | S304Y | Ig-like C2-type 2 | 0.1000 | 0.9000 | 1.0000 | 1.0000 | 0.9989 | 0.0804 | Lung Adenocarcinoma; Lung Non-Small Cell Lung Carcinoma |
| | NM_002529 | | | | | | | | |
| | NP_002520 | | | | | | | | |
| 2 | D306E | Ig-like C2-type 2 | 0.1000 | 0.9000 | 1.0000 | 1.0000 | 0.0641 | 0.0058 | Pancreas Ductal Adenocarcinoma; Unknown Primary Undifferentiated Small Cell Carcinoma |
| | G307A | | | | | | | | |
| | NM_002529 | | | | | | | | |
| | NP_002520 | | | | | | | | |
| 4 | P309S | Ig-like C2-type 2 | 0.2000 | 0.9000 | 0.0000 | 1.0000 | 0.9933 | 0.0000 | Stomach Adenocarcinoma; Lung Adenocarcinoma; Unknown Primary Adenocarcinoma |
| | A310E | | | | | | | | |
| | A310S | | | | | | | | |
| | P311L | | | | | | | | |
| | NM_002529 | | | | | | | | |
| | NP_ 002520 | | | | | | | | |
| 7 | R314G | Ig-like C2-type 2 | 0.3500 | 0.9000 | 1.0000 | 0.8852 | 0.9024 | 0.1258 | Lung Adenocarcinoma; Head and Neck Squamous Cell Carcinoma; Bone Marrow Multiple Myeloma |
| | R314H | | | | | | | | |
| | R314L | | | | | | | | |
| | R314P | | | | | | | | |
| | NM_002529 | | | | | | | | |
| | NP_002520 | | | | | | | | |
| 4 | N318S | Ig-like C2-type 2 | 0.2000 | 0.9000 | 1.0000 | 0.9836 | 0.9415 | 0.1550 | Head and Neck Squamous Cell Carcinoma; Skin Melanoma; Ovary Epithelial Carcinoma |
| | G319S | | | | | | | | |
| | S320F | | | | | | | | |
| | NM_002529 | | | | | | | | |
| | NP_002520 | | | | | | | | |
| 8 | G319S | Ig-like C2-type 2 | 0.4000 | 0.9000 | 1.0000 | 0.9344 | 0.8913 | 0.2091 | Colon Adenocarcinoma; Breast Carcinoma; Skin Melanoma^{1, 2, 3}; Esophagus and Stomach Carcinoma⁴ |
| | S320F^{1, 2, 3} | | | | | | | | |
| | V321M^{1, 2, 4} | | | | | | | | |
| | NM_002529 | | | | | | | | |
| | NP_002520 | | | | | | | | |
| 5 | N323S | Ig-like C2-type 2 | 0.2500 | 0.9000 | 0.6000 | 0.9672 | 0.9886 | 0.0362 | Breast Invasive Ductal Carcinoma; Liver Cholangiocarcinoma : Uterus Endometrial Adenocarcinoma Endometrioid |
| | E324D | | | | | | | | |
| | E324K | | | | | | | | |
| | NM_002529 | | | | | | | | |
| | NP_002520 | | | | | | | | |
| 2 | S326R | Ig-like C2-type 2 | 0.1000 | 0.9000 | 1.0000 | 1.0000 | 0.9746 | 0.0785 | Lung Adenocarcinoma; Uterus Endometrial Adenocarcinoma |
| | NM_002529 | | | | | | | | |
| | NP_002520 | | | | | | | | |
| 10 | I328V | Ig-like C2-type 2 | 0.1500 | 0.9000 | 1.0000 | 0.9836 | 0.9957 | 0.0181 | Liver Hepatocellular Carcinoma; Pancreas Ductal Adenocarcinoma; Bladder Urothelial Carcinoma |
| | F329V | | | | | | | | |
| | T330A | | | | | | | | |
| | NM_002529 | | | | | | | | |
| | NP_002520 | | | | | | | | |
| 6 | P335L | Ig-like C2-type 2 | 0.3000 | 0.9000 | 0.0000 | 0.6885 | 0.9072 | 0.0000 | Skin Melanoma; Colon Adenocarcinoma; Breast Carcinoma |
| | A336E | | | | | | | | |
| | A337P | | | | | | | | |
| | A337T | | | | | | | | |
| | NM_002529 | | | | | | | | |
| | NP_002520 | | | | | | | | |
| 14 | T340I | Ig-like C2-type 2 | 0.7000 | 0.9000 | 1.0000 | 0.8361 | 0.2163 | 0.0614 | Colon Adenocarcinoma; Breast Ductal Carcinoma in Situ; Bone Marrow Multiple Myeloma |
| | V341M | | | | | | | | |
| | R342Q | | | | | | | | |
| | R342W | | | | | | | | |
| | NM_002529 | | | | | | | | |
| | NP_002520 | | | | | | | | |
| 2 | G344E | Ig-like C2-type 2 | 0.1000 | 0.9000 | 1.0000 | 1.0000 | 0.9862 | 0.0888 | Lung Adenocarcinoma; Lung Carcinosarcoma |
| | G344W | | | | | | | | |
| | NM_002529 | | | | | | | | |
| | NP_002520 | | | | | | | | |
| 5 | L346P | Ig-like C2-type 2 | 0.2500 | 0.9000 | 0.4000 | 0.9180 | 0.9893 | 0.0434 | Colon Adenocarcinoma; Uterus Endometrial Adenocarcinoma Endometrioid; Brain Medulloblastoma |
| | R347C | | | | | | | | |
| | R347G | | | | | | | | |
| | R347H | | | | | | | | |
| | NM_002529 | | | | | | | | |
| | NP_002520 | | | | | | | | |
| 2 | N349K | Ig-like C2-type 2 | 0.1000 | 0.9000 | 1.0000 | 0.9672 | 0.9852 | 0.0000 | Breast Invasive Ductal Carcinoma; Unknown Primary Malignant Neoplasm |
| | N349S | | | | | | | | |
| | NM_002529 | | | | | | | | |
| | NP_002520 | | | | | | | | |
| 1 | G357S | Ig-like C2-type 2 | 0.0500 | 0.9000 | 1.0000 | 1.0000 | 0.9989 | 0.0045 | Skin Melanoma |
| | NM_002529 | | | | | | | | |
| | NP_002520 | | | | | | | | |
| 4 | Y359C | Ig-like C2-type 2 | 0.2000 | 0.9000 | 1.0000 | 0.9672 | 0.9969 | 0.0868 | Lung Adenocarcinoma; Colon Adenocarcinoma; Liver Cholangiocarcinoma |
| | T360M | | | | | | | | |
| | L361R | | | | | | | | |
| | NM_002529 | | | | | | | | |
| | NP_002520 | | | | | | | | |
| 4 | P366L | | 0.2000 | 0.0500 | 1.0000 | 0.9672 | 0.9818 | 0.0000 | Brain Glioblastoma; Unknown Primary Adenocarcinoma; Unknown Primary Melanoma |
| | P366R | | | | | | | | |
| | P366S | | | | | | | | |
| | P366T | | | | | | | | |
| | NM_002529 | | | | | | | | |
| | NP_ 002520 | | | | | | | | |
| 2 | G368C | | 0.1000 | 0.0500 | 1.0000 | 1.0000 | 0.9985 | 0.0045 | Lung Adenocarcinoma |
| | NM_002529 | | | | | | | | |
| | NP_002520 | | | | | | | | |
| 5 | S371F | | 0.2500 | 0.0500 | 1.0000 | 0.9672 | 0.9922 | 0.0073 | Lung Squamous Cell Carcinoma; Breast Invasive Lobular Carcinoma; Soft Tissue Liposarcoma |
| | A372S | | | | | | | | |
| | A372T | | | | | | | | |
| | NM_002529 | | | | | | | | |
| | NP_002520 | | | | | | | | |
| 7 | I374N | | 0.3500 | 0.0500 | 1.0000 | 0.9508 | 0.5446 | 0.0067 | Lung Adenocarcinoma; Brain Glioblastoma; Lung Squamous Cell Carcinoma |
| | M375I | | | | | | | | |
| | M375V | | | | | | | | |
| | NM_002529 | | | | | | | | |
| | NP_002520 | | | | | | | | |
| 3 | M379T | | 0.1500 | 0.0500 | 1.0000 | 0.9672 | 0.9937 | 0.0000 | Unknown Primary Melanoma; Unknown Primary Carcinoma; Small Intestine Gist |
| | D380G | | | | | | | | |
| | N381S | | | | | | | | |
| | NM_002529 | | | | | | | | |
| | NP_002520 | | | | | | | | |
| 3 | E388D | | 0.1500 | 0.0500 | 1.0000 | 1.0000 | 0.9981 | 0.0000 | Colon Adenocarcinoma; Uterus Endometrial Adenocarcinoma Endometrioid; Breast Invasive Lobular Carcinoma |
| | E388K | | | | | | | | |
| | D389Y | | | | | | | | |
| | NM_002529 | | | | | | | | |
| | NP_002520 | | | | | | | | |
| 2 | P392S | | 0.1000 | 0.0500 | 1.0000 | 0.9508 | 0.9981 | 0.0000 | Bone Marrow Multiple Myeloma; Unknown Primary Carcinoma |
| | V393F | | | | | | | | |
| | NM_002529 | | | | | | | | |
| | NP_002520 | | | | | | | | |
| 3 | F395L | | 0.1500 | 0.0500 | 1.0000 | 0.9672 | 0.9980 | 0.0000 | Colon Adenocarcinoma; Breast Invasive Ductal Carcinoma; Head and Neck Squamous Cell Carcinoma |
| | NM_002529 | | | | | | | | |
| | NP_002520 | | | | | | | | |
| 8 | P397L | | 0.4000 | 0.0500 | 1.0000 | 0.8033 | 0.9980 | 0.0000 | Lung Adenocarcinoma; Ovary Serous Carcinoma; Lung Squamous Cell Carcinoma |
| | V398L | | | | | | | | |
| | NM_002529 | | | | | | | | |
| | NP_002520 | | | | | | | | |
| 3 | S402I | | 0.1500 | 0.0500 | 1.0000 | 1.0000 | 0.9980 | 0.0033 | Breast Carcinoma; Kidney Renal Cell Carcinoma; Skin Squamous Cell Carcinoma |
| | S402R | | | | | | | | |
| | NM_002529 | | | | | | | | |
| | NP_002520 | | | | | | | | |
| 1 | S404P | | 0.0500 | 0.0500 | 1.0000 | 1.0000 | 0.8904 | 0.0000 | Breast Carcinoma |
| | NM_002529 | | | | | | | | |
| | NP_002520 | | | | | | | | |
| 6 | D406Y | | 0.3000 | 0.0500 | 1.0000 | 0.9344 | 0.9906 | 0.0030 | Lung Adenocarcinoma; Colon Adenocarcinoma; Breast Invasive Ductal Carcinoma |
| | P407L | | | | | | | | |
| | P407R | | | | | | | | |
| | V408G | | | | | | | | |
| | NM_002529 | | | | | | | | |
| | NP_002520 | | | | | | | | |
| 3 | K410N | | 0.1500 | 0.0500 | 1.0000 | 0.9836 | 0.4940 | 0.0036 | Breast Carcinoma; Brain Glioblastoma; Unknown Primary Carcinoma |
| | K411N | | | | | | | | |
| | K411del | | | | | | | | |
| | NM_002529 | | | | | | | | |
| | NP_002520 | | | | | | | | |
| 10 | E413K | | 0.5000 | 0.0500 | 0.7000 | 0.7541 | 0.9604 | 0.0039 | Colon Adenocarcinoma; Skin Squamous Cell Carcinoma; Breast Invasive Ductal Carcinoma |
| | E413Q | | | | | | | | |
| | NM_002529 | | | | | | | | |
| | NP_002520 | | | | | | | | |
| 3 | P415S | | 0.1500 | 0.0500 | 0.0000 | 1.0000 | 0.8472 | 0.0000 | Unknown Primary Malignant Neoplasm; Lung Adenocarcinoma |
| | F416S | | | | | | | | |
| | G417V | | | | | | | | |
| | NM_002529 | | | | | | | | |
| | NP_002520 | | | | | | | | |
| 1 | S419L | | 0.0500 | 0.0500 | 1.0000 | 0.9672 | 0.9972 | 0.0000 | Breast Invasive Lobular Carcinoma |
| | NM_002529 | | | | | | | | |
| | NP_002520 | | | | | | | | |
| 2 | A421T | | 0.1000 | 0.0500 | 1.0000 | 1.0000 | 0.9972 | 0.0047 | Lung Adenocarcinoma; Stomach Adenocarcinoma Diffuse Type |
| | V422L | | | | | | | | |
| | NM_002529 | | | | | | | | |
| | NP_002520 | | | | | | | | |
| 4 | A425S | | 0.2000 | 0.0500 | 1.0000 | 1.0000 | 0.9972 | 0.0000 | Ovary Serous Carcinoma; Unknown Primary Carcinoma; Uterus Endometrial Adenocarcinoma Endometrioid |
| | V426I | | | | | | | | |
| | NM_002529 | | | | | | | | |
| | NP_002520 | | | | | | | | |
| 1 | L432R | | 0.0500 | 0.0500 | 1.0000 | 1.0000 | 0.9914 | 0.0012 | Breast Invasive Ductal Carcinoma |
| | NM_002529 | | | | | | | | |
| | NP_002520 | | | | | | | | |
| 4 | T434M | | 0.2000 | 0.0500 | 1.0000 | 0.9672 | 0.4824 | 0.0044 | Colon Adenocarcinoma; Skin Melanoma; Unknown Primary Carcinoma |
| | NM_002529 | | | | | | | | |
| | NP_002520 | | | | | | | | |
| 4 | N440K | | 0.2000 | 0.0500 | 0.2500 | 0.7377 | 0.9375 | 0.0000 | Skin Melanoma; Kidney Medullary Carcinoma; Soft Tissue Hemangioendothelio ma |
| | N440S | | | | | | | | |
| | NM_002529 | | | | | | | | |
| | NP_002520 | | | | | | | | |
| 6 | R444P | | 0.3000 | 0.0500 | 1.0000 | 0.0000 | 0.6725 | 0.0000 | Breast Carcinoma; Breast Invasive Ductal Carcinoma; Lung Non-Small Cell Lung Carcinoma |
| | R444Q | | | | | | | | |
| | R444W | | | | | | | | |
| | NM_002529 | | | | | | | | |
| | NP_002520 | | | | | | | | |
| 3 | K447M | | 0.1500 | 0.0500 | 1.0000 | 1.0000 | 0.9265 | 0.0010 | Colon Adenocarcinoma; Bladder Urothelial Carcinoma |
| | K447N | | | | | | | | |
| | K447T | | | | | | | | |
| | NM_002529 | | | | | | | | |
| | NP_002520 | | | | | | | | |
| 14 | R452C | | 0.7000 | 0.0500 | 1.0000 | 0.7377 | 0.9873 | 0.0000 | Lung Adenocarcinoma; Lung Squamous Cell Carcinoma; Gastroesophageal Junction Adenocarcinoma |
| | R452G | | | | | | | | |
| | P453L | | | | | | | | |
| | P453Q | | | | | | | | |
| | P453T | | | | | | | | |
| | A454T | | | | | | | | |
| | NM_002529 | | | | | | | | |
| | NP_002520 | | | | | | | | |
| 6 | P453L | | 0.3000 | 0.0500 | 1.0000 | 0.9180 | 0.9735 | 0.0059 | Lung Squamous Cell Carcinoma; Soft Tissue Leiomyosarcoma; Adrenal Gland Neuroblastoma |
| | P453Q | | | | | | | | |
| | P453T | | | | | | | | |
| | A454T | | | | | | | | |
| | V455M | | | | | | | | |
| | NM_002529 | | | | | | | | |
| | NP_002520 | | | | | | | | |
| 1 | A457V | | 0.0500 | 0.0500 | 1.0000 | 1.0000 | 0.9960 | 0.0025 | Unknown Primary Squamous Cell Carcinoma |
| | NM_002529 | | | | | | | | |
| | NP_002520 | | | | | | | | |
| 3 | D460N | | 0.1500 | 0.0500 | 0.3333 | 0.9836 | 0.9960 | 0.0000 | Lung Adenocarcinoma; Skin Melanoma; Lung Non-Small Cell Lung Carcinoma |
| | G461R | | | | | | | | |
| | NM_002529 | | | | | | | | |
| | NP_002520 | | | | | | | | |
| 2 | S465F | | 0.1000 | 0.0500 | 1.0000 | 1.0000 | 0.9949 | 0.0047 | Kidney Renal Cell Carcinoma; Skin Basal Cell Carcinoma |
| | S465del | | | | | | | | |
| | NM_002529 | | | | | | | | |
| | NP_002520 | | | | | | | | |
| 1 | F468L | | 0.0500 | 0.0500 | 1.0000 | 1.0000 | 0.9949 | 0.0002 | Unknown Primary Carcinoma |
| | NM_002529 | | | | | | | | |
| | NP_002520 | | | | | | | | |
| 3 | L471F | | 0.1500 | 0.0500 | 1.0000 | 1.0000 | 0.4800 | 0.0027 | Lung Adenocarcinoma; Head and Neck Squamous Cell Carcinoma; Lung Sarcomatoid Carcinoma |
| | G472S | | | | | | | | |
| | NM_002529 | | | | | | | | |
| | NP_002520 | | | | | | | | |
| 8 | S475C | | 0.4000 | 0.0500 | 0.6250 | 0.9672 | 0.9497 | 0.0040 | Unknown Primary Adenocarcinoma; Lung Adenocarcinoma; Colon Adenocarcinoma |
| | S475F | | | | | | | | |
| | S475T | | | | | | | | |
| | L476M | | | | | | | | |
| | S477Y | | | | | | | | |
| | S477_insS | | | | | | | | |
| | NM_002529 | | | | | | | | |
| | NP_002520 | | | | | | | | |
| 4 | L476M | | 0.2000 | 0.0500 | 0.2500 | 1.0000 | 0.9054 | 0.0014 | Lung Adenocarcinoma; Skin Melanoma; Unknown Primary Adenocarcinoma |
| | S477Y | | | | | | | | |
| | S477_insS | | | | | | | | |
| | P478L | | | | | | | | |
| | NM_002529 | | | | | | | | |
| | NP_002520 | | | | | | | | |
| 4 | S477Y | | 0.2000 | 0.0500 | 0.2500 | 0.9836 | 0.9794 | 0.0012 | Lung Adenocarcinoma; Brain Glioblastoma; Skin Melanoma |
| | S477_insS | | | | | | | | |
| | P478L | | | | | | | | |
| | T479I | | | | | | | | |
| | NM_002529 | | | | | | | | |
| | NP_002520 | | | | | | | | |
| 4 | P478L | | 0.2000 | 0.0500 | 0.2500 | 0.9672 | 0.9794 | 0.0006 | Lung Adenocarcinoma; Brain Glioblastoma; Skin Melanoma |
| | T479I | | | | | | | | |
| | E480K | | | | | | | | |
| | E480Q | | | | | | | | |
| | NM_002529 | | | | | | | | |
| | NP_002520 | | | | | | | | |
| 3 | S484Y | | 0.1500 | 0.0500 | 1.0000 | 0.8361 | 0.9960 | 0.0000 | Lung Adenocarcinoma; Colon Adenocarcinoma; Pancreas Ductal Adenocarcinoma |
| | G485R | | | | | | | | |
| | L486I | | | | | | | | |
| | NM_002529 | | | | | | | | |
| | NP_002520 | | | | | | | | |
| 3 | G485R | | 0.1500 | 0.0500 | 1.0000 | 0.8361 | 0.9394 | 0.0000 | Colon Adenocarcinoma; Pancreas Ductal Adenocarcinoma; Bladder Urothelial Carcinoma |
| | L486I | | | | | | | | |
| | Q487L | | | | | | | | |
| | NM_002529 | | | | | | | | |
| | NP_002520 | | | | | | | | |
| 4 | L486I | | 0.2000 | 0.0500 | 1.0000 | 0.8361 | 0.9175 | 0.0047 | Colon Adenocarcinoma; Lung Squamous Cell Carcinoma; Pancreas Ductal Adenocarcinoma |
| | Q487L | | | | | | | | |
| | G488C | | | | | | | | |
| | G488S | | | | | | | | |
| | NM_002529 | | | | | | | | |
| | NP_002520 | | | | | | | | |
| 5 | Q487L | | 0.2500 | 0.0500 | 1.0000 | 0.9508 | 0.9204 | 0.0100 | Colon Adenocarcinoma; Kidney Renal Cell Carcinoma; Lung Squamous Cell Carcinoma |
| | G488C | | | | | | | | |
| | G488S | | | | | | | | |
| | H489Q | | | | | | | | |
| | H489Y | | | | | | | | |
| | NM_002529 | | | | | | | | |
| | NP_002520 | | | | | | | | |
| 5 | P494T | | 0.2500 | 0.0500 | 1.0000 | 0.9508 | 0.9935 | 0.0000 | Ovary Serous Carcinoma; Pancreas Carcinoma; Soft Tissue Ewing Sarcoma |
| | Q495R | | | | | | | | |
| | NM_002529 | | | | | | | | |
| | NP_002520 | | | | | | | | |
| 1 | A500T | | 0.0500 | 0.0500 | 1.0000 | 1.0000 | 0.9324 | 0.0021 | Lymph Node Lymphoma Follicular Lymphoma |
| | NM_002529 | | | | | | | | |
| | NP_002520 | | | | | | | | |
| 4 | V502A | | 0.2000 | 0.0500 | 0.2500 | 1.0000 | 0.9950 | 0.0019 | Breast Carcinoma; Skin Melanoma; Bile Duct Adenocarcinoma |
| | H503N | | | | | | | | |
| | H503Y | | | | | | | | |
| | NM_002529 | | | | | | | | |
| | NP_002520 | | | | | | | | |
| 10 | R507C | | 0.5000 | 0.0500 | 1.0000 | 0.5902 | 0.8988 | 0.0108 | Bladder Urothelial Carcinoma; Lung Adenocarcinoma; Breast Invasive Ductal Carcinoma |
| | R507H | | | | | | | | |
| | R508Q | | | | | | | | |
| | R508W | | | | | | | | |
| | NM_002529 | | | | | | | | |
| | NP_002520 | | | | | | | | |
| 4 | I510T | Protein kinase | 0.2000 | 1.0000 | 1.0000 | 0.8525 | 0.9837 | 0.1606 | Unknown Primary Adenocarcinoma; Unknown Primary Melanoma; Uterus Endometrial Adenocarcinoma Endometrioid |
| | V511M | | | | | | | | |
| | L512F | | | | | | | | |
| | L512R | | | | | | | | |
| | NM_002529 | | | | | | | | |
| | NP_002520 | | | | | | | | |
| 1 | E515K | Protein kinase | 0.0500 | 1.0000 | 1.0000 | 1.0000 | 0.9976 | 0.0050 | Unknown Primary Melanoma |
| | NM_002529 | | | | | | | | |
| | NP_002520 | | | | | | | | |
| 2 | G517R | Protein kinase | 0.1000 | 1.0000 | 0.0000 | 1.0000 | 0.9976 | 0.0000 | Skin Melanoma; Rectum Adenocarcinoma |
| | E518K | | | | | | | | |
| | NM_002529 | | | | | | | | |
| | NP_002520 | | | | | | | | |
| 1 | A520T | Protein kinase | 0.0500 | 1.0000 | 1.0000 | 1.0000 | 0.9976 | 0.0499 | Uterus Endometrial Adenocarcinoma |
| | NM_002529 | | | | | | | | |
| | NP_002520 | | | | | | | | |
| 1 | G522W | Protein kinase | 0.0500 0.2000 | 1.0000 | 1.0000 | 1.0000 | 0.9976 | 0.0249 | Eye Lacrimal Duct Carcinoma |
| | NM_002529 | | | | | | | | |
| | NP_002520 | | | | | | | | |
| 4 | L526F | Protein kinase | | 1.0000 | 0.2500 | 1.0000 | 0.9749 | 0.0081 | Lung Adenocarcinoma; Colon Adenocarcinoma; Unknown Primary Melanoma |
| | L526P | | | | | | | | |
| | A527T | | | | | | | | |
| | NM_002529 | | | | | | | | |
| | NP_002520 | | | | | | | | |
| 1 | H530Y | Protein kinase | 0.0500 | 1.0000 | 1.0000 | 1.0000 | 0.7968 | 0.0356 | Esophagus Adenocarcinoma |
| | NM_002529 | | | | | | | | |
| | NP_002520 | | | | | | | | |
| 1 | L533Q | Protein kinase | 0.0500 | 1.0000 | 1.0000 | 1.0000 | 0.9923 | 0.0496 | Lung Adenocarcinoma |
| | NM_002529 | | | | | | | | |
| | NP_002520 | | | | | | | | |
| 2 | D537E | Protein kinase | 0.1000 | 1.0000 | 1.0000 | 1.0000 | 0.9767 | 0.0874 | Colon Adenocarcinoma; Ovary Serous Carcinoma |
| | D537N | | | | | | | | |
| | NM_002529 | | | | | | | | |
| | NP_002520 | | | | | | | | |
| 4 | M539L | Protein kinase | 0.2000 | 1.0000 | 1.0000 | 1.0000 | 0.9663 | 0.0000 | Lung Adenocarcinoma; Breast Invasive Ductal Carcinoma |
| | M539R | | | | | | | | |
| | L540Q | | | | | | | | |
| | V541M | | | | | | | | |
| | NM_002529 | | | | | | | | |
| | NP_002520 | | | | | | | | |
| 1 | V543A | Protein kinase | 0.0500 | 1.0000 | 1.0000 | 1.0000 | 0.9923 | 0.0050 | Uterus Leiomyosarcoma |
| | NM_002529 | | | | | | | | |
| | NP_002520 | | | | | | | | |
| 1 | K547T | Protein kinase | 0.0500 | 1.0000 | 1.0000 | 1.0000 | 0.9939 | 0.0445 | Head and Neck Squamous Cell Carcinoma |
| | NM_002529 | | | | | | | | |
| | NP_002520 | | | | | | | | |
| 5 | A549T | Protein kinase | 0.2500 | 1.0000 | 0.0000 | 0.8689 | 0.6778 | 0.0000 | Lung Adenocarcinoma; Colon Adenocarcinoma; Breast Invasive Ductal Carcinoma |
| | A549V | | | | | | | | |
| | S550Y | | | | | | | | |
| | E551D | | | | | | | | |
| | E551V | | | | | | | | |
| | NM_002529 | | | | | | | | |
| | NP_002520 | | | | | | | | |
| 4 | S550Y | Protein kinase | 0.2000 | 1.0000 | 0.0000 | 1.0000 | 0.9961 | 0.0000 | Lung Adenocarcinoma; Lung Non-Small Cell Lung Carcinoma |
| | E551D | | | | | | | | |
| | E551V | | | | | | | | |
| | S552R | | | | | | | | |
| | NM_002529 | | | | | | | | |
| | NP_002520 | | | | | | | | |
| 4 | E551D | Protein kinase | 0.2000 | 1.0000 | 0.2500 | 1.0000 | 0.9961 | 0.0427 | Lung Adenocarcinoma; Lung Non-Small Cell Lung Carcinoma; Unknown Primary Adenocarcinoma |
| | E551V | | | | | | | | |
| | S552R | | | | | | | | |
| | A553T | | | | | | | | |
| | NM_002529 | | | | | | | | |
| | NP_002520 | | | | | | | | |
| 6 | S552R | Protein kinase | 0.3000 | 1.0000 | 1.0000 | 0.8852 | 0.8492 | 0.1121 | Lung Adenocarcinoma; Unknown Primary Adenocarcinoma; Pleura Mesothelioma |
| | A553T | | | | | | | | |
| | R554P | | | | | | | | |
| | R554Q | | | | | | | | |
| | R554W | | | | | | | | |
| | NM_002529 | | | | | | | | |
| | NP_002520 | | | | | | | | |
| 1 | D556N | Protein kinase | 0.0500 | 1.0000 | 1.0000 | 1.0000 | 0.9982 | 0.0499 | Unknown Primary Melanoma |
| | NM_002529 | | | | | | | | |
| | NP_002520 | | | | | | | | |
| 2 | R559H | Protein kinase | 0.1000 | 1.0000 | 0.0000 | 0.9508 | 0.9982 | 0.0000 | Lung Adenocarcinoma; Colon Adenocarcinoma |
| | NM_002529 | | | | | | | | |
| | NP_002520 | | | | | | | | |
| 1 | A561T | Protein kinase | 0.0500 | 1.0000 | 0.0000 | 1.0000 | 0.9982 | 0.0000 | Lung Adenocarcinoma |
| | NM_002529 | | | | | | | | |
| | NP_002520 | | | | | | | | |
| 1 | M566K | Protein kinase | 0.0500 | 1.0000 | 1.0000 | 0.9836 | 0.9939 | 0.0000 | Colon Adenocarcinoma |
| | NM_002529 | | | | | | | | |
| | NP_002520 | | | | | | | | |
| 1 | Q570R | Protein kinase | 0.0500 | 1.0000 | 1.0000 | 1.0000 | 0.9939 | 0.0050 | Bone Marrow Leukemia Non-Lymphocytic Acute Myelocytic |
| | NM_002529 | | | | | | | | |
| | NP_002520 | | | | | | | | |
| 8 | V573M | Protein kinase | 0.4000 | 1.0000 | 0.1250 | 0.9016 | 0.9977 | 0.0000 | Lung Non-Small Cell Lung Carcinoma; Breast Carcinoma; Ovary Serous Carcinoma |
| | R574C | | | | | | | | |
| | R574H | | | | | | | | |
| | F575L | | | | | | | | |
| | NM_002529 | | | | | | | | |
| | NP_002520 | | | | | | | | |
| 3 | G577S | Protein kinase | 0.1500 | 1.0000 | 0.0000 | 0.9180 | 0.9982 | 0.0000 | Colon Adenocarcinoma; Brain Glioblastoma; Lung Non-Small Cell Lung Carcinoma |
| | V578I | | | | | | | | |
| | NM_002529 | | | | | | | | |
| | NP_002520 | | | | | | | | |
| 1 | T580I | Protein kinase | 0.0500 | 1.0000 | 0.0000 | 1.0000 | 0.9982 | 0.0000 | Lung Large Cell Neuroendocrine Carcinoma |
| | NM_002529 | | | | | | | | |
| | NP_002520 | | | | | | | | |
| 6 | R583C | Protein kinase | 0.3000 | 1.0000 | 1.0000 | 0.8689 | 0.9679 | 0.0000 | Lung Squamous Cell Carcinoma; Ovary Serous Carcinoma; Head and Neck Squamous Cell Carcinoma |
| | R583L | | | | | | | | |
| | NM_002529 | | | | | | | | |
| | NP_002520 | | | | | | | | |
| 1 | L585R | Protein kinase | 0.0500 | 1.0000 | 1.0000 | 1.0000 | 0.9939 | 0.0000 | Ovary Serous Carcinoma |
| | NM_002529 | | | | | | | | |
| | NP_002520 | | | | | | | | |
| 1 | M587T | Protein kinase | 0.0500 | 1.0000 | 1.0000 | 1.0000 | 0.9939 | 0.0497 | Breast Carcinoma |
| | NM_002529 | | | | | | | | |
| | NP_002520 | | | | | | | | |
| 1 | Y591C | Protein kinase | 0.0500 | 1.0000 | 1.0000 | 1.0000 | 0.9939 | 0.0497 | Lung Adenocarcinoma |
| | NM_002529 | | | | | | | | |
| | NP_002520 | | | | | | | | |
| 7 | R593W | Protein kinase | 0.3500 | 1.0000 | 0.5714 | 0.7541 | 0.7196 | 0.0649 | Colon Adenocarcinoma; Lung Adenocarcinoma; Breast Invasive Ductal Carcinoma |
| | H594Q | | | | | | | | |
| | G595R | | | | | | | | |
| | NM_002529 | | | | | | | | |
| | NP_002520 | | | | | | | | |
| 5 | R599H | Protein kinase | 0.2500 | 1.0000 | 1.0000 | 0.9508 | 0.9982 | 0.0000 | Lung Adenocarcinoma; Breast Invasive Ductal Carcinoma; Unknown Primary Adenocarcinoma |
| | NM_002529 | | | | | | | | |
| | NP_002520 | | | | | | | | |
| 3 | R602Q | Protein kinase | 0.1500 | 1.0000 | 0.0000 | 1.0000 | 0.9952 | 0.0000 | Lung Adenocarcinoma; Head and Neck Squamous Cell Carcinoma; Bone Marrow Multiple Myeloma |
| | S603P | | | | | | | | |
| | NM_002529 | | | | | | | | |
| | NP_002520 | | | | | | | | |
| 2 | P606H | Protein kinase | 0.1000 | 1.0000 | 1.0000 | 1.0000 | 0.9963 | 0.0926 | Uterus Endometrial Adenocarcinoma Endometrioid; Lung Sarcomatoid Carcinoma |
| | D607N | | | | | | | | |
| | NM_002529 | | | | | | | | |
| | NP_002520 | | | | | | | | |
| 1 | K609N | Protein kinase | 0.0500 | 1.0000 | 0.0000 | 1.0000 | 0.9316 | 0.0000 | Lung Adenocarcinoma |
| | NM_002529 | | | | | | | | |
| | NP_002520 | | | | | | | | |
| 2 | A612S | Protein kinase | 0.1000 | 1.0000 | 1.0000 | 1.0000 | 0.9963 | 0.0000 | Prostate Acinar Adenocarcinoma; Breast Adenoid Cystic Carcinoma |
| | A612V | | | | | | | | |
| | NM_002529 | | | | | | | | |
| | NP_002520 | | | | | | | | |
| 6 | G614A | Protein kinase | 0.3000 | 1.0000 | 0.5000 | 1.0000 | 0.9763 | 0.0262 | Lung Adenocarcinoma; Brain Glioblastoma; Skin Melanoma |
| | G614V | | | | | | | | |
| | E615K | | | | | | | | |
| | E615Q | | | | | | | | |
| | D616H | | | | | | | | |
| | D616N | | | | | | | | |
| | NM_002529 | | | | | | | | |
| | NP_002520 | | | | | | | | |
| 1 | A618V | Protein kinase | 0.0500 | 1.0000 | 1.0000 | 1.0000 | 0.9963 | 0.0000 | Nasopharynx and Paranasal Sinuses Undifferentiated Carcinoma |
| | NM_002529 | | | | | | | | |
| | NP_002520 | | | | | | | | |
| 1 | G620C | Protein kinase | 0.0500 | 1.0000 | 0.0000 | 1.0000 | 0.9963 | 0.0000 | Lung Adenocarcinoma |
| | NM_002529 | | | | | | | | |
| | NP_002520 | | | | | | | | |
| 2 | G623C | Protein kinase | 0.1000 | 1.0000 | 1.0000 | 0.9672 | 0.7246 | 0.0000 | Breast Carcinoma; Skin Squamous Cell Carcinoma |
| | NM_002529 | | | | | | | | |
| | NP_002520 | | | | | | | | |
| 1 | Q626K | Protein kinase | 0.0500 | 1.0000 | 1.0000 | 1.0000 | 0.9963 | 0.0446 | Breast Ductal Carcinoma in Situ |
| | NM_002529 | | | | | | | | |
| | NP_002520 | | | | | | | | |
| 2 | V630A | Protein kinase | 0.1000 | 1.0000 | 1.0000 | 1.0000 | 0.9928 | 0.0000 | Bladder Urothelial Carcinoma; Unknown Primary Melanoma |
| | A631D | | | | | | | | |
| | NM_002529 | | | | | | | | |
| | NP_002520 | | | | | | | | |
| 7 | A636E | Protein kinase | 0.3500 | 1.0000 | 0.5714 | 0.9672 | 0.9953 | 0.1363 | Colon Adenocarcinoma; Skin Melanoma; Lung Small Cell Undifferentiated Carcinoma |
| | A636T | | | | | | | | |
| | A636V | | | | | | | | |
| | G637E | | | | | | | | |
| | G637W | | | | | | | | |
| | M638V | | | | | | | | |
| | NM_002529 | | | | | | | | |
| | NP_002520 | | | | | | | | |
| 6 | G637E | Protein kinase | 0.3000 | 1.0000 | 1.0000 | 0.9672 | 0.9951 | 0.0578 | Ovary Serous Carcinoma; Skin Squamous Cell Carcinoma; Bile Duct Adenocarcinoma |
| | G637W | | | | | | | | |
| | M638V | | | | | | | | |
| | V639L | | | | | | | | |
| | V639M | | | | | | | | |
| | NM_002529 | | | | | | | | |
| | NP_ 002520 | | | | | | | | |
| 6 | M638V | Protein kinase | 0.3000 | 1.0000 | 1.0000 | 0.9672 | 0.9916 | 0.0000 | Lung Adenocarcinoma; Ovary Serous Carcinoma; Lung Non-Small Cell Lung Carcinoma |
| | V639L | | | | | | | | |
| | V639M | | | | | | | | |
| | Y640C | | | | | | | | |
| | NM_002529 | | | | | | | | |
| | NP_002520 | | | | | | | | |
| 6 | V639L | Protein kinase | 0.3000 | 1.0000 | 1.0000 | 0.9836 | 0.9821 | 0.0000 | Lung Adenocarcinoma; Ovary Serous Carcinoma; Lung Non-Small Cell Lung Carcinoma |
| | V639M | | | | | | | | |
| | Y640C | | | | | | | | |
| | L641M | | | | | | | | |
| | NM_002529 | | | | | | | | |
| | NP_002520 | | | | | | | | |
| 6 | Y640C | Protein kinase | 0.3000 | 1.0000 | 0.0000 | 0.9836 | 0.9821 | 0.0000 | Lung Adenocarcinoma; Colon Adenocarcinoma; Lung Non-Small Cell Lung Carcinoma |
| | L641M | | | | | | | | |
| | A642S | | | | | | | | |
| | A642V | | | | | | | | |
| | NM_002529 | | | | | | | | |
| | NP_002520 | | | | | | | | |
| 1 | L644M | Protein kinase | 0.0500 | 1.0000 | 0.0000 | 1.0000 | 0.7447 | 0.0000 | Lung Adenocarcinoma |
| | NM_002529 | | | | | | | | |
| | NP_002520 | | | | | | | | |
| 7 | V647G | Protein kinase | 0.3500 | 1.0000 | 0.7143 | 0.8525 | 0.9733 | 0.0000 | Lung Adenocarcinoma; Colon Adenocarcinoma; Head and Neck |
| | V647L | | | | | | | | |
| | NM_002529 | | | | | | | | |
| | NP_002520 | | | | | | | | |
| | | | | | | | | | Squamous Cell Carcinoma |
| 6 | R649L | Protein kinase | 0.3000 | 1.0000 | 0.5000 | 1.0000 | 0.9446 | 0.1267 | Lung Adenocarcinoma; Colon Adenocarcinoma; Ovary Serous Carcinoma |
| | R649W | | | | | | | | |
| | NM_002529 | | | | | | | | |
| | NP_002520 | | | | | | | | |
| 1 | L651M | Protein kinase | 0.0500 | 1.0000 | 0.0000 | 1.0000 | 0.9359 | 0.0000 | Lung Adenocarcinoma |
| | NM_002529 | | | | | | | | |
| | NP_002520 | | | | | | | | |
| 6 | R654C | Protein kinase | 0.3000 | 1.0000 | 0.5000 | 0.9180 | 0.9951 | 0.1246 | Lung Adenocarcinoma; Breast Carcinoma; Colon Adenocarcinoma |
| | R654H | | | | | | | | |
| | N655Y | | | | | | | | |
| | NM_002529 | | | | | | | | |
| | NP_002520 | | | | | | | | |
| 2 | L657P | Protein kinase | 0.1000 | 1.0000 | 1.0000 | 1.0000 | 0.8658 | 0.0000 | Colon Adenocarcinoma; Pleura Mesothelioma |
| | L657V | | | | | | | | |
| | NM_002529 | | | | | | | | |
| | NP_002520 | | | | | | | | |
| 2 | Q660L | Protein kinase | 0.1000 | 1.0000 | 0.0000 | 1.0000 | 0.9922 | 0.0000 | Skin Melanoma |
| | G661E | | | | | | | | |
| | NM_002529 | | | | | | | | |
| | NP_002520 | | | | | | | | |
| 2 | V663E | Protein kinase | 0.1000 | 1.0000 | 0.0000 | 1.0000 | 0.9909 | 0.0000 | Lung Adenocarcinoma; Skin Melanoma |
| | V664I | | | | | | | | |
| | NM_002529 | | | | | | | | |
| | NP_002520 | | | | | | | | |
| 1 | I666T | Protein kinase | 0.0500 | 1.0000 | 0.0000 | 1.0000 | 0.9867 | 0.0000 | Lung Non-Small Cell Lung Carcinoma |
| | NM_002529 | | | | | | | | |
| | NP_002520 | | | | | | | | |
| 1 | M671T | Protein kinase | 0.0500 | 1.0000 | 1.0000 | 1.0000 | 0.9867 | 0.0441 | Colon Adenocarcinoma |
| | NM_002529 | | | | | | | | |
| | NP_002520 | | | | | | | | |
| 2 | D674E | Protein kinase | 0.1000 | 1.0000 | 0.0000 | 1.0000 | 0.2552 | 0.0000 | Lung Adenocarcinoma; Skin Merkel Cell Carcinoma |
| | D674N | | | | | | | | |
| | NM_002529 | | | | | | | | |
| | NP_002520 | | | | | | | | |
| 1 | S677N | Protein kinase | 0.0500 | 1.0000 | 1.0000 | 1.0000 | 0.9952 | 0.0050 | Uterus Endometrial Adenocarcinoma Papillary Serous |
| | NM_002529 | | | | | | | | |
| | NP_002520 | | | | | | | | |
| 5 | D679N^{1,2,5} | Protein kinase | 0.2500 | 1.0000 | 1.0000 | 0.9508 | 0.9935 | 0.2137 | Lung Small Cell Undifferentiated Carcinoma; Prostate Acinar Adenocarcinoma; Uterus Endometrial Adenocarcinoma Endometrioid; Uterine Corpus Endometrioid Carcinoma ^{2,5} |
| | D679Y | | | | | | | | |
| | Y680H | | | | | | | | |
| | NM_002529 | | | | | | | | |
| | NP_002520 | | | | | | | | |
| 9 | R682C | Protein kinase | 0.4500 | 1.0000 | 0.3333 | 0.0000 | 0.9932 | 0.0000 | Colon Adenocarcinoma; Lung Adenocarcinoma; Lung Squamous Cell Carcinoma |
| | R682H | | | | | | | | |
| | R682S | | | | | | | | |
| | V683G | | | | | | | | |
| | G684E | | | | | | | | |
| | NM_002529 | | | | | | | | |
| | NP_002520 | | | | | | | | |
| 8 | R686G | Protein kinase | 0.4000 | 1.0000 | 0.2500 | 0.9672 | 0.9957 | 0.0401 | Lung Adenocarcinoma; Colon Adenocarcinoma; Skin Melanoma |
| | R686H | | | | | | | | |
| | T687I | | | | | | | | |
| | M688I | | | | | | | | |
| | NM_002529 | | | | | | | | |
| | NP_002520 | | | | | | | | |
| 3 | T687I | Protein kinase | 0.1500 | 1.0000 | 1.0000 | 1.0000 | 0.9957 | 0.1493 | Skin Melanoma; Unknown Primary Melanoma; Uterus Endometrial Adenocarcinoma Papillary Serous |
| | M688I | | | | | | | | |
| | L689M | | | | | | | | |
| | NM_002529 | | | | | | | | |
| | NP_002520 | | | | | | | | |
| 3 | M688I | Protein kinase | 0.1500 | 1.0000 | 1.0000 | 1.0000 | 0.9957 | 0.1493 | Unknown Primary Melanoma; Lung Small Cell Undifferentiated Carcinoma; Uterus Endometrial Adenocarcinoma Papillary Serous |
| | L689M | | | | | | | | |
| | P690H | | | | | | | | |
| | NM_002529 | | | | | | | | |
| | NP_002520 | | | | | | | | |
| 6 | R692C | Protein kinase | 0.3000 | 1.0000 | 0.5000 | 0.9180 | 0.9957 | 0.1240 | Colon Adenocarcinoma; Skin Melanoma; Unknown Primary Adenocarcinoma |
| | R692H | | | | | | | | |
| | NM_002529 | | | | | | | | |
| | NP_002520 | | | | | | | | |
| 4 | P695S | Protein kinase | 0.2000 | 1.0000 | 1.0000 | 0.9836 | 0.9957 | 0.1778 | Skin Melanoma; Prostate Acinar Adenocarcinoma; Uterus Endometrial Adenocarcinoma Endometrioid |
| | P696L | | | | | | | | |
| | E697K | | | | | | | | |
| | NM_002529 | | | | | | | | |
| | NP_002520 | | | | | | | | |
| 1 | I699V | Protein kinase | 0.0500 | 1.0000 | 1.0000 | 1.0000 | 0.9878 | 0.0442 | Colon Adenocarcinoma |
| | NM_002529 | | | | | | | | |
| | NP_002520 | | | | | | | | |
| 12 | R702C | Protein kinase | 0.6000 | 1.0000 | 0.1667 | 0.8361 | 0.9957 | 0.0423 | Lung Adenocarcinoma; Ovary Serous Carcinoma; Skin Melanoma |
| | R702H | | | | | | | | |
| | R702L | | | | | | | | |
| | R702S | | | | | | | | |
| | NM_002529 | | | | | | | | |
| | NP_ 002520 | | | | | | | | |
| 2 | T705S | Protein kinase | 0.1000 | 1.0000 | 1.0000 | 0.0000 | 0.9917 | 0.0000 | Breast Carcinoma; Unknown Primary Adenocarcinoma |
| | T706K | | | | | | | | |
| | NM_002529 | | | | | | | | |
| | NP_002520 | | | | | | | | |
| 5 | D709N | Protein kinase | 0.2500 | 1.0000 | 0.0000 | 0.9508 | 0.9957 | 0.0000 | Lung Adenocarcinoma; Skin Melanoma; Unknown Primary Melanoma |
| | D709Y | | | | | | | | |
| | V710M | | | | | | | | |
| | NM_002529 | | | | | | | | |
| | NP_002520 | | | | | | | | |
| 1 | S712R | Protein kinase | 0.0500 | 1.0000 | 0.0000 | 1.0000 | 0.9957 | 0.0000 | Colon Adenocarcinoma |
| | NM_002529 | | | | | | | | |
| | NP_002520 | | | | | | | | |
| 3 | V715M | Protein kinase | 0.1500 | 1.0000 | 1.0000 | 0.9836 | 0.9957 | 0.0000 | Colon Adenocarcinoma; Breast Invasive Ductal Carcinoma; Uterus Endometrial Adenocarcinoma |
| | NM_002529 | | | | | | | | |
| | NP_002520 | | | | | | | | |
| 3 | E719D | Protein kinase | 0.1500 | 1.0000 | 0.3333 | 1.0000 | 0.9950 | 0.0415 | Colon Adenocarcinoma; Breast Invasive Ductal Carcinoma; Unknown Primary Melanoma |
| | E719K | | | | | | | | |
| | NM_002529 | | | | | | | | |
| | NP_002520 | | | | | | | | |
| 1 | Y723C | Protein kinase | 0.0500 | 1.0000 | 1.0000 | 1.0000 | 0.9857 | 0.0493 | Leukemia Lymphocytic Chronic |
| | NM_002529 | | | | | | | | |
| | NP_002520 | | | | | | | | |
| 2 | K725M | Protein kinase | 0.1000 | 1.0000 | 1.0000 | 1.0000 | 0.9857 | 0.0099 | Lung Adenocarcinoma; Breast Carcinoma |
| | K725T | | | | | | | | |
| | NM_002529 | | | | | | | | |
| | NP_002520 | | | | | | | | |
| 1 | W728R | Protein kinase | 0.0500 | 1.0000 | 1.0000 | 1.0000 | 0.9857 | 0.0493 | Lung Adenocarcinoma |
| | NM_002529 | | | | | | | | |
| | NP_002520 | | | | | | | | |
| 2 | N733S | Protein kinase | 0.1000 | 1.0000 | 1.0000 | 0.9016 | 0.9902 | 0.0000 | Stomach Adenocarcinoma; Unknown Primary Melanoma |
| | T734M | | | | | | | | |
| | NM_002529 | | | | | | | | |
| | NP_002520 | | | | | | | | |
| 2 | A736E | Protein kinase | 0.1000 | 1.0000 | 1.0000 | 1.0000 | 0.9744 | 0.0487 | Lung Adenocarcinoma; Uterus Endometrial Adenocarcinoma Papillary Serous |
| | A736T | | | | | | | | |
| | NM_002529 | | | | | | | | |
| | NP_002520 | | | | | | | | |
| 11 | T741M | Protein kinase | 0.5500 | 1.0000 | 1.0000 | 0.9016 | 0.9975 | 0.0000 | Lung Adenocarcinoma; Colon Adenocarcinoma; Kidney Renal Cell Carcinoma |
| | NM_002529 | | | | | | | | |
| | NP_002520 | | | | | | | | |
| 9 | G743R | Protein kinase | 0.4500 | 1.0000 | 1.0000 | 0.6066 | 0.9574 | 0.0000 | Lung Adenocarcinoma; Breast Carcinoma; Skin Melanoma |
| | R744C | | | | | | | | |
| | R744H | | | | | | | | |
| | NM_002529 | | | | | | | | |
| | NP_ 002520 | | | | | | | | |
| 7 | E747K | Protein kinase | 0.3500 | 1.0000 | 1.0000 | 0.9344 | 0.9837 | 0.1707 | Lung Adenocarcinoma; Lung Non-Small Cell Lung Carcinoma; Liver Hepatocellular Carcinoma |
| | R748L | | | | | | | | |
| | R748Q | | | | | | | | |
| | R748W | | | | | | | | |
| | P749Q | | | | | | | | |
| | NM_002529 | | | | | | | | |
| | NP_002520 | | | | | | | | |
| 11 | R748L | Protein kinase | 0.5500 | 1.0000 | 0.5455 | 0.9180 | 0.9887 | 0.1753 | Lung Adenocarcinoma; Breast Carcinoma; Lung Non-Small Cell Lung Carcinoma |
| | R748Q | | | | | | | | |
| | R748W | | | | | | | | |
| | P749Q | | | | | | | | |
| | R750C | | | | | | | | |
| | R750H | | | | | | | | |
| | R750L | | | | | | | | |
| | NM_002529 | | | | | | | | |
| | NP_002520 | | | | | | | | |
| 7 | P749Q | Protein kinase | 0.3500 | 1.0000 | 0.2857 | 0.9836 | 0.9975 | 0.0701 | Lung Adenocarcinoma; Breast Carcinoma; Head and Neck Squamous Cell Carcinoma |
| | R750C | | | | | | | | |
| | R750H | | | | | | | | |
| | R750L | | | | | | | | |
| | A751D | | | | | | | | |
| | NM_002529 | | | | | | | | |
| | NP_002520 | | | | | | | | |
| 3 | P753Q | Protein kinase | 0.1500 | 1.0000 | 0.0000 | 1.0000 | 0.9975 | 0.0000 | Lung Adenocarcinoma; Breast Carcinoma; Unknown Primary Carcinoma |
| | P754T | | | | | | | | |
| | E755Q | | | | | | | | |
| | NM_002529 | | | | | | | | |
| | NP_002520 | | | | | | | | |
| 9 | M760I | Protein kinase | 0.4500 | 1.0000 | 1.0000 | 0.5574 | 0.9763 | 0.0000 | Breast Carcinoma; Lung Adenocarcinoma; Colon Adenocarcinoma |
| | M760V | | | | | | | | |
| | R761Q | | | | | | | | |
| | R761W | | | | | | | | |
| | G762R | | | | | | | | |
| | NM_002529 | | | | | | | | |
| | NP_002520 | | | | | | | | |
| 8 | R761Q | Protein kinase | 0.4000 | 1.0000 | 1.0000 | 0.5574 | 0.9744 | 0.0000 | Lung Adenocarcinoma; Colon Adenocarcinoma; Breast Carcinoma |
| | R761W | | | | | | | | |
| | G762R | | | | | | | | |
| | C763F | | | | | | | | |
| | NM_002529 | | | | | | | | |
| | NP_002520 | | | | | | | | |
| 2 | R766L | Protein kinase | 0.1000 | 1.0000 | 1.0000 | 0.9344 | 0.9744 | 0.0910 | Lung Adenocarcinoma |
| | R766W | | | | | | | | |
| | NM_002529 | | | | | | | | |
| | NP_002520 | | | | | | | | |
| 1 | P768S | Protein kinase | 0.0500 | 1.0000 | 1.0000 | 1.0000 | 0.9975 | 0.0446 | Uterus Endometrial Adenocarcinoma Papillary Serous |
| | NM_002529 | | | | | | | | |
| | NP_002520 | | | | | | | | |
| 5 | R771H | Protein kinase | 0.2500 | 1.0000 | 1.0000 | 0.9508 | 0.7407 | 0.0000 | Lung Adenocarcinoma; Pancreas Ductal Adenocarcinoma; Unknown Primary Adenocarcinoma |
| | H772R | | | | | | | | |
| | NM_002529 | | | | | | | | |
| | NP_002520 | | | | | | | | |
| 4 | D776E | Protein kinase | 0.2000 | 1.0000 | 1.0000 | 1.0000 | 0.4962 | 0.0136 | Lung Adenocarcinoma; Colon Adenocarcinoma; Lung Non-Small Cell Lung Carcinoma |
| | D776N | | | | | | | | |
| | V777A | | | | | | | | |
| | NM_002529 | | | | | | | | |
| | NP_002520 | | | | | | | | |
| 8 | A779T | Protein kinase | 0.4000 | 1.0000 | 1.0000 | 0.9344 | 0.6010 | 0.0000 | Colon Adenocarcinoma; Ovary Serous Carcinoma; Unknown Primary Adenocarcinoma |
| | R780G | | | | | | | | |
| | R780W | | | | | | | | |
| | NM_002529 | | | | | | | | |
| | NP_002520 | | | | | | | | |
| 4 | P788L | | 0.2000 | 0.0500 | 1.0000 | 0.9836 | 0.9659 | 0.0000 | Colon Adenocarcinoma; Breast Carcinoma; Unknown Primary Adenocarcinoma |
| | P788S | | | | | | | | |
| | NM_002529 | | | | | | | | |
| | NP_002520 | | | | | | | | |
| 4 | V790I | | 0.2000 | 0.0500 | 1.0000 | 0.9180 | 0.9461 | 0.0000 | Lung Adenocarcinoma; Ovary Serous Carcinoma; Kidney Renal Cell Carcinoma |
| | V790L | | | | | | | | |
| | Y791H | | | | | | | | |
| | NM_002529 | | | | | | | | |
| | NP_002520 | | | | | | | | |

| | | | | | | | | | |
|---|---|---|---|---|---|---|---|---|---|
| ¹Ovarian Cancer Gene Database, ocgene.bioinfo-minzhao.org/gene_mutation.cgi?gene=4914, downloaded on May 31, 2016. ²Pediatric Cancer Gene Database, pedican.bioinfo-minzhao.org/gene_mutation.cgi?gene=4914, downloaded on May 31, 2016. ³Catalog of Somatic Mutations in Cancer (COSMIC) database, cancer.sanger.ac.uk/cosmic/mutation/ overview?id=1688778, downloaded on May 31, 2016. ⁴Catalog of Somatic Mutations in Cancer (COSMIC) database, cancer.sanger.ac.uk/cosmic/mutation/ overview?id=1259646, downloaded on May 31, 2016. ⁵Catalog of Somatic Mutations in Cancer (COSMIC) database, cancer.sanger.ac.uk/cosmic/mutation/overview?id=897427, downloaded on May 31, 2016. | | | | | | | | | |

**Table 2. Detected TrkB Protein Mutations Resulting from NTRK2 Point Mutations Detected in Cancer Biopsy Samples**

| Mutated Samples | Mutations Ref.Transcript/Protein | Domain | Hotspot Score | Domain Score | co-Alteration Score | Exac Score | Conservation Score | Total Score | Disease |
|---|---|---|---|---|---|---|---|---|---|
| 1 | W7R | | 0.0500 | 0.0500 | 0.0000 | 1.0000 | 0.9920 | 0.0000 | Breast Carcinoma |
| | NM_006180 | | | | | | | | |
| | NP_006171 | | | | | | | | |
| 3 | G9E | | 0.1500 | 0.0500 | 1.0000 | 1.0000 | 0.9828 | 0.0010 | Lung Adenocarcinoma; Soft Tissue Inflammatory Myofibroblastic Tumor |
| | G9V | | | | | | | | |
| | P10H | | | | | | | | |
| | NM_006180 | | | | | | | | |
| | NP_006171 | | | | | | | | |
| 1 | R14W | | 0.0500 | 0.0500 | 1.0000 | 1.0000 | 0.9975 | 0.0025 | Skin Squamous Cell Carcinoma |
| | NM_006180 | | | | | | | | |
| | NP_006171 | | | | | | | | |
| 2 | V23A | | 0.1000 | 0.0500 | 1.0000 | 0.9836 | 0.0000 | 0.0000 | Lung Non-Small Cell Lung Carcinoma; Unknown Primary Undifferentiated Neuroendocrine Carcinoma |
| | V23M | | | | | | | | |
| | NM_006180 | | | | | | | | |
| | NP_006171 | | | | | | | | |
| 1 | W26R | | 0.0500 | 0.0500 | 1.0000 | 0.9836 | 0.9920 | 0.0024 | Lung Adenocarcinoma |
| | NM_006180 | | | | | | | | |
| | NP_ 006171 | | | | | | | | |
| 3 | A28D | | 0.1500 | 0.0500 | 0.0000 | 1.0000 | 0.9628 | 0.0000 | Lung Adenocarcinoma; Gastroesophageal Junction Adenocarcinoma |
| | A29T | | | | | | | | |
| | NM_006180 | | | | | | | | |
| | NP_ 006171 | | | | | | | | |
| 1 | A31T | | 0.0500 | 0.0500 | 1.0000 | 0.9836 | 0.9511 | 0.0000 | Lung Adenosquamous Carcinoma |
| | NM_006180 | | | | | | | | |
| | NP_ 006171 | | | | | | | | |
| 3 | T34A | LRRNT | 0.1500 | 0.3000 | 0.3333 | 0.8525 | 0.5091 | 0.0000 | Breast Invasive Ductal Carcinoma; Kidney Renal Cell Carcinoma; Skin Melanoma |
| | T34R | | | | | | | | |
| | S35F | | | | | | | | |
| | NM_006180 | | | | | | | | |
| | NP_006171 | | | | | | | | |
| 1 | K37R | LRRNT | 0.0500 | 0.3000 | 1.0000 | 1.0000 | 0.0667 | 0.0000 | Bone Marrow Leukemia Non-Lymphocytic Acute Myelocytic |
| | NM_006180 | | | | | | | | |
| | NP_006171 | | | | | | | | |
| 1 | R42Q | LRRNT | 0.0500 | 0.3000 | 0.0000 | 1.0000 | 0.9975 | 0.0000 | Lung Adenocarcinoma |
| | NM_006180 | | | | | | | | |
| | NP_006171 | | | | | | | | |
| 5 | C45F | LRRNT | 0.2500 | 0.3000 | 1.0000 | 0.9836 | 0.9333 | 0.0510 | Lung Adenocarcinoma; Head and Neck Squamous Cell Carcinoma; Uterus Endometrial Adenocarcinoma Endometrioid |
| | C45R | | | | | | | | |
| | C45Y | | | | | | | | |
| | S46R | | | | | | | | |
| | D47N | | | | | | | | |
| | NM_006180 | | | | | | | | |
| | NP_ 006171 | | | | | | | | |
| 1 | G51D | LRRNT | 0.0500 | 0.3000 | 1.0000 | 1.0000 | 0.9975 | 0.0150 | Skin Melanoma |
| | NM_006180 | | | | | | | | |
| | NP_ 006171 | | | | | | | | |
| 1 | V53A | LRRNT | 0.0500 | 0.3000 | 1.0000 | 1.0000 | 0.9920 | 0.0015 | Unknown Primary Squamous Cell Carcinoma |
| | NM_006180 | | | | | | | | |
| | NP_006171 | | | | | | | | |
| 5 | P56L | LRRNT | 0.2500 | 0.3000 | 0.0000 | 1.0000 | 0.8209 | 0.0000 | Breast Ductal Carcinoma in Situ; Brain Glioblastoma; Unknown Primary Carcinoma |
| | P56S | | | | | | | | |
| | R57S | | | | | | | | |
| | NM_006180 | | | | | | | | |
| | NP_006171 | | | | | | | | |
| 1 | P60H | LRRNT | 0.0500 | 0.3000 | 1.0000 | 0.9836 | 0.9522 | 0.0000 | Lung Adenocarcinoma |
| | NM_006180 | | | | | | | | |
| | NP_006171 | | | | | | | | |
| 3 | P65H | | 0.1500 | 0.0500 | 1.0000 | 1.0000 | 0.8478 | 0.0028 | Thyroid Papillary Carcinoma; Stomach Adenocarcinoma Diffuse Type; Eye Intraocular Melanoma |
| | P65T | | | | | | | | |
| | E66D | | | | | | | | |
| | NM_006180 | | | | | | | | |
| | NP_006171 | | | | | | | | |
| 3 | T69P | | 0.1500 | 0.0500 | 1.0000 | 1.0000 | 0.9957 | 0.0025 | Kidney Urothelial Carcinoma; Uterus Carcinosarcoma; Bone Sarcoma |
| | T69S | | | | | | | | |
| | E70K | | | | | | | | |
| | NM_006180 | | | | | | | | |
| | NP_006171 | | | | | | | | |
| 1 | F72L | | 0.0500 | 0.0500 | 1.0000 | 1.0000 | 0.8906 | 0.0022 | Colon Adenocarcinoma |
| | NM_006180 | | | | | | | | |
| | NP_006171 | | | | | | | | |
| 2 | A74S | | 0.1000 | 0.0500 | 0.0000 | 1.0000 | 0.5829 | 0.0000 | Lung Non-Small Cell Lung Carcinoma; Soft Tissue Ewing Sarcoma |
| | N75K | | | | | | | | |
| | NM_006180 | | | | | | | | |
| | NP_006171 | | | | | | | | |
| 1 | R78K | | 0.0500 | 0.0500 | 1.0000 | 1.0000 | 0.9533 | 0.0012 | Brain Oligodendroglioma |
| | NM_006180 | | | | | | | | |
| | NP_ 006171 | | | | | | | | |
| 3 | E80Q | | 0.1500 | 0.0500 | 1.0000 | 1.0000 | 0.9575 | 0.0000 | Colon Adenocarcinoma; Lung Squamous Cell Carcinoma; Lung Atypical Carcinoid |
| | I81F | | | | | | | | |
| | I82V | | | | | | | | |
| | NM_006180 | | | | | | | | |
| | NP_006171 | | | | | | | | |
| 1 | E84K | | 0.0500 | 0.0500 | 1.0000 | 1.0000 | 0.9976 | 0.0012 | Head and Neck |
| | NM_006180 | | | | | | | | Squamous Cell Carcinoma |
| | NP_006171 | | | | | | | | |
| 5 | E88K | | 0.2500 | 0.0500 | 1.0000 | 1.0000 | 0.9976 | 0.0115 | Lung Adenocarcinoma; Lung Non-Small Cell Lung Carcinoma; Unknown Primary Melanoma |
| | E88Q | | | | | | | | |
| | A89T | | | | | | | | |
| | NM_006180 | | | | | | | | |
| | NP_ 006171 | | | | | | | | |
| 2 | T97A | LRR1 | 0.1000 | 0.3000 | 1.0000 | 1.0000 | 0.9924 | 0.0000 | Lung Adenocarcinoma; Ovary Granulosa Cell Tumor |
| | I98V | | | | | | | | |
| | NM_006180 | | | | | | | | |
| | NP_006171 | | | | | | | | |
| 2 | D100N | LRR1 | 0.1000 | 0.3000 | 1.0000 | 1.0000 | 0.9981 | 0.0299 | Unknown Primary Malignant Neoplasm; Duodenum Adenocarcinoma |
| | S101F | | | | | | | | |
| | NM_006180 | | | | | | | | |
| | NP_006171 | | | | | | | | |
| 1 | F105L | LRR1 | 0.0500 | 0.3000 | 1.0000 | 1.0000 | 0.9935 | 0.0133 | Skin Squamous Cell Carcinoma |
| | NM_006180 | | | | | | | | |
| | NP_006171 | | | | | | | | |
| 1 | A110E | LRR1 | 0.0500 | 0.3000 | 1.0000 | 1.0000 | 0.9981 | 0.0150 | Lung Adenocarcinoma |
| | NM_006180 | | | | | | | | |
| | NP_006171 | | | | | | | | |
| 1 | K113R | LRR1 | 0.0500 | 0.3000 | 1.0000 | 1.0000 | 0.8998 | 0.0135 | Ovary Serous Carcinoma |
| | NM_006180 | | | | | | | | |
| | NP_ 006171 | | | | | | | | |
| 2 | I120N | LRR2 | 0.1000 | 0.3000 | 1.0000 | 0.9836 | 0.9481 | 0.0210 | Pancreas Ductal Adenocarcinoma; Unknown Primary Adenocarcinoma |
| | I120V | | | | | | | | |
| | NM_006180 | | | | | | | | |
| | NP_006171 | | | | | | | | |
| 1 | F122I | LRR2 | 0.0500 | 0.3000 | 1.0000 | 0.9836 | 0.9049 | 0.0000 | Colon Carcinoid Tumor |
| | NM_006180 | | | | | | | | |
| | NP_006171 | | | | | | | | |
| 2 | R124Q | LRR2 | 0.1000 | 0.3000 | 1.0000 | 0.8525 | 0.5752 | 0.0000 | Ovary Serous Carcinoma; Lung Squamous Cell Carcinoma |
| | N125K | | | | | | | | |
| | NM_006180 | | | | | | | | |
| | NP_006171 | | | | | | | | |
| 2 | R132S | LRR2 | 0.1000 | 0.3000 | 0.0000 | 1.0000 | 0.3113 | 0.0000 | Lung Non-Small Cell Lung Carcinoma; Lymph Node Lymphoma Diffuse Large B Cell |
| | K133N | | | | | | | | |
| | NM_006180 | | | | | | | | |
| | NP_006171 | | | | | | | | |
| 2 | R136C | LRR2 | 0.1000 | 0.3000 | 1.0000 | 0.8689 | 0.9066 | 0.0211 | Colon Adenocarcinoma; Breast Ductal Carcinoma in Situ |
| | R136H | | | | | | | | |
| | NM_006180 | | | | | | | | |
| | NP_006171 | | | | | | | | |
| 2 | L138F | | 0.1000 | 0.0500 | 1.0000 | 1.0000 | 0.9977 | 0.0045 | Lung Adenocarcinoma; Thymus Carcinoma |
| | D139H | | | | | | | | |
| | NM_006180 | | | | | | | | |
| | NP_006171 | | | | | | | | |
| 5 | V146A | | 0.2500 | 0.0500 | 0.4000 | 1.0000 | 0.7006 | 0.0000 | Lung Adenocarcinoma; Colon Adenocarcinoma; Lung Small Cell Undifferentiated Carcinoma |
| | V146L | | | | | | | | |
| | G147S | | | | | | | | |
| | NM_006180 | | | | | | | | |
| | NP_006171 | | | | | | | | |
| 3 | W158C | LRRCT | 0.1500 | 0.3000 | 0.0000 | 1.0000 | 0.8642 | 0.0000 | Lung Adenocarcinoma; Liver Hepatocellular Carcinoma; Gastroesophageal Junction Adenocarcinoma |
| | I159F | | | | | | | | |
| | I159M | | | | | | | | |
| | NM_006180 | | | | | | | | |
| | NP_006171 | | | | | | | | |
| 1 | K166T | LRRCT | 0.0500 | 0.3000 | 1.0000 | 0.9836 | 0.9938 | 0.0000 | Breast Invasive Ductal Carcinoma |
| | NM_006180 | | | | | | | | |
| | NP_006171 | | | | | | | | |
| 1 | P169S | LRRCT | 0.0500 | 0.3000 | 1.0000 | 1.0000 | 0.9612 | 0.0072 | Soft Tissue Inflammatory Myofibroblastic Tumor |
| | NM_006180 | | | | | | | | |
| | NP_006171 | | | | | | | | |
| 2 | Q172K | LRRCT | 0.1000 | 0.3000 | 0.0000 | 1.0000 | 0.9650 | 0.0000 | Lung Adenocarcinoma |
| | D173Y | | | | | | | | |
| | NM_006180 | | | | | | | | |
| | NP_ 006171 | | | | | | | | |
| 1 | Y175H | LRRCT | 0.0500 | 0.3000 | 1.0000 | 1.0000 | 0.9938 | 0.0133 | Lung Small Cell Carcinoma |
| | NM_006180 | | | | | | | | |
| | NP_006171 | | | | | | | | |
| 1 | P185L | LRRCT | 0.0500 | 0.3000 | 1.0000 | 1.0000 | 0.9982 | 0.0150 | Skin Squamous Cell Carcinoma |
| | NM_006180 | | | | | | | | |
| | NP_006171 | | | | | | | | |
| 3 | A187E | LRRCT | 0.1500 | 0.3000 | 1.0000 | 0.8197 | 0.9226 | 0.0000 | Head and Neck Squamous Cell Carcinoma; Stomach Adenocarcinoma; Uterus Endometrial Adenocarcinoma Endometrioid |
| | A187S | | | | | | | | |
| | NM_006180 | | | | | | | | |
| | NP_006171 | | | | | | | | |
| 1 | I191T | LRRCT | 0.0500 | 0.3000 | 1.0000 | 1.0000 | 0.9938 | 0.0015 | Breast Carcinoma |
| | NM_006180 | | | | | | | | |
| | NP_006171 | | | | | | | | |
| 1 | N193S | LRRCT | 0.0500 | 0.3000 | 1.0000 | 1.0000 | 0.9938 | 0.0015 | Uterus Endometrial Adenocarcinoma |
| | NM_006180 | | | | | | | | |
| | NP_006171 | | | | | | | | |
| 2 | G195A | LRRCT | 0.1000 | 0.3000 | 1.0000 | 1.0000 | 0.9297 | 0.0000 | Lung Small Cell Undifferentiated Carcinoma; Unknown Primary Malignant Neoplasm |
| | L196F | | | | | | | | |
| | NM_006180 | | | | | | | | |
| | NP_006171 | | | | | | | | |
| 3 | S198T | Ig-likeC2-type1 | 0.1500 | 0.9000 | 0.0000 | 1.0000 | 0.9971 | 0.0000 | Lung Adenocarcinoma; Bone Marrow Leukemia Lymphocytic Chronic; Leukemia Lymphocytic Chronic |
| | A199T | | | | | | | | |
| | NM_006180 | | | | | | | | |
| | NP_006171 | | | | | | | | |
| 3 | A202D | Ig-likeC2-type1 | 0.1500 | 0.9000 | 1.0000 | 0.9344 | 0.9888 | 0.0000 | Lung Adenocarcinoma; Unknown Primary Carcinoma; Anus Squamous Cell Carcinoma |
| | A203S | | | | | | | | |
| | NM_006180 | | | | | | | | |
| | NP_006171 | | | | | | | | |
| 1 | T207I | Ig-likeC2-type1 | 0.0500 | 0.9000 | 1.0000 | 1.0000 | 0.9983 | 0.0402 | Skin Melanoma |
| | NM_006180 | | | | | | | | |
| | NP_006171 | | | | | | | | |
| 5 | E209D | Ig-likeC2-type1 | 0.2500 | 0.9000 | 0.4000 | 1.0000 | 0.9395 | 0.0743 | Lung Adenocarcinoma; Skin Melanoma; Unknown Primary Adenocarcinoma |
| | E209K | | | | | | | | |
| | E210V | | | | | | | | |
| | NM_006180 | | | | | | | | |
| | NP_006171 | | | | | | | | |
| 4 | A221V | Ig-likeC2-type1 | 0.2000 | 0.9000 | 1.0000 | 1.0000 | 0.9741 | 0.0000 | Lung Small Cell Undifferentiated Carcinoma; Uterus Endometrial Adenocarcinoma Endometrioid; Duodenum Adenocarcinoma |
| | G222D | | | | | | | | |
| | D223H | | | | | | | | |
| | NM_006180 | | | | | | | | |
| | NP_006171 | | | | | | | | |
| 4 | G222D | Ig-likeC2-type1 | 0.2000 | 0.9000 | 1.0000 | 1.0000 | 0.9828 | 0.0000 | Lung Small Cell Undifferentiated Carcinoma; Uterus Endometrial Adenocarcinoma Endometrioid; Fallopian Tube Serous Carcinoma |
| | D223H | | | | | | | | |
| | P224S | | | | | | | | |
| | NM_006180 | | | | | | | | |
| | NP_006171 | | | | | | | | |
| 4 | D223H | Ig-likeC2-type1 | 0.2000 | 0.9000 | 1.0000 | 1.0000 | 0.9417 | 0.0000 | Lung Small Cell Undifferentiated Carcinoma; Kidney Renal Cell Carcinoma; Fallopian Tube Serous Carcinoma |
| | P224S | | | | | | | | |
| | V225I | | | | | | | | |
| | NM_006180 | | | | | | | | |
| | NP_006171 | | | | | | | | |
| 1 | M228T | Ig-likeC2-type1 | 0.0500 | 0.9000 | 1.0000 | 1.0000 | 0.9942 | 0.0447 | Liver Hepatocellular Carcinoma |
| | NM_006180 | | | | | | | | |
| | NP_006171 | | | | | | | | |
| 1 | W230L | Ig-likeC2-type1 | 0.0500 | 0.9000 | 1.0000 | 1.0000 | 0.9984 | 0.0449 | Breast Metaplastic Carcinoma |
| | NM_006180 | | | | | | | | |
| | NP_006171 | | | | | | | | |
| 1 | N234Y | Ig-likeC2-type1 | 0.0500 | 0.9000 | 1.0000 | 1.0000 | 0.9946 | 0.0400 | Bladder Urothelial Carcinoma |
| | NM_006180 | | | | | | | | |
| | NP_006171 | | | | | | | | |
| 5 | M240I | Ig-likeC2-type1 | 0.2500 | 0.9000 | 0.4000 | 1.0000 | 0.9968 | 0.0425 | Skin Melanoma; Unknown Primary Adenocarcinoma; Unknown Primary Melanoma |
| | N241D | | | | | | | | |
| | E242K | | | | | | | | |
| | NM_006180 | | | | | | | | |
| | NP_006171 | | | | | | | | |
| 4 | S249F | Ig-likeC2-type1 | 0.2000 | 0.9000 | 1.0000 | 0.9016 | 0.7991 | 0.0000 | Unknown Primary Melanoma; Soft Tissue Leiomyosarcoma; Ovary Carcinosarcoma |
| | S249Y | | | | | | | | |
| | NM_006180 | | | | | | | | |
| | NP_006171 | | | | | | | | |
| 3 | R251G | Ig-likeC2-type1 | 0.1500 | 0.9000 | 1.0000 | 1.0000 | 0.9427 | 0.1273 | Breast Invasive Ductal Carcinoma; Lung Non-Small Cell Lung Carcinoma; Gastroesophageal Junction Adenocarcinoma |
| | R251K | | | | | | | | |
| | I252V | | | | | | | | |
| | NM_006180 | | | | | | | | |
| | NP_006171 | | | | | | | | |
| 1 | N254S | Ig-likeC2-type1 | 0.0500 | 0.9000 | 1.0000 | 1.0000 | 0.9925 | 0.0400 | Colon Adenocarcinoma |
| | NM_006180 | | | | | | | | |
| | NP_006171 | | | | | | | | |
| 4 | S256L | Ig-likeC2-type1 | 0.2000 | 0.9000 | 1.0000 | 1.0000 | 0.9976 | 0.1520 | Lung Adenocarcinoma; Soft Tissue Sarcoma; Unknown Primary Melanoma |
| | S257F | | | | | | | | |
| | D258N | | | | | | | | |
| | NM_006180 | | | | | | | | |
| | NP_ 006171 | | | | | | | | |
| 1 | G261 R | Ig-likeC2-type1 | 0.0500 | 0.9000 | 1.0000 | 0.9836 | 0.9976 | 0.0395 | Lung Adenocarcinoma |
| | NM_006180 | | | | | | | | |
| | NP_006171 | | | | | | | | |
| 1 | I264M | Ig-likeC2-type1 | 0.0500 | 0.9000 | 1.0000 | 1.0000 | 0.9976 | 0.0402 | Head and Neck Squamous Cell Carcinoma |
| | NM_006180 | | | | | | | | |
| | NP_ 006171 | | | | | | | | |
| 2 | C266S | Ig-likeC2-type1 | 0.1000 | 0.9000 | 1.0000 | 1.0000 | 0.9976 | 0.0898 | Colon Adenocarcinoma; Bladder Urothelial Carcinoma |
| | C266Y | | | | | | | | |
| | NM_006180 | | | | | | | | |
| | NP_006171 | | | | | | | | |
| 5 | A268V | Ig-likeC2-type1 | 0.2500 | 0.9000 | 0.0000 | 1.0000 | 0.9976 | 0.0000 | Lung Adenocarcinoma; Gastroesophageal Junction Adenocarcinoma; Rectum Adenocarcinoma |
| | NM_006180 | | | | | | | | |
| | NP_006171 | | | | | | | | |
| 1 | V272E | Ig-likeC2-type1 | 0.0500 | 0.9000 | 0.0000 | 1.0000 | 0.9925 | 0.0000 | Lung Adenocarcinoma |
| | NM_006180 | | | | | | | | |
| | NP_ 006171 | | | | | | | | |
| 2 | V279A | Ig-likeC2-type1 | 0.1000 | 0.9000 | 0.0000 | 0.9836 | 0.9927 | 0.0000 | Lung Non-Small Cell Lung Carcinoma; Lymph Node Lymphoma Follicular Lymphoma |
| | N280I | | | | | | | | |
| | NM_006180 | | | | | | | | |
| | NP_006171 | | | | | | | | |
| 3 | A286P | | 0.1500 | 0.0500 | 1.0000 | 1.0000 | 0.9986 | 0.0070 | Liver Hepatocellular Carcinoma; Unknown Primary Adenocarcinoma |
| | A286T | | | | | | | | |
| | NM_006180 | | | | | | | | |
| | NP_ 006171 | | | | | | | | |
| 1 | I289V | | 0.0500 | 0.0500 | 1.0000 | 1.0000 | 0.9950 | 0.0000 | Stomach Adenocarcinoma |
| | NM_006180 | | | | | | | | |
| | NP_006171 | | | | | | | | |
| 6 | L292I | | 0.3000 | 0.0500 | 1.0000 | 1.0000 | 0.9840 | 0.0133 | Unknown Primary Squamous Cell Carcinoma; Unknown Primary Carcinoma; Unknown Primary Malignant Neoplasm |
| | E293D | | | | | | | | |
| | E293K | | | | | | | | |
| | S294F | | | | | | | | |
| | NM_006180 | | | | | | | | |
| | NP_006171 | | | | | | | | |
| 7 | E293D | | 0.3500 | 0.0500 | 0.5714 | 1.0000 | 0.9861 | 0.0089 | Unknown Primary Squamous Cell Carcinoma; Bone Marrow Multiple Myeloma; Skin Melanoma |
| | E293K | | | | | | | | |
| | S294F | | | | | | | | |
| | P295S | | | | | | | | |
| | NM_006180 | | | | | | | | |
| | NP_ 006171 | | | | | | | | |
| 5 | S294F | Ig-likeC2-type2 | 0.2500 | 0.9000 | 0.4000 | 1.0000 | 0.9986 | 0.0861 | Bone Marrow Multiple Myeloma; Skin Melanoma; Unknown Primary Melanoma |
| | P295S | | | | | | | | |
| | T296I | | | | | | | | |
| | NM_006180 | | | | | | | | |
| | NP_ 006171 | | | | | | | | |
| 1 | P304L | Ig-likeC2-type2 | 0.0500 | 0.9000 | 1.0000 | 1.0000 | 0.9986 | 0.0449 | Colon Adenocarcinoma |
| | NM_006180 | | | | | | | | |
| | NP_ 006171 | | | | | | | | |
| 2 | N310del | Ig-likeC2-type2 | 0.1000 | 0.9000 | 1.0000 | 1.0000 | 0.9964 | 0.0802 | Lung Adenocarcinoma; Head and Neck Neuroblastoma |
| | P311H | | | | | | | | |
| | NM_006180 | | | | | | | | |
| | NP_ 006171 | | | | | | | | |
| 5 | A314E | Ig-likeC2-type2 | 0.2500 | 0.9000 | 1.0000 | 0.9672 | 0.9984 | 0.0610 | Colon Adenocarcinoma; Lung Non-Small Cell Lung Carcinoma; Unknown Primary Undifferentiated Neuroendocrine Carcinoma |
| | A314G | | | | | | | | |
| | A314V | | | | | | | | |
| | L315F | | | | | | | | |
| | NM_006180 | | | | | | | | |
| | NP_006171 | | | | | | | | |
| 1 | Y319C | Ig-likeC2-type2 | 0.0500 | 0.9000 | 1.0000 | 1.0000 | 0.9945 | 0.0448 | Lung Non-Small Cell Lung Carcinoma |
| | NM_006180 | | | | | | | | |
| | NP_006171 | | | | | | | | |
| 2 | G321V | Ig-likeC2-type2 | 0.1000 | 0.9000 | 1.0000 | 1.0000 | 0.9984 | 0.0899 | Unknown Primary Adenocarcinoma |
| | NM_006180 | | | | | | | | |
| | NP_006171 | | | | | | | | |
| 1 | E326D | Ig-likeC2-type2 | 0.0500 | 0.9000 | 0.0000 | 1.0000 | 0.9666 | 0.0000 | Lung Adenocarcinoma |
| | NM_006180 | | | | | | | | |
| | NP_006171 | | | | | | | | |
| 1 | K328Q | Ig-likeC2-type2 | 0.0500 | 0.9000 | 1.0000 | 0.9672 | 0.9945 | 0.0387 | Lymph Node Lymphoma Diffuse Large B Cell |
| | NM_006180 | | | | | | | | |
| | NP_ 006171 | | | | | | | | |
| 2 | C331F | Ig-likeC2-type2 | 0.1000 | 0.9000 | 0.0000 | 1.0000 | 0.9984 | 0.0000 | Lung Adenocarcinoma; Uterus Leiomyosarcoma |
| | C331Y | | | | | | | | |
| | NM_006180 | | | | | | | | |
| | NP_ 006171 | | | | | | | | |
| 1 | H335L | Ig-likeC2-type2 | 0.0500 | 0.9000 | 1.0000 | 1.0000 | 0.9945 | 0.0400 | Lung Adenocarcinoma |
| | NM_006180 | | | | | | | | |
| | NP_ 006171 | | | | | | | | |
| 2 | E341K | Ig-likeC2-type2 | 0.1000 | 0.9000 | 1.0000 | 1.0000 | 0.9964 | 0.0897 | Colon Adenocarcinoma; Gallbladder Glomus Tumor |
| | E341V | | | | | | | | |
| | NM_006180 | | | | | | | | |
| | NP_006171 | | | | | | | | |
| 1 | H343D | Ig-likeC2-type2 | 0.0500 | 0.9000 | 1.0000 | 1.0000 | 0.9984 | 0.0449 | Pancreas Carcinoma |
| | NM_006180 | | | | | | | | |
| | NP_006171 | | | | | | | | |
| 3 | D349Y | Ig-likeC2-type2 | 0.1500 | 0.9000 | 1.0000 | 1.0000 | 0.4799 | 0.0648 | Lung Adenocarcinoma; Unknown Primary Adenocarcinoma; Adrenal Gland Cortical Carcinoma |
| | N350I | | | | | | | | |
| | N350K | | | | | | | | |
| | NM_006180 | | | | | | | | |
| | NP_006171 | | | | | | | | |
| 1 | M354I | Ig-likeC2-type2 | 0.0500 | 0.9000 | 1.0000 | 1.0000 | 0.9984 | 0.0402 | Skin Melanoma |
| | NM_006180 | | | | | | | | |
| | NP_006171 | | | | | | | | |
| 2 | G357R | Ig-likeC2-type2 | 0.1000 | 0.9000 | 1.0000 | 1.0000 | 0.9984 | 0.0851 | Stomach Adenocarcinoma; Unknown Primary Melanoma |
| | D358Y | | | | | | | | |
| | NM_006180 | | | | | | | | |
| | NP_006171 | | | | | | | | |
| 1 | D370Y | | 0.0500 | 0.0500 | 1.0000 | 1.0000 | 0.9984 | 0.0025 | Lung Adenocarcinoma |
| | NM_006180 | | | | | | | | |
| | NP_006171 | | | | | | | | |
| 1 | Q373L | | 0.0500 | 0.0500 | 1.0000 | 1.0000 | 0.9945 | 0.0025 | Liver Cholangiocarcinoma |
| | NM_006180 | | | | | | | | |
| | NP_ 006171 | | | | | | | | |
| 1 | H377Y | | 0.0500 | 0.0500 | 1.0000 | 1.0000 | 0.9984 | 0.0025 | Skin Squamous Cell Carcinoma; |
| | NM_006180 | | | | | | | | |
| | NP_006171 | | | | | | | | |
| 2 | M379T | | 0.1000 | 0.0500 | 0.0000 | 1.0000 | 0.9945 | 0.0000 | Lung Adenocarcinoma; Bladder Urothelial Carcinoma |
| | M379V | | | | | | | | |
| | NM_006180 | | | | | | | | |
| | NP_ 006171 | | | | | | | | |
| 7 | D385G | | 0.3500 | 0.0500 | 0.1429 | 0.9836 | 0.7950 | 0.0018 | Colon Adenocarcinoma; Lung Adenocarcinoma; Breast Invasive Ductal Carcinoma |
| | D386N | | | | | | | | |
| | G387C | | | | | | | | |
| | NM_006180 | | | | | | | | |
| | NP_ 006171 | | | | | | | | |
| 7 | D386N | | 0.3500 | 0.0500 | 0.5714 | 0.9836 | 0.7731 | 0.0069 | Lung Adenocarcinoma; Colon Adenocarcinoma; Breast Carcinoma |
| | G387C | | | | | | | | |
| | A388V | | | | | | | | |
| | NM_006180 | | | | | | | | |
| | NP_ 006171 | | | | | | | | |
| 3 | G387C | | 0.1500 | 0.0500 | 1.0000 | 1.0000 | 0.8177 | 0.0061 | Lung Adenocarcinoma; Breast Carcinoma; Lung Large Cell Carcinoma |
| | A388V | | | | | | | | |
| | N389I | | | | | | | | |
| | NM_006180 | | | | | | | | |
| | NP_006171 | | | | | | | | |
| 3 | A388V | | 0.1500 | 0.0500 | 1.0000 | 1.0000 | 0.5854 | 0.0044 | Breast Carcinoma; Unknown Primary Squamous Cell Carcinoma; Lung Large Cell Carcinoma |
| | N389I | | | | | | | | |
| | P390R | | | | | | | | |
| | NM_006180 | | | | | | | | |
| | NP_006171 | | | | | | | | |
| 4 | D394H | | 0.2000 | 0.0500 | 1.0000 | 1.0000 | 0.9535 | 0.0033 | Colon Adenocarcinoma; Unknown Primary Adenocarcinoma; Bladder Urothelial Carcinoma |
| | D394N | | | | | | | | |
| | D394Y | | | | | | | | |
| | V395del | | | | | | | | |
| | NM_006180 | | | | | | | | |
| | NP_006171 | | | | | | | | |
| 1 | E398K | | 0.0500 | 0.0500 | 1.0000 | 1.0000 | 0.9960 | 0.0025 | Unknown Primary Melanoma |
| | NM_006180 | | | | | | | | |
| | NP_006171 | | | | | | | | |
| 3 | G401A | | 0.1500 | 0.0500 | 1.0000 | 1.0000 | 0.9815 | 0.0000 | Lung Adenocarcinoma; Skin Melanoma; Soft Tissue Liposarcoma |
| | G401E | | | | | | | | |
| | G401R | | | | | | | | |
| | NM_006180 | | | | | | | | |
| | NP_006171 | | | | | | | | |
| 3 | G408R | | 0.1500 | 0.0500 | 1.0000 | 0.9672 | 0.9985 | 0.0000 | Lung Adenocarcinoma; Breast Invasive Ductal Carcinoma |
| | NM_006180 | | | | | | | | |
| | NP_006171 | | | | | | | | |
| 1 | T410N | | 0.0500 | 0.0500 | 1.0000 | 0.9836 | 0.9989 | 0.0002 | Bone Marrow Lymph Proliferative Disease |
| | NM_006180 | | | | | | | | |
| | NP_006171 | | | | | | | | |
| 1 | S414I | | 0.0500 | 0.0500 | 1.0000 | 1.0000 | 0.9740 | 0.0024 | Lung Adenocarcinoma |
| | NM_006180 | | | | | | | | |
| | NP_006171 | | | | | | | | |
| 1 | E416G | | 0.0500 | 0.0500 | 1.0000 | 0.9836 | 0.7364 | 0.0000 | Thymus Thymoma Lymphocytic |
| | NM_006180 | | | | | | | | |
| | NP_006171 | | | | | | | | |
| 1 | S419F | | 0.0500 | 0.0500 | 1.0000 | 1.0000 | 0.9989 | 0.0012 | Breast Carcinoma |
| | NM_006180 | | | | | | | | |
| | NP_006171 | | | | | | | | |
| 2 | T423I | | 0.1000 | 0.0500 | 1.0000 | 1.0000 | 0.3050 | 0.0000 | Brain Glioblastoma; Uterus Endometrial Adenocarcinoma Endometrioid |
| | T423S | | | | | | | | |
| | NM_006180 | | | | | | | | |
| | NP_006171 | | | | | | | | |
| 10 | T426I | | 0.5000 | 0.0500 | 0.5000 | 0.8689 | 0.8171 | 0.0000 | Skin Melanoma; Lung Adenocarcinoma; Colon Adenocarcinoma |
| | G427S | | | | | | | | |
| | R428Q | | | | | | | | |
| | NM_006180 | | | | | | | | |
| | NP_006171 | | | | | | | | |
| 6 | H430Y | | 0.3000 | 0.0500 | 1.0000 | 0.7869 | 0.9989 | 0.0000 | Colon Adenocarcinoma; Bone Marrow Multiple Myeloma; Liver Cholangiocarcinoma |
| | NM_006180 | | | | | | | | |
| | NP_006171 | | | | | | | | |
| 3 | S432L | | 0.1500 | 0.0500 | 1.0000 | 1.0000 | 0.9989 | 0.0000 | Lung Adenocarcinoma; Lung Non-Small Cell Lung Carcinoma; Gallbladder Adenocarcinoma |
| | NM_006180 | | | | | | | | |
| | NP_006171 | | | | | | | | |
| 1 | A435V | | 0.0500 | 0.0500 | 1.0000 | 1.0000 | 0.9989 | 0.0025 | Soft Tissue Paraganglioma |
| | NM_006180 | | | | | | | | |
| | NP_006171 | | | | | | | | |
| 4 | A440S | | 0.2000 | 0.0500 | 1.0000 | 0.9836 | 0.9989 | 0.0044 | Colon Adenocarcinoma; Breast Carcinoma; Breast Ductal Carcinoma in Situ |
| | A440T | | | | | | | | |
| | A440V | | | | | | | | |
| | NM_006180 | | | | | | | | |
| | NP_006171 | | | | | | | | |
| 2 | V442L | | 0.1000 | 0.0500 | 1.0000 | 0.9344 | 0.9989 | 0.0000 | Lung Adenocarcinoma |
| | V442M | | | | | | | | |
| | NM_006180 | | | | | | | | |
| | NP_ 006171 | | | | | | | | |
| 1 | C446Y | | 0.0500 | 0.0500 | 1.0000 | 1.0000 | 0.9989 | 0.0003 | Lung Adenocarcinoma |
| | NM_006180 | | | | | | | | |
| | NP_ 006171 | | | | | | | | |
| 1 | V449I | | 0.0500 | 0.0500 | 1.0000 | 0.9344 | 0.9736 | 0.0000 | Pancreas Ductal Adenocarcinoma |
| | NM_006180 | | | | | | | | |
| | NP_006171 | | | | | | | | |
| 1 | F452L | | 0.0500 | 0.0500 | 1.0000 | 1.0000 | 0.9247 | 0.0021 | Stomach Adenocarcinoma |
| | NM_006180 | | | | | | | | |
| | NP_ 006171 | | | | | | | | |
| 2 | L454I | | 0.1000 | 0.0500 | 1.0000 | 1.0000 | 0.9191 | 0.0000 | Bladder Urothelial Carcinoma; Stomach Adenocarcinoma |
| | K455N | | | | | | | | |
| | NM_006180 | | | | | | | | |
| | NP_006171 | | | | | | | | |
| 2 | R458G | | 0.1000 | 0.0500 | 1.0000 | 1.0000 | 0.9946 | 0.0000 | Liver Cholangiocarcinoma; Uterus Endometrial Adenocarcinoma Endometrioid |
| | NM_006180 | | | | | | | | |
| | NP_006171 | | | | | | | | |
| 3 | S460F | | 0.1500 | 0.0500 | 1.0000 | 1.0000 | 0.9737 | 0.0073 | Lung Adenocarcinoma; Liver Cholangiocarcinoma; Soft Tissue Synovial Sarcoma |
| | S460T | | | | | | | | |
| | S460Y | | | | | | | | |
| | NM_006180 | | | | | | | | |
| | NP_ 006171 | | | | | | | | |
| 1 | M464V | | 0.0500 | 0.0500 | 1.0000 | 1.0000 | 0.9953 | 0.0000 | Soft Tissue Sarcoma |
| | NM_006180 | | | | | | | | |
| | NP_006171 | | | | | | | | |
| 3 | V475A | | 0.1500 | 0.0500 | 0.0000 | 1.0000 | 0.9916 | 0.0000 | Lung Adenocarcinoma; Skin Squamous Cell Carcinoma; Lung Adenosquamous Carcinoma |
| | K476E | | | | | | | | |
| | K476I | | | | | | | | |
| | NM_006180 | | | | | | | | |
| | NP_006171 | | | | | | | | |
| 7 | G480D | | 0.3500 | 0.0500 | 1.0000 | 0.9344 | 0.9733 | 0.0000 | Lung Adenocarcinoma; Colon Adenocarcinoma; Lung Non-Small Cell Lung Carcinoma |
| | V481I | | | | | | | | |
| | G482R | | | | | | | | |
| | G482VNM_006180 | | | | | | | | |
| | NP_006171 | | | | | | | | |
| 7 | V481I | | 0.3500 | 0.0500 | 0.7143 | 0.9344 | 0.9734 | 0.0035 | Lung Non-Small Cell Lung Carcinoma; Lung Adenocarcinoma; Colon Adenocarcinoma |
| | G482R | | | | | | | | |
| | G482V | | | | | | | | |
| | P483T | | | | | | | | |
| | NM_006180 | | | | | | | | |
| | NP_ 006171 | | | | | | | | |
| 4 | G482R | | 0.2000 | 0.0500 | 0.5000 | 0.9836 | 0.9982 | 0.0043 | Colon Adenocarcinoma; Kidney Renal Cell Carcinoma; Lung Non-Small Cell Lung Carcinoma |
| | G482V | | | | | | | | |
| | P483T | | | | | | | | |
| | A484T | | | | | | | | |
| | NM_006180 | | | | | | | | |
| | NP_ 006171 | | | | | | | | |
| 3 | V486F | | 0.1500 | 0.0500 | 0.0000 | 0.9672 | 0.9984 | 0.0000 | Colon Adenocarcinoma; Lung Non-Small Cell Lung Carcinoma; Bladder Urothelial Carcinoma |
| | V486I | | | | | | | | |
| | NM_006180 | | | | | | | | |
| | NP_006171 | | | | | | | | |
| 2 | P496R | | 0.1000 | 0.0500 | 0.0000 | 1.0000 | 0.9984 | 0.0000 | Lung Adenocarcinoma; Brain Astrocytoma; |
| | P496S | | | | | | | | |
| | NM_006180 | | | | | | | | |
| | NP_006171 | | | | | | | | |
| 2 | H498N | | 0.1000 | 0.0500 | 1.0000 | 1.0000 | 0.9984 | 0.0047 | Lung Adenocarcinoma; Skin Melanoma |
| | H498Y | | | | | | | | |
| | NM_006180 | | | | | | | | |
| | NP_ 006171 | | | | | | | | |
| 1 | S501C | | 0.0500 | 0.0500 | 1.0000 | 1.0000 | 0.9984 | 0.0025 | Gastroesophageal Junction Adenocarcinoma |
| | NM_006180 | | | | | | | | |
| | NP_006171 | | | | | | | | |
| 1 | G503W | | 0.0500 | 0.0500 | 1.0000 | 1.0000 | 0.9982 | 0.0022 | Lung Adenocarcinoma |
| | NM_006180 | | | | | | | | |
| | NP_006171 | | | | | | | | |
| 3 | P514L | | 0.1500 | 0.0500 | 1.0000 | 1.0000 | 0.9984 | 0.0024 | Unknown Primary Adenocarcinoma; Stomach Adenocarcinoma; Unknown Primary Urothelial Carcinoma |
| | P514S | | | | | | | | |
| | D515N | | | | | | | | |
| | NM_006180 | | | | | | | | |
| | NP_006171 | | | | | | | | |
| 1 | V517I | | 0.0500 | 0.0500 | 1.0000 | 1.0000 | 0.9984 | 0.0000 | Gastroesophageal Junction Adenocarcinoma |
| | NM_006180 | | | | | | | | |
| | NP_006171 | | | | | | | | |
| 1 | I519del | | 0.0500 | 0.0500 | 0.0000 | 1.0000 | 0.9944 | 0.0000 | Breast Carcinoma |
| | NM_006180 | | | | | | | | |
| | NP_006171 | | | | | | | | |
| 1 | M521L | | 0.0500 | 0.0500 | 1.0000 | 1.0000 | 0.9944 | 0.0012 | Gastroesophageal Junction Adenocarcinoma |
| | NM_006180 | | | | | | | | |
| | NP_006171 | | | | | | | | |
| 1 | I524F | | 0.0500 | 0.0500 | 1.0000 | 1.0000 | 0.9944 | 0.0025 | Unknown Primary Carcinoma |
| | NM_006180 | | | | | | | | |
| | NP_006171 | | | | | | | | |
| 1 | E528K | | 0.0500 | 0.0500 | 0.0000 | 1.0000 | 0.9984 | 0.0000 | Skin Melanoma |
| | NM_006180 | | | | | | | | |
| | NP_006171 | | | | | | | | |
| 1 | P530L | | 0.0500 | 0.0500 | 1.0000 | 1.0000 | 0.9984 | 0.0012 | Colon Adenocarcinoma |
| | NM_006180 | | | | | | | | |
| | NP_006171 | | | | | | | | |
| 2 | Q539H | | 0.1000 | 0.0500 | 1.0000 | 1.0000 | 0.5850 | 0.0029 | Liver Cholangiocarcinoma; Lung Small Cell Undifferentiated Carcinoma |
| | NM_006180 | | | | | | | | |
| | NP_006171 | | | | | | | | |
| 1 | F545V | | 0.0500 | 0.0500 | 1.0000 | 1.0000 | 0.9939 | 0.0000 | Lung Adenocarcinoma |
| | NM_006180 | | | | | | | | |
| | NP_006171 | | | | | | | | |
| 1 | Q547R | | 0.0500 | 0.0500 | 1.0000 | 1.0000 | 0.9957 | 0.0000 | Lung Adenocarcinoma |
| | NM_006180 | | | | | | | | |
| | NP_ 006171 | | | | | | | | |
| 1 | I549M | | 0.0500 | 0.0500 | 1.0000 | 1.0000 | 0.9989 | 0.0022 | Breast Ductal Carcinoma in Situ |
| | NM_006180 | | | | | | | | |
| | NP_006171 | | | | | | | | |
| 2 | H552Q | | 0.1000 | 0.0500 | 0.0000 | 1.0000 | 0.6398 | 0.0000 | Lung Adenocarcinoma; Soft Tissue Leiomyosarcoma |
| | N553S | | | | | | | | |
| | NM_006180 | | | | | | | | |
| | NP_006171 | | | | | | | | |
| 4 | R558K | Protein kinase | 0.2000 | 1.0000 | 0.2500 | 1.0000 | 0.9810 | 0.0400 | Lung Adenocarcinoma; Brain Glioblastoma; Unknown Primary Melanoma |
| | E559D | | | | | | | | |
| | E559K | | | | | | | | |
| | NM_006180 | | | | | | | | |
| | NP_006171 | | | | | | | | |
| 5 | G561S | Protein kinase | 0.2500 | 1.0000 | 0.4000 | 1.0000 | 0.9989 | 0.0925 | Skin Melanoma; Lung Adenocarcinoma; Bladder Urothelial Carcinoma |
| | E562K | | | | | | | | |
| | G563R | | | | | | | | |
| | G563V | | | | | | | | |
| | NM_006180 | | | | | | | | |
| | NP_006171 | | | | | | | | |
| 5 | E562K | Protein kinase | 0.2500 | 1.0000 | 0.4000 | 1.0000 | 0.9989 | 0.0696 | Skin Melanoma; Breast Invasive Ductal Carcinoma; Bladder Urothelial Carcinoma |
| | G563R | | | | | | | | |
| | G563V | | | | | | | | |
| | A564T | | | | | | | | |
| | NM_006180 | | | | | | | | |
| | NP_006171 | | | | | | | | |
| 1 | G566E | Protein kinase | 0.0500 | 1.0000 | 1.0000 | 1.0000 | 0.9989 | 0.0499 | Unknown Primary Melanoma |
| | NM_006180 | | | | | | | | |
| | NP_006171 | | | | | | | | |
| 1 | F569L | Protein kinase | 0.0500 | 1.0000 | 1.0000 | 1.0000 | 0.9987 | 0.0250 | Gastroesophageal Junction Adenocarcinoma |
| | NM_006180 | | | | | | | | |
| | NP_006171 | | | | | | | | |
| 1 | Y574H | Protein kinase | 0.0500 | 1.0000 | 1.0000 | 1.0000 | 0.9954 | 0.0000 | Bone Sarcoma |
| | NM_006180 | | | | | | | | |
| | NP_006171 | | | | | | | | |
| 6 | C577S | Protein kinase | 0.3000 | 1.0000 | 0.5000 | 1.0000 | 0.9653 | 0.0905 | Ovary Epithelial Carcinoma; Lung Adenocarcinoma; Bladder Urothelial Carcinoma |
| | P578H | | | | | | | | |
| | P578L | | | | | | | | |
| | P578S | | | | | | | | |
| | P578T | | | | | | | | |
| | E579D | | | | | | | | |
| | NM_006180 | | | | | | | | |
| | NP_ 006171 | | | | | | | | |
| 6 | P578H | Protein kinase | 0.3000 | 1.0000 | 0.5000 | 1.0000 | 0.9647 | 0.0181 | Lung Adenocarcinoma; Bladder Urothelial Carcinoma; Ovary Epithelial Carcinoma |
| | P578L | | | | | | | | |
| | P578S | | | | | | | | |
| | P578T | | | | | | | | |
| | E579D | | | | | | | | |
| | Q580P | | | | | | | | |
| | NM_006180 | | | | | | | | |
| | NP_ 006171 | | | | | | | | |
| 3 | E579D | Protein kinase | 0.1500 | 1.0000 | 1.0000 | 1.0000 | 0.9469 | 0.0187 | Colon Adenocarcinoma; Peritoneum Serous Carcinoma; Stomach Leiomyosarcoma |
| | Q580P | | | | | | | | |
| | D581N | | | | | | | | |
| | NM_006180 | | | | | | | | |
| | NP_006171 | | | | | | | | |
| 3 | Q580P | Protein kinase | 0.1500 | 1.0000 | 1.0000 | 1.0000 | 0.9965 | 0.0629 | Colon Adenocarcinoma; Stomach Adenocarcinoma Intestinal Type; Peritoneum Serous Carcinoma |
| | D581N | | | | | | | | |
| | K582T | | | | | | | | |
| | NM_006180 | | | | | | | | |
| | NP_006171 | | | | | | | | |
| 1 | L584F | Protein kinase | 0.0500 | 1.0000 | 1.0000 | 0.9836 | 0.9987 | 0.0000 | Breast Carcinoma |
| | NM_006180 | | | | | | | | |
| | NP_006171 | | | | | | | | |
| 1 | T589S | Protein kinase | 0.0500 | 1.0000 | 1.0000 | 1.0000 | 0.9969 | 0.0000 | Rectum Adenocarcinoma |
| | NM_006180 | | | | | | | | |
| | NP_ 006171 | | | | | | | | |
| 1 | D592A | Protein kinase | 0.0500 | 1.0000 | 1.0000 | 1.0000 | 0.9906 | 0.0495 | Unknown Primary Melanoma |
| | NM_006180 | | | | | | | | |
| | NP_006171 | | | | | | | | |
| 1 | D595E | Protein kinase | 0.0500 | 1.0000 | 0.0000 | 1.0000 | 0.9456 | 0.0000 | Lung Small Cell Undifferentiated Carcinoma |
| | NM_006180 | | | | | | | | |
| | NP_006171 | | | | | | | | |
| 4 | R598C | Protein kinase | 0.2000 | 1.0000 | 1.0000 | 1.0000 | 0.9953 | 0.1991 | Lung Adenocarcinoma; Breast Carcinoma; Soft Tissue Sarcoma |
| | R598S | | | | | | | | |
| | K599M | | | | | | | | |
| | D600H | | | | | | | | |
| | NM_006180 | | | | | | | | |
| | NP_006171 | | | | | | | | |
| 3 | H602N | Protein kinase | 0.1500 | 1.0000 | 1.0000 | 1.0000 | 0.9964 | 0.1495 | Lung Adenocarcinoma; Lung Squamous Cell Carcinoma; Unknown Primary Sarcomatoid Carcinoma |
| | R603S | | | | | | | | |
| | NM_006180 | | | | | | | | |
| | NP_006171 | | | | | | | | |
| 1 | L608M | Protein kinase | 0.0500 | 1.0000 | 1.0000 | 1.0000 | 0.9344 | 0.0047 | Breast Invasive Ductal Carcinoma |
| | NM_006180 | | | | | | | | |
| | NP_006171 | | | | | | | | |
| 3 | H615L | Protein kinase | 0.1500 | 1.0000 | 0.0000 | 1.0000 | 0.9912 | 0.0000 | Lung Non-Small Cell Lung Carcinoma; Liver Hepatocellular Carcinoma; Kidney Sarcomatoid Carcinoma |
| | H615Y | | | | | | | | |
| | I616T | | | | | | | | |
| | NM_006180 | | | | | | | | |
| | NP_006171 | | | | | | | | |
| 1 | K618R | Protein kinase | 0.0500 | 1.0000 | 1.0000 | 0.9836 | 0.9887 | 0.0049 | Colon Adenocarcinoma |
| | NM_006180 | | | | | | | | |
| | NP_006171 | | | | | | | | |
| 1 | V622I | Protein kinase | 0.0500 | 1.0000 | 1.0000 | 0.9508 | 0.9961 | 0.0000 | Unknown Primary Carcinoma |
| | NM_006180 | | | | | | | | |
| | NP_006171 | | | | | | | | |
| 3 | V624L | Protein kinase | 0.1500 | 1.0000 | 0.0000 | 1.0000 | 0.9961 | 0.0000 | Breast Carcinoma; Skin Melanoma; Unknown Primary Carcinoma |
| | V624M | | | | | | | | |
| | E625K | | | | | | | | |
| | NM_006180 | | | | | | | | |
| | NP_006171 | | | | | | | | |
| 1 | D627N | Protein kinase | 0.0500 | 1.0000 | 1.0000 | 0.9836 | 0.9961 | 0.0000 | Stomach Adenocarcinoma |
| | NM_006180 | | | | | | | | |
| | NP_006171 | | | | | | | | |
| 2 | L629I | Protein kinase | 0.1000 | 1.0000 | 0.0000 | 1.0000 | 0.9924 | 0.0000 | Lung Non-Small Cell Lung Carcinoma; Lung Squamous Cell Carcinoma |
| | I630V | | | | | | | | |
| | NM_006180 | | | | | | | | |
| | NP_ 006171 | | | | | | | | |
| 1 | V632I | Protein kinase | 0.0500 | 1.0000 | 1.0000 | 1.0000 | 0.9961 | 0.0498 | Skin Melanoma |
| | NM_006180 | | | | | | | | |
| | NP_006171 | | | | | | | | |
| 1 | E634Q | Protein kinase | 0.0500 | 1.0000 | 1.0000 | 1.0000 | 0.9961 | 0.0498 | Gastroesophageal Junction Adenocarcinoma |
| | NM_006180 | | | | | | | | |
| | NP_ 006171 | | | | | | | | |
| 3 | H638L | Protein kinase | 0.1500 | 1.0000 | 0.0000 | 1.0000 | 0.9950 | 0.0000 | Lung Adenocarcinoma; Colon Adenocarcinoma; Uterus Endometrial Adenocarcinoma |
| | G639R | | | | | | | | |
| | G639V | | | | | | | | |
| | NM_006180 | | | | | | | | |
| | NP_006171 | | | | | | | | |
| 1 | R646M | Protein kinase | 0.0500 | 1.0000 | 1.0000 | 1.0000 | 0.9961 | 0.0249 | Soft Tissue Perivascular Epithelioid Cell Tumor |
| | NM_006180 | | | | | | | | |
| | NP_006171 | | | | | | | | |
| 2 | H648Q | Protein kinase | 0.1000 | 1.0000 | 1.0000 | 1.0000 | 0.0229 | 0.0017 | Breast Carcinoma; Unknown Primary Melanoma |
| | G649S | | | | | | | | |
| | NM_006180 | | | | | | | | |
| | NP_006171 | | | | | | | | |
| 5 | A652V | Protein kinase | 0.2500 | 1.0000 | 1.0000 | 0.9508 | 0.9963 | 0.0947 | Lung Adenocarcinoma; Breast Invasive Ductal Carcinoma; Lung Non-Small Cell Lung Carcinoma |
| | V653M | | | | | | | | |
| | L654V | | | | | | | | |
| | NM_006180 | | | | | | | | |
| | NP_ 006171 | | | | | | | | |
| 5 | A656D | Protein kinase | 0.2500 | 1.0000 | 0.0000 | 1.0000 | 0.9952 | 0.0000 | Lung Adenocarcinoma; Breast Carcinoma; Lung Non-Small Cell Lung Carcinoma |
| | E657K | | | | | | | | |
| | E657Q | | | | | | | | |
| | G658D | | | | | | | | |
| | NM_006180 | | | | | | | | |
| | NP_ 006171 | | | | | | | | |
| 2 | P660L | Protein kinase | 0.1000 | 1.0000 | 1.0000 | 0.0000 | 0.9952 | 0.0000 | Lung Adenocarcinoma; Head and Neck Adenoid Cystic Carcinoma |
| | P660T | | | | | | | | |
| | NM_006180 | | | | | | | | |
| | NP_006171 | | | | | | | | |
| 2 | T662M | Protein kinase | 0.1000 | 1.0000 | 1.0000 | 1.0000 | 0.9952 | 0.0995 | Liver Cholangiocarcinoma; Lymph Node Lymphoma Mediastinal B-Cell(Pmbcl) |
| | NM_006180 | | | | | | | | |
| | NP_006171 | | | | | | | | |
| 5 | L664M | Protein kinase | 0.2500 | 1.0000 | 1.0000 | 0.9508 | 0.9902 | 0.1997 | Lung Adenocarcinoma; Colon Adenocarcinoma; Head and Neck Squamous Cell Carcinoma |
| | T665M | | | | | | | | |
| | T665S | | | | | | | | |
| | Q666L | | | | | | | | |
| | Q666R | | | | | | | | |
| | NM_006180 | | | | | | | | |
| | NP_006171 | | | | | | | | |
| 1 | Q668L | Protein kinase | 0.0500 | 1.0000 | 1.0000 | 1.0000 | 0.9869 | 0.0000 | Colon Adenocarcinoma |
| | NM_006180 | | | | | | | | |
| | NP_006171 | | | | | | | | |
| 3 | L670M | Protein kinase | 0.1500 | 1.0000 | 1.0000 | 1.0000 | 0.9646 | 0.0381 | Lung Adenocarcinoma; Lung Squamous Cell Carcinoma; Bone Marrow Leukemia Non-Lymphocytic Acute Myelocytic |
| | H671R | | | | | | | | |
| | NM_006180 | | | | | | | | |
| | NP_006171 | | | | | | | | |
| 2 | A673G | Protein kinase | 0.1000 | 1.0000 | 1.0000 | 1.0000 | 0.9580 | 0.0000 | Colon Adenocarcinoma; Lung Non-Small Cell Lung Carcinoma |
| | Q674H | | | | | | | | |
| | NM_006180 | | | | | | | | |
| | NP_ 006171 | | | | | | | | |
| 6 | A677T | Protein kinase | 0.3000 | 1.0000 | 0.6667 | 1.0000 | 0.9952 | 0.1765 | Colon Adenocarcinoma; Gastroesophageal Junction Adenocarcinoma; Bladder Urothelial Carcinoma |
| | A678T | | | | | | | | |
| | A678V | | | | | | | | |
| | G679D | | | | | | | | |
| | NM_006180 | | | | | | | | |
| | NP_006171 | | | | | | | | |
| 6 | A678T | Protein kinase | 0.3000 | 1.0000 | 0.6667 | 0.9836 | 0.9952 | 0.1636 | Gastroesophageal Junction Adenocarcinoma; Colon Adenocarcinoma; Bladder Urothelial Carcinoma |
| | A678V | | | | | | | | |
| | G679D | | | | | | | | |
| | M680I | | | | | | | | |
| | NM_006180 | | | | | | | | |
| | NP_006171 | | | | | | | | |
| 1 | Y682C | Protein kinase | 0.0500 | 1.0000 | 1.0000 | 1.0000 | 0.9869 | 0.0493 | Adrenal Gland Cortical Carcinoma |
| | NM_006180 | | | | | | | | |
| | NP_006171 | | | | | | | | |
| 7 | A684E | Protein kinase | 0.3500 | 1.0000 | 1.0000 | 1.0000 | 0.9952 | 0.0697 | Lung Adenocarcinoma; Head and Neck Squamous Cell Carcinoma; Pancreas Ductal Adenocarcinoma |
| | A684T | | | | | | | | |
| | A684V | | | | | | | | |
| | S685Y | | | | | | | | |
| | NM_006180 | | | | | | | | |
| | NP_006171 | | | | | | | | |
| 2 | V689M | Protein kinase | 0.1000 | 1.0000 | 1.0000 | 1.0000 | 0.9952 | 0.0890 | Uterus Endometrial Adenocarcinoma Endometrioid; Breast Lobular Carcinoma in Situ |
| | NM_006180 | | | | | | | | |
| | NP_006171 | | | | | | | | |
| 3 | R691C | Protein kinase | 0.1500 | 1.0000 | 1.0000 | 1.0000 | 0.9952 | 0.1335 | Other; Ovary Serous Carcinoma; Unknown Primary Adenocarcinoma |
| | D692N | | | | | | | | |
| | NM_006180 | | | | | | | | |
| | NP_006171 | | | | | | | | |
| 5 | C698R | Protein kinase | 0.2500 | 1.0000 | 0.0000 | 1.0000 | 0.7190 | 0.0000 | Lung Adenocarcinoma; Breast Carcinoma; Breast Invasive Ductal Carcinoma |
| | C698W | | | | | | | | |
| | L699P | | | | | | | | |
| | V700F | | | | | | | | |
| | NM_006180 | | | | | | | | |
| | NP_ 006171 | | | | | | | | |
| 1 | E702D | Protein kinase | 0.0500 | 1.0000 | 1.0000 | 1.0000 | 0.9952 | 0.0498 | Lung Non-Small Cell Lung Carcinoma |
| | NM_006180 | | | | | | | | |
| | NP_006171 | | | | | | | | |
| 1 | V706M | Protein kinase | 0.0500 | 1.0000 | 1.0000 | 1.0000 | 0.9952 | 0.0050 | Skin Melanoma |
| | NM_006180 | | | | | | | | |
| | NP_006171 | | | | | | | | |
| 3 | G709R | Protein kinase | 0.1500 | 1.0000 | 1.0000 | 1.0000 | 0.9952 | 0.0149 | Lung Non-Small Cell Lung Carcinoma; Uterus Endometrial Adenocarcinoma Papillary Serous; Leukemia Lymphocytic |
| | D710Y | | | | | | | | |
| | NM_006180 | | | | | | | | |
| | NP_006171 | | | | | | | | |
| 4 | S714A | Protein kinase | 0.2000 | 1.0000 | 0.2500 | 1.0000 | 0.9931 | 0.0421 | Skin Melanoma; Lung Non-Small Cell Lung Carcinoma; Prostate Acinar Adenocarcinoma |
| | R715Q | | | | | | | | |
| | R715W | | | | | | | | |
| | D716N | | | | | | | | |
| | NM_006180 | | | | | | | | |
| | NP_006171 | | | | | | | | |
| 4 | V725G | Protein kinase | 0.2000 | 1.0000 | 0.5000 | 1.0000 | 0.9979 | 0.0862 | Lung Adenocarcinoma; Lung Non-Small Cell Lung Carcinoma; Liver Hepatocellular Carcinoma |
| | G726C | | | | | | | | |
| | G727D | | | | | | | | |
| | NM_006180 | | | | | | | | |
| | NP_006171 | | | | | | | | |
| 1 | T729S | Protein kinase | 0.0500 | 1.0000 | 1.0000 | 1.0000 | 0.9954 | 0.0498 | Lung Squamous Cell Carcinoma |
| | NM_006180 | | | | | | | | |
| | NP_006171 | | | | | | | | |
| 2 | M736I | Protein kinase | 0.1000 | 1.0000 | 0.0000 | 1.0000 | 0.9987 | 0.0000 | Lung Adenocarcinoma; Unknown Primary Sarcomatoid Carcinoma |
| | P737T | | | | | | | | |
| | NM_006180 | | | | | | | | |
| | NP_ 006171 | | | | | | | | |
| 3 | I741N | Protein kinase | 0.1500 | 1.0000 | 1.0000 | 0.9836 | 0.9957 | 0.0788 | Lung Adenocarcinoma; Breast Invasive Ductal Carcinoma; Lung Sarcoma |
| | I741V | | | | | | | | |
| | M742L | | | | | | | | |
| | NM_006180 | | | | | | | | |
| | NP_ 006171 | | | | | | | | |
| 1 | R744K | Protein kinase | 0.0500 | 1.0000 | 1.0000 | 1.0000 | 0.9988 | 0.0050 | Skin Basal Cell Carcinoma |
| | NM_006180 | | | | | | | | |
| | NP_006171 | | | | | | | | |
| 10 | F746I | Protein kinase | 0.5000 | 1.0000 | 0.7000 | 0.9836 | 0.9985 | 0.2458 | Colon Adenocarcinoma; Liver Hepatocellular Carcinoma^{1, 2, 3}; Bladder Urothelial Carcinoma; Large Intestine/Colon Carcinoma²; Lymphoid |
| | T747M | | | | | | | | |
| | T748M^{1, 2, 3} | | | | | | | | |
| | NM_006180 | | | | | | | | |
| | NP_006171 | | | | | | | | |
| | | | | | | | | | Neoplasm³ |
| 10 | T747M | Protein kinase | 0.5000 | 1.0000 | 0.4000 | 0.9836 | 0.9988 | 0.1424 | Colon Adenocarcinoma; Bladder Urothelial Carcinoma; Lung Adenocarcinoma |
| | T748M | | | | | | | | |
| | E749K | | | | | | | | |
| | NM_006180 | | | | | | | | |
| | NP_ 006171 | | | | | | | | |
| 6 | T748M | Protein kinase | 0.3000 | 1.0000 | 0.0000 | 0.9836 | 0.9988 | 0.0000 | Lung Adenocarcinoma; Colon Adenocarcinoma; Liver Hepatocellular Carcinoma |
| | E749K | | | | | | | | |
| | S750N | | | | | | | | |
| | NM_006180 | | | | | | | | |
| | NP_006171 | | | | | | | | |
| 1 | V752I | Protein kinase | 0.0500 | 1.0000 | 0.0000 | 1.0000 | 0.9989 | 0.0000 | Colon Adenocarcinoma |
| | NM_006180 | | | | | | | | |
| | NP_ 006171 | | | | | | | | |
| 4 | S754T | Protein kinase | 0.2000 | 1.0000 | 0.5000 | 1.0000 | 0.9989 | 0.0946 | Lung Adenocarcinoma; Lung Non-Small Cell Lung Carcinoma; Unknown Primary Adenocarcinoma |
| | L755M | | | | | | | | |
| | G756W | | | | | | | | |
| | NM_006180 | | | | | | | | |
| | NP_ 006171 | | | | | | | | |
| 4 | V758E | Protein kinase | 0.2000 | 1.0000 | 0.0000 | 1.0000 | 0.9981 | 0.0000 | Lung Adenocarcinoma; Ovary Serous Carcinoma; Unknown Primary Carcinoma |
| | V758L | | | | | | | | |
| | V758M | | | | | | | | |
| | NM_006180 | | | | | | | | |
| | NP_006171 | | | | | | | | |
| 5 | W760R | Protein kinase | 0.2500 | 1.0000 | 0.0000 | 1.0000 | 0.9894 | 0.0000 | Lung Adenocarcinoma; Colon Adenocarcinoma; Unknown Primary Melanoma |
| | E761D | | | | | | | | |
| | E761Q | | | | | | | | |
| | I762M | | | | | | | | |
| | NM_006180 | | | | | | | | |
| | NP_006171 | | | | | | | | |
| 2 | G766D | Protein kinase | 0.1000 | 1.0000 | 0.0000 | 1.0000 | 0.9989 | 0.0000 | Brain Glioblastoma; Lung Small Cell Undifferentiated Carcinoma |
| | G766S | | | | | | | | |
| | NM_006180 | | | | | | | | |
| | NP_ 006171 | | | | | | | | |
| 1 | P769T | Protein kinase | 0.0500 | 1.0000 | 0.0000 | 1.0000 | 0.9989 | 0.0000 | Lung Adenocarcinoma |
| | NM_006180 | | | | | | | | |
| | NP_006171 | | | | | | | | |
| 2 | L773M | Protein kinase | 0.1000 | 1.0000 | 0.0000 | 1.0000 | 0.9989 | 0.0000 | Colon Adenocarcinoma |
| | NM_006180 | | | | | | | | |
| | NP_006171 | | | | | | | | |
| 1 | E777Q | Protein kinase | 0.0500 | 1.0000 | 1.0000 | 1.0000 | 0.9989 | 0.0050 | Lymph Node Lymphoma Diffuse Large B Cell |
| | NM_006180 | | | | | | | | |
| | NP_006171 | | | | | | | | |
| 1 | I779M | Protein kinase | 0.0500 | 1.0000 | 1.0000 | 1.0000 | 0.1928 | 0.0000 | Lung Adenocarcinoma |
| | NM_006180 | | | | | | | | |
| | NP_ 006171 | | | | | | | | |
| 3 | I782M | Protein kinase | 0.1500 | 1.0000 | 1.0000 | 1.0000 | 0.8857 | 0.0000 | Lung Adenocarcinoma; Unknown Primary Carcinoma; Brain Meningioma |
| | T783I | | | | | | | | |
| | Q784H | | | | | | | | |
| | NM_006180 | | | | | | | | |
| | NP_006171 | | | | | | | | |
| 3 | T783I | Protein kinase | 0.1500 | 1.0000 | 1.0000 | 1.0000 | 0.9455 | 0.0473 | Unknown Primary Carcinoma; Uterus Endometrial Adenocarcinoma; Brain Meningioma |
| | Q784H | | | | | | | | |
| | G785V | | | | | | | | |
| | NM_006180 | | | | | | | | |
| | NP_ 006171 | | | | | | | | |
| 4 | Q784H | Protein kinase | 0.2000 | 1.0000 | 1.0000 | 0.9672 | 0.9587 | 0.1757 | Colon Adenocarcinoma; Unknown Primary Carcinoma; Uterus Endometrial Adenocarcinoma |
| | G785V | | | | | | | | |
| | R786Q | | | | | | | | |
| | NM_006180 | | | | | | | | |
| | NP_006171 | | | | | | | | |
| 4 | G785V | Protein kinase | 0.2000 | 1.0000 | 1.0000 | 0.9672 | 0.9981 | 0.1795 | Colon Adenocarcinoma; Uterus Endometrial Adenocarcinoma; Bile Duct Adenocarcinoma |
| | R786Q | | | | | | | | |
| | V787F | | | | | | | | |
| | NM_006180 | | | | | | | | |
| | NP_006171 | | | | | | | | |
| 4 | R786Q | Protein kinase | 0.2000 | 1.0000 | 1.0000 | 0.9672 | 0.8640 | 0.1539 | Colon Adenocarcinoma; Stomach Adenocarcinoma; Bile Duct Adenocarcinoma |
| | V787F | | | | | | | | |
| | L788M | | | | | | | | |
| | NM_006180 | | | | | | | | |
| | NP_006171 | | | | | | | | |
| 3 | V787F | Protein kinase | 0.1500 | 1.0000 | 1.0000 | 1.0000 | 0.8193 | 0.1229 | Colon Adenocarcinoma; Stomach Adenocarcinoma; Bile Duct Adenocarcinoma |
| | L788M | | | | | | | | |
| | Q789E | | | | | | | | |
| | NM_006180 | | | | | | | | |
| | NP_006171 | | | | | | | | |
| 4 | R792C | Protein kinase | 0.2000 | 1.0000 | 0.2500 | 0.9344 | 0.9780 | 0.0026 | Lung Adenocarcinoma; Brain Medulloblastoma; Breast Neuroendocrine Carcinoma |
| | T793A | | | | | | | | |
| | T793M | | | | | | | | |
| | NM_006180 | | | | | | | | |
| | NP_ 006171 | | | | | | | | |
| 1 | P795T | Protein kinase | 0.0500 | 1.0000 | 1.0000 | 1.0000 | 0.9981 | 0.0050 | Lung Adenocarcinoma |
| | NM_006180 | | | | | | | | |
| | NP_ 006171 | | | | | | | | |
| 1 | E797K | Protein kinase | 0.0500 | 1.0000 | 1.0000 | 1.0000 | 0.9986 | 0.0050 | Lung Adenocarcinoma |
| | NM_006180 | | | | | | | | |
| | NP_006171 | | | | | | | | |
| 1 | Y799N | Protein kinase | 0.0500 | 1.0000 | 0.0000 | 1.0000 | 0.9949 | 0.0000 | Lung Adenocarcinoma |
| | NM_006180 | | | | | | | | |
| | NP_ 006171 | | | | | | | | |
| 1 | M802L | Protein kinase | 0.0500 | 1.0000 | 1.0000 | 1.0000 | 0.9949 | 0.0445 | Esophagus Adenocarcinoma |
| | NM_006180 | | | | | | | | |
| | NP_006171 | | | | | | | | |
| 3 | G804E | Protein kinase | 0.1500 | 1.0000 | 1.0000 | 1.0000 | 0.9973 | 0.1247 | Colon Adenocarcinoma; Lung Non-Small Cell Lung Carcinoma; Unknown Primary Adenocarcinoma |
| | C805R | | | | | | | | |
| | C805Y | | | | | | | | |
| | NM_006180 | | | | | | | | |
| | NP_006171 | | | | | | | | |
| 1 | P810T | Protein kinase | 0.0500 | 1.0000 | 1.0000 | 1.0000 | 0.9986 | 0.0250 | Lung Adenocarcinoma |
| | NM_006180 | | | | | | | | |
| | NP_006171 | | | | | | | | |
| 1 | M812I | Protein kinase | 0.0500 | 1.0000 | 0.0000 | 0.9836 | 0.8600 | 0.0000 | Skin Melanoma |
| | NM_006180 | | | | | | | | |
| | NP_006171 | | | | | | | | |
| 1 | G818D | Protein kinase | 0.0500 | 1.0000 | 1.0000 | 1.0000 | 0.8511 | 0.0426 | Appendix Adenocarcinoma |
| | NM_006180 | | | | | | | | |
| | NP_006171 | | | | | | | | |
| 3 | T821N | Protein kinase | 0.1500 | 1.0000 | 1.0000 | 1.0000 | 0.9986 | 0.0867 | Liver Hepatocellular Carcinoma; Gastroesophageal Junction Adenocarcinoma; Uterus Endometrial Adenocarcinoma Papillary Serous |
| | T821S | | | | | | | | |
| | L822F | | | | | | | | |
| | NM_006180 | | | | | | | | |
| | NP_006171 | | | | | | | | |
| 1 | N825D | | 0.0500 | 0.0500 | 1.0000 | 0.9508 | 0.9949 | 0.0021 | Bone Marrow Multiple Myeloma |
| | NM_006180 | | | | | | | | |
| | NP_006171 | | | | | | | | |
| 2 | A829S | | 0.1000 | 0.0500 | 1.0000 | 1.0000 | 0.9986 | 0.0045 | Lung Adenocarcinoma; Unknown Primary Adenocarcinoma |
| | NM_006180 | | | | | | | | |
| | NP_006171 | | | | | | | | |
| 2 | P831L | | 0.1000 | 0.0500 | 1.0000 | 0.9672 | 0.9986 | 0.0000 | Lung Non-Small Cell Lung Carcinoma; Pancreas Ductal Adenocarcinoma |
| | NM_006180 | | | | | | | | |
| | NP_006171 | | | | | | | | |

| | | | | | | | | | |
|---|---|---|---|---|---|---|---|---|---|
| Pediatric Cancer Gene Database, pedican.bioinfo-minzhao.org/gene_mutation.cgi?gene=4915, downloaded on May 31, 2016. ² Endometrial Cancer Gene Database, ecgene.bioinfo-minzhao.org/gene_mutation.cgi?gene=4915, downloaded on May 31, 2016. ³ Catalog of Somatic Mutations in Cancer (COSMIC) database, cancer.sanger.ac.uk/cosmic/mutation/ overview?id=1636266, downloaded on May 31, 2016. | | | | | | | | | |

**Table 3. Detected TrkB Protein Mutations Resulting from NTRK2 Point Mutations Detected in Cancer Biopsy Samples (Confirmed Expression)**

| Mutated Disease | Mutations Samples | Domain | Hotspot Score | Domain Score | co-Alteration Score | Exac Score | Conservation Score | Total Score |
|---|---|---|---|---|---|---|---|---|
| 5 | A314E | Ig-like C2-type 2 | 0.25 | 0.90 | 1.00 | 0.97 | 1.00 | 0.22 |
| | A314G | | | | | | | |
| | A314V | | | | | | | |
| | L315F | | | | | | | |
| 2 | V689M | Protein kinase | 0.10 | 1.00 | 1.00 | 1.00 | 1.00 | 0.10 |
| 5 | M240I | Ig-like C2-type 1 | 0.25 | 0.90 | 0.40 | 1.00 | 1.00 | 0.09 |
| | N241D | | | | | | | |
| | E242K | | | | | | | |
| 1 | I264M | Ig-like C2-type 1 | 0.05 | 0.90 | 1.00 | 1.00 | 1.00 | 0.04 |
| 4 | A440S | | 0.20 | 0.05 | 1.00 | 0.98 | 1.00 | 0.01 |
| | A440T | | | | | | | |
| | A440V | | | | | | | |
| 10 | T426I, G427S, R428Q | | 0.50 | 0.05 | 0.50 | 0.87 | 0.82 | 0.01 |
| 3 | G401A, G401E, G401R | | 0.15 | 0.05 | 1.00 | 1.00 | 0.98 | 0.01 |

**Table 4. Detected TrkC Protein Mutations Resulting from NTRK3 Point Mutations Detected in Cancer Biopsy Samples**

| Mutated Samples | Mutations Ref.Transcript/Protein | Domain | Hotspot Score | Domain Score | co-Alteration Score | Exac Score | Conservation Score | Total Score | Disease |
|---|---|---|---|---|---|---|---|---|---|
| 3 | S4C | | 0.1500 | 0.0500 | 1.0000 | 1.0000 | 0.9813 | 0.0069 | Lung Adenocarcinoma; Lung Squamous Cell Carcinoma |
| | S4F | | | | | | | | |
| | L5I | | | | | | | | |
| | NM_001012338 | | | | | | | | |
| | NP_ 001012338 | | | | | | | | |
| 5 | P7L | | 0.2500 | 0.0500 | 0.4000 | 1.0000 | 0.9773 | 0.0033 | Lung Adenocarcinoma; Colon Adenocarcinoma; Unknown Primary Adenocarcinoma |
| | P7R | | | | | | | | |
| | A8D | | | | | | | | |
| | K9E | | | | | | | | |
| | K9N | | | | | | | | |
| | NM_001012338 | | | | | | | | |
| | NP_001012338 | | | | | | | | |
| 1 | R14P | | 0.0500 | 0.0500 | 1.0000 | 1.0000 | 0.9813 | 0.0025 | Breast Carcinoma |
| | NM_001012338 | | | | | | | | |
| | NP_001012338 | | | | | | | | |
| 1 | G19E | | 0.0500 | 0.0500 | 0.0000 | 1.0000 | 0.7246 | 0.0000 | Colon Adenocarcinoma |
| | NM_001012338 | | | | | | | | |
| | NP_001012338 | | | | | | | | |
| 4 | V21F | | 0.2000 | 0.0500 | 1.0000 | 0.0000 | 0.7777 | 0.0000 | Colon Adenocarcinoma; Breast Invasive Ductal Carcinoma; Head and Neck Squamous Cell Carcinoma |
| | V21I | | | | | | | | |
| | NM_001012338 | | | | | | | | |
| | NP_001012338 | | | | | | | | |
| 1 | Y25C | | 0.0500 | 0.0500 | 1.0000 | 1.0000 | 0.9688 | 0.0000 | Lymphoma Follicular Lymphoma |
| | NM_001012338 | | | | | | | | |
| | NP_001012338 | | | | | | | | |
| 1 | G27A | | 0.0500 | 0.0500 | 1.0000 | 1.0000 | 0.9858 | 0.0025 | Breast Carcinoma |
| | NM_001012338 | | | | | | | | |
| | NP_001012338 | | | | | | | | |
| 4 | N35S | | 0.2000 | 0.0500 | 0.2500 | 1.0000 | 0.7479 | 0.0017 | Skin Melanoma; Liver Hepatocellular Carcinoma; Unknown Primary Melanoma |
| | C36W | | | | | | | | |
| | V37A | | | | | | | | |
| | NM_001012338 | | | | | | | | |
| | NP_001012338 | | | | | | | | |
| 1 | S39R | | 0.0500 | 0.0500 | 1.0000 | 1.0000 | 0.9691 | 0.0022 | Lung Adenocarcinoma |
| | NM_001012338 | | | | | | | | |
| | NP_001012338 | | | | | | | | |
| 7 | C45W | | 0.3500 | 0.0500 | 1.0000 | 0.9508 | 0.9581 | 0.0000 | Lung Adenocarcinoma; Breast Carcinoma; Breast Invasive Ductal Carcinoma |
| | R46P | | | | | | | | |
| | R46W | | | | | | | | |
| | NM_001012338 | | | | | | | | |
| | NP_ 001012338 | | | | | | | | |
| 2 | P48L | | 0.1000 | 0.0500 | 0.0000 | 1.0000 | 0.9775 | 0.0000 | Lung Adenocarcinoma; Bladder Urothelial Carcinoma |
| | D49G | | | | | | | | |
| | NM_001012338 | | | | | | | | |
| | NP_001012338 | | | | | | | | |
| 1 | P55S | | 0.0500 | 0.0500 | 1.0000 | 1.0000 | 0.9860 | 0.0022 | Uterus Endometrial Adenocarcinoma Endometrioid |
| | NM_001012338 | | | | | | | | |
| | NP_001012338 | | | | | | | | |
| 2 | G63W | | 0.1000 | 0.0500 | 1.0000 | 1.0000 | 0.8998 | 0.0045 | Unknown Primary Carcinoma |
| | N64K | | | | | | | | |
| | NM_001012338 | | | | | | | | |
| | NP_001012338 | | | | | | | | |
| 2 | G67E | | 0.1000 | 0.0500 | 1.0000 | 0.9672 | 0.9885 | 0.0000 | Colon Adenocarcinoma; Breast Carcinoma |
| | NM_001012338 | | | | | | | | |
| | NP_001012338 | | | | | | | | |
| 1 | A69T | | 0.0500 | 0.0500 | 1.0000 | 1.0000 | 0.0000 | 0.0000 | Colon Adenocarcinoma |
| | NM_001012338 | | | | | | | | |
| | NP_001012338 | | | | | | | | |
| 2 | I71V | | 0.1000 | 0.0500 | 1.0000 | 0.6885 | 0.0000 | 0.0000 | Breast Carcinoma; Liver Cholangiocarcinoma |
| | NM_001012338 | | | | | | | | |
| | NP_001012338 | | | | | | | | |
| 3 | D75G | | 0.1500 | 0.0500 | 0.0000 | 1.0000 | 0.9835 | 0.0000 | Colon Adenocarcinoma; Thyroid Papillary Carcinoma |
| | D75N | | | | | | | | |
| | I76T | | | | | | | | |
| | NM_001012338 | | | | | | | | |
| | NP_ 001012338 | | | | | | | | |
| 3 | R78K | | 0.1500 | 0.0500 | 1.0000 | 0.9672 | 0.9545 | 0.0012 | Lung Squamous Cell Carcinoma; Bladder Urothelial Carcinoma; Skin Basal Cell Carcinoma |
| | R78S | | | | | | | | |
| | NM_001012338 | | | | | | | | |
| | NP_001012338 | | | | | | | | |
| 2 | S82F | | 0.1000 | 0.0500 | 0.0000 | 1.0000 | 0.9859 | 0.0000 | Skin Melanoma |
| | I83V | | | | | | | | |
| | NM_001012338 | | | | | | | | |
| | NP_ 001012338 | | | | | | | | |
| 2 | I85M | | 0.1000 | 0.0500 | 0.0000 | 1.0000 | 0.4043 | 0.0000 | Lung Adenocarcinoma; Colon Adenocarcinoma |
| | NM_001012338 | | | | | | | | |
| | NP_001012338 | | | | | | | | |
| 9 | R89C | | 0.4500 | 0.0500 | 0.0000 | 0.9344 | 0.9578 | 0.0000 | Colon Adenocarcinoma; Lung Adenocarcinoma; Breast Carcinoma |
| | R89H | | | | | | | | |
| | R89S | | | | | | | | |
| | S90N | | | | | | | | |
| | NM_001012338 | | | | | | | | |
| | NP_ 001012338 | | | | | | | | |
| 4 | N95S | | 0.2000 | 0.0500 | 1.0000 | 0.9836 | 0.8553 | 0.0024 | Colon Adenocarcinoma; Uterus Endometrial Adenocarcinoma; Bladder Carcinoma |
| | A96S | | | | | | | | |
| | A96T | | | | | | | | |
| | NM_001012338 | | | | | | | | |
| | NP_ 001012338 | | | | | | | | |
| 4 | D98G | | 0.2000 | 0.0500 | 0.5000 | 0.9836 | 0.9928 | 0.0016 | Lung Adenocarcinoma; Breast Carcinoma; Skin Melanoma |
| | D98N | | | | | | | | |
| | M99I | | | | | | | | |
| | NM_001012338 | | | | | | | | |
| | NP_001012338 | | | | | | | | |
| 1 | L101I | | 0.0500 | 0.0500 | 1.0000 | 1.0000 | 0.9950 | 0.0022 | Lung Squamous Cell Carcinoma |
| | NM_001012338 | | | | | | | | |
| | NP_001012338 | | | | | | | | |
| 1 | K111N | LRR 1 | 0.0500 | 0.3000 | 1.0000 | 1.0000 | 0.9968 | 0.0015 | Breast Carcinoma |
| | NM_001012338 | | | | | | | | |
| | NP_001012338 | | | | | | | | |
| 5 | S113T | LRR 1 | 0.2500 | 0.3000 | 1.0000 | 1.0000 | 0.9930 | 0.0577 | Lung Adenocarcinoma; Colon Adenocarcinoma; Head and Neck Squamous Cell Carcinoma |
| | G114E | | | | | | | | |
| | L115F | | | | | | | | |
| | L115P | | | | | | | | |
| | L115R | | | | | | | | |
| | NM_001012338 | | | | | | | | |
| | NP_ 001012338 | | | | | | | | |
| 9 | G114E | LRR 1 | 0.4500 | 0.3000 | 0.6667 | 0.9508 | 0.9057 | 0.0597 | Colon Adenocarcinoma; Unknown Primary Melanoma; Lung Adenocarcinoma |
| | L115F | | | | | | | | |
| | L115P | | | | | | | | |
| | L115R | | | | | | | | |
| | R116Q | | | | | | | | |
| | R116W | | | | | | | | |
| | NM_001012338 | | | | | | | | |
| | NP_001012338 | | | | | | | | |
| 9 | L115F | LRR 1 | 0.4500 | 0.3000 | 0.6667 | 0.9344 | 0.8790 | 0.0627 | Colon Adenocarcinoma; Unknown Primary Melanoma; Lung Adenocarcinoma |
| | L115P | | | | | | | | |
| | L115R | | | | | | | | |
| | R116Q | | | | | | | | |
| | R116W | | | | | | | | |
| | S117N | | | | | | | | |
| | NM_001012338 | | | | | | | | |
| | NP_ 001012338 | | | | | | | | |
| 5 | Q119H | LRR 1 | 0.2500 | 0.3000 | 1.0000 | 1.0000 | 0.9500 | 0.0629 | Lung Adenocarcinoma; Lung Squamous Cell Carcinoma; Liver Hepatocellular Carcinoma |
| | Q119K | | | | | | | | |
| | P120H | | | | | | | | |
| | R121G | | | | | | | | |
| | R121K | | | | | | | | |
| | NM_001012338 | | | | | | | | |
| | NP_001012338 | | | | | | | | |
| 4 | F123L | LRR 1 | 0.2000 | 0.3000 | 0.0000 | 1.0000 | 0.9799 | 0.0000 | Lung Adenocarcinoma; Colon Adenocarcinoma; Lung Squamous Cell Carcinoma |
| | A124V | | | | | | | | |
| | K125E | | | | | | | | |
| | K125N | | | | | | | | |
| | NM_001012338 | | | | | | | | |
| | NP_001012338 | | | | | | | | |
| 4 | A124V | LRR 1 | 0.2000 | 0.3000 | 0.0000 | 1.0000 | 0.7283 | 0.0000 | Lung Adenocarcinoma; Colon Adenocarcinoma; Pancreas Ductal Adenocarcinoma |
| | K125E | | | | | | | | |
| | K125N | | | | | | | | |
| | N126K | | | | | | | | |
| | NM_001012338 | | | | | | | | |
| | NP_ 001012338 | | | | | | | | |
| 4 | K125E | | 0.2000 | 0.0500 | 0.0000 | 1.0000 | 0.7283 | 0.0000 | Lung Adenocarcinoma; Colon Adenocarcinoma; Pancreas Ductal Adenocarcinoma |
| | K125N | | | | | | | | |
| | N126K | | | | | | | | |
| | P127H | | | | | | | | |
| | NM_001012338 | | | | | | | | |
| | NP_ 001012338 | | | | | | | | |
| 6 | R130C | LRR 2 | 0.3000 | 0.3000 | 1.0000 | 0.9344 | 0.9523 | 0.0464 | Lung Adenocarcinoma; Breast Carcinoma; Breast Invasive Ductal Carcinoma |
| | R130H | | | | | | | | |
| | NM_001012338 | | | | | | | | |
| | NP_ 001012338 | | | | | | | | |
| 2 | N133H | LRR 2 | 0.1000 | 0.3000 | 1.0000 | 1.0000 | 0.9920 | 0.0149 | Lung Adenocarcinoma; Unknown Primary Adenocarcinoma |
| | L134Q | | | | | | | | |
| | NM_001012338 | | | | | | | | |
| | NP_001012338 | | | | | | | | |
| 7 | R138Q | LRR 2 | 0.3500 | 0.3000 | 1.0000 | 0.8197 | 0.9974 | 0.0000 | Lung Adenocarcinoma; Colon Adenocarcinoma; Lung Non-Small Cell Lung Carcinoma |
| | R138W | | | | | | | | |
| | NM_001012338 | | | | | | | | |
| | NP_001012338 | | | | | | | | |
| 1 | T140N | LRR 2 | 0.0500 | 0.3000 | 0.0000 | 1.0000 | 0.9974 | 0.0000 | Lung Non-Small Cell Lung Carcinoma |
| | NM_001012338 | | | | | | | | |
| | NP_001012338 | | | | | | | | |
| 5 | F147L | LRR 2 | 0.2500 | 0.3000 | 1.0000 | 0.8033 | 0.9974 | 0.0000 | Lung Adenocarcinoma; Breast Invasive Ductal Carcinoma; Stomach Adenocarcinoma |
| | Q148H | | | | | | | | |
| | T149M | | | | | | | | |
| | T149R | | | | | | | | |
| | NM_001012338 | | | | | | | | |
| | NP_ 001012338 | | | | | | | | |
| 6 | L152I | | 0.3000 | 0.0500 | 1.0000 | 0.9508 | 0.9974 | 0.0129 | Lung Adenocarcinoma; Colon Adenocarcinoma; Breast Carcinoma |
| | R153Q | | | | | | | | |
| | E154K | | | | | | | | |
| | NM_001012338 | | | | | | | | |
| | NP_001012338 | | | | | | | | |
| 4 | Q156H | | 0.2000 | 0.0500 | 1.0000 | 1.0000 | 0.1641 | 0.0006 | Lung Adenocarcinoma; Skin Melanoma; Bile Duct Adenocarcinoma |
| | Q156R | | | | | | | | |
| | L157M | | | | | | | | |
| | E158K | | | | | | | | |
| | NM_001012338 | | | | | | | | |
| | NP_001012338 | | | | | | | | |
| 4 | L157M | | 0.2000 | 0.0500 | 0.5000 | 1.0000 | 0.6264 | 0.0026 | Lung Adenocarcinoma; Skin Melanoma; Lung Non-Small Cell Lung Carcinoma |
| | E158K | | | | | | | | |
| | Q159H | | | | | | | | |
| | Q159K | | | | | | | | |
| | NM_001012338 | | | | | | | | |
| | NP_001012338 | | | | | | | | |
| 1 | F161I | LRRCT | 0.0500 | 0.3000 | 1.0000 | 1.0000 | 0.9933 | 0.0149 | Breast Ductal Carcinoma in Situ |
| | NM_001012338 | | | | | | | | |
| | NP_001012338 | | | | | | | | |
| 6 | N163T | LRRCT | 0.3000 | 0.3000 | 0.5000 | 0.9836 | 0.9948 | 0.0000 | Lung Adenocarcinoma; Unknown Primary Adenocarcinoma; Colon Adenocarcinoma |
| | C164G | | | | | | | | |
| | C164S | | | | | | | | |
| | S165N | | | | | | | | |
| | NM_001012338 | | | | | | | | |
| | NP_001012338 | | | | | | | | |
| 1 | R169S | LRRCT | 0.0500 | 0.3000 | 0.0000 | 1.0000 | 0.9979 | 0.0000 | Lung Adenocarcinoma |
| | NM_001012338 | | | | | | | | |
| | NP_001012338 | | | | | | | | |
| 2 | M171L | LRRCT | 0.1000 | 0.3000 | 1.0000 | 0.9508 | 0.9956 | 0.0000 | Lung Adenocarcinoma |
| | Q172H | | | | | | | | |
| | NM_001012338 | | | | | | | | |
| | NP_ 001012338 | | | | | | | | |
| 1 | W174L | LRRCT | 0.0500 | 0.3000 | 0.0000 | 1.0000 | 0.9979 | 0.0000 | Lung Adenocarcinoma |
| | NM_001012338 | | | | | | | | |
| | NP_001012338 | | | | | | | | |
| 1 | E176K | LRRCT | 0.0500 | 0.3000 | 1.0000 | 1.0000 | 0.9979 | 0.0150 | Skin Melanoma |
| | NM_001012338 | | | | | | | | |
| | NP_001012338 | | | | | | | | |
| 4 | G178E | LRRCT | 0.2000 | 0.3000 | 1.0000 | 1.0000 | 0.9979 | 0.0599 | Unknown Primary Melanoma; Lung Small Cell Undifferentiated Carcinoma; Unknown Primary Malignant Neoplasm |
| | G178V | | | | | | | | |
| | E179K | | | | | | | | |
| | NM_001012338 | | | | | | | | |
| | NP_001012338 | | | | | | | | |
| 3 | S184C | LRRCT | 0.1500 | 0.3000 | 1.0000 | 0.9836 | 0.4394 | 0.0179 | Lung Adenocarcinoma; Bone Marrow Multiple Myeloma; Skin Melanoma |
| | S184N | | | | | | | | |
| | S184R | | | | | | | | |
| | NM_001012338 | | | | | | | | |
| | NP_001012338 | | | | | | | | |
| 5 | Y188C | LRRCT | 0.2500 | 0.3000 | 0.0000 | 1.0000 | 0.9951 | 0.0000 | Lung Adenocarcinoma; Lung Squamous Cell Carcinoma |
| | Y188F | | | | | | | | |
| | Y188H | | | | | | | | |
| | C189F | | | | | | | | |
| | NM_001012338 | | | | | | | | |
| | NP_001012338 | | | | | | | | |
| 1 | A192T | LRRCT | 0.0500 | 0.3000 | 1.0000 | 1.0000 | 0.5408 | 0.0041 | Gastroesophageal Junction Adenocarcinoma |
| | NM_001012338 | | | | | | | | |
| | NP_001012338 | | | | | | | | |
| 5 | G194D | LRRCT | 0.2500 | 0.3000 | 1.0000 | 1.0000 | 0.9748 | 0.0658 | Brain Glioblastoma; Lung Non-Small Cell Lung Carcinoma; Bladder Urothelial Carcinoma |
| | S195C | | | | | | | | |
| | S195F | | | | | | | | |
| | Q196K | | | | | | | | |
| | NM_001012338 | | | | | | | | |
| | NP_ 001012338 | | | | | | | | |
| 6 | L199H | LRRCT | 0.3000 | 0.3000 | 0.0000 | 0.9180 | 0.9301 | 0.0000 | Colon Adenocarcinoma; Breast Invasive Ductal Carcinoma; Lung Non-Small Cell Lung Carcinoma |
| | L199P | | | | | | | | |
| | L199V | | | | | | | | |
| | F200V | | | | | | | | |
| | R201C | | | | | | | | |
| | NM_001012338 | | | | | | | | |
| | NP_ 001012338 | | | | | | | | |
| 4 | F200V | LRRCT | 0.2000 | 0.3000 | 0.2500 | 0.9180 | 0.8985 | 0.0077 | Colon Adenocarcinoma; Brain Glioblastoma; Lung Non-Small Cell Lung Carcinoma |
| | R201C | | | | | | | | |
| | M202I | | | | | | | | |
| | NM_001012338 | | | | | | | | |
| | NP_ 001012338 | | | | | | | | |
| 1 | S205G | LRRCT | 0.0500 | 0.3000 | 1.0000 | 1.0000 | 0.9933 | 0.0133 | Esophagus Adenocarcinoma |
| | NM_001012338 | | | | | | | | |
| | NP_001012338 | | | | | | | | |
| 10 | D208E | LRRCT | 0.5000 | 0.3000 | 0.4000 | 0.3279 | 0.9648 | 0.0148 | Soft Tissue Sarcoma; Breast Invasive Ductal Carcinoma; Lung Non-Small Cell Lung Carcinoma |
| | D208N | | | | | | | | |
| | L209I | | | | | | | | |
| | L209P | | | | | | | | |
| | L209R | | | | | | | | |
| | L209V | | | | | | | | |
| | P210S | | | | | | | | |
| | NM_001012338 | | | | | | | | |
| | NP_ 001012338 | | | | | | | | |
| 1 | I212M | Ig-like C2-type 1 | 0.0500 | 0.9000 | 1.0000 | 1.0000 | 0.9978 | 0.0449 | Lung Non-Small Cell Lung Carcinoma |
| | NM_001012338 | | | | | | | | |
| | NP_001012338 | | | | | | | | |
| 1 | S215T | Ig-like C2-type 1 | 0.0500 | 0.9000 | 1.0000 | 1.0000 | 0.9978 | 0.0000 | Lung Adenocarcinoma |
| | NM_001012338 | | | | | | | | |
| | NP_001012338 | | | | | | | | |
| 5 | V217A | Ig-like C2-type 1 | 0.2500 | 0.9000 | 1.0000 | 0.9672 | 0.9145 | 0.0000 | Lung Non-Small Cell Lung Carcinoma; Colon Adenocarcinoma; Pancreas Carcinoma |
| | V217I | | | | | | | | |
| | N218S | | | | | | | | |
| | NM_001012338 | | | | | | | | |
| | NP_ 001012338 | | | | | | | | |
| 7 | V2211 | Ig-like C2-type 1 | 0.3500 | 0.9000 | 1.0000 | 0.9180 | 0.8870 | 0.1832 | Unknown Primary Melanoma; Skin Melanoma; Uterus Endometrial Adenocarcinoma Endometrioid |
| | R222Q | | | | | | | | |
| | E223D | | | | | | | | |
| | NM_001012338 | | | | | | | | |
| | NP_001012338 | | | | | | | | |
| 1 | A227T | Ig-like C2-type 1 | 0.0500 | 0.9000 | 1.0000 | 1.0000 | 0.9587 | 0.0043 | Skin Basal Cell Carcinoma |
| | NM_001012338 | | | | | | | | |
| | NP_001012338 | | | | | | | | |
| 1 | T230S | Ig-like C2-type 1 | 0.0500 | 0.9000 | 1.0000 | 1.0000 | 0.9929 | 0.0400 | Lung Squamous Cell Carcinoma |
| | NM_001012338 | | | | | | | | |
| | NP_001012338 | | | | | | | | |
| 2 | N232S | Ig-like C2-type 1 | 0.1000 | 0.9000 | 0.0000 | 0.9836 | 0.9953 | 0.0000 | Brain Glioblastoma; Lung Non-Small Cell Lung Carcinoma |
| | G233F | | | | | | | | |
| | NM_001012338 | | | | | | | | |
| | NP_ 001012338 | | | | | | | | |
| 2 | G235E | Ig-like C2-type 1 | 0.1000 | 0.9000 | 0.0000 | 1.0000 | 0.9745 | 0.0000 | Skin Melanoma; Lung Squamous Cell Carcinoma |
| | G235R | | | | | | | | |
| | NM_001012338 | | | | | | | | |
| | NP_ 001012338 | | | | | | | | |
| 5 | P239H | Ig-like C2-type 1 | 0.2500 | 0.9000 | 0.2000 | 1.0000 | 0.9968 | 0.0381 | Lung Adenocarcinoma; Bladder Urothelial Carcinoma; Unknown Primary Melanoma |
| | P239S | | | | | | | | |
| | P239T | | | | | | | | |
| | D240G | | | | | | | | |
| | D240H | | | | | | | | |
| | NM_001012338 | | | | | | | | |
| | NP_ 001012338 | | | | | | | | |
| 6 | D242N | Ig-like C2-type 1 | 0.3000 | 0.9000 | 0.5000 | 1.0000 | 0.9584 | 0.1120 | Lung Small Cell Carcinoma; Lung Adenocarcinoma; |
| | W243C | | | | | | | | |
| | I244T | | | | | | | | |
| | NM_001012338 | | | | | | | | Colon Adenocarcinoma |
| | NP_001012338 | | | | | | | | |
| 3 | L248M | Ig-like C2-type 1 | 0.1500 | 0.9000 | 0.0000 | 1.0000 | 0.9724 | 0.0000 | Lung Adenocarcinoma; Colon Adenocarcinoma; Gastroesophageal Junction Adenocarcinoma |
| | Q249H | | | | | | | | |
| | NM_001012338 | | | | | | | | |
| | NP_001012338 | | | | | | | | |
| 5 | N252S | Ig-like C2-type 1 | 0.2500 | 0.9000 | 0.6000 | 0.9836 | 0.9789 | 0.0808 | Head and Neck Squamous Cell Carcinoma; Lung Non-Small Cell Lung Carcinoma; Unknown Primary Carcinoma |
| | N252T | | | | | | | | |
| | T253N | | | | | | | | |
| | H254Q | | | | | | | | |
| | NM_001012338 | | | | | | | | |
| | NP_001012338 | | | | | | | | |
| 4 | T253N | Ig-like C2-type 1 | 0.2000 | 0.9000 | 0.0000 | 1.0000 | 0.9767 | 0.0000 | Lung Non-Small Cell Lung Carcinoma; Head and Neck Squamous Cell Carcinoma; Lunq Sarcoma |
| | H254Q | | | | | | | | |
| | Q255H | | | | | | | | |
| | NM_001012338 | | | | | | | | |
| | NP_001012338 | | | | | | | | |
| 4 | H254Q | Ig-like C2-type 1 | 0.2000 | 0.9000 | 0.0000 | 1.0000 | 0.9766 | 0.0000 | Lung Non-Small Cell Lung Carcinoma; Unknown Primary Adenocarcinoma; Lung Sarcoma |
| | Q255H | | | | | | | | |
| | T256N | | | | | | | | |
| | NM_001012338 | | | | | | | | |
| | NP_ 001012338 | | | | | | | | |
| 1 | W260R | Ig-like C2-type 1 | 0.0500 | 0.9000 | 1.0000 | 1.0000 | 0.9919 | 0.0045 | Lung Adenocarcinoma |
| | NM_001012338 | | | | | | | | |
| | NP_001012338 | | | | | | | | |
| 1 | N262S | Ig-like C2-type 1 | 0.0500 | 0.9000 | 1.0000 | 1.0000 | 0.9919 | 0.0399 | Ovary Carcinoma |
| | NM_001012338 | | | | | | | | |
| | NP_001012338 | | | | | | | | |
| 1 | I266V | Ig-like C2-type 1 | 0.0500 | 0.9000 | 1.0000 | 1.0000 | 0.9919 | 0.0399 | Kidney Renal Cell Carcinoma |
| | NM_001012338 | | | | | | | | |
| | NP_001012338 | | | | | | | | |
| 8 | T269A | Ig-like C2-type 1 | 0.4000 | 0.9000 | 0.6250 | 0.9672 | 0.9701 | 0.1004 | Lung Adenocarcinoma; Head and Neck Squamous Cell Carcinoma; Skin Melanoma |
| | T269M | | | | | | | | |
| | L270M | | | | | | | | |
| | L270Q | | | | | | | | |
| | L270V | | | | | | | | |
| | V271L | | | | | | | | |
| | V271M | | | | | | | | |
| | NM_001012338 | | | | | | | | |
| | NP_001012338 | | | | | | | | |
| 2 | V273M | Ig-like C2-type 1 | 0.1000 | 0.9000 | 1.0000 | 1.0000 | 0.9727 | 0.0438 | Lung Adenocarcinoma; Rectum Squamous Cell Carcinoma |
| | V273del | | | | | | | | |
| | NM_001012338 | | | | | | | | |
| | NP_ 001012338 | | | | | | | | |
| 5 | E276D | Ig-like C2-type 1 | 0.2500 | 0.9000 | 1.0000 | 0.9836 | 0.9454 | 0.1586 | Breast Invasive Ductal Carcinoma; Unknown Primary Adenocarcinoma; Stomach Adenocarcinoma |
| | D277E | | | | | | | | |
| | D277G | | | | | | | | |
| | D277N | | | | | | | | |
| | NM_001012338 | | | | | | | | |
| | NP_ 001012338 | | | | | | | | |
| 1 | G279D | Ig-like C2-type 1 | 0.0500 | 0.9000 | 1.0000 | 1.0000 | 0.9534 | 0.0429 | Head and Neck Squamous Cell Carcinoma |
| | NM_001012338 | | | | | | | | |
| | NP_001012338 | | | | | | | | |
| 12 | T281I | Ig-like C2-type 1 | 0.6000 | 0.9000 | 1.0000 | 0.9508 | 0.9594 | 0.2223 | Lung Adenocarcinoma^{2, 3, 4, 12}; Unknown Primary Carcinoma; Breast Invasive Ductal Carcinoma |
| | T281P | | | | | | | | |
| | L282M² | | | | | | | | |
| | T283A | | | | | | | | |
| | T283K^{2, 4, 12} | | | | | | | | |
| | T283M | | | | | | | | |
| | NM_001012338 | | | | | | | | |
| | NP_001012338 | | | | | | | | |
| 2 | E287D | Ig-like C2-type 1 | 0.1000 | 0.9000 | 1.0000 | 1.0000 | 0.9751 | 0.0816 | Uterus Endometrial Adenocarcinoma Papillary Serous; Eye Intraocular |
| | E287Q | | | | | | | | |
| | NM_001012338 | | | | | | | | |
| | NP_ 001012338 | | | | | | | | |
| | | | | | | | | | Melanoma |
| 2 | V289A | Ig-like C2-type 1 | 0.1000 | 0.9000 | 1.0000 | 1.0000 | 0.9919 | 0.0670 | Ovary Serous Carcinoma; Uterus Endometrial Adenocarcinoma Endometrioid |
| | V290A | | | | | | | | |
| | NM_001012338 | | | | | | | | |
| | NP_001012338 | | | | | | | | |
| 5 | M292I | Ig-like C2-type 1 | 0.2500 | 0.9000 | 0.4000 | 1.0000 | 0.9714 | 0.0261 | Breast Invasive Ductal Carcinoma; Unknown Primary Adenocarcinoma; Thyroid Papillary Carcinoma |
| | M292V | | | | | | | | |
| | S293R | | | | | | | | |
| | N294T | | | | | | | | |
| | NM_001012338 | | | | | | | | |
| | NP_001012338 | | | | | | | | |
| 3 | S296I | Ig-like C2-type 1 | 0.1500 | 0.9000 | 1.0000 | 1.0000 | 0.9809 | 0.1208 | Head and Neck Squamous Cell Carcinoma; Lung Squamous Cell Carcinoma; Bone Marrow Leukemia Non-Lymphocytic Acute Myelocytic |
| | S296R | | | | | | | | |
| | V297I | | | | | | | | |
| | NM_001012338 | | | | | | | | |
| | NP_001012338 | | | | | | | | |
| 1 | L299del | Ig-like C2-type 1 | 0.0500 | 0.9000 | 1.0000 | 1.0000 | 0.9967 | 0.0401 | Colon Adenocarcinoma |
| | NM_001012338 | | | | | | | | |
| | NP_001012338 | | | | | | | | |
| 1 | V301F | | 0.0500 | 0.0500 | 1.0000 | 1.0000 | 0.9967 | 0.0025 | Lung Adenocarcinoma |
| | NM_001012338 | | | | | | | | |
| | NP_001012338 | | | | | | | | |
| 18 | P304L | | 0.9000 | 0.0500 | 0.6667 | 0.7213 | 0.9965 | 0.0000 | Unknown Primary Melanoma; Colon Adenocarcinoma; Breast Invasive Ductal Carcinoma |
| | P304S | | | | | | | | |
| | P304T | | | | | | | | |
| | P305Q | | | | | | | | |
| | P305R | | | | | | | | |
| | P305S | | | | | | | | |
| | P305T | | | | | | | | |
| | R306C | | | | | | | | |
| | R306H | | | | | | | | |
| | R306P | | | | | | | | |
| | NM_001012338 | | | | | | | | |
| | NP_ 001012338 | | | | | | | | |
| 6 | V308L | | 0.3000 | 0.0500 | 0.0000 | 0.9672 | 0.9677 | 0.0000 | Lung Adenocarcinoma; Breast Invasive Ductal Carcinoma; Lung Non-Small Cell Lung Carcinoma |
| | S309I | | | | | | | | |
| | NM_001012338 | | | | | | | | |
| | NP_001012338 | | | | | | | | |
| 6 | E312K | Ig-like C2-type 2 | 0.3000 | 0.9000 | 0.0000 | 1.0000 | 0.9858 | 0.0000 | Lung Adenocarcinoma; Colon Adenocarcinoma; Skin Melanoma |
| | E312Q | | | | | | | | |
| | P313T | | | | | | | | |
| | E314A | | | | | | | | |
| | E314D | | | | | | | | |
| | E314Q | | | | | | | | |
| | NM_001012338 | | | | | | | | |
| | NP_ 001012338 | | | | | | | | |
| 4 | R316C | Ig-like C2-type 2 | 0.2000 | 0.9000 | 0.0000 | 0.9016 | 0.9965 | 0.0000 | Colon Adenocarcinoma; Breast Invasive Ductal Carcinoma; Skin Melanoma |
| | R316H | | | | | | | | |
| | NM_001012338 | | | | | | | | |
| | NP_001012338 | | | | | | | | |
| 1 | C320F | Ig-like C2-type 2 | 0.0500 | 0.9000 | 1.0000 | 1.0000 | 0.9965 | 0.0401 | Lung Non-Small Cell Lung Carcinoma |
| | NM_001012338 | | | | | | | | |
| | NP_001012338 | | | | | | | | |
| 6 | E322A | Ig-like C2-type 2 | 0.3000 | 0.9000 | 0.1667 | 1.0000 | 0.9942 | 0.0388 | Lung Adenocarcinoma; Unknown Primary Melanoma; Unknown Primary Carcinoma |
| | E322K | | | | | | | | |
| | E322Q | | | | | | | | |
| | F323L | | | | | | | | |
| | NM_001012338 | | | | | | | | |
| | NP_001012338 | | | | | | | | |
| 8 | V325M | Ig-like C2-type 2 | 0.4000 | 0.9000 | 0.5000 | 0.8689 | 0.9965 | 0.1185 | Lung Adenocarcinoma; Colon Adenocarcinoma; Breast Carcinoma |
| | R326C | | | | | | | | |
| | R326G | | | | | | | | |
| | R326H | | | | | | | | |
| | R326L | | | | | | | | |
| | R326P | | | | | | | | |
| | NM_001012338 | | | | | | | | |
| | NP_001012338 | | | | | | | | |
| 5 | N328S | Ig-like C2-type 2 | 0.2500 | 0.9000 | 1.0000 | 0.9836 | 0.9951 | 0.1101 | Lung Adenocarcinoma; Colon Adenocarcinoma; Skin Melanoma |
| | P329N | | | | | | | | |
| | P329S | | | | | | | | |
| | P330Q | | | | | | | | |
| | NM_001012338 | | | | | | | | |
| | NP_001012338 | | | | | | | | |
| 2 | T332M | Ig-like C2-type 2 | 0.1000 | 0.9000 | 1.0000 | 0.7377 | 0.9965 | 0.0592 | Colon Adenocarcinoma; Esophagus Adenocarcinoma |
| | NM_001012338 | | | | | | | | |
| | NP_001012338 | | | | | | | | |
| 1 | H334Q | Ig-like C2-type 2 | 0.0500 | 0.9000 | 0.0000 | 0.9836 | 0.7412 | 0.0000 | Lung Adenocarcinoma |
| | NM_001012338 | | | | | | | | |
| | NP_001012338 | | | | | | | | |
| 2 | L336R | Ig-like C2-type 2 | 0.1000 | 0.9000 | 1.0000 | 1.0000 | 0.9897 | 0.0000 | Ovary Serous Carcinoma; Unknown Primary Melanoma |
| | H337R | | | | | | | | |
| | NM_001012338 | | | | | | | | |
| | NP_ 001012338 | | | | | | | | |
| 3 | G339K | Ig-like C2-type 2 | 0.1500 | 0.9000 | 0.0000 | 1.0000 | 0.9170 | 0.0000 | Lung Adenocarcinoma; Rectum Adenocarcinoma; Skin Squamous Cell Carcinoma |
| | Q340H Q340K | | | | | | | | |
| | NM_001012338 | | | | | | | | |
| | NP_001012338 | | | | | | | | |
| 5 | R343L | Ig-like C2-type 2 | 0.2500 | 0.9000 | 1.0000 | 1.0000 | 0.9924 | 0.0837 | Lung Adenocarcinoma; Colon Adenocarcinoma; Bladder Urothelial Carcinoma |
| | E344G | | | | | | | | |
| | E344V | | | | | | | | |
| | S345F | | | | | | | | |
| | NM_001012338 | | | | | | | | |
| | NP_ 001012338 | | | | | | | | |
| 5 | E344G | Ig-like C2-type 2 | 0.2500 | 0.9000 | 1.0000 | 1.0000 | 0.9924 | 0.1954 | Colon Adenocarcinoma; Bladder Urothelial Carcinoma; Gastroesophageal Junction Adenocarcinoma |
| | E344V | | | | | | | | |
| | S345F | | | | | | | | |
| | K346N | | | | | | | | |
| | NM_001012338 | | | | | | | | |
| | NP_001012338 | | | | | | | | |
| 6 | H349Y | Ig-like C2-type 2 | 0.3000 | 0.9000 | 1.0000 | 1.0000 | 0.6005 | 0.1564 | Lung Adenocarcinoma; Bone Marrow Multiple Myeloma; Lung Squamous Cell Carcinoma |
| | V350E | | | | | | | | |
| | E351D | | | | | | | | |
| | NM_001012338 | | | | | | | | |
| | NP_ 001012338 | | | | | | | | |
| 3 | Y353F | Ig-like C2-type 2 | 0.1500 | 0.9000 | 1.0000 | 1.0000 | 0.9920 | 0.1269 | Lung Adenocarcinoma; Lung Small Cell Undifferentiated Carcinoma |
| | Q354K | | | | | | | | |
| | NM_001012338 | | | | | | | | |
| | NP_001012338 | | | | | | | | |
| 7 | G356E | Ig-like C2-type 2 | 0.3500 | 0.9000 | 0.0000 | 1.0000 | 0.9168 | 0.0000 | Skin Melanoma; Lung Adenocarcinoma; Unknown Primary Melanoma |
| | G356R | | | | | | | | |
| | G356Y | | | | | | | | |
| | E357D | | | | | | | | |
| | NM_001012338 | | | | | | | | |
| | NP_001012338 | | | | | | | | |
| 2 | S359F | Ig-like C2-type 2 | 0.1000 | 0.9000 | 0.0000 | 1.0000 | 0.9960 | 0.0000 | Skin Melanoma; Eye Intraocular Squamous Cell Carcinoma |
| | NM_001012338 | | | | | | | | |
| | NP_001012338 | | | | | | | | |
| 3 | G361N | Ig-like C2-type 2 | 0.1500 | 0.9000 | 0.0000 | 1.0000 | 0.9960 | 0.0000 | Lung Non-Small Cell Lung Carcinoma; Unknown Primary Squamous Cell Carcinoma; Skin Adnexal Carcinoma |
| | G361S | | | | | | | | |
| | C362F | | | | | | | | |
| | NM_001012338 | | | | | | | | |
| | NP_001012338 | | | | | | | | |
| 1 | L364F | Ig-like C2-type 2 | 0.0500 | 0.9000 | 1.0000 | 0.9836 | 0.9960 | 0.0441 | Colon Adenocarcinoma |
| | NM_001012338 | | | | | | | | |
| | NP_001012338 | | | | | | | | |
| 2 | H370N | Ig-like C2-type 2 | 0.1000 | 0.9000 | 0.0000 | 1.0000 | 0.9953 | 0.0000 | Lung Adenocarcinoma; Skin Melanoma |
| | NM_001012338 | | | | | | | | |
| | NP_001012338 | | | | | | | | |
| 1 | N372K | Ig-like C2-type 2 | 0.0500 | 0.9000 | 0.0000 | 1.0000 | 0.9204 | 0.0000 | Colon Adenocarcinoma |
| | NM_001012338 | | | | | | | | |
| | NP_001012338 | | | | | | | | |
| 1 | Y376N | Ig-like C2-type 2 | 0.0500 | 0.9000 | 1.0000 | 1.0000 | 0.9869 | 0.0444 | Lung Adenocarcinoma |
| | NM_001012338 | | | | | | | | |
| | NP_001012338 | | | | | | | | |
| 6 | L378V | Ig-like C2-type 2 | 0.3000 | 0.9000 | 1.0000 | 0.9836 | 0.9551 | 0.0884 | Breast Invasive Ductal Carcinoma; Head and Neck Squamous Cell Carcinoma; Skin Melanoma |
| | I379V | | | | | | | | |
| | A380P | | | | | | | | |
| | A380V | | | | | | | | |
| | NM_001012338 | | | | | | | | |
| | NP_001012338 | | | | | | | | |
| 3 | N382H | Ig-like C2-type 2 | 0.1500 | 0.9000 | 1.0000 | 1.0000 | 0.9880 | 0.0930 | Rectum Adenocarcinoma; Esophagus Adenocarcinoma; Soft Tissue Synovial Sarcoma |
| | N382I | | | | | | | | |
| | N382T | | | | | | | | |
| | NM_001012338 | | | | | | | | |
| | NP_001012338 | | | | | | | | |
| 1 | L384M | | 0.0500 | 0.0500 | 1.0000 | 1.0000 | 0.9319 | 0.0021 | Lung Adenocarcinoma |
| | NM_001012338 | | | | | | | | |
| | NP_001012338 | | | | | | | | |
| 5 | N388K | | 0.2500 | 0.0500 | 1.0000 | 0.8361 | 0.9829 | 0.0000 | Colon Adenocarcinoma; Brain Glioblastoma; Unknown Primary Adenocarcinoma |
| | Q389E | | | | | | | | |
| | Q389H | | | | | | | | |
| | NM_001012338 | | | | | | | | |
| | NP_ 001012338 | | | | | | | | |
| 9 | N392S | | 0.4500 | 0.0500 | 1.0000 | 0.7213 | 0.3707 | 0.0000 | Lung Adenocarcinoma; Skin Melanoma; Soft Tissue Sarcoma |
| | G393D | | | | | | | | |
| | G393S | | | | | | | | |
| | H394Q | | | | | | | | |
| | NM_001012338 | | | | | | | | |
| | NP_ 001012338 | | | | | | | | |
| 5 | L396I | | 0.2500 | 0.0500 | 1.0000 | 0.9836 | 0.9498 | 0.0106 | Lung Non-Small Cell Lung Carcinoma; Lung Adenocarcinoma; Bladder Urothelial Carcinoma |
| | K397N | | | | | | | | |
| | E398D | | | | | | | | |
| | E398K | | | | | | | | |
| | NM_001012338 | | | | | | | | |
| | NP_ 001012338 | | | | | | | | |
| 5 | K397N | | 0.2500 | 0.0500 | 0.4000 | 0.9836 | 0.9498 | 0.0042 | Lung Non-Small Cell Lung Carcinoma; Lung Adenocarcinoma; Skin Melanoma |
| | E398D | | | | | | | | |
| | E398K | | | | | | | | |
| | P399L | | | | | | | | |
| | NM_001012338 | | | | | | | | |
| | NP_ 001012338 | | | | | | | | |
| 5 | P401Q | | 0.2500 | 0.0500 | 1.0000 | 1.0000 | 0.9966 | 0.0119 | Skin Melanoma; Unknown Primary Melanoma; Unknown Primary Malignant Neoplasm |
| | P401S | | | | | | | | |
| | NM_001012338 | | | | | | | | |
| | NP_001012338 | | | | | | | | |
| 4 | T404M | | 0.2000 | 0.0500 | 0.2500 | 0.8689 | 0.9940 | 0.0020 | Lung Adenocarcinoma; Brain Glioblastoma; Lung Small Cell Undifferentiated Carcinoma |
| | T404S | | | | | | | | |
| | D405N | | | | | | | | |
| | D405V | | | | | | | | |
| | NM_001012338 | | | | | | | | |
| | NP_ 001012338 | | | | | | | | |
| 1 | I408M | | 0.0500 | 0.0500 | 1.0000 | 1.0000 | 0.0000 | 0.0000 | Head and Neck Squamous Cell Carcinoma |
| | NM_001012338 | | | | | | | | |
| | NP_001012338 | | | | | | | | |
| 5 | D411E | | 0.2500 | 0.0500 | 1.0000 | 0.9344 | 0.4354 | 0.0048 | Breast Carcinoma; Lung Adenocarcinoma; Skin Melanoma |
| | D411N | | | | | | | | |
| | E412K | | | | | | | | |
| | NM_001012338 | | | | | | | | |
| | NP_001012338 | | | | | | | | |
| 4 | P415H | | 0.2000 | 0.0500 | 1.0000 | 1.0000 | 0.9974 | 0.0093 | Lung Non-Small Cell Lung Carcinoma; Unknown Primary Melanoma; |
| | P415S | | | | | | | | |
| | T416I | | | | | | | | |
| | P417L | | | | | | | | |
| | NM_001012338 | | | | | | | | Head and Neck Neuroblastoma |
| | NP_001012338 | | | | | | | | |
| 3 | T416I | | 0.1500 | 0.0500 | 1.0000 | 1.0000 | 0.9959 | 0.0075 | Stomach Adenocarcinoma; Unknown Primary Melanoma; Ovary Carcinosarcoma |
| | P417L | | | | | | | | |
| | P418H | | | | | | | | |
| | NM_001012338 | | | | | | | | |
| | NP_001012338 | | | | | | | | |
| 2 | V421L | | 0.1000 | 0.0500 | 1.0000 | 1.0000 | 0.9974 | 0.0050 | Lung Adenocarcinoma; Ovary Serous Carcinoma |
| | NM_001012338 | | | | | | | | |
| | NP_001012338 | | | | | | | | |
| 1 | H423Q | | 0.0500 | 0.0500 | 1.0000 | 1.0000 | 0.7735 | 0.0002 | Lung Squamous Cell Carcinoma |
| | NM_001012338 | | | | | | | | |
| | NP_001012338 | | | | | | | | |
| 2 | P425S | | 0.1000 | 0.0500 | 1.0000 | 0.9836 | 0.9733 | 0.0048 | Skin Melanoma; Unknown Primary Melanoma |
| | E426K | | | | | | | | |
| | NM_001012338 | | | | | | | | |
| | NP_001012338 | | | | | | | | |
| 4 | T429I | | 0.2000 | 0.0500 | 0.5000 | 1.0000 | 0.9960 | 0.0037 | Lung Adenocarcinoma; Lung Small Cell Undifferentiated Carcinoma; Brain Oligodendroglioma |
| | F430V | | | | | | | | |
| | G431V | | | | | | | | |
| | G431W | | | | | | | | |
| | NM_001012338 | | | | | | | | |
| | NP_001012338 | | | | | | | | |
| 1 | S433F | | 0.0500 | 0.0500 | 1.0000 | 1.0000 | 0.9978 | 0.0025 | Skin Melanoma |
| | NM_001012338 | | | | | | | | |
| | NP_001012338 | | | | | | | | |
| 4 | A435E | | 0.2000 | 0.0500 | 1.0000 | 1.0000 | 0.9965 | 0.0093 | Lung Adenocarcinoma; Breast Invasive Ductal Carcinoma; Lung Squamous Cell Carcinoma |
| | V436A | | | | | | | | |
| | V436F | | | | | | | | |
| | G437E | | | | | | | | |
| | NM_001012338 | | | | | | | | |
| | NP_001012338 | | | | | | | | |
| 1 | A439P | | 0.0500 | 0.0500 | 1.0000 | 1.0000 | 0.9978 | 0.0025 | Lung Squamous Cell Carcinoma |
| | NM_001012338 | | | | | | | | |
| | NP_001012338 | | | | | | | | |
| 4 | V448A | | 0.2000 | 0.0500 | 0.2500 | 1.0000 | 0.9685 | 0.0021 | Lung Adenocarcinoma; Stomach Adenocarcinoma; Lung Large Cell Neuroendocrine Carcinoma |
| | L449P | | | | | | | | |
| | F450L | | | | | | | | |
| | NM_001012338 | | | | | | | | |
| | NP_001012338 | | | | | | | | |
| 8 | L449P | | 0.4000 | 0.0500 | 0.3750 | 0.9672 | 0.7053 | 0.0013 | Lung Adenocarcinoma; Colon Adenocarcinoma; Kidney Renal Cell Carcinoma |
| | F450L | | | | | | | | |
| | V451I | | | | | | | | |
| | NM_001012338 | | | | | | | | |
| | NP_ 001012338 | | | | | | | | |
| 13 | F450L | | 0.6500 | 0.0500 | 0.6154 | 0.9672 | 0.8075 | 0.0043 | Lung Adenocarcinoma; Colon Adenocarcinoma; Kidney Renal Cell Carcinoma |
| | V451I | | | | | | | | |
| | M452I | | | | | | | | |
| | M452K | | | | | | | | |
| | M452L | | | | | | | | |
| | M452V | | | | | | | | |
| | NM_001012338 | | | | | | | | |
| | NP_ 001012338 | | | | | | | | |
| 2 | K455N | | 0.1000 | 0.0500 | 1.0000 | 1.0000 | 0.9693 | 0.0036 | Head and Neck Squamous Cell Carcinoma; Soft Tissue Angiosarcoma |
| | K455R | | | | | | | | |
| | NM_001012338 | | | | | | | | |
| | NP_001012338 | | | | | | | | |
| 9 | G457C | | 0.4500 | 0.0500 | 0.0000 | 0.9672 | 0.9645 | 0.0000 | Lung Adenocarcinoma; Colon Adenocarcinoma; Skin Melanoma |
| | G457V | | | | | | | | |
| | R458P | | | | | | | | |
| | R459G | | | | | | | | |
| | R459W | | | | | | | | |
| | NM_001012338 | | | | | | | | |
| | NP_ 001012338 | | | | | | | | |
| 7 | R458P | | 0.3500 | 0.0500 | 0.0000 | 0.9672 | 0.9546 | 0.0000 | Lung Adenocarcinoma; Colon Adenocarcinoma; Skin Melanoma |
| | R459G | | | | | | | | |
| | R459W | | | | | | | | |
| | S460T | | | | | | | | |
| | NM_001012338 | | | | | | | | |
| | NP_ 001012338 | | | | | | | | |
| 8 | R459G | | 0.4000 | 0.0500 | 0.0000 | 0.9672 | 0.9584 | 0.0000 | Lung Adenocarcinoma; Colon Adenocarcinoma; Skin Melanoma |
| | R459W | | | | | | | | |
| | S460T | | | | | | | | |
| | K461R | | | | | | | | |
| | NM_001012338 | | | | | | | | |
| | NP_ 001012338 | | | | | | | | |
| 5 | G463R | | 0.2500 | 0.0500 | 1.0000 | 0.9836 | 0.9865 | 0.0109 | Head and Neck Squamous Cell Carcinoma; Unknown Primary Melanoma; Uterus Endometrial Adenocarcinoma Endometrioid |
| | G463V | | | | | | | | |
| | M464I | | | | | | | | |
| | NM_001012338 | | | | | | | | |
| | NP_001012338 | | | | | | | | |
| 8 | G466C | | 0.4000 | 0.0500 | 1.0000 | 0.9672 | 0.9903 | 0.0027 | Breast Invasive Ductal Carcinoma; Ovary Serous Carcinoma; Lung Non-Small Cell Lung Carcinoma |
| | P467H | | | | | | | | |
| | P467S | | | | | | | | |
| | V468L | | | | | | | | |
| | V468M | | | | | | | | |
| | NM_001012338 | | | | | | | | |
| | NP_ 001012338 | | | | | | | | |
| 8 | P467H | | 0.4000 | 0.0500 | 1.0000 | 0.9672 | 0.9964 | 0.0028 | Lung Non-Small Cell Lung Carcinoma; Breast Invasive Ductal Carcinoma; Ovary Serous Carcinoma |
| | P467S | | | | | | | | |
| | V468L | | | | | | | | |
| | V468M | | | | | | | | |
| | A469D | | | | | | | | |
| | NM_001012338 | | | | | | | | |
| | NP_001012338 | | | | | | | | |
| 2 | G473C | | 0.1000 | 0.0500 | 1.0000 | 1.0000 | 0.9912 | 0.0050 | Lung Adenocarcinoma |
| | E474G | | | | | | | | |
| | NM_001012338 | | | | | | | | |
| | NP_ 001012338 | | | | | | | | |
| 3 | S477L | | 0.1500 | 0.0500 | 0.0000 | 1.0000 | 0.9953 | 0.0000 | Lung Adenocarcinoma; Anus Squamous Cell Carcinoma |
| | A478G | | | | | | | | |
| | NM_001012338 | | | | | | | | |
| | NP_ 001012338 | | | | | | | | |
| 3 | G487C | | 0.1500 | 0.0500 | 0.0000 | 0.9672 | 0.9557 | 0.0000 | Colon Adenocarcinoma; Brain Glioblastoma; Uterus Endometrial Adenocarcinoma Endometrioid |
| | G487S | | | | | | | | |
| | I488T | | | | | | | | |
| | NM_001012338 | | | | | | | | |
| | NP_001012338 | | | | | | | | |
| 7 | T490K | | 0.3500 | 0.0500 | 0.0000 | 0.8361 | 0.9635 | 0.0000 | Colon Adenocarcinoma; Lung Adenocarcinoma; Breast Invasive Ductal Carcinoma |
| | T490M | | | | | | | | |
| | P491H | | | | | | | | |
| | S492L | | | | | | | | |
| | NM_001012338 | | | | | | | | |
| | NP_001012338 | | | | | | | | |
| 1 | L494M | | 0.0500 | 0.0500 | 0.0000 | 1.0000 | 0.3947 | 0.0000 | Lung Non-Small Cell Lung Carcinoma |
| | NM_001012338 | | | | | | | | |
| | NP_001012338 | | | | | | | | |
| 13 | A496E | | 0.6500 | 0.0500 | 0.6154 | 0.7869 | 0.9890 | 0.0008 | Lung Adenocarcinoma; Skin Squamous Cell Carcinoma; Breast Invasive Ductal Carcinoma |
| | A496V | | | | | | | | |
| | G497R | | | | | | | | |
| | G497V | | | | | | | | |
| | G497W | | | | | | | | |
| | NM_001012338 | | | | | | | | |
| | NP_001012338 | | | | | | | | |
| 5 | D499N | | 0.2500 | 0.0500 | 1.0000 | 0.8525 | 0.9972 | 0.0000 | Lung Adenocarcinoma; Stomach Adenocarcinoma; Unknown Primary Melanoma |
| | NM_001012338 | | | | | | | | |
| | NP_001012338 | | | | | | | | |
| 1 | V501L | | 0.0500 | 0.0500 | 0.0000 | 1.0000 | 0.9972 | 0.0000 | Lung Adenocarcinoma |
| | NM_001012338 | | | | | | | | |
| | NP_001012338 | | | | | | | | |
| 11 | T506A | | 0.5500 | 0.0500 | 0.7273 | 0.8852 | 0.9634 | 0.0094 | Lung Adenocarcinoma; Colon Adenocarcinoma; Kidney Renal Cell Carcinoma |
| | T506S | | | | | | | | |
| | R507C | | | | | | | | |
| | R507H | | | | | | | | |
| | R507P | | | | | | | | |
| | I508T | | | | | | | | |
| | NM_001012338 | | | | | | | | |
| | NP_001012338 | | | | | | | | |
| 11 | R507C | | 0.5500 | 0.0500 | 0.7273 | 0.9016 | 0.9484 | 0.0086 | Colon Adenocarcinoma; Lung Adenocarcinoma; Brain Glioblastoma |
| | R507H | | | | | | | | |
| | R507P | | | | | | | | |
| | I508T | | | | | | | | |
| | P509L | | | | | | | | |
| | P509S | | | | | | | | |
| | NM_001012338 | | | | | | | | |
| | NP_ 001012338 | | | | | | | | |
| 4 | I511T | | 0.2000 | 0.0500 | 1.0000 | 1.0000 | 0.8334 | 0.0077 | Lung Adenocarcinoma; Gastroesophageal Junction Adenocarcinoma; Uterus Endometrial Adenocarcinoma Endometrioid |
| | E512K | | | | | | | | |
| | N513I | | | | | | | | |
| | N513K | | | | | | | | |
| | NM_001012338 | | | | | | | | |
| | NP_001012338 | | | | | | | | |
| 5 | E512K | | 0.2500 | 0.0500 | 1.0000 | 1.0000 | 0.8571 | 0.0036 | Lung Adenocarcinoma; Breast Invasive Ductal Carcinoma; Gastroesophageal Junction Adenocarcinoma |
| | N5131 | | | | | | | | |
| | N513K | | | | | | | | |
| | P514H | | | | | | | | |
| | P514S | | | | | | | | |
| | NM_001012338 | | | | | | | | |
| | NP_ 001012338 | | | | | | | | |
| 1 | Y516F | | 0.0500 | 0.0500 | 1.0000 | 1.0000 | 0.8632 | 0.0022 | Unknown Primary Carcinoma |
| | NM_001012338 | | | | | | | | |
| | NP_001012338 | | | | | | | | |
| 8 | R518C | | 0.4000 | 0.0500 | 0.2500 | 0.9016 | 0.9563 | 0.0011 | Colon Adenocarcinoma; Brain Glioblastoma; Lung Adenocarcinoma |
| | R518H | | | | | | | | |
| | Q519E | | | | | | | | |
| | Q519L | | | | | | | | |
| | G520E | | | | | | | | |
| | NM_001012338 | | | | | | | | |
| | NP_ 001012338 | | | | | | | | |
| 4 | Q519E | | 0.2000 | 0.0500 | 0.2500 | 1.0000 | 0.9834 | 0.0020 | Lung Adenocarcinoma; Colon Adenocarcinoma; Breast Invasive Ductal Carcinoma |
| | Q519L | | | | | | | | |
| | G520E | | | | | | | | |
| | H521N | | | | | | | | |
| | NM_001012338 | | | | | | | | |
| | NP_001012338 | | | | | | | | |
| 3 | G520E | | 0.1500 | 0.0500 | 1.0000 | 1.0000 | 0.9799 | 0.0056 | Colon Adenocarcinoma; Unknown Primary Adenocarcinoma; Stomach Adenocarcinoma Diffuse Type |
| | H521N | | | | | | | | |
| | N522K | | | | | | | | |
| | NM_001012338 | | | | | | | | |
| | NP_001012338 | | | | | | | | |
| 4 | P526A | | 0.2000 | 0.0500 | 0.0000 | 0.9836 | 0.9853 | 0.0000 | Lung Adenocarcinoma; Unknown Primary Adenocarcinoma |
| | P526Q | | | | | | | | |
| | D527E | | | | | | | | |
| | NM_001012338 | | | | | | | | |
| | NP_001012338 | | | | | | | | |
| 1 | Y529N | | 0.0500 | 0.0500 | 1.0000 | 1.0000 | 0.9913 | 0.0012 | Ovary Epithelial Carcinoma |
| | NM_001012338 | | | | | | | | |
| | NP_001012338 | | | | | | | | |
| 1 | Q531R | | 0.0500 | 0.0500 | 1.0000 | 0.9836 | 0.8616 | 0.0019 | Head and Neck Squamous Cell Carcinoma |
| | NM_001012338 | | | | | | | | |
| | NP_001012338 | | | | | | | | |
| 6 | I533F | | 0.3000 | 0.0500 | 1.0000 | 1.0000 | 0.9912 | 0.0000 | Lung Adenocarcinoma; Breast Invasive Ductal Carcinoma; Head and Neck Squamous Cell Carcinoma |
| | I533L | | | | | | | | |
| | K534E | | | | | | | | |
| | K534R | | | | | | | | |
| | R535M | | | | | | | | |
| | NM_001012338 | | | | | | | | |
| | NP_001012338 | | | | | | | | |
| 5 | K534E | | 0.2500 | 0.0500 | 0.4000 | 1.0000 | 0.9940 | 0.0044 | Lung Adenocarcinoma; Unknown Primary Melanoma |
| | K534R | | | | | | | | |
| | R535M | | | | | | | | |
| | R536I | | | | | | | | |
| | R536T | | | | | | | | |
| | NM_001012338 | | | | | | | | |
| | NP_ 001012338 | | | | | | | | |
| 5 | R535M | | 0.2500 | 0.0500 | 0.4000 | 1.0000 | 0.9967 | 0.0044 | Lung Adenocarcinoma; Lung Squamous Cell Carcinoma; Unknown Primary Melanoma |
| | R536I | | | | | | | | |
| | R536T | | | | | | | | |
| | D537E | | | | | | | | |
| | D537Y | | | | | | | | |
| | NM_001012338 | | | | | | | | |
| | NP_001012338 | | | | | | | | |
| 5 | R536I | | 0.2500 | 0.0500 | 0.4000 | 1.0000 | 0.9954 | 0.0044 | Lung Adenocarcinoma; Lung Squamous Cell Carcinoma; Unknown Primary Melanoma |
| | R536T | | | | | | | | |
| | D537E | | | | | | | | |
| | D537Y | | | | | | | | |
| | I538N | | | | | | | | |
| | NM_001012338 | | | | | | | | |
| | NP_ 001012338 | | | | | | | | |
| 6 | D537E | Protein kinase | 0.3000 | 1.0000 | 1.0000 | 0.9672 | 0.9956 | 0.2375 | Skin Melanoma^{2, 6}; Lung Squamous Cell Carcinoma; Uterus Endometrial Adenocarcinoma Endometrioid^{2, 5}; Liver Carcinoma⁶ |
| | D537Y^{2,5} | | | | | | | | |
| | I538N | | | | | | | | |
| | V539M^{2, 6} | | | | | | | | |
| | NM001012338 | | | | | | | | |
| | _ NP_001012338 | | | | | | | | |
| 5 | I538N | Protein kinase | 0.2500 | 1.0000 | 1.0000 | 0.9672 | 0.5976 | 0.1128 | Skin Melanoma; Stomach Adenocarcinoma; Gallbladder Adenocarcinoma |
| | V539M | | | | | | | | |
| | L540M | | | | | | | | |
| | NM_001012338 | | | | | | | | |
| | NP_ 001012338 | | | | | | | | |
| 3 | R542L | Protein kinase | 0.1500 | 1.0000 | 1.0000 | 1.0000 | 0.4907 | 0.0678 | Kidney Renal Cell Carcinoma; Stomach Adenocarcinoma; Esophagus Adenocarcinoma |
| | R542del | | | | | | | | |
| | E543D | | | | | | | | |
| | NM_001012338 | | | | | | | | |
| | NP_001012338 | | | | | | | | |
| 3 | G545C | Protein kinase | 0.1500 | 1.0000 | 1.0000 | 1.0000 | 0.9967 | 0.1229 | Lung Adenocarcinoma; Soft Tissue Inflammatory Myofibroblastic Tumor |
| | G545D | | | | | | | | |
| | NM_001012338 | | | | | | | | |
| | NP_001012338 | | | | | | | | |
| 3 | G547E | Protein kinase | 0.1500 | 1.0000 | 0.0000 | 1.0000 | 0.9967 | 0.0000 | Lung Adenocarcinoma; Breast Carcinoma; Gastroesophageal Junction Adenocarcinoma |
| | G547V | | | | | | | | |
| | A548_P562del¹ | | | | | | | | |
| | NM_001012338 | | | | | | | | |
| | NP_001012338 | | | | | | | | |
| 3 | A548_P562del¹ | Protein kinase | 0.1500 | 1.0000 | 1.0000 | 1.0000 | 0.9945 | 0.1243 | Breast Carcinoma; Gastroesophageal Junction Adenocarcinoma; Skin Squamous Cell Carcinoma |
| | G550R | | | | | | | | |
| | K551E | | | | | | | | |
| | NM_001012338 | | | | | | | | |
| | NP_001012338 | | | | | | | | |
| 11 | A548_P562del¹ | Protein kinase | 0.5500 | 1.0000 | 0.8182 | 0.8852 | 0.9967 | 0.1184 | Breast Carcinoma; Lung Adenocarcinoma; Colon Adenocarcinoma |
| | L560V | | | | | | | | |
| | P562L | | | | | | | | |
| | P562Q | | | | | | | | |
| | P562R | | | | | | | | |
| | P562T | | | | | | | | |
| | NM_001012338 | | | | | | | | |
| | NP_ 001012338 | | | | | | | | |
| 3 | D565H | Protein kinase | 0.1500 | 1.0000 | 0.0000 | 1.0000 | 0.9945 | 0.0000 | Lung Non-Small Cell Lung Carcinoma; Lung Squamous Cell Carcinoma; Pancreas Ductal Adenocarcinoma |
| | K566N | | | | | | | | |
| | M567T | | | | | | | | |
| | NM_001012338 | | | | | | | | |
| | NP_001012338 | | | | | | | | |
| 3 | K566N | Protein kinase | 0.1500 | 1.0000 | 0.0000 | 1.0000 | 0.9945 | 0.0000 | Lung Non-Small Cell Lung Carcinoma; Pancreas Ductal Adenocarcinoma; Cervix Squamous Cell Carcinoma |
| | M567T | | | | | | | | |
| | L568F | | | | | | | | |
| | NM_001012338 | | | | | | | | |
| | NP_001012338 | | | | | | | | |
| 3 | M567T | Protein kinase | 0.1500 | 1.0000 | 0.0000 | 1.0000 | 0.9945 | 0.0000 | Lung Non-Small Cell Lung Carcinoma; Pancreas Ductal Adenocarcinoma; Cervix Squamous Cell Carcinoma |
| | L568F | | | | | | | | |
| | V569L | | | | | | | | |
| | NM_001012338 | | | | | | | | |
| | NP_001012338 | | | | | | | | |
| 1 | K572N | Protein kinase | 0.0500 | 1.0000 | 0.0000 | 1.0000 | 0.9967 | 0.0000 | Lung Adenocarcinoma |
| | NM_001012338 | | | | | | | | |
| | NP_001012338 | | | | | | | | |
| 7 | K575E | Protein kinase | 0.3500 | 1.0000 | 0.5714 | 1.0000 | 0.9493 | 0.1743 | Skin Melanoma; Unknown Primary Malignant Neoplasm; Unknown Primary Undifferentiated Neuroendocrine Carcinoma |
| | D576N | | | | | | | | |
| | P577S | | | | | | | | |
| | NM_001012338 | | | | | | | | |
| | NP_001012338 | | | | | | | | |
| 1 | L579M | Protein kinase | 0.0500 | 1.0000 | 1.0000 | 1.0000 | 0.4716 | 0.0236 | Lung Squamous Cell Carcinoma |
| | NM_001012338 | | | | | | | | |
| | NP_001012338 | | | | | | | | |
| 9 | A581D | Protein kinase | 0.4500 | 1.0000 | 0.6667 | 0.9344 | 0.5079 | 0.0300 | Breast Ductal Carcinoma in Situ; Head and Neck Squamous Cell Carcinoma; Skin Melanoma |
| | R582Q | | | | | | | | |
| | R582W | | | | | | | | |
| | K583T | | | | | | | | |
| | NM_001012338 | | | | | | | | |
| | NP_ 001012338 | | | | | | | | |
| 14 | R582Q | Protein kinase | 0.7000 | 1.0000 | 0.7857 | 0.9344 | 0.5448 | 0.1703 | Skin Melanoma; Unknown Primary Melanoma; Breast Ductal Carcinoma in Situ |
| | R582W | | | | | | | | |
| | K583T | | | | | | | | |
| | D584E | | | | | | | | |
| | D584N | | | | | | | | |
| | NM_001012338 | | | | | | | | |
| | NP_001012338 | | | | | | | | |
| 3 | Q586K | Protein kinase | 0.1500 | 1.0000 | 0.0000 | 1.0000 | 0.9955 | 0.0000 | Lung Adenocarcinoma; Unknown Primary Carcinoma |
| | Q586L | | | | | | | | |
| | NM_001012338 | | | | | | | | |
| | NP_ 001012338 | | | | | | | | |
| 1 | E588Q | Protein kinase | 0.0500 | 1.0000 | 0.0000 | 1.0000 | 0.9973 | 0.0000 | Colon Adenocarcinoma |
| | NM_001012338 | | | | | | | | |
| | NP_001012338 | | | | | | | | |
| 2 | E590D | Protein kinase | 0.1000 | 1.0000 | 0.0000 | 0.9672 | 0.7326 | 0.0000 | Lung Adenocarcinoma; Skin Melanoma |
| | E590K | | | | | | | | |
| | NM_001012338 | | | | | | | | |
| | NP_ 001012338 | | | | | | | | |
| 1 | L592I | Protein kinase | 0.0500 | 1.0000 | 0.0000 | 1.0000 | 0.9973 | 0.0000 | Lung Adenocarcinoma |
| | NM_001012338 | | | | | | | | |
| | NP_001012338 | | | | | | | | |
| 4 | L595P | Protein kinase | 0.2000 | 1.0000 | 0.0000 | 1.0000 | 0.5097 | 0.0000 | Lung Adenocarcinoma; Unknown Primary Adenocarcinoma |
| | Q596K | | | | | | | | |
| | H597N | | | | | | | | |
| | H597Q | | | | | | | | |
| | NM_001012338 | | | | | | | | |
| | NP_ 001012338 | | | | | | | | |
| 4 | Q596K | Protein kinase | 0.2000 | 1.0000 | 0.0000 | 1.0000 | 0.5097 | 0.0000 | Lung Adenocarcinoma |
| | H597N | | | | | | | | |
| | H597Q | | | | | | | | |
| | E598G | | | | | | | | |
| | NM_001012338 | | | | | | | | |
| | NP_ 001012338 | | | | | | | | |
| 5 | H597N | Protein kinase | 0.2500 | 1.0000 | 0.0000 | 1.0000 | 0.6061 | 0.0000 | Lung Adenocarcinoma; Uterus Endometrial Adenocarcinoma Endometrioid |
| | H597Q | | | | | | | | |
| | E598G | | | | | | | | |
| | H599L | | | | | | | | |
| | H599Y | | | | | | | | |
| | NM_001012338 | | | | | | | | |
| | NP_001012338 | | | | | | | | |
| 4 | E598G | Protein kinase | 0.2000 | 1.0000 | 0.0000 | 1.0000 | 0.9931 | 0.0000 | Lung Adenocarcinoma; Prostate Acinar Adenocarcinoma; Uterus Endometrial Adenocarcinoma Endometrioid |
| | H599L | | | | | | | | |
| | H599Y | | | | | | | | |
| | I600V | | | | | | | | |
| | NM_001012338 | | | | | | | | |
| | NP_001012338 | | | | | | | | |
| 5 | H599L | Protein kinase | 0.2500 | 1.0000 | 0.4000 | 1.0000 | 0.9940 | 0.0445 | Lung Adenocarcinoma; Prostate Acinar Adenocarcinoma; Uterus Endometrial Adenocarcinoma Endometrioid |
| | H599Y | | | | | | | | |
| | I600V | | | | | | | | |
| | V601A | | | | | | | | |
| | V601I | | | | | | | | |
| | NM_001012338 | | | | | | | | |
| | NP_ 001012338 | | | | | | | | |
| 4 | I600V | Protein kinase | 0.2000 | 1.0000 | 1.0000 | 1.0000 | 0.9931 | 0.0000 | Lung Adenocarcinoma; Colon Adenocarcinoma; Prostate Acinar Adenocarcinoma |
| | V601A | | | | | | | | |
| | V601I | | | | | | | | |
| | K602R | | | | | | | | |
| | NM_001012338 | | | | | | | | |
| | NP_ 001012338 | | | | | | | | |
| 5 | V601A | Protein kinase | 0.2500 | 1.0000 | 1.0000 | 1.0000 | 0.9951 | 0.1113 | Lung Adenocarcinoma; Colon Adenocarcinoma; Lung Non-Small Cell Lung Carcinoma |
| | V601I | | | | | | | | |
| | K602R | | | | | | | | |
| | F603L | | | | | | | | |
| | NM_001012338 | | | | | | | | |
| | NP_001012338 | | | | | | | | |
| 6 | K602R | Protein kinase | 0.3000 | 1.0000 | 0.5000 | 1.0000 | 0.9936 | 0.1365 | Lung Adenocarcinoma; Colon Adenocarcinoma; Lung Non-Small Cell Lung Carcinoma |
| | F603L | | | | | | | | |
| | Y604F | | | | | | | | |
| | Y604H | | | | | | | | |
| | Y604N | | | | | | | | |
| | NM_001012338 | | | | | | | | |
| | NP_ 001012338 | | | | | | | | |
| 6 | F603L | Protein kinase | 0.3000 | 1.0000 | 0.0000 | 1.0000 | 0.9868 | 0.0000 | Lung Adenocarcinoma; Lung Non-Small Cell Lung Carcinoma; Lung Squamous Cell Carcinoma |
| | Y604F | | | | | | | | |
| | Y604H | | | | | | | | |
| | Y604N | | | | | | | | |
| | G605V | | | | | | | | |
| | NM_001012338 | | | | | | | | |
| | NP_ 001012338 | | | | | | | | |
| 19 | C607F | Protein kinase | 0.9500 | 1.0000 | 0.8421 | 0.9180 | 0.9966 | 0.6144 | Lung Adenocarcinoma^{2 (G608C), 7}; Head and Neck Squamous Cell Carcinoma; Skin Melanoma; Large Intestine Adenocarcinoma^{2, 12 (G608S), 8}; Pancreas Carcinoma^{8, 12}; Urinary Tract Carcinoma^{2 (D609N)}, ⁹; Kidney Carcinoma⁹; Upper Aerodigestive Tract Squamous Cell Carcinoma^{2 (D609V), 10, 12}; Colorectal Cancer¹¹ |
| | G608C^{2, 7} | | | | | | | | |
| | G608E | | | | | | | | |
| | G608S^{2, 8, 11, 12} | | | | | | | | |
| | D609G | | | | | | | | |
| | D609H | | | | | | | | |
| | D609N^{2, 9} | | | | | | | | |
| | D609V^{2, 10, 12} | | | | | | | | |
| | NM001012338 | | | | | | | | |
| | _ NP_001012338 | | | | | | | | |
| 20 | G608C^{2, 7} | Protein kinase | 1.0000 | 1.0000 | 1.0000 | 0.9180 | 0.9966 | 0.7348 | Head and Neck Squamous Cell Carcinoma; Skin Melanoma; Lung Adenocarcinoma^{2 (G608C), 7}; Large Intestine Adenocarcinoma^{2 (G608S), 8, 12}; Pancreas Carcinoma^{8, 12}; Urinary Tract Carcinoma^{2 (D609N)}, ⁹; Kidney Carcinoma⁹; Upper Aerodigestive Tract Squamous Cell Carcinoma^{2 (D609V), 10, 12}; Colorectal Cancer¹¹ |
| | G608E | | | | | | | | |
| | G608S^{2, 8, 11, 12} | | | | | | | | |
| | D609G | | | | | | | | |
| | D609H | | | | | | | | |
| | D609N^{2, 9} | | | | | | | | |
| | D609V^{2, 10, 12} | | | | | | | | |
| | G610R | | | | | | | | |
| | NM_001012338 | | | | | | | | |
| | NP_001012338 | | | | | | | | |
| 5 | P612A | Protein kinase | 0.2500 | 1.0000 | 1.0000 | 0.9672 | 0.9973 | 0.1531 | Bone Marrow Multiple Myeloma; Lung Non-Small Cell Lung Carcinoma; Lung Squamous Cell Carcinoma |
| | P612L | | | | | | | | |
| | P612S | | | | | | | | |
| | P612T | | | | | | | | |
| | NM_001012338 | | | | | | | | |
| | NP_001012338 | | | | | | | | |
| 1 | M615I | Protein kinase | 0.0500 | 1.0000 | 0.0000 | 1.0000 | 0.9973 | 0.0000 | Lung Adenocarcinoma |
| | NM_001012338 | | | | | | | | |
| | NP_001012338 | | | | | | | | |
| 3 | K621N | Protein kinase | 0.1500 | 1.0000 | 0.0000 | 1.0000 | 0.9517 | 0.0000 | Lung Adenocarcinoma; Gastroesophageal Junction Adenocarcinoma |
| | NM_001012338 | | | | | | | | |
| | NP_001012338 | | | | | | | | |
| 3 | G623E | Protein kinase | 0.1500 | 1.0000 | 0.0000 | 1.0000 | 0.9973 | 0.0000 | Skin Melanoma; Lung Non-Small Cell Lung Carcinoma; Kidney Urothelial Carcinoma |
| | D624Y | | | | | | | | |
| | L625M | | | | | | | | |
| | NM_001012338 | | | | | | | | |
| | NP_ 001012338 | | | | | | | | |
| 3 | D624Y | Protein kinase | 0.1500 | 1.0000 | 1.0000 | 1.0000 | 0.9023 | 0.1015 | Lung Non-Small Cell Lung Carcinoma; Unknown Primary Malignant Neoplasm; Kidney Urothelial Carcinoma |
| | L625M | | | | | | | | |
| | N626K | | | | | | | | |
| | NM_001012338 | | | | | | | | |
| | NP_001012338 | | | | | | | | |
| 4 | L625M | Protein kinase | 0.2000 | 1.0000 | 1.0000 | 1.0000 | 0.9130 | 0.1217 | Ovary Serous Carcinoma; Lung Non-Small Cell Lung Carcinoma; Gastroesophageal Junction Adenocarcinoma |
| | N626K | | | | | | | | |
| | K627N | | | | | | | | |
| | K627R | | | | | | | | |
| | NM_001012338 | | | | | | | | |
| | NP_001012338 | | | | | | | | |
| 4 | N626K | Protein kinase | 0.2000 | 1.0000 | 1.0000 | 1.0000 | 0.9116 | 0.1367 | Ovary Serous Carcinoma; Gastroesophageal Junction Adenocarcinoma; Unknown Primary Melanoma |
| | K627N | | | | | | | | |
| | K627R | | | | | | | | |
| | F628L | | | | | | | | |
| | NM_001012338 | | | | | | | | |
| | NP_001012338 | | | | | | | | |
| 5 | K627N | Protein kinase | 0.2500 | 1.0000 | 1.0000 | 1.0000 | 0.9858 | 0.1868 | Breast Ductal Carcinoma in Situ; Ovary Serous Carcinoma; Gastroesophageal Junction Adenocarcinoma |
| | K627R | | | | | | | | |
| | F628L | | | | | | | | |
| | L629F | | | | | | | | |
| | L629I | | | | | | | | |
| | NM_001012338 | | | | | | | | |
| | NP_ 001012338 | | | | | | | | |
| 4 | A631V | Protein kinase | 0.2000 | 1.0000 | 0.2500 | 1.0000 | 0.9961 | 0.0423 | Lung Adenocarcinoma; Head and Neck Squamous Cell Carcinoma; Skin Melanoma |
| | H632N | | | | | | | | |
| | H632Y | | | | | | | | |
| | NM_001012338 | | | | | | | | |
| | NP_ 001012338 | | | | | | | | |
| 6 | P634L | Protein kinase | 0.3000 | 1.0000 | 0.6667 | 1.0000 | 0.9961 | 0.1153 | Lung Adenocarcinoma; Head and Neck Squamous Cell Carcinoma; Colon Adenocarcinoma |
| | P634T | | | | | | | | |
| | D635H | | | | | | | | |
| | A636E | | | | | | | | |
| | A636V | | | | | | | | |
| | NM_001012338 | | | | | | | | |
| | NP_001012338 | | | | | | | | |
| 7 | D635H | Protein kinase | 0.3500 | 1.0000 | 0.7143 | 0.9836 | 0.9940 | 0.2330 | Lung Adenocarcinoma; Head and Neck Squamous Cell Carcinoma; Colon Adenocarcinoma |
| | A636E | | | | | | | | |
| | A636V | | | | | | | | |
| | M6371 | | | | | | | | |
| | M637K | | | | | | | | |
| | M637V | | | | | | | | |
| | NM_001012338 | | | | | | | | |
| | NP_ 001012338 | | | | | | | | |
| 6 | Q643E | Protein kinase | 0.3000 | 1.0000 | 1.0000 | 0.0000 | 0.9858 | 0.0000 | Lung Adenocarcinoma; Lung Non-Small Cell Lung Carcinoma; Lymph Node Lymphoma Diffuse Large B Cell |
| | Q643H | | | | | | | | |
| | P644T | | | | | | | | |
| | R645C | | | | | | | | |
| | R645L | | | | | | | | |
| | R645S | | | | | | | | |
| | NM_001012338 | | | | | | | | |
| | NP_ 001012338 | | | | | | | | |
| 6 | A647D | Protein kinase | 0.3000 | 1.0000 | 0.0000 | 0.9836 | 0.9614 | 0.0000 | Lung Adenocarcinoma; Breast Carcinoma; Head and Neck Squamous Cell Carcinoma |
| | A647I | | | | | | | | |
| | K648N | | | | | | | | |
| | G649S | | | | | | | | |
| | G649V | | | | | | | | |
| | NM_001012338 | | | | | | | | |
| | NP_ 001012338 | | | | | | | | |
| 4 | K648N | Protein kinase | 0.2000 | 1.0000 | 0.0000 | 1.0000 | 0.9744 | 0.0000 | Lung Adenocarcinoma; Lung Squamous Cell Carcinoma; Skin Squamous Cell Carcinoma |
| | G649S | | | | | | | | |
| | G649V | | | | | | | | |
| | E650V | | | | | | | | |
| | NM_001012338 | | | | | | | | |
| | NP_ 001012338 | | | | | | | | |
| 4 | G649S | Protein kinase | 0.2000 | 1.0000 | 0.2500 | 1.0000 | 0.9925 | 0.0372 | Lung Adenocarcinoma; Skin Squamous Cell Carcinoma; Nasopharynx and Paranasal Sinuses Undifferentiated Carcinoma |
| | G649V | | | | | | | | |
| | E650V | | | | | | | | |
| | L651P | | | | | | | | |
| | NM_001012338 | | | | | | | | |
| | NP_001012338 | | | | | | | | |
| 5 | E650V | Protein kinase | 0.2500 | 1.0000 | 1.0000 | 1.0000 | 0.9932 | 0.2130 | Lung Adenocarcinoma^{2, 13}; Kidney Urothelial Carcinoma; Soft Tissue Angiosarcoma |
| | L651P | | | | | | | | |
| | G652R^{2, 13} | | | | | | | | |
| | G652V² | | | | | | | | |
| | NM_001012338 | | | | | | | | |
| | NP_001012338 | | | | | | | | |
| 6 | L651P | Protein kinase | 0.3000 | 1.0000 | 1.0000 | 1.0000 | 0.9937 | 0.2792 | Lung Adenocarcinoma Lung Squamous Cell Carcinoma; Unknown Primary Melanoma |
| | G652R^{2, 13} | | | | | | | | |
| | G652V² | | | | | | | | |
| | L653F | | | | | | | | |
| | L653P | | | | | | | | |
| | NM_001012338 | | | | | | | | |
| | NP_001012338 | | | | | | | | |
| 4 | Q655K | Protein kinase | 0.2000 | 1.0000 | 0.2500 | 1.0000 | 0.9943 | 0.0476 | Lung Non-Small Cell Lung Carcinoma; Lung Adenocarcinoma; Skin Melanoma |
| | Q655del | | | | | | | | |
| | M656R | | | | | | | | |
| | NM_001012338 | | | | | | | | |
| | NP_001012338 | | | | | | | | |
| 2 | H658N | Protein kinase | 0.1000 | 1.0000 | 1.0000 | 1.0000 | 0.9961 | 0.0926 | Lung Adenocarcinoma; Unknown Primary Melanoma |
| | H658Y | | | | | | | | |
| | NM_001012338 | | | | | | | | |
| | NP_ 001012338 | | | | | | | | |
| 2 | A660T | Protein kinase | 0.1000 | 1.0000 | 1.0000 | 1.0000 | 0.9925 | 0.0000 | Lung Adenocarcinoma; Colon Adenocarcinoma |
| | S661G | | | | | | | | |
| | NM_001012338 | | | | | | | | |
| | NP_ 001012338 | | | | | | | | |
| 8 | I663V | Protein kinase | 0.4000 | 1.0000 | 0.2500 | 1.0000 | 0.9952 | 0.0772 | Lung Adenocarcinoma; Colon Adenocarcinoma; Ovary Serous Carcinoma |
| | A664P | | | | | | | | |
| | A664S | | | | | | | | |
| | S665L | | | | | | | | |
| | NM_001012338 | | | | | | | | |
| | NP_001012338 | | | | | | | | |
| 5 | M667I | Protein kinase | 0.2500 | 1.0000 | 0.0000 | 1.0000 | 0.9917 | 0.0000 | Colon Adenocarcinoma; Head and Neck Squamous Cell Carcinoma; Skin Melanoma |
| | M667L | | | | | | | | |
| | V668M | | | | | | | | |
| | Y669C | | | | | | | | |
| | Y669S | | | | | | | | |
| | NM_001012338 | | | | | | | | |
| | NP_ 001012338 | | | | | | | | |
| 1 | S672Y | Protein kinase | 0.0500 | 1.0000 | 0.0000 | 1.0000 | 0.9961 | 0.0000 | Lung Adenocarcinoma |
| | NM_001012338 | | | | | | | | |
| | NP_001012338 | | | | | | | | |
| 1 | F675S | Protein kinase | 0.0500 | 1.0000 | 1.0000 | 1.0000 | 0.9888 | 0.0494 | Unknown Primary Adenocarcinoma |
| | NM_001012338 | | | | | | | | |
| | NP_001012338 | | | | | | | | |
| 7 | H677Y | Protein kinase | 0.3500 | 1.0000 | 0.5714 | 1.0000 | 0.9951 | 0.1792 | Pancreas Ductal Adenocarcinoma; Ovary Serous Carcinoma; Gastroesophageal Junction Adenocarcinoma |
| | R678Q | | | | | | | | |
| | D679G | | | | | | | | |
| | D679N | | | | | | | | |
| | NM_001012338 | | | | | | | | |
| | NP_001012338 | | | | | | | | |
| 1 | R683S | Protein kinase | 0.0500 | 1.0000 | 0.0000 | 1.0000 | 0.9961 | 0.0000 | Breast Ductal Carcinoma in Situ |
| | NM_001012338 | | | | | | | | |
| | NP_001012338 | | | | | | | | |
| 1 | C685F | Protein kinase | 0.0500 | 1.0000 | 1.0000 | 1.0000 | 0.9961 | 0.0498 | Pancreas Neuroendocrine Carcinoma |
| | NM_001012338 | | | | | | | | |
| | NP_001012338 | | | | | | | | |
| 2 | V687A | Protein kinase | 0.6000 | 1.0000 | 0.7500 | 0.9836 | 0.9955 | 0.2385 | Unknown Primary Melanoma; Unknown Primary Malignant Neoplasm; Lung Adenocarcinoma; Mouth Carcinoma¹⁴; Upper Aerodigestive Tract Carcinoma¹⁵ |
| | V687I^{14, 15} | | | | | | | | |
| | G688R | | | | | | | | |
| | A689E | | | | | | | | |
| | A689V | | | | | | | | |
| | NM_001012338 | | | | | | | | |
| | NP_001012338 | | | | | | | | |
| 6 | V693L | Protein kinase | 0.3000 | 1.0000 | 0.0000 | 0.9836 | 0.9925 | 0.0000 | Colon Adenocarcinoma; Rectum Adenocarcinoma; Lung Adenocarcinoma |
| | K694N | | | | | | | | |
| | I695F | | | | | | | | |
| | I695T | | | | | | | | |
| | NM_001012338 | | | | | | | | |
| | NP_001012338 | | | | | | | | |
| 7 | K694N | Protein kinase | 0.3500 | 1.0000 | 0.0000 | 0.9836 | 0.9930 | 0.0000 | Colon Adenocarcinoma; Lung Adenocarcinoma; Breast Invasive Ductal Carcinoma |
| | I695F | | | | | | | | |
| | I695T | | | | | | | | |
| | G696R | | | | | | | | |
| | G696W | | | | | | | | |
| | NM_001012338 | | | | | | | | |
| | NP_ 001012338 | | | | | | | | |
| 6 | I695F | Protein kinase | 0.3000 | 1.0000 | 0.0000 | 0.9836 | 0.9925 | 0.0000 | Colon Adenocarcinoma; Breast Invasive Ductal Carcinoma; Skin Melanoma |
| | I695T | | | | | | | | |
| | G696R | | | | | | | | |
| | G696W | | | | | | | | |
| | D697N | | | | | | | | |
| | NM_001012338 | | | | | | | | |
| | NP_001012338 | | | | | | | | |
| 5 | G699S | Protein kinase | 0.2500 | 1.0000 | 0.0000 | 1.0000 | 0.9946 | 0.0000 | Colon Adenocarcinoma; Head and Neck Squamous Cell Carcinoma; Skin Melanoma |
| | M700T | | | | | | | | |
| | S701F | | | | | | | | |
| | NM_001012338 | | | | | | | | |
| | NP_001012338 | | | | | | | | |
| 3 | M700T | Protein kinase | 0.1500 | 1.0000 | 0.0000 | 1.0000 | 0.9937 | 0.0000 | Colon Adenocarcinoma; Head and Neck Squamous Cell Carcinoma; Skin Melanoma |
| | S701F | | | | | | | | |
| | R702I | | | | | | | | |
| | NM_001012338 | | | | | | | | |
| | NP_001012338 | | | | | | | | |
| 3 | V704F | Protein kinase | 0.1500 | 1.0000 | 0.0000 | 1.0000 | 0.9937 | 0.0000 | Head and Neck Squamous Cell Carcinoma; Lung Non-Small Cell Lung Carcinoma; Uterus Leiomyosarcoma |
| | Y705N | | | | | | | | |
| | S706I | | | | | | | | |
| | NM_001012338 | | | | | | | | |
| | NP_001012338 | | | | | | | | |
| 3 | Y705N | Protein kinase | 0.1500 | 1.0000 | 1.0000 | 0.9344 | 0.9937 | 0.1165 | Head and Neck Squamous Cell Carcinoma; Gallbladder Adenocarcinoma; Uterus Leiomyosarcoma |
| | S706I | | | | | | | | |
| | T707M | | | | | | | | |
| | NM_001012338 | | | | | | | | |
| | NP_001012338 | | | | | | | | |
| 4 | S706I | Protein kinase | 0.2000 | 1.0000 | 1.0000 | 0.9344 | 0.9961 | 0.1629 | Head and Neck Squamous Cell Carcinoma; Unknown Primary Melanoma; Gallbladder Adenocarcinoma |
| | T707M | | | | | | | | |
| | D708N | | | | | | | | |
| | NM_001012338 | | | | | | | | |
| | NP_001012338 | | | | | | | | |
| 3 | Y710C | Protein kinase | 0.1500 | 1.0000 | 0.0000 | 1.0000 | 0.9888 | 0.0000 | Colon Adenocarcinoma; Gastroesophageal Junction Adenocarcinoma; Pancreas Neuroendocrine Carcinoma |
| | Y710H | | | | | | | | |
| | NM_001012338 | | | | | | | | |
| | NP_001012338 | | | | | | | | |
| 2 | L712F | Protein kinase | 0.1000 | 1.0000 | 1.0000 | 1.0000 | 0.9797 | 0.0000 | Bladder Urothelial Carcinoma; Unknown Primary Melanoma |
| | L712P | | | | | | | | |
| | NM_001012338 | | | | | | | | |
| | NP_ 001012338 | | | | | | | | |
| 12 | N714S | Protein kinase | 0.6000 | 1.0000 | 1.0000 | 0.9508 | 0.9784 | 0.0000 | Unknown Primary Melanoma; Breast Carcinoma; Skin Melanoma |
| | P715L | | | | | | | | |
| | P715S | | | | | | | | |
| | S716Y | | | | | | | | |
| | NM_001012338 | | | | | | | | |
| | NP_ 001012338 | | | | | | | | |
| 6 | P715L | Protein kinase | 0.3000 | 1.0000 | 1.0000 | 1.0000 | 0.9875 | 0.1481 | Unknown Primary Melanoma; Breast Invasive Ductal Carcinoma; Bone Marrow Multiple Myeloma |
| | P715S | | | | | | | | |
| | S716Y | | | | | | | | |
| | G717R | | | | | | | | |
| | NM_001012338 | | | | | | | | |
| | NP_ 001012338 | | | | | | | | |
| 3 | D719N | Protein kinase | 0.1500 | 1.0000 | 1.0000 | 1.0000 | 0.9771 | 0.0000 | Skin Melanoma; Unknown Primary Adenocarcinoma; Lung Small Cell Undifferentiated Carcinoma |
| | F720I | | | | | | | | |
| | F720L | | | | | | | | |
| | NM_001012338 | | | | | | | | |
| | NP_001012338 | | | | | | | | |
| 2 | W723R | Protein kinase | 0.1000 | 1.0000 | 1.0000 | 1.0000 | 0.9797 | 0.0980 | Lung Adenocarcinoma; Skin Squamous Cell Carcinoma |
| | C724F | | | | | | | | |
| | NM_001012338 | | | | | | | | |
| | NP_001012338 | | | | | | | | |
| 1 | V726L | Protein kinase | 0.0500 | 1.0000 | 0.0000 | 1.0000 | 0.9973 | 0.0000 | Skin Melanoma |
| | NM_001012338 | | | | | | | | |
| | NP_001012338 | | | | | | | | |
| 7 | T730N | Protein kinase | 0.3500 | 1.0000 | 0.7143 | 1.0000 | 0.9964 | 0.2237 | Lung Adenocarcinoma; Adrenal Gland Cortical Carcinoma; Colon Adenocarcinoma |
| | M731I | | | | | | | | |
| | M731L | | | | | | | | |
| | L732I | | | | | | | | |
| | NM_001012338 | | | | | | | | |
| | NP_ 001012338 | | | | | | | | |
| 11 | R735C | Protein kinase | 0.5500 | 1.0000 | 0.1818 | 1.0000 | 0.9973 | 0.0884 | Colon Adenocarcinoma; Lung Adenocarcinoma; Skin Melanoma |
| | R735H | | | | | | | | |
| | R735S | | | | | | | | |
| | W736C | | | | | | | | |
| | NM_001012338 | | | | | | | | |
| | NP_001012338 | | | | | | | | |
| 3 | P738H | Protein kinase | 0.1500 | 1.0000 | 1.0000 | 1.0000 | 0.9817 | 0.1473 | Lung Adenocarcinoma; Ovary Epithelial Carcinoma; Ovary Clear Cell Carcinoma |
| | P738S | | | | | | | | |
| | NM_001012338 | | | | | | | | |
| | NP_001012338 | | | | | | | | |
| 2 | S741C | Protein kinase | 0.1000 | 1.0000 | 1.0000 | 1.0000 | 0.9944 | 0.0099 | Skin Melanoma; Lung Squamous Cell Carcinoma |
| | S741I | | | | | | | | |
| | NM_001012338 | | | | | | | | |
| | NP_001012338 | | | | | | | | |
| 13 | Y744F | Protein kinase | 0.6500 | 1.0000 | 1.0000 | 0.9672 | 0.9792 | 0.5315 | Gastroesophageal Junction Adenocarcinoma; Stomach Adenocarcinoma; Colon Adenocarcinoma |
| | R745P | | | | | | | | |
| | R745W | | | | | | | | |
| | K746R | | | | | | | | |
| | K746T | | | | | | | | |
| | NM_001012338 | | | | | | | | |
| | NP_ 001012338 | | | | | | | | |
| 1 | T749K | Protein kinase | 0.0500 | 1.0000 | 1.0000 | 1.0000 | 0.9967 | 0.0050 | Lung Adenocarcinoma |
| | NM_001012338 | | | | | | | | |
| | NP_001012338 | | | | | | | | |
| 3 | S751N | Protein kinase | 0.1500 | 1.0000 | 0.0000 | 1.0000 | 0.9967 | 0.0000 | Lung Adenocarcinoma; Unknown Primary Melanoma |
| | D752N | | | | | | | | |
| | V753L | | | | | | | | |
| | NM_001012338 | | | | | | | | |
| | NP_001012338 | | | | | | | | |
| 4 | D752N | Protein kinase | 0.2000 | 1.0000 | 0.0000 | 1.0000 | 0.9967 | 0.0000 | Lung Adenocarcinoma; Lung Squamous Cell Carcinoma; Unknown Primary Melanoma |
| | V753L | | | | | | | | |
| | W754C | | | | | | | | |
| | W754L | | | | | | | | |
| | NM_001012338 | | | | | | | | |
| | NP_001012338 | | | | | | | | |
| 5 | V753L | Protein kinase | 0.2500 | 1.0000 | 0.0000 | 1.0000 | 0.9967 | 0.0000 | Lung Adenocarcinoma; Breast Carcinoma; Lung Squamous Cell Carcinoma |
| | W754C | | | | | | | | |
| | W754L | | | | | | | | |
| | S755R | | | | | | | | |
| | NM_001012338 | | | | | | | | |
| | NP_001012338 | | | | | | | | |
| 4 | G757E | Protein kinase | 0.2000 | 1.0000 | 0.2500 | 1.0000 | 0.9967 | 0.0278 | Lung Adenocarcinoma; Skin Melanoma; Soft Tissue Leiomyosarcoma |
| | G757R | | | | | | | | |
| | G757W | | | | | | | | |
| | NM_001012338 | | | | | | | | |
| | NP_001012338 | | | | | | | | |
| 2 | I759M | Protein kinase | 0.1000 | 1.0000 | 0.0000 | 1.0000 | 0.8619 | 0.0000 | Lung Non-Small Cell Lung Carcinoma; Unknown Primary Squamous Cell Carcinoma |
| | L760F | | | | | | | | |
| | NM_001012338 | | | | | | | | |
| | NP_001012338 | | | | | | | | |
| 4 | E762D | Protein kinase | 0.2000 | 1.0000 | 0.2500 | 1.0000 | 0.8792 | 0.0000 | Colon Adenocarcinoma; Unknown Primary Melanoma; Lung Small Cell Undifferentiated Carcinoma |
| | E762K | | | | | | | | |
| | NM_001012338 | | | | | | | | |
| | NP_001012338 | | | | | | | | |
| 1 | F764I | Protein kinase | 0.0500 | 1.0000 | 1.0000 | 1.0000 | 0.9902 | 0.0495 | Unknown Primary Adenocarcinoma |
| | NM_001012338 | | | | | | | | |
| | NP_001012338 | | | | | | | | |
| 1 | Y766F | Protein kinase | 0.0500 | 1.0000 | 1.0000 | 1.0000 | 0.9902 | 0.0443 | Lung Small Cell Carcinoma |
| | NM_001012338 | | | | | | | | |
| | NP_001012338 | | | | | | | | |
| 7 | K768E | Protein kinase | 0.3500 | 1.0000 | 0.5714 | 0.8852 | 0.9902 | 0.0000 | Gastroesophageal Junction Adenocarcinoma; Skin Melanoma; Pancreas Ductal Adenocarcinoma |
| | K768R | | | | | | | | |
| | NM_001012338 | | | | | | | | |
| | NP_001012338 | | | | | | | | |
| 2 | F772L | Protein kinase | 0.1000 | 1.0000 | 1.0000 | 1.0000 | 0.9967 | 0.0695 | Lung Adenocarcinoma; Lung Large Cell Neuroendocrine Carcinoma |
| | Q773K | | | | | | | | |
| | NM_001012338 | | | | | | | | |
| | NP_001012338 | | | | | | | | |
| 5 | T777M | Protein kinase | 0.2500 | 1.0000 | 0.4000 | 0.9836 | 0.9844 | 0.0904 | Skin Melanoma; Prostate Acinar Adenocarcinoma; Soft Tissue Neuroblastoma |
| | E778K | | | | | | | | |
| | E778V | | | | | | | | |
| | NM_001012338 | | | | | | | | |
| | NP_001012338 | | | | | | | | |
| 4 | E781K | Protein kinase | 0.2000 | 1.0000 | 1.0000 | 1.0000 | 0.9952 | 0.0445 | Lung Adenocarcinoma; Breast Invasive Ductal Carcinoma; Pancreas Ductal Adenocarcinoma |
| | C782R | | | | | | | | |
| | C782S | | | | | | | | |
| | I783N | | | | | | | | |
| | NM_001012338 | | | | | | | | |
| | NP_001012338 | | | | | | | | |
| 4 | C782R | Protein kinase | 0.2000 | 1.0000 | 1.0000 | 1.0000 | 0.9952 | 0.0911 | Lung Adenocarcinoma; Head and Neck Squamous Cell Carcinoma; Pancreas Ductal Adenocarcinoma |
| | C782S | | | | | | | | |
| | I783N | | | | | | | | |
| | T784S | | | | | | | | |
| | NM_001012338 | | | | | | | | |
| | NP_001012338 | | | | | | | | |
| 8 | G786C | Protein kinase | 0.4000 | 1.0000 | 1.0000 | 0.9180 | 0.9977 | 0.2156 | Stomach Adenocarcinoma; Lung Adenocarcinoma; Kidney Renal Cell Carcinoma |
| | G786S | | | | | | | | |
| | R787C | | | | | | | | |
| | R787H | | | | | | | | |
| | R787S | | | | | | | | |
| | NM_001012338 | | | | | | | | |
| | NP_ 001012338 | | | | | | | | |
| 4 | L789F | Protein kinase | 0.2000 | 1.0000 | 0.2500 | 0.9672 | 0.7282 | 0.0059 | Lung Squamous Cell Carcinoma; Lung Adenocarcinoma; Rectum Adenocarcinoma |
| | E790V | | | | | | | | |
| | R791Q | | | | | | | | |
| | R791W | | | | | | | | |
| | NM_001012338 | | | | | | | | |
| | NP_001012338 | | | | | | | | |
| 4 | E790V | Protein kinase | 0.2000 | 1.0000 | 0.2500 | 0.9672 | 0.9404 | 0.0152 | Lung Adenocarcinoma; Lung Squamous Cell Carcinoma; Rectum Adenocarcinoma |
| | R791Q | | | | | | | | |
| | R791W | | | | | | | | |
| | P792H | | | | | | | | |
| | NM_001012338 | | | | | | | | |
| | NP_001012338 | | | | | | | | |
| 6 | R791Q | Protein kinase | 0.3000 | 1.0000 | 0.0000 | 0.7869 | 0.9603 | 0.0000 | Lung Adenocarcinoma; Colon Adenocarcinoma; Breast Ductal Carcinoma in Situ |
| | R791W | | | | | | | | |
| | P792H | | | | | | | | |
| | R793L | | | | | | | | |
| | R793Q | | | | | | | | |
| | NM_001012338 | | | | | | | | |
| | NP_001012338 | | | | | | | | |
| 3 | P796L | Protein kinase | 0.1500 | 1.0000 | 1.0000 | 1.0000 | 0.9977 | 0.1497 | Lung Adenocarcinoma; Unknown Primary Malignant Neoplasm; Brain Oligodendroglioma |
| | P796S | | | | | | | | |
| | NM_001012338 | | | | | | | | |
| | NP_001012338 | | | | | | | | |
| 5 | D801N | Protein kinase | 0.2500 | 1.0000 | 1.0000 | 0.9672 | 0.9977 | 0.2111 | Unknown Primary Melanoma; Head and Neck Squamous Cell Carcinoma; Gallbladder Adenocarcinoma |
| | NM_001012338 | | | | | | | | |
| | NP_001012338 | | | | | | | | |
| 5 | G805R | Protein kinase | 0.2500 | 1.0000 | 0.4000 | 1.0000 | 0.9967 | 0.0997 | Unknown Primary Melanoma; Lung Adenocarcinoma; Ovary Serous Carcinoma |
| | G805W | | | | | | | | |
| | C806S | | | | | | | | |
| | W807G | | | | | | | | |
| | NM_001012338 | | | | | | | | |
| | NP_001012338 | | | | | | | | |
| 4 | C806S | Protein kinase | 0.2000 | 1.0000 | 0.2500 | 1.0000 | 0.9964 | 0.0498 | Lung Adenocarcinoma; Ovary Serous Carcinoma; Stomach Adenocarcinoma Diffuse Type |
| | W807G | | | | | | | | |
| | Q808H | | | | | | | | |
| | NM_001012338 | | | | | | | | |
| | NP_001012338 | | | | | | | | |
| 4 | W807G | Protein kinase | 0.2000 | 1.0000 | 0.2500 | 1.0000 | 0.7716 | 0.0386 | Lung Adenocarcinoma; Ovary Serous Carcinoma; Stomach Adenocarcinoma Diffuse Type |
| | Q808H | | | | | | | | |
| | R809W | | | | | | | | |
| | NM_001012338 | | | | | | | | |
| | NP_001012338 | | | | | | | | |
| 6 | Q808H | Protein kinase | 0.3000 | 1.0000 | 0.5000 | 0.9836 | 0.8478 | 0.1103 | Lung Adenocarcinoma; Skin Melanoma; Ovary Serous Carcinoma |
| | R809W | | | | | | | | |
| | E810K | | | | | | | | |
| | NM_001012338 | | | | | | | | |
| | NP_ 001012338 | | | | | | | | |
| 5 | Q812H | Protein kinase | 0.2500 | 1.0000 | 0.4000 | 1.0000 | 0.9557 | 0.0936 | Lung Non-Small Cell Lung Carcinoma; Lung Adenocarcinoma; Gallbladder Adenocarcinoma |
| | Q813E | | | | | | | | |
| | Q813K | | | | | | | | |
| | R814Q | | | | | | | | |
| | NM_001012338 | | | | | | | | |
| | NP_001012338 | | | | | | | | |
| 6 | Q813E | Protein kinase | 0.3000 | 1.0000 | 0.5000 | 1.0000 | 0.8436 | 0.0799 | Lung Non-Small Cell Lung Carcinoma; Lung Adenocarcinoma; Kidney Chromophobe Carcinoma |
| | Q813K | | | | | | | | |
| | R814Q | | | | | | | | |
| | L815M | | | | | | | | |
| | NM_001012338 | | | | | | | | |
| | NP_001012338 | | | | | | | | |
| 1 | E819K | Protein kinase | 0.0500 | 1.0000 | 1.0000 | 1.0000 | 0.9977 | 0.0446 | Skin Melanoma |
| | NM_001012338 | | | | | | | | |
| | NP_001012338 | | | | | | | | |
| 2 | K822R | Protein kinase | 0.1000 | 1.0000 | 1.0000 | 0.9508 | 0.8995 | 0.0000 | Colon Adenocarcinoma; Unknown Primary Adenocarcinoma |
| | NM_001012338 | | | | | | | | |
| | NP_001012338 | | | | | | | | |
| 6 | L824F | Protein kinase | 0.3000 | 1.0000 | 0.5000 | 0.9508 | 0.9136 | 0.0326 | Unknown Primary Melanoma; Lung Adenocarcinoma; Colon Adenocarcinoma |
| | H825R | | | | | | | | |
| | H825Y | | | | | | | | |
| | A826G | | | | | | | | |
| | A826S | | | | | | | | |
| | A826V | | | | | | | | |
| | NM_001012338 | | | | | | | | |
| | NP_001012338 | | | | | | | | |
| 7 | H825R | Protein kinase | 0.3500 | 1.0000 | 0.1429 | 0.9508 | 0.9309 | 0.0203 | Lung Adenocarcinoma; Unknown Primary Melanoma; Colon Adenocarcinoma |
| | H825Y | | | | | | | | |
| | A826G | | | | | | | | |
| | A826S | | | | | | | | |
| | A826V | | | | | | | | |
| | L827F | | | | | | | | |
| | NM_001012338 | | | | | | | | |
| | NP_ 001012338 | | | | | | | | |
| 7 | A826G | Protein kinase | 0.3500 | 1.0000 | 0.0000 | 0.9836 | 0.9822 | 0.0000 | Lung Adenocarcinoma; Ovary Serous Carcinoma; Skin Melanoma |
| | A826S | | | | | | | | |
| | A826V | | | | | | | | |
| | L827F | | | | | | | | |
| | G828E | | | | | | | | |
| | G828W | | | | | | | | |
| | NM_001012338 | | | | | | | | |
| | NP_ 001012338 | | | | | | | | |
| 1 | A830D | Protein kinase | 0.0500 | 1.0000 | 1.0000 | 1.0000 | 0.9634 | 0.0048 | Bone Marrow Multiple Myeloma |
| | NM_001012338 | | | | | | | | |
| | NP_001012338 | | | | | | | | |
| 6 | P832A | Protein kinase | 0.3000 | 1.0000 | 0.5000 | 1.0000 | 0.9246 | 0.0438 | Lung Adenocarcinoma; Breast Carcinoma; Skin Melanoma |
| | P832R | | | | | | | | |
| | P832T | | | | | | | | |
| | 1833V | | | | | | | | |
| | Y834C | | | | | | | | |
| | Y834N | | | | | | | | |
| | NM_001012338 | | | | | | | | |
| | NP_001012338 | | | | | | | | |
| 2 | D836E | Protein kinase | 0.1000 | 1.0000 | 1.0000 | 1.0000 | 0.9796 | 0.0980 | Prostate Acinar Adenocarcinoma; Lung Carcinosarcoma |
| | D836N | | | | | | | | |
| | NM_001012338 | | | | | | | | |
| | NP_ 001012338 | | | | | | | | |

| | | | | | | | | | |
|---|---|---|---|---|---|---|---|---|---|
| ¹ In some biopsy samples, mutation in the NTRK3 gene results in a TrkC protein lacking amino acids 548 to 562 in the wildtype TrkC protein (e.g., NP_001012338). 2 Pediatric Cancer Gene Database, pedican.bioinfo-minzhao.org/gene_mutation.cgi?gene=4916, downloaded on May 31, 2016. ³ Catalog of Somatic Mutations in Cancer (COSMIC) database, cancer.sanger.ac.uk/cosmic/mutation/ overview?id=401588, downloaded on May 31, 2016. ⁴ Catalog of Somatic Mutations in Cancer (COSMIC) database, cancer.sanger.ac.uk/cosmic/mutation/ overview?id=48622, downloaded on May 31, 2016. ⁵ Catalog of Somatic Mutations in Cancer (COSMIC) database, cancer.sanger.ac.uk/cosmic/mutation/ overview?id=966118, downloaded on May 31, 2016. ⁶ Catalog of Somatic Mutations in Cancer (COSMIC) database, cancer.sanger.ac.uk/cosmic/mutation/ overview?id= 1708512, downloaded on May 31, 2016. ⁷ Catalog of Somatic Mutations in Cancer (COSMIC) database, cancer.sanger.ac.uk/cosmic/mutation/ overview?id=1517968, downloaded on May 31, 2016. ⁸ Catalog of Somatic Mutations in Cancer (COSMIC) database, cancer.sanger.ac.uk/cosmic/mutation/ overview?id=88799, downloaded on May 31, 2016. ⁹ Catalog of Somatic Mutations in Cancer (COSMIC) database, cancer.sanger.ac.uk/cosmic/mutation/ overview?id=471203, downloaded on May 31, 2016. ¹⁰ Catalog of Somatic Mutations in Cancer (COSMIC) database, cancer.sanger.ac.uk/cosmic/mutation/ overview?id= 124878, downloaded on May 31, 2016. ¹¹ Bardelli et al., Science, 300(5621):949, 2003. ¹² Genevois et al., Proc. Nat. Acad. Sci. U.S.A. 110(8):3017-3022, 2013. ¹³ Catalog of Somatic Mutations in Cancer (COSMIC) database, cancer.sanger.ac.uk/cosmic/mutation/ overview?id=1517970, downloaded on May 31, 2016. ¹⁴ Ovarian Cancer Gene Database, ocgene.bioinfo-minzhao.org/gene_mutation.cgi?gene=4916, downloaded on May 31, 2016. ¹⁵ Catalog of Somatic Mutations in Cancer (COSMIC) database, cancer.sanger.ac.uk/cosmic/mutation/ overview?id=3711772, downloaded on May 31, 2016. | | | | | | | | | |

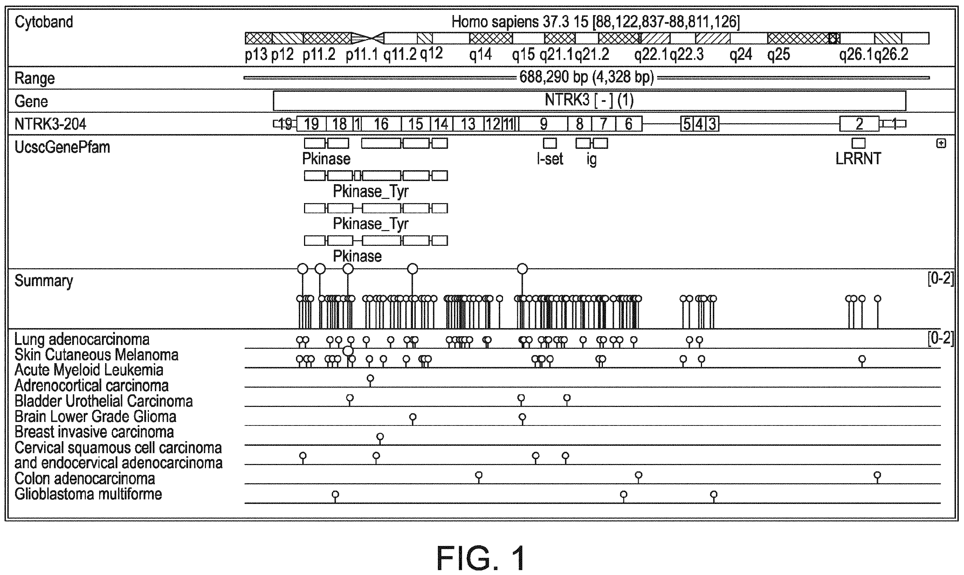

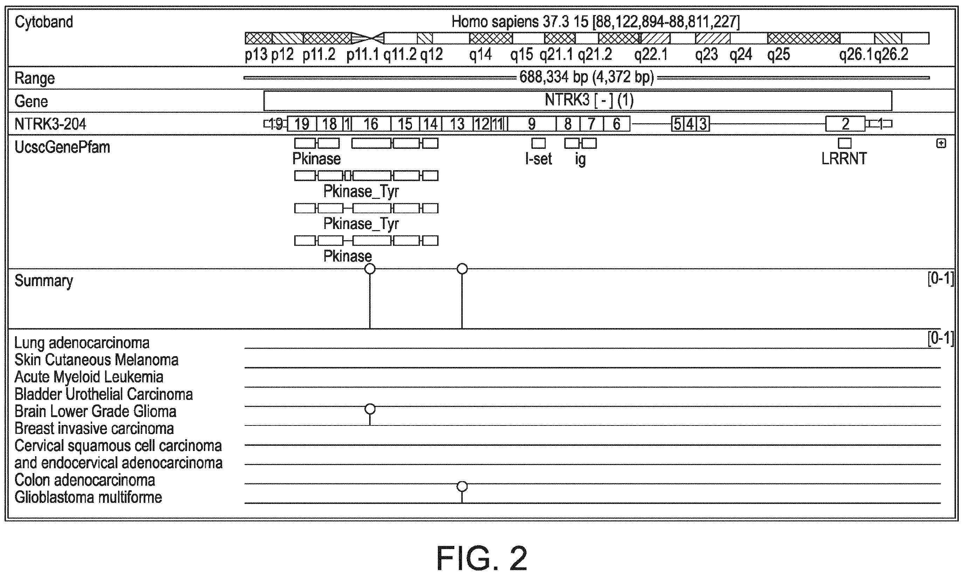

The point mutations observed in NTRK1, NTRK2, and NTRK3 appear to be more common in cancers that are associated with carcinogens known to generate point mutations (e.g., tobacco and UV exposure). As an example, the location of point mutations detected in NTRK3 are shown in Figure 1 and the location of point mutations detected in NTRK3 have confirmed expression above background are shown in Figure 2.

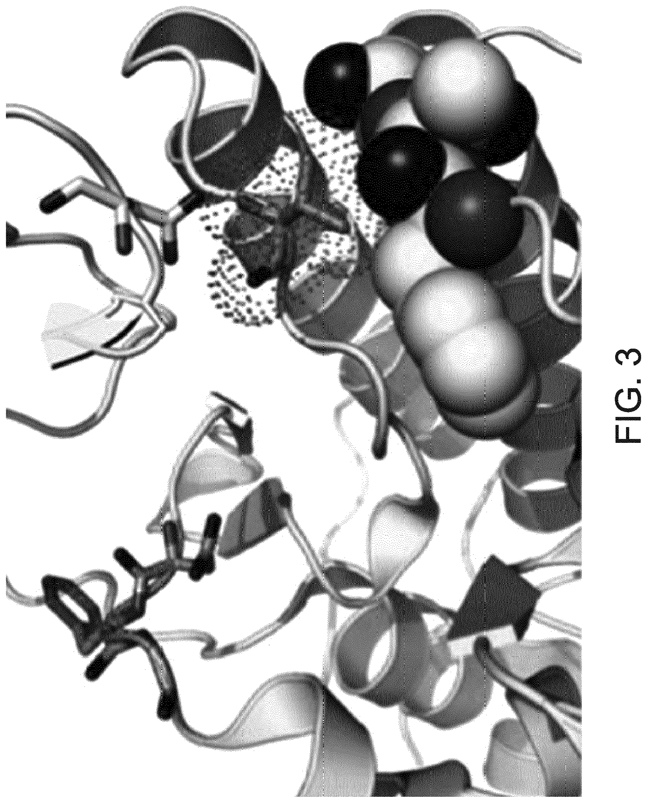

The data in Tables 1 and 2 show that a point mutation in NTRK2 that results in a substitution of the valine at amino acid position 689 in the TrkB protein with a different amino acid is present in cancer tissue. An examination of the crystal structure of TrkB suggests that the valine at amino acid position 689 of TrkB interacts with an asparagine at amino acid position 529 in the juxta-membrane domain, which may allow for the stabilization of the auto-inhibited conformation of the TrkB kinase (Figure 3). When the valine at amino acid position 689 is mutated to a methionine, the large sidechain is predicted to clash with amino acids 746 to 748 in the C-terminal domain and result in a conformation change that may destabilize the auto-inhibited conformation of the TrkB kinase (Figure 3).

The point mutations identified in the NTRK1, NTRK2, and NTRK3 genes may be used, for example, to select subjects for treatment of a Trk inhibitor, used to identify subjects that have an increased likelihood of having a positive response to treatment with a Trk inhibitor, used to determine a subject's risk of developing a cancer, and used to assist in the diagnosis of a cancer in a subject.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A Trk inhibitor for use in treating a subject identified as having a cancer cell that has:
at least one point mutation in a NTRK1 gene that results in the expression of a TrkA protein comprising a mutation at one or more amino acid position(s) selected from the group consisting of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750;
at least one point mutation in a NTRK2 gene that results in the expression of a TrkB protein comprising a mutation at one or more amino acid position(s) selected from the group consisting of 240, 241, 242, 251, 252, 256, 257, 258, 264, 314, 315, 401, 426, 427, 428, 440, 598, 599, 600, 602, 603, 664, 665, 677, 678, 679, 680, 689, 692, 746, 747, 748, 749, 784, 785, 786, 787, 788, 789, 804, and 805, or an amino acid substitution of Q666L; and/or
at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 609, 610, 612, 624, 625, 626, 627, 628, 629, 634, 635, 636, 637, 650, 651, 652, 653, 678, 679, 687, 688, 689, 705, 706, 707, 708, 715, 716, 717, 730, 738, 744, 745, 746, 786, 787, 796, 801, 808, 809, and 810, or an amino acid substitution of G608C, G608E, M731I, M731L, or L732I;
wherein the Trk inhibitor comprises a compound of Formula I: 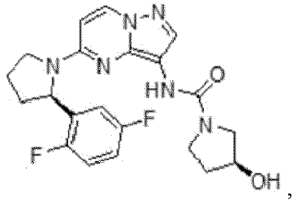 or a pharmaceutically acceptable salt thereof.

2. The Trk inhibitor for use of claim 1, wherein the subject is a subject identified as having a cancer cell that has at least one point mutation in a NTRK1 gene that results in the expression of a TrkA protein comprising a mutation at one or more amino acid position(s) selected from the group consisting of 241, 314, 318, 319, 320, 321, 510, 511, 512, 552, 553, 554, 636, 637, 638, 649, 654, 655, 679, 680, 687, 688, 689, 690, 692, 695, 696, 697, 747, 748, 749, and 750.

3. The Trk inhibitor for use of claim 2, wherein the TrkA protein comprises one or more amino acid substitutions selected from the group consisting of S241F, S241H, S241Y, R314G, R314H, R314L, R314P, N318S, G319S, S320F, V321M, I510T, V511M, L512F, L512R, S552R, A553T, R554P, R554Q, R554W, A636E, A636T, A636V, G637E, G637W, M638V, R649L, R649W, R654C, R654H, N655Y, D679N, D679Y, Y680H, T687I, M688I, L689M, P690H, R692C, R692H, P695S, P696L, E697K, E747K, R748L, R748Q, R748W, P749Q, R750C, R750H, and R750L.

4. The Trk inhibitor for use of claim 1, wherein the subject is a subject identified as having a cancer cell that has at least one point mutation in a NTRK2 gene that results in the expression of a TrkB protein comprising a mutation at one or more amino acid position(s) selected from the group consisting of 240, 241, 242, 251, 252, 256, 257, 258, 264, 314, 315, 401, 426, 427, 428, 440, 598, 599, 600, 602, 603, 664, 665, 677, 678, 679, 680, 689, 692, 746, 747, 748, 749, 784, 785, 786, 787, 788, 789, 804, and 805, or an amino acid substitution of Q666L.

5. The Trk inhibitor for use of claim 4, wherein the TrkB protein comprises one or more amino acid substitutions selected from the group consisting of M240I, N241D, E242K, R251G, R251K, I252V, S256L, S257F, D258N, I264M, A314E, A314G, A314V, L315F, G401A, G401E, G401R, T426I, G427S, R428Q, A440S, A440T, A440V, R598C, R598S, K599M, D600H, H602N, R603S, L664M, T665M, T665S, A677T, A678T, A678V, G679D, M680I, V689M, D692N, F746I, T747M, T748M, E749K, Q784H, G785V, R786Q, V787F, L788M, Q789E, G804E, C805R, and C805Y.

6. The Trk inhibitor for use of claim 1, wherein the subject is a subject identified as having a cancer cell that has at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 609, 610, 612, 624, 625, 626, 627, 628, 629, 634, 635, 636, 637, 650, 651, 652, 653, 678, 679, 687, 688, 689, 705, 706, 707, 708, 715, 716, 717, 730, 738, 744, 745, 746, 786, 787, 796, 801, 808, 809, and 810, or an amino acid substitution of G608C, G608E, M731I, M731L, or L732I.

7. The Trk inhibitor for use of claim 1, wherein the subject is a subject identified as having a cancer cell that has at least one point mutation in a NTRK3 gene that results in the expression of a TrkC protein comprising a mutation at one or more amino acid position(s) selected from the group consisting of 221, 222, 223, 242, 243, 244, 269, 270, 271, 276, 277, 281, 282, 283, 296, 297, 325, 326, 328, 329, 344, 345, 346, 349, 350, 351, 353, 354, 537, 538, 539, 540, 545, 550, 551, 560, 562, 575, 576, 577, 582, 583, 584, 601, 602, 603, 604, 607, 609, 610, 612, 624, 625, 626, 627, 628, 629, 634, 635, 636, 637, 650, 651, 652, 653, 678, 679, 687, 688, 689, 705, 706, 707, 708, 715, 716, 717, 730, 738, 744, 745, 746, 786, 787, 796, 801, 808, 809, and 810.

8. The Trk inhibitor for use of claim 7, wherein the TrkC protein comprises one or more amino acid substitutions selected from the group consisting of V221I, R222Q, E223D, D242N, W243C, I244T, T269A, T269M, T270M, T270Q, T270V, V271L, V271M, E276D, D277E, D277G, D277N, T281I, T281P, T282M, T283A, T283K, T283M, S296I, S296R, V297I, V325M, R326C, R326G, R326H, R326L, R326P, N328S, P329N, P329S, P330Q, E344G, E344V, S345F, K346N, H349Y, V350E, E351D, Y353F, Q354K, D537E, D537Y, I538N, V539M, L540M, G545C, G545D, G550R, K551E, L560V, P562L, P562Q, P562R, P562T, K575E, D576N, P577S, P582Q, P582W, K583%, D584E, D584N, V601A, V601I, K602R, F603L, Y604F, Y604H, Y604N, C607F, D609G, D609H, D609N, D609V, G610R, P612A, P612L, P612S, P612T, D624Y, L625M, N626K, K627N, K627R, F628L, L629F, L629I, P634L, P634T, D635H, A636E, A636V, M637I, M637K, M637V, E650V, L651P, G652R, G652V, L653F, L653P, R678Q, D679G, D679N, V687A, V687I, G688R, A689E, A689V, Y705N, S706I, T707M, D708N, P715L, P715S, S716Y, G717R, T730N, P738H, P738S, Y744F, R745P, R745W, K746R, K746T, G786C, G786S, R787C, R787H, R787S, P796L, P796S, D801N, Q808H, R809W, and E810K.

9. The Trk inhibitor for use of any one of claims 1-8, wherein the cancer is selected from the group consisting of: adenocarcinoma, adrenal gland cortical carcinoma, adrenal gland neuroblastoma, anus squamous cell carcinoma, appendix adenocarcinoma, bladder urothelial carcinoma, bile duct adenocarcinoma, bladder carcinoma, bladder urothelial carcinoma, bone chordoma, bone marrow leukemia lymphocytic chronic, bone marrow leukemia non-lymphocytic acute myelocytic, bone marrow lymph proliferative disease, bone marrow multiple myeloma, bone sarcoma, brain astrocytoma, brain glioblastoma, brain medulloblastoma, brain meningioma, brain oligodendroglioma, breast adenoid cystic carcinoma, breast carcinoma, breast ductal carcinoma in situ, breast invasive ductal carcinoma, breast invasive lobular carcinoma, breast metaplastic carcinoma, cervix neuroendocrine carcinoma, cervix squamous cell carcinoma, colon adenocarcinoma, colon carcinoid tumor, duodenum adenocarcinoma, endometrioid tunor, esophagus adenocarcinoma, esophagus and stomach carcinoma, eye intraocular melanoma, eye intraocular squamous cell carcinoma, eye lacrimal duct carcinoma, fallopian tube serous carcinoma, gallbladder adenocarcinoma, gallbladder glomus tumor, gastroesophageal junction adenocarcinoma, head and neck adenoid cystic carcinoma, head and neck carcinoma, head and neck neuroblastoma, head and neck squamous cell carcinoma, kidney chromophore carcinoma, kidney medullary carcinoma, kidney renal cell carcinoma, kidney renal papillary carcinoma, kidney sarcomatoid carcinoma, kidney urothelial carcinoma, kidney carcinoma, leukemia lymphocytic, leukemia lymphocytic chronic, liver cholangiocarcinoma, liver hepatocellular carcinoma, liver carcinoma, lung adenocarcinoma, lung adenosquamous carcinoma, lung atypical carcinoid, lung carcinosarcoma, lung large cell neuroendocrine carcinoma, lung non-small cell lung carcinoma, lung sarcoma, lung sarcomatoid carcinoma, lung small cell carcinoma, lung small cell undifferentiated carcinoma, lung squamous cell carcinoma, upper aerodigestive tract squamous cell carcinoma, upper aerodigestive tract carcinoma, lymph node lymphoma diffuse large B cell, lymph node lymphoma follicular lymphoma, lymph node lymphoma mediastinal B-cell, lymph node lymphoma plasmablastic lung adenocarcinoma, lymphoma follicular lymphoma, lymphoma, non-Hodgkins, nasopharynx and paranasal sinuses undifferentiated carcinoma, ovary carcinoma, ovary carcinosarcoma, ovary clear cell carcinoma, ovary epithelial carcinoma, ovary granulosa cell tumor, ovary serous carcinoma, pancreas carcinoma, pancreas ductal adenocarcinoma, pancreas neuroendocrine carcinoma, peritoneum mesothelioma, peritoneum serous carcinoma, placenta choriocarcinoma, pleura mesothelioma, prostate acinar adenocarcinoma, prostate carcinoma, rectum adenocarcinoma, rectum squamous cell carcinoma, skin adnexal carcinoma, skin basal cell carcinoma, skin melanoma, skin Merkel cell carcinoma, skin squamous cell carcinoma, small intestine adenocarcinoma, small intestine gastrointestinal stromal tumors (GISTs), large intestine/colon carcinoma, large intestine adenocarcinoma, soft tissue angiosarcoma, soft tissue Ewing sarcoma, soft tissue hemangioendothelioma, soft tissue inflammatory myofibroblastic tumor, soft tissue leiomyosarcoma, soft tissue liposarcoma, soft tissue neuroblastoma, soft tissue paraganglioma, soft tissue perivascular epitheliod cell tumor, soft tissue sarcoma, soft tissue synovial sarcoma, stomach adenocarcinoma, stomach adenocarcinoma diffuse-type, stomach adenocarcinoma intestinal type, stomach adenocarcinoma intestinal type, stomach leiomyosarcoma, thymus carcinoma, thymus thymoma lymphocytic, thyroid papillary carcinoma, unknown primary adenocarcinoma, unknown primary carcinoma, unknown primary malignant neoplasm, lymphoid neoplasm, unknown primary melanoma, unknown primary sarcomatoid carcinoma, unknown primary squamous cell carcinoma, unknown undifferentiated neuroendocrine carcinoma, unknown primary undifferentiated small cell carcinoma, uterus carcinosarcoma, uterus endometrial adenocarcinoma, uterus endometrial adenocarcinoma endometrioid, uterus endometrial adenocarcinoma papillary serous, and uterus leiomyosarcoma, optionally wherein the subject is previously identified or diagnosed as having the cancer.

10. The Trk inhibitor for use of claim 1, wherein the subject has been identified as having a cancer cell that has the at least one point mutation in a NTRK1 gene, the at least one point mutation in a NTRK2 gene, and/or the at least one point mutation in a NTRK3 gene by performance of an assay to determine the presence of the at least one point mutation in a NTRK1 gene, the at least one point mutation in a NTRK2 gene, and/or the at least one point mutation in a NTRK3 gene, in a cancer cell in a sample from the subject.

11. The Trk inhibitor for use of claim 10, wherein the assay is selected from the group consisting of: denaturing gradient gel electrophoresis (DGGE), temperature gradient gel electrophoresis (TGGE), temperature gradient capillary electrophoresis, a single strand conformational polymorphism assay, a molecular beacon assay, a dynamic hybridization assay, a PCR-based assay, and denaturing high performance liquid chromatography.

12. The Trk inhibitor for use of claim 10, wherein the assay comprises sequencing a segment of the NTRK1 gene comprising the at least one point mutation, a segment of the NTRK2 gene comprising the at least one point mutation, and/or a segment of the NTRK3 gene comprising the at least one point mutation.

13. The Trk inhibitor for use of any one of claims 1-12, wherein the Trk inhibitor is a crystalline form of the compound of Formula I: 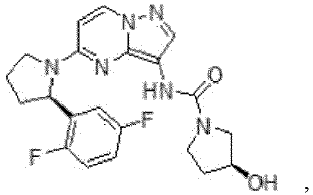 or a hydrogen sulfate salt thereof.
